# EUROPEAN PATENT APPLICATION

(11) **EP 4 625 045 A2**
(43) Date of publication of application: **01.10.2025**
(21) Application number: 25166786.1
(22) Date of filing: 27.03.2025
(51) Int. Cl.: G03F 7/038, G03F 7/039, G03F 7/09, G03F 7/11

(54) **COMPOSITION FOR FORMING FILM, METHOD FOR FORMING ORGANIC FILM, PATTERNING PROCESS, MONOMER, AND POLYMER**

(30) Priority: 27.03.2024 JP 2024052520; 11.07.2024 JP 2024112023; 23.08.2024 JP 2024141836
(71) Applicant: SHIN-ETSU CHEMICAL CO., LTD., Tokyo 1000005 (JP)
(72) Inventor: KORI, Daisuke, Niigata (JP); YAMAMOTO, Yasuyuki, Niigata (JP); HATAKEYAMA, Jun, Niigata (JP)
(74) Representative: Schicker, Silvia

(57) **Abstract**

The present invention is a composition for forming a film including a polymer having an aromatic group having a pentafluorosulfanyl group as a substituent. An object of the present invention is to provide a composition for forming a film that has a less environmental load and excellent coatability on a substrate.

## Description

### TECHNICAL FIELD

The present invention relates to a composition for forming an organic film, a method for forming an organic film, a patterning process, a monomer, and a polymer.

### BACKGROUND ART

As LSIs advance toward higher integration and higher processing speed, miniaturization of pattern rule is progressing rapidly. This is because the spread of high-speed communication of 5th generation mobile networks and artificial intelligence (AI) has progressed, and high-performance devices for processing these are needed. As a cutting-edge technology for miniaturization, 5-nm node devices have been mass-produced by extreme ultraviolet ray (EUV) lithography at a wavelength of 13.5 nm. Furthermore, the use of EUV lithography is being studied also for
next-generation 3-nm node and the following-generation 2-nm node devices.

It is known that in a monolayer resist method, which is employed as a typical resist patterning process, as the thinning of resist patterns progresses as described above, patterning becomes difficult and that a multilayer resist method, in which a pattern is formed by laminating films having different dry etching properties to form a pattern with a high aspect ratio on an uneven substrate, is excellent as a fine pattern processing method. Thus, there have been developed and put into practical use a three-layer resist method (Patent Document 2) in which a photoresist layer made of an organic photosensitive polymer used in a monolayer resist method is combined with a middle layer made of a silicon-based polymer or a silicon-based CVD film, and a underlayer made of an organic polymer.

In this three-layer resist method, for example, an organic film made of a novolak or the like is formed uniformly as a resist underlayer film on a substrate to be processed; a silicon-containing film is formed thereon as a resist middle layer film; and an ordinary organic photoresist film is formed thereon as a resist upper layer film. In dry etching using a fluorine-based gas plasma, an organic resist upper layer film has favorable etching selectivity to a silicon-containing resist middle layer film, and therefore, a resist pattern is transferred to the silicon-containing resist middle layer film by dry etching using a fluorine-based gas plasma. According to this method, the pattern can be transferred to the silicon-containing film even when using a resist composition that causes difficulties in forming a pattern having a sufficient film thickness for directly processing the substrate to be processed or when using a resist composition that does not have sufficient dry etching resistance for processing the substrate. In addition, by subsequently transferring the pattern by dry etching with an oxygen-based gas plasma, it is possible to obtain a pattern in an organic film having sufficient dry etching resistance for processing.

Many techniques are already publicly known (e.g. Patent Document 2) regarding organic underlayer films as described above. However, in association with recent progress in miniaturization, the need for excellent filling property in addition to dry etching property is rising. There is a demand for an organic underlayer film material that enables uniform film formation even on an underlying substrate to be processed having a complex form or material, and that has a filling property that allows spaces in a required pattern to be filled without gaps.

When a semiconductor substrate or the like is manufactured, the above-described organic underlayer film is formed using a coater/developer that can perform treatments, such as a spin-coating process, a EBR process, and baking process. The EBR (Edge Bead Removal) process is a process of removing, after forming a film on a substrate (wafer) by spin-coating, coating film on the edge of the substrate with a remover, for the purpose of preventing the contamination of a substrate-conveying arm of the coater/developer. The removers used in the EBR process includes a mixed solution of propylene glycol monomethyl ether acetate and propylene glycol monomethyl ether (30 mass% :70 mass%), and such removers are widely used in the EBR processes of resist films and resist underlayer films (silicon-containing resist middle layer films and organic underlayer films).

Due to the effect of a remover in an EBR process, a state (a hump) where a peripheral portion of an organic underlayer film is thick occurs in some cases. In the above-described dry etching process in processing a substrate, the hump causes a defect, and therefore, an organic underlayer film in which the hump is prevented is desired.

Not only an organic underlayer film but also a resist material used in photolithography using the above organic photosensitive polymer are formed to be a film by applying a solution by a method, such as spin coating, as with the organic underlayer film and then evaporating the solvent by being baked. As with the organic underlayer film, the film is desired to have a uniform thickness after baking and be flat, and the requirements for uniformity and flatness are becoming stricter every year.

In recent years, there is a demand for a thick resist film for 3D-NAND memory applications, and even greater flatness is required. As the film thickness increases, it becomes more difficult to achieve flatness of the film. On the other hand, as miniaturization progresses, the film thickness is becoming thinner, and in this case, the risk of occurring of a pinhole defect and the like is increasing.

The above describes examples of a film material using organic substances used in semiconductor processing materials. However, even in the case of a material for forming a film that does not use organic substances, it is of great industrial advantage to obtain a material that forms a film with uniform in-plane film thickness without making a pinhole.

In recent years, the health effects of perfluoroalkyl compounds (PFAS) have been pointed out, and there is a movement to impose restrictions on the manufacturing and sale of PFAS compounds under REACH in Europa. Perfluoroalkyl compounds have a wide range of uses, and because of their properties due to their structure, such as repelling water and oil, being resistant to heat and chemicals, and not absorbing light, they are used in a wide range of applications, such as a water repellent, a surface treatment agent, an emulsifier, a fire extinguishing agent, and a coating agent. Therefore, there is an urgent need to develop alternative materials that do not have a PFAS structure.

As an example of the material using the above-mentioned perfluoroalkyl compound, a surfactant having a fluoroalkyl group or a silicone chain is highly effective in reducing surface tension, and in particular, a fluoroalkyl group-based surfactant that has a low risk of generating silicon-derived particles after dry ashing of a resist film is widely used (Patent Documents 3 and 4). In addition to the resist materials, fluorine-based surfactants are used also in top coats formed on an upper layer of a resist and in anti-reflective films formed on an underlayer under a resist (Patent Document 5).

In view of the future tightening of regulations, it is necessary to use a material that do not fall under the PFAS regulations. For example, a surfactant having a trifluoromethoxy group or a pentafluorosulfanyl group and their use have been proposed as the above-mentioned surfactants (Patent Document 6). In addition, in the field of a resist material, resist materials using a photo-acid generator have also been proposed (Patent Document 7).

### CITATION LIST

### PATENT LITERATURE

Patent Document 1: JP4355943B2, etc.
Patent Document 2: JP2004-205685A
Patent Document 3: JPH06-186735A
Patent Document 4: JPH06-214380A
Patent Document 5: JP2010-139822A
Patent Document 6: JP2008-526792A
Patent Document 7: WO2023/223624A1

### SUMMARY OF INVENTION

### TECHNICAL PROBLEM

The present invention has been made in view of the above-described circumstances. An object of the present invention is to provide a composition for forming a film that has a less environmental load and excellent coatability on a substrate.

### SOLUTION TO PROBLEM

To solve the above problems, the present invention provides a composition for forming a film comprising a polymer having an aromatic group having a pentafluorosulfanyl group as a substituent.

In this event, the polymer is preferably a polymer (a) having a repeating unit "a" having an aromatic group having a pentafluorosulfanyl group as a substituent.

The composition for forming a film including the polymer with such a structure is considered to be able to utilize a synergistic effect of: interaction between aromatic rings; and an effect similar to the "fluorophilic" effect under discussion for a perfluoroalkyl group, the effect by which the behavior of the hydrophobic group is enhanced by that the presence of an aromatic group having a pentafluorosulfanyl group as a repeating unit facilitates formation of an aggregated structure through the two interactive effects between the pentafluorosulfanyl groups and between the benzene rings. Therefore, the inventive composition for forming a film can provide a material for forming a film that can be applied not only to a material for forming a film including only various polymers, but also to a material for forming a film including organic polymers, organic compounds, or inorganic substances, such as silicon, titanium, and zirconium.

Further, the repeating unit "a" is preferably represented by any one of the following formulae (a1) to (a4).

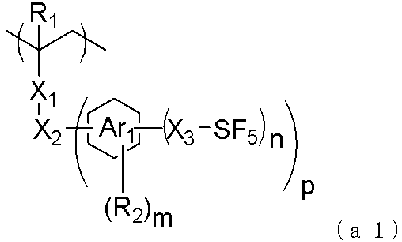

In the above formula, "p" represents an integer of 1 to 3; "n" represents an integer of 0 to 5; "m" represents an integer of 0 to 4; R₁ represents a hydrogen atom or a methyl group; R₂ represents a hydrocarbyl group having 1 to 6 carbon atoms, a hydrocarbyloxy group having 1 to 12 carbon atoms, a hydrocarbyloxycarbonyl group having 2 to 6 carbon atoms, a hydrocarbylcarbonyloxy group having 2 to 12 carbon atoms, a hydroxy group, a carboxy group, a halogen atom, a trifluoromethoxy group, a cyano group, or a nitro group; X₁ represents a single bond, an ester bond, an ether bond, a sulfonic acid ester group, a sulfonamide group, an amide bond, or a phenylene group; X₂ represents a single bond or a hydrocarbylene group having 1 to 20 carbon atoms when "p" represents 1, and X₂ represents a (p+1)-valent hydrocarbon group having 1 to 20 carbon atoms when "p" represents 2 or 3, and the hydrocarbylene group and the (p+1)-valent hydrocarbon group may contain at least one atom selected from the group consisting of an oxygen atom, a sulfur atom, a nitrogen atom, and a halogen atom; X₃ represents a single bond or an ether bond; and Ar₁ each independently represents an (m+n+1)-valent group derived from benzene or naphthalene, provided that at least one of "n"s in the formula represents 1 or more.

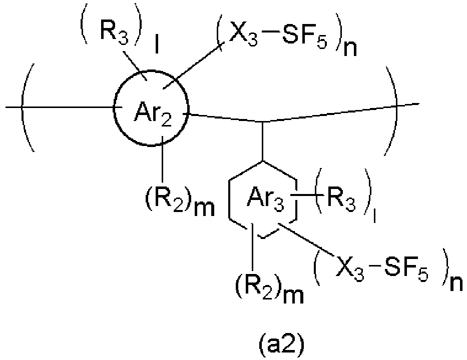

In the above formula, "n" and "m" represent the same as defined above, "l" represents an integer of 0 to 3, and at least one of the two "n"s and the two "1"s in the formula represents 1 or more; R₂ and X₃ represent the same as defined above; and R₃ represents the following general formula (a2-1); Ar₂ each independently represents an (n+m+l+2)-valent aromatic hydrocarbon group having 6 to 30 carbon atoms; and Ar₃ represents an (n+m+l+1)-valent group derived from benzene or naphthalene.

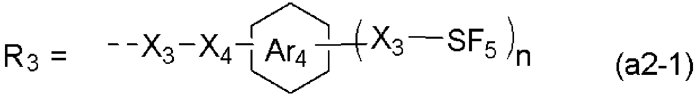

In the above formula, X₃ and "n" represent the same as above; X₄ represents a single bond or a hydrocarbylene group having 1 to 12 carbon atoms, and the hydrocarbylene group may have at least one selected from the group consisting of a hydroxy group, a saturated hydrocarbyloxy group having 1 to 6 carbon atoms, an ether bond, an ester bond, and an amide bond; and Ar₄ represents an (n+1)-valent group derived from benzene or naphthalene.

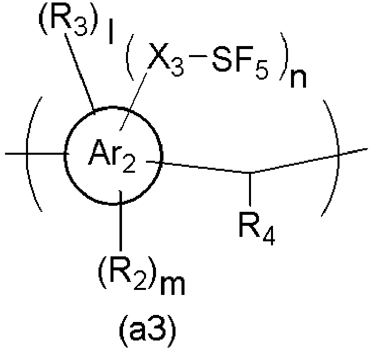

In the above formula, "n", "m", "l", Ar₂, R₂, R₃, and X₃ represent the same as defined above; R₄ represents a hydrogen atom or a hydrocarbyl group having 1 to 10 carbon atoms, and the hydrocarbyl group may have an oxygen atom but does not have a substituent having SF₅; and at least one of "n" and "l" in the formula represents 1 or more.

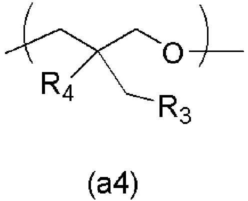

In the above formula, R₃ and R₄ represent the same as defined above.

The polymer having the repeating units shown above can utilize the interactive effects between the molecules effectively and can provide an excellent material for forming a film.

In this event, the repeating unit "a" is preferably represented by the formula (a1).

When considering easiness in manufacturing a material and a range of combination with polymers and compounds for forming an organic film, it is advantageous to have a repeating unit represented by (a1).

The polymer (a) preferably comprises further a repeating unit "b1" having a hydrophilic group selected from the group consisting of an ether bond, an ester bond, a hydroxy group, a carboxy group, a sulfonamide bond, a sulfonimide bond, a sulfo group, a lactone ring, a sultone ring, a carbonate bond, a urethane bond, and an amide bond.

The inventive material for forming a film is excellent in film-formability as mentioned before, but by incorporating the above hydrophilic group with a repeating unit, the polymer can contribute as a relaxing unit for the aggregated structure mentioned before and makes it possible to expand its versatility as a material for forming a film. For example, it is also possible to provide a material for forming a film that can be applicable to a variety of film thickness (independent of the concentration of a solution) by adjusting a surface activating ability.

Further, the composition for forming a film is preferably a composition for forming an organic film comprising (A) a resin or compound for forming an organic film; (B) the polymer (a); and (C) a solvent.

By combining the polymer (a) described above with a resin or compound for forming an organic film and a solvent, the composition can be used as a composition for forming an organic film.

Further, the component (B) is preferably contained in an amount of 0.01 to 5 parts by mass based on 100 parts by mass of the component (A).

By adding the polymer (a) in such a range, it is possible to enhance film-formability of the composition for forming an organic film. Therefore, although there are a variety of resins or compounds, such as resins having variety of repeating units, compounds with a rigid structure and high crystallinity, and compounds having a substituent with high polarity, the inventive polymer (a) can provide a composition for forming an organic film with excellent film-formability even when using such resins or compounds having different properties.

Furthermore, the composition for forming a film preferably comprises (D) a photo-acid generator or (E) a thermal acid generator further.

By using such a composition for forming an organic film, it is possible to impart photosensitivity or the like to a composition for forming an organic film and apply the inventive composition for forming an organic film to a resist material used for photolithography.

Further, the present invention provide a method for forming an organic film to be used in a manufacturing process of a semiconductor device, the method comprising:
spin-coating a substrate to be processed with the composition for forming a film above; and
forming a cured film by heating the substrate coated with the composition for forming a film at a temperature of 100°C or higher and 600°C or lower for 10 to 600 seconds.

The inventive composition for forming an organic film is useful especially in the case: where a pattern in a complicated form on a substrate to be processed is filled by spin-coating; an organic film having excellent in-plane uniformity is formed; and the organic film on the edge of the substrate is removed while preventing a hump in the EBR process.

Further, the present invention provides a patterning process comprising:
forming an organic film on a body to be processed by using the composition for forming a film above;
forming a resist middle layer film on the organic film by using a silicon-containing resist middle layer film material;
forming a resist upper layer film on the resist middle layer film by using a resist upper layer film material comprising a photoresist composition;
forming a circuit pattern in the resist upper layer film;
transferring the pattern to the resist middle layer film by etching while using the resist upper layer film having the formed circuit pattern as a mask;
transferring the pattern to the organic film by etching while using the resist middle layer film having the transferred pattern as a mask; and
further transferring the pattern in the body to be processed by etching while using the organic film having the transferred pattern as a mask.

Further, the present invention provides a patterning process comprising:
forming an organic film on a body to be processed by using the composition for forming a film above;
forming a resist middle layer film on the organic film by using a silicon-containing resist middle layer film material;
forming an organic antireflective film or an adhesive film on the resist middle layer film;
forming a resist upper layer film on the organic antireflective film or the adhesive film by using a resist upper layer film material comprising a photoresist composition;
forming a circuit pattern in the resist upper layer film;
transferring the pattern to the organic antireflective film or the adhesive film and to the resist middle layer film by etching while using the resist upper layer film having the formed circuit pattern as a mask;
transferring the pattern to the organic film by etching while using the resist middle layer film having the transferred pattern as a mask; and
further transferring the pattern to the body to be processed by etching while using the organic film having the transferred pattern as a mask.

Further, the present invention provides a patterning process comprising:
forming an organic film on a body to be processed by using the composition for forming a film above;
forming an inorganic hard mask selected from the group consisting of a silicon oxide film, a silicon nitride film, and a silicon oxynitride film on the organic film;
forming a resist upper layer film on the inorganic hard mask by using a resist upper layer film material comprising a photoresist composition;
forming a circuit pattern in the resist upper layer film;
transferring the pattern to the inorganic hard mask by etching while using the resist upper layer film having the formed circuit pattern as a mask;
transferring the pattern to the organic film by etching while using the inorganic hard mask having the transferred pattern as a mask; and
further transferring the pattern to the body to be processed by etching while using the organic film having the transferred pattern as a mask.

Further, the present invention provides a patterning process comprising:
forming an organic film on a body to be processed by using the composition for forming a film above;
forming an inorganic hard mask selected from the group consisting of a silicon oxide film, a silicon nitride film, and a silicon oxynitride film on the organic film;
forming an organic antireflective film or an adhesive film on the inorganic hard mask;
forming a resist upper layer film on the organic antireflective film or the adhesive film by using a resist upper layer film material comprising a photoresist composition;
forming a circuit pattern in the resist upper layer film;
transferring the pattern to the organic antireflective film or the adhesive film and to the inorganic hard mask by etching while using the resist upper layer film having the formed circuit pattern as a mask;
transferring the pattern to the organic film by etching while using the inorganic hard mask having the transferred pattern as a mask; and
further transferring the pattern to the body to be processed by etching while using the organic film having the transferred pattern as a mask.

As described above, the inventive composition for forming an organic film can be used suitably for different patterning processes, such as a three-layer resist process using a silicon-containing resist middle layer film or an inorganic hard mask and a four-layer resist process using an organic antireflective film in addition to these. By using such patterning processes of the present invention, it is possible to transfer and form a circuit pattern of a resist upper layer film in a body to be processed with high precision.

Further, the inorganic hard mask is preferably formed by a CVD method or an ALD method.

In the present patterning process, for example, an inorganic hard mask can be formed by such methods.

Further, the circuit pattern is preferably formed by a lithography using light having a wavelength of 10 nm or more and 300 nm or less, a direct writing with an electron beam, nanoimprinting, or a combination thereof.

Further, the circuit pattern is preferably developed with an alkaline development or an organic solvent.

In the inventive patterning process, such a circuit pattern forming means and developing means can be suitably used.

Further, the body to be processed is preferably a semiconductor device substrate or the semiconductor device substrate coated with any of a metal film, a metal carbide film, a metal oxide film, a metal nitride film, a metal oxycarbide film, and a metal oxynitride film.

In this event, the metal constituting the body to be processed is preferably silicon, titanium, tungsten, hafnium, zirconium, chromium, germanium, copper, silver, gold, aluminum, indium, gallium, arsenic, palladium, iron, tantalum, iridium, molybdenum, or an alloy thereof.

The inventive patterning process makes it possible to process the above-described body to be processed to form a pattern.

Further, the present invention provides a patterning process comprising:
forming a resist film on a substrate by using the composition for forming a film above;
exposing the resist film to a high-energy beam; and
developing the exposed resist film by using a developer.

By using the inventive composition for forming a film, it is possible to perform the above-described exposure to light to perform a pattern.

In this event, the high-energy beam is preferably a g-line, an i-line, a KrF excimer laser beam, an ArF excimer laser beam, an ultraviolet ray, an electron beam, or an extreme ultraviolet ray having a wavelength of 3 to 15 nm.

By using the above-described high-energy beam, it is possible to form a fine pattern.

Further, the present invention provide a monomer represented by the following general formula (a5).

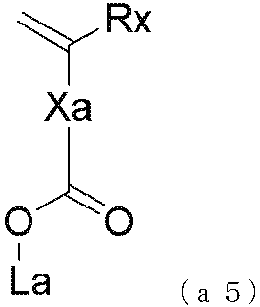

In the formula, Rₓ represents a hydrogen atom or a methyl group; Xₐ represents a single bond, a phenylene group, a naphthylene group, or a linking group having 1 to 12 carbon atoms and having an ester bond, an ether bond, or a lactone ring, and may have an oxygen atom, a nitrogen atom, a sulfur atom, and/or a halogen atom; and La represents an acid-labile group and has an aromatic group having at least one pentafluorosulfanyl group as a substituent.

The inventive monomer is preferably represented by the following general formula (a6).

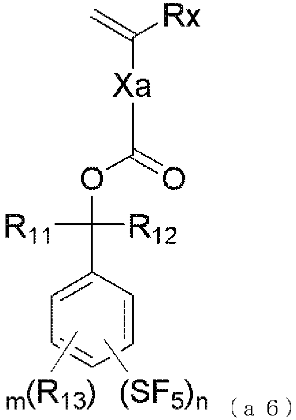

In the formula, Xₐ and Rₓ represent the same defined above; R₁₁ and R₁₂ each independently represent a linear, branched, or cyclic alkyl group having 1 to 12 carbon atoms, a linear, branched, or cyclic alkenyl group having 2 to 12 carbon atoms, a linear, branched, or cyclic alkynyl group having 2 to 12 carbon atoms, or an aryl group having 5 to 10 carbon atoms, each of which may have an oxygen atom and/or a sulfur atom, and R₁₁ and R₁₂ may bond to each other to form a ring; R₁₃ each independently represents a hydrogen atom, a halogen atom, an alkyl group having 1 to 4 carbon atoms, or an alkoxy group; "m" represents an integer of 0 to 4; and "n" represents an integer of 1 to 3.

The above monomers are promising candidates to replace conventional monomers into which a fluorine substituent has been introduced as monomers that is not subject to the PFAS regulations.

The present invention provides a polymer having a repeating unit represented by the following general formula (a7) and having a weight-average molecular weight in a range of 100 to 500,000.

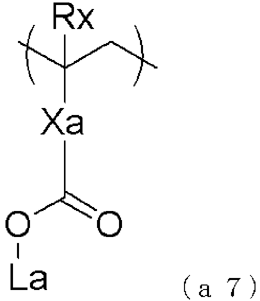

In the formula, Rₓ represents a hydrogen atom or a methyl group; Xₐ represents a single bond, a phenylene group, a naphthylene group, or a linking group having 1 to 12 carbon atoms and having an ester bond, an ether bond, or a lactone ring, and may have an oxygen atom, a nitrogen atom, a sulfur atom, and/or a halogen atom; La represents an acid-labile group and has an aromatic group having at least one pentafluorosulfanyl group as a substituent.

The inventive polymer preferably have a repeating unit represented by the following general formula (a8) and preferably have a weight-average molecular weight in a range of 100 to 500,000.

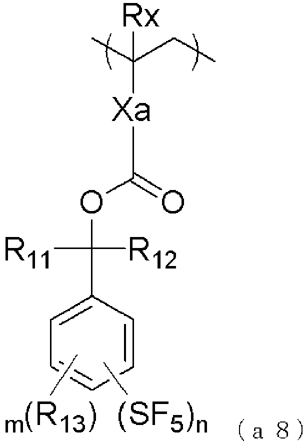

In the formula, Xₐ and Rₓ represent the same defined above; R₁₁ and R₁₂ each independently represent a linear, branched, or cyclic alkyl group having 1 to 12 carbon atoms, a linear, branched, or cyclic alkenyl group having 2 to 12 carbon atoms, a linear, branched, or cyclic alkynyl group having 2 to 12 carbon atoms, or an aryl group having 5 to 10 carbon atoms, each of which may have an oxygen atom and/or a sulfur atom, and R₁₁ and R₁₂ may bond to each other to form a ring; R₁₃ each independently represents a hydrogen atom, a halogen atom, an alkyl group having 1 to 4 carbon atoms, or an alkoxy group; "m" represents an integer of 0 to 4; and "n" represents an integer of 1 to 3.

The above-described polymers are promising candidates as polymers that do not fall under the PFAS regulations and can replace conventional polymers having a fluorine substituent introduced.

### ADVANTAGEOUS EFFECTS OF INVENTION

As described above, the present invention can apply to a composition for forming a film that is excellent in film-formability (in-plane uniformity) on a substrate (wafer) and filling property, and can provide a composition for forming an organic film that is excellent in film-formability on the organic film and prevents a hump in a EBR process when the material for forming an organic film combined with a resin or compound for forming an organic film is used for an organic underlayer film. In addition, also when the composition for forming an organic film is used as a resist material in combination with a photo-acid generator or the like, it is possible to process a fine pattern as a resist material for photolithography with excellent in-plane uniformity. In addition, by combining with a hydrophilic unit to make a copolymer, it can be applied to not only various materials for forming an organic film but also materials for forming a film having a metal atom, such as silicon, and useful as a versatile material for forming a film that achieves high film-formability. The inventive composition for forming an organic film is extremely useful as a material for an organic film used in a multilayer resist process, such as a two-layer resist process, a three-layer resist process using a silicon-containing resist underlayer film or an inorganic hard mask, and a four-layer resist process using a silicon-containing resist underlayer film or an inorganic hard mask and an organic antireflective film, or as material for forming a film for manufacturing semiconductor device such as a photoresist material. Furthermore, the inventive composition for forming a film does not have any problem in terms of environmental impact.

### BRIEF DESCRIPTION OF DRAWINGS

FIG. 1 is an example of a graph showing the height of hump of a composition for forming an organic film with hump suppressed, measured using a stylus profiler;
FIG. 2 is an example of a graph showing the height of hump of a composition for forming an organic film with hump not-suppressed, measured using a stylus profiler;
FIG. 3 is an explanatory diagram for an example of a patterning process according to the inventive three-layer resist process; and
FIG. 4 is an explanatory diagram of a method for evaluating the filling property in Examples.

### DESCRIPTION OF EMBODIMENTS

As described above, it has been desired to develop a material for forming a film that is excellent in hump characteristics and film-formability, can achieve high in-plane uniformity and filling property, and can withstand environmental regulations.

For example, when an organic film is to be formed, a resin for forming an organic film and additives are dissolved in an organic solvent to form a composition; the composition is applied with a coater/developer onto a substrate on which a structure, wiring, etc. is formed; the composition is spread by rotating the substrate; the composition on the edge is removed in an EBR process; and then the composition is baked to form an organic film.

When the above composition has insufficient surface-active effect, it is thought that surfactants are distributed non-uniformly in a film to generate a gap in filling a hole or trench having a very high aspect ratio, and that a hump is generated in the periphery of the organic film if the resin for forming an organic film or the additives have poor solubility in the remover used in the EBR process.

The inventors performed diligent studies to obtain a material for forming a film that has excellent film-formability, which are desired characteristics in recent years, such as high flatness after being applied and a good coatability at the outermost edge of a substrate (wafer), and has less occurrence of a defect after coating, developing, and etching. As a result, they found that use of a polymer having a specific substituent can provide a composition for forming an organic film that is excellent in film-formability, high filling property, and preventing a hump in the EBR process, and have completed the present invention. The inventive composition for forming a film has a polymer having an aromatic group having a pentafluorosulfanyl group as a substituent is a structure not subject to PFAS regulations and thus is expected to be an industrially useful material under consideration also in terms of environments.

That is, the present invention is a composition for forming a film comprising a polymer having an aromatic group having a pentafluorosulfanyl group as a substituent.

Hereinafter, the present invention will be described in detail, but the present invention is not limited thereto.

### [Composition for Forming Film]

The present invention provides a composition for forming a film containing a polymer having an aromatic group having a pentafluorosulfanyl group as a substituent, that does not fall under the category of a perfluoroalkyl compound (PFAS).

By using a composition for forming an organic film containing this polymer, it is possible to form a film that has excellent coatability in terms of a coating defect, such as a pinhole on a substrate (wafer), film formability (in-plane uniformity), and filling property. Furthermore, by using this composition for forming an organic film as an organic underlayer film material, it is possible to provide an organic film that has an excellent process margin when used as an organic underlayer film for a multilayer resist, and it is also possible to provide a method for forming an organic film and a patterning process using this composition for forming an organic film. Furthermore, this composition for forming an organic film is also useful as a photoresist material, and has excellent film flatness after coating, and is a resist material with less defects not only after coating but also after development, and it is possible to provide a patterning process using this. The above-mentioned composition is useful as a material for forming a film with a less environmental load because it is possible to provide a material for forming a film that does not fall under the category of PFAS.

The polymer is preferably a polymer (a) having a repeating unit "a" having an aromatic group having a pentafluorosulfanyl group as a substituent. The substituent is preferably -SF₅ or -OSF₅ substituted directly on an aromatic ring.

The repeating unit "a" of the polymer is preferably represented by any one of the following formulae (a1) to (a4).

The repeating unit (a1) represented by the general formula (a1) is shown below.

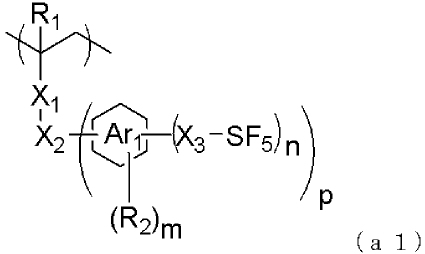

In the above formula, "p" represents an integer of 1 to 3; "n" represents an integer of 0 to 5; "m" represents an integer of 0 to 4; R₁ represents a hydrogen atom or a methyl group; R₂ represents a hydrocarbyl group having 1 to 6 carbon atoms, a hydrocarbyloxy group having 1 to 12 carbon atoms, a hydrocarbyloxycarbonyl group having 2 to 6 carbon atoms, a hydrocarbylcarbonyloxy group having 2 to 12 carbon atoms, a hydroxy group, a carboxy group, a halogen atom, a trifluoromethoxy group, a cyano group, or a nitro group; X₁ represents a single bond, an ester bond, an ether bond, a sulfonic acid ester group, a sulfonamide group, an amide bond, or a phenylene group; X₂ represents a single bond or a hydrocarbylene group having 1 to 20 carbon atoms when "p" represents 1, and X₂ represents a (p+1)-valent hydrocarbon group having 1 to 20 carbon atoms when "p" represents 2 or 3, and the hydrocarbylene group and the (p+1)-valent hydrocarbon group may contain at least one atom selected from the group consisting of an oxygen atom, a sulfur atom, a nitrogen atom, and a halogen atom; X₃ represents a single bond or an ether bond; and Ar₁ each independently represents an (m+n+1)-valent group derived from benzene or naphthalene, provided that at least one of "n"s in the formula represents 1 or more.

In the above general formula (a1), "n" represents an integer from 0 to 5, n=1 or 2 preferably from the viewpoint of film-formability, and n=1 preferably from the viewpoint of a readily available raw material. Further, "p" is an integer of 1 to 3, and p=1 or 2 preferably from the viewpoint of ease of production. "m" is an integer from 0 to 4, and m=0 preferably from the viewpoint of versatility of a raw material, that is, the substituent on the aromatic group is preferably a hydrogen.

In the above general formula (a1), R₁ is a hydrogen atom or a methyl group. R₂ is a hydrocarbyl group having 1 to 6 carbon atoms, a hydrocarbyloxy group having 1 to 12 carbon atoms, a hydrocarbyloxycarbonyl group having 2 to 6 carbon atoms, a hydrocarbylcarbonyloxy group having 2 to 12 carbon atoms, a hydroxy group, a carboxy group , a halogen atom, a trifluoromethoxy group, a cyano group, or a nitro group.

In the above general formula (a1), X₁ is a single bond, an ester bond, an ether bond, an amide bond, or a phenylene group; X₂ represents a single bond or a hydrocarbylene group having 1 to 20 carbon atoms when "p" is 1, X₂ represents a (p+1)-valent hydrocarbon group having 1 to 20 carbon atoms when "p" is 2 or 3, and these hydrocarbylene group and (p+1)-valent hydrocarbon group may include at least one selected from the group consisting of an oxygen atom, a sulfur atom, a nitrogen atom, and a halogen atom; and X₃ is a single bond or an ether bond.

The hydrocarbylene group represented by X₂ may be saturated or unsaturated, and may be linear, branched, or cyclic. The specific examples include: alkanediyl groups having 1 to 20 carbon atoms, such as a methanediyl group, an ethane-1,1-diyl group, an ethane-1,2-diyl group, a propane-1,3-diyl group, a butane-1,4-diyl group, a pentane-1,5-diyl group, a hexane-1,6-diyl group, a heptane-1,7-diyl group, an octane-1,8-diyl group, a nonane-1,9-diyl group, a decane-1,10-diyl group, an undecane-1,11-diyl group, and a dodecane-1,12-diyl group; cyclic saturated hydrocarbylene groups having 3 to 20 carbon atoms, such as a cyclopentanediyl group, a cyclohexanediyl group, a norbornanediyl group, and an adamantanediyl group; unsaturated aliphatic hydrocarbylene group having 2 to 20 carbon atoms, such as a vinylene group and a propene-1,3-diyl group; arylene groups having 6 to 20 carbon atoms, such as a phenylene group and a naphthylene group; and groups obtained by combining these. Further, the (p+1)-valent hydrocarbon groups having 1 to 20 carbon atoms represented by X₂ may be saturated or unsaturated, and may be linear, branched, or cyclic. Specific examples thereof include groups obtained by further removing one or two hydrogen atoms from the above-mentioned hydrocarbylene group, and a group obtained by substituting one or two hydrogen atoms of the above-mentioned hydrocarbylene group with a hydrocarbylene group.

It is preferable that at least one of X₁ and X₂ is not a single bond.

The repeating unit (a2) represented by the general formula (a2) is shown below.

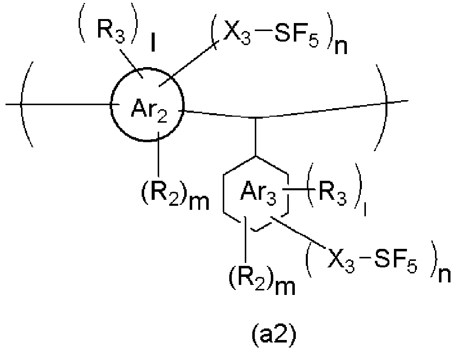

In the above general formula, "n" and "m" represent the same as defined above, "l" represents an integer of 0 to 3, and at least one of the two "n"s and the two "1"s in the formula represents 1 or more; R₂ and X₃ represent the same as defined above; R₃ represents the following general formula (a2-1); Ar₂ each independently represents an (n+m+l+2)-valent aromatic hydrocarbon group having 6 to 30 carbon atoms; and Ar₃ represents an (n+m+l+1)-valent group derived from benzene or naphthalene.

In the above general formula (a2), "n" is an integer of 0 to 5, "m" is an integer of 0 to 4, "l" is an integer of 0 to 3, and at least one of the two "n"s and the two "l"s in the formula is 1 or more; R₂ and X₃ represent the same as defined above; R₃ is represented by the following general formula (a2-1); Ar₂ is each independently an (n+m+l+2)-valent aromatic hydrocarbon group having 6 to 30 carbon atoms. The (n+m+l+2)-valent aromatic hydrocarbon group having 6 to 30 carbon atoms is preferably derived from benzene, naphthalene, anthracene, phenanthrene, pyrene, fluorene, biphenyl, diphenylmethane, trisphenyl, fluorene bisphenol, fluorene bisnaphthol, or the like, may have a substituted or unsubstituted hydroxy group and a saturated hydrocarbyl group having 1 to 12 carbon atoms, and these may be bonded to benzene or naphthalene having a substituted or unsubstituted hydroxy group; and Ar₃ is an (n+m+l+1)-valent group derived from benzene or naphthalene.

R₃ is represented by the following formula(a2-1).

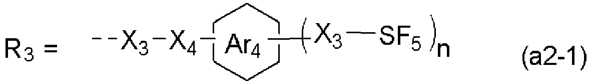

In the above general formula, X₃ and "n" represent the same as above; X₄ represents a single bond or a hydrocarbylene group having 1 to 12 carbon atoms, and the hydrocarbylene group may have at least one selected from the group consisting of a hydroxy group, a saturated hydrocarbyloxy group having 1 to 6 carbon atoms, an ether bond, an ester bond, and an amide bond; and Ar₄ represents an (n+1)-valent group derived from benzene or naphthalene.

The repeating unit (a3) represented by the formula (a3) is shown below.

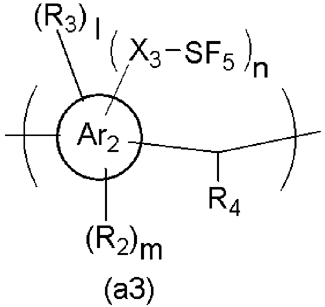

In the above formula, "n", "m", "l", Ar₂, R₂, R₃, and X₃ represent the same as defined above; R₄ represents a hydrogen atom or a hydrocarbyl group having 1 to 10 carbon atoms, and the hydrocarbyl group may have an oxygen atom but does not have a substituent having SF₅; and at least one of "n" and "l" in the formula represents 1 or more.

The repeating unit (a4) represented by the formula (a4) is shown below.

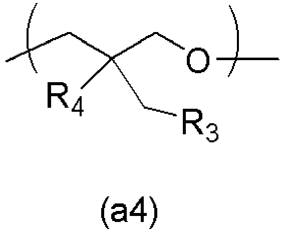

In the formula, R₃ and R₄ represent the same as defined above.

The number of substituents on the aromatic ring having SF₅ in the above repeating units (a1) to (a4) is more preferably n=1 from the viewpoint of film-formability control and a readily available raw material.

The polymer is preferably a polymer having at least one selected from the repeating unit (a1) (hereinafter also referred to as polymer (a1)).

Monomers that provide a repeating unit (a1), providing a polymer having an aromatic group having a pentafluorosulfanyl group as a substituent, include those shown below but are not limited thereto. Incidentally, R₁ in the following formulae represents the same as defined above.

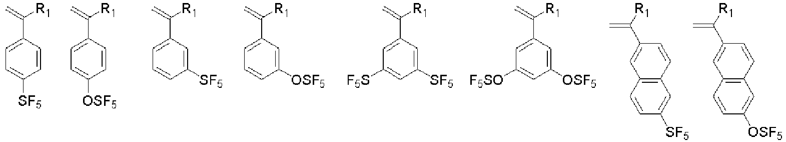

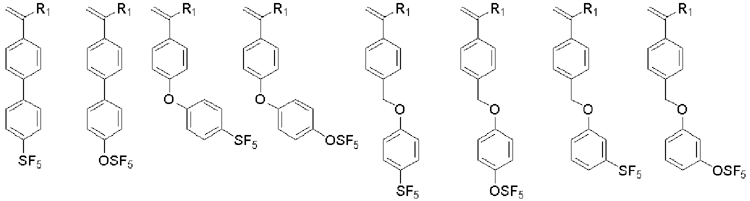

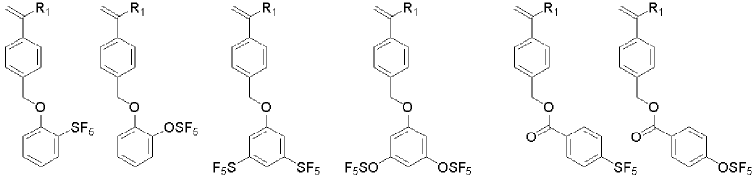

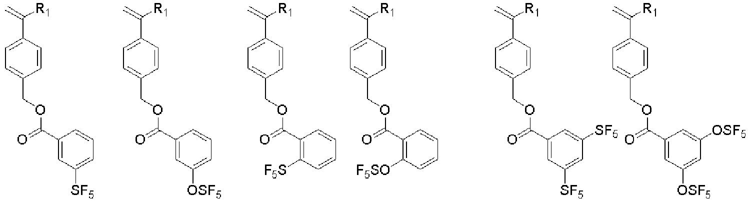

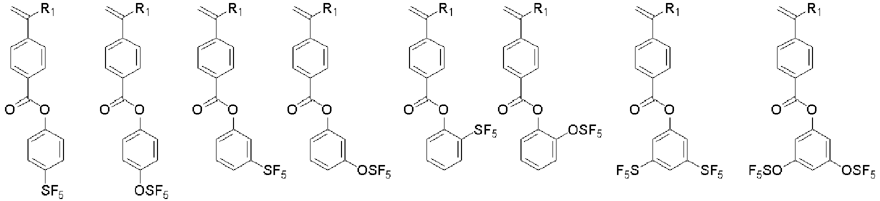

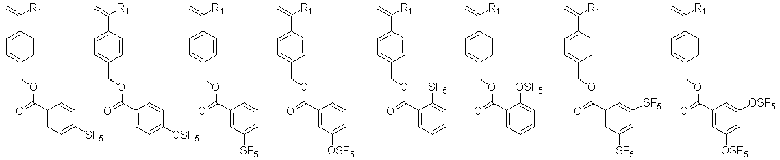

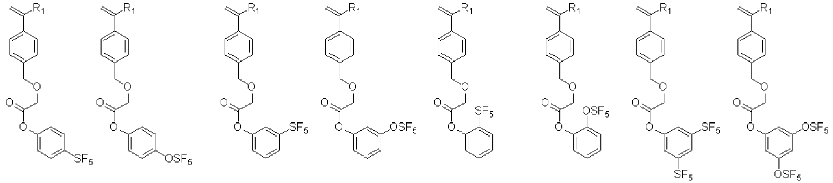

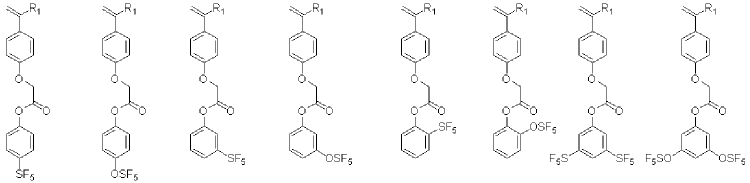

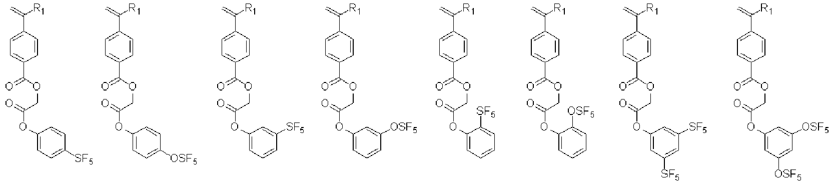

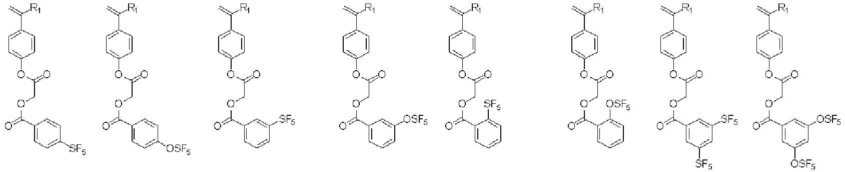

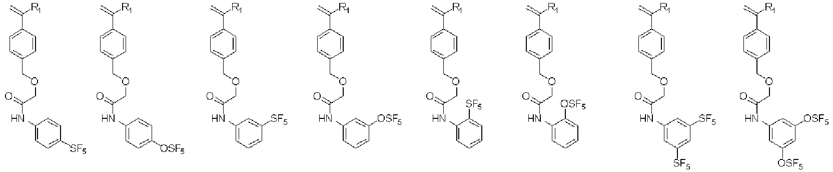

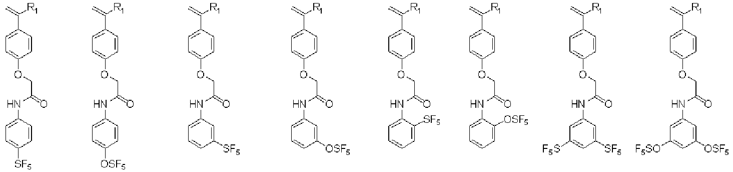

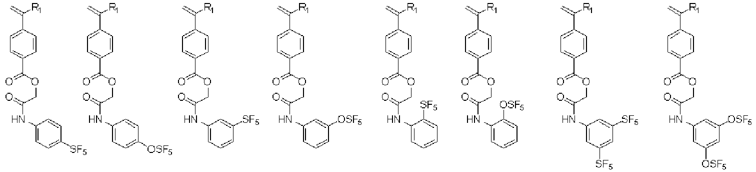

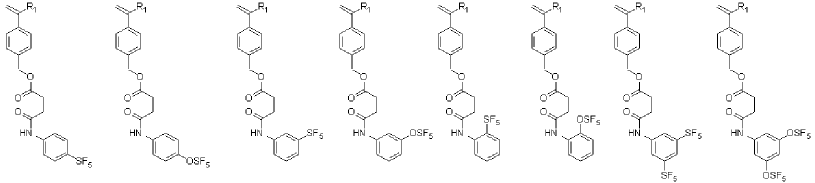

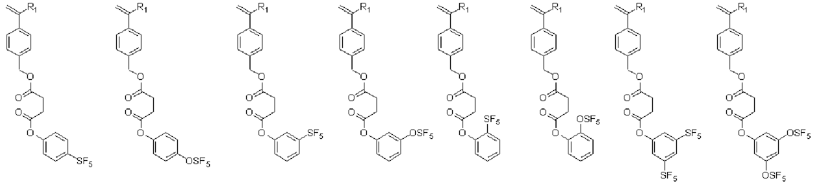

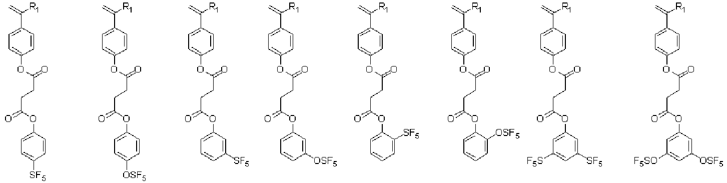

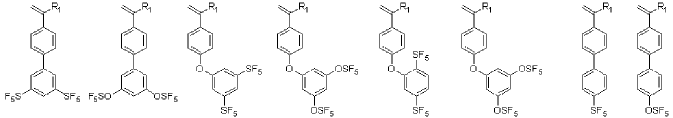

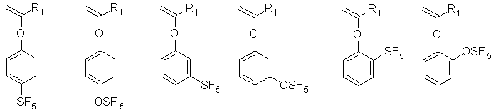

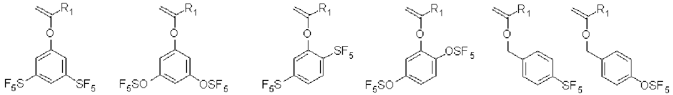

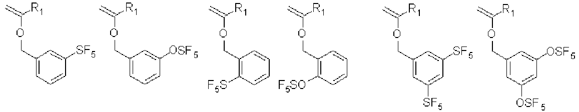

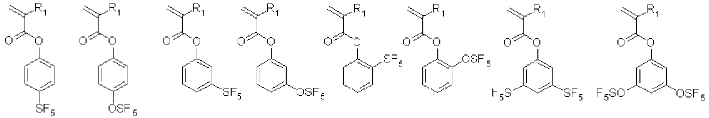

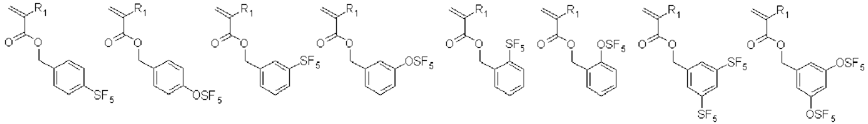

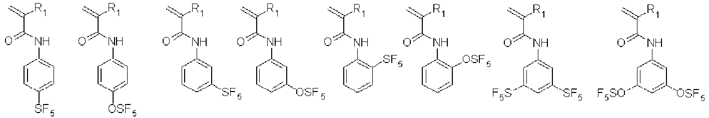

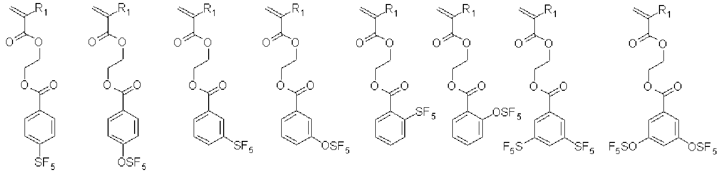

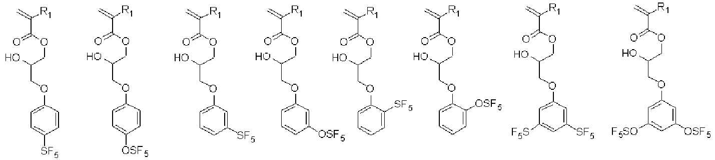

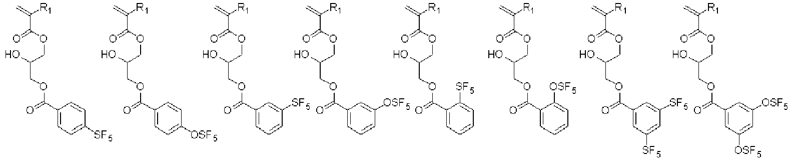

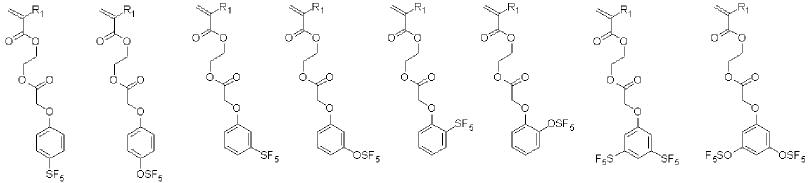

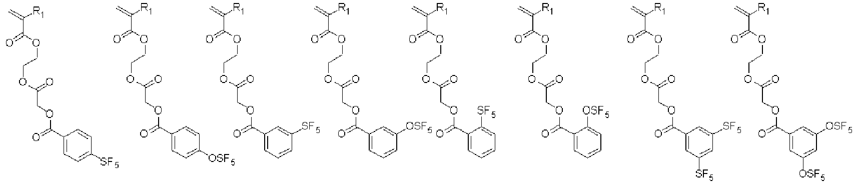

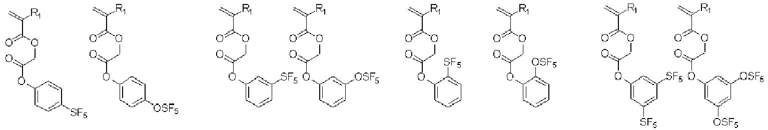

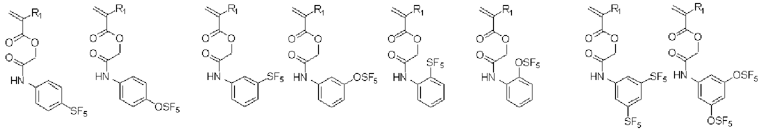

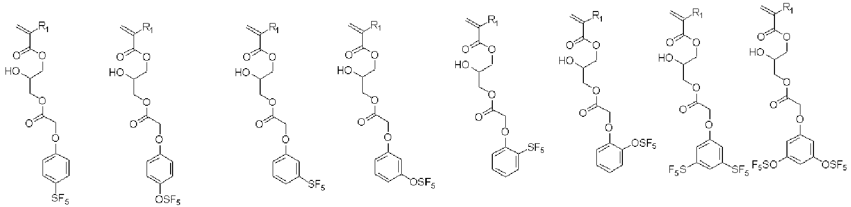

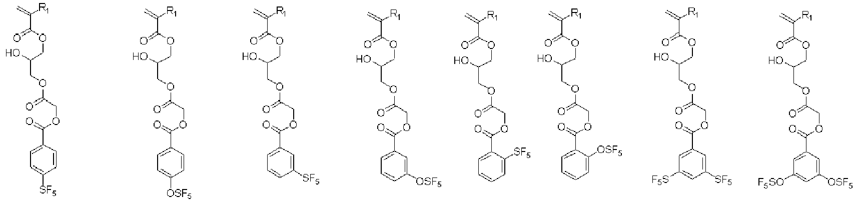

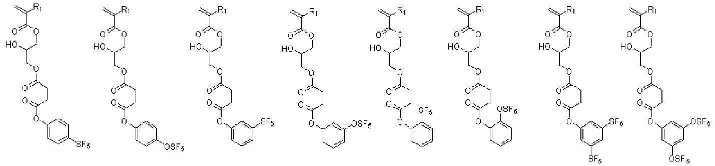

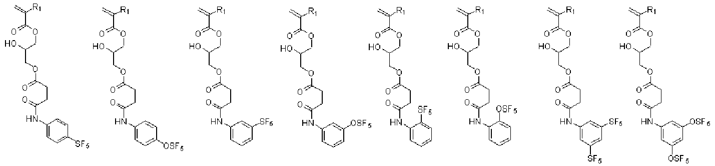

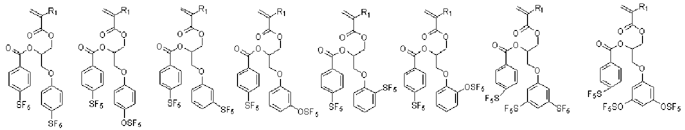

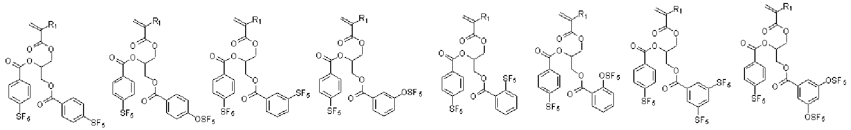

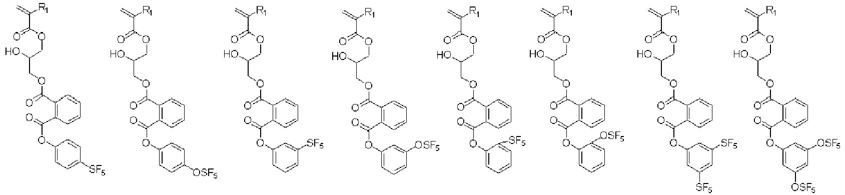

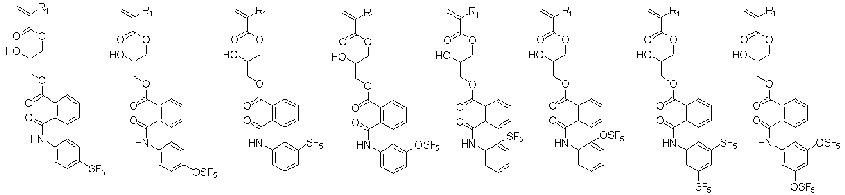

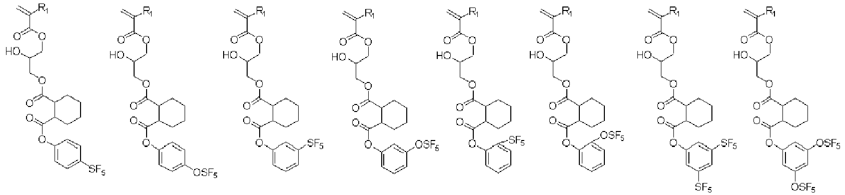

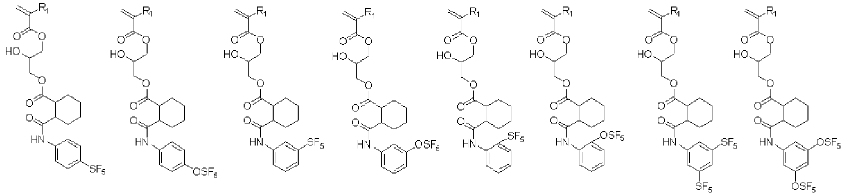

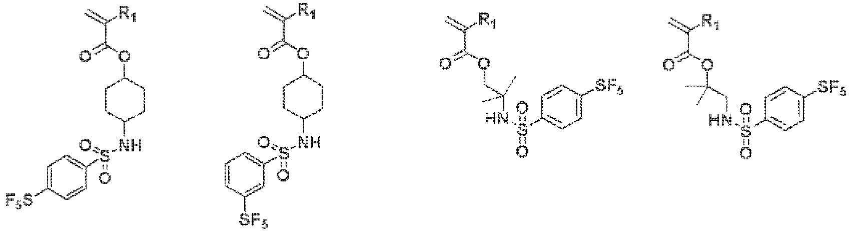

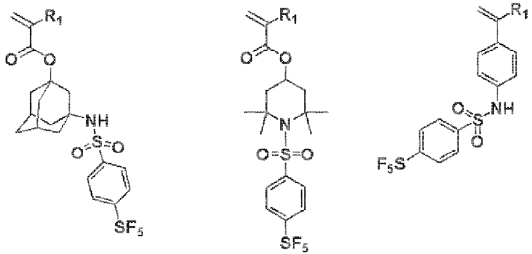

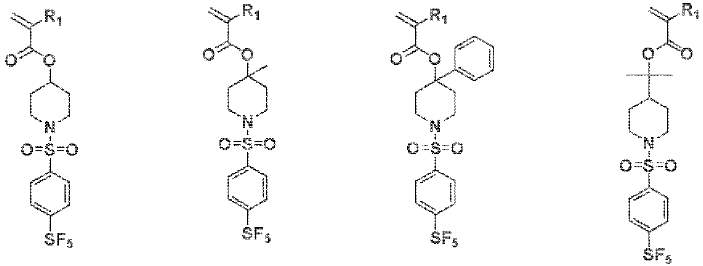

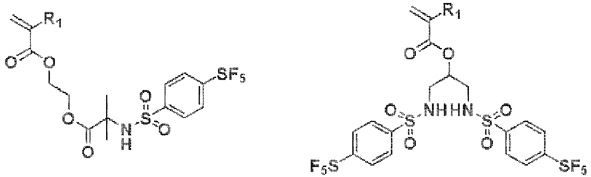

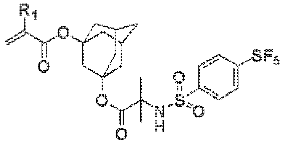

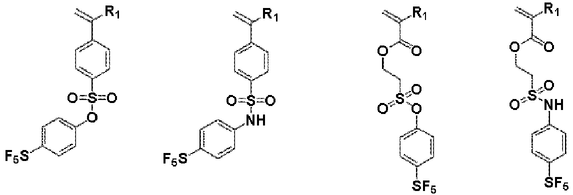

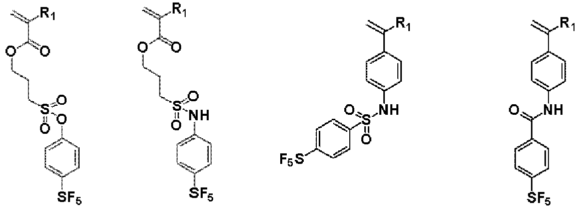

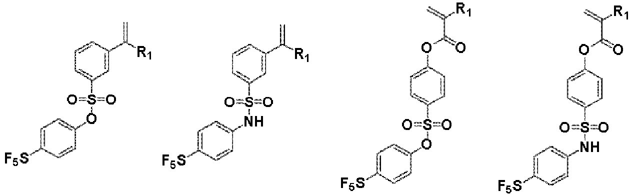

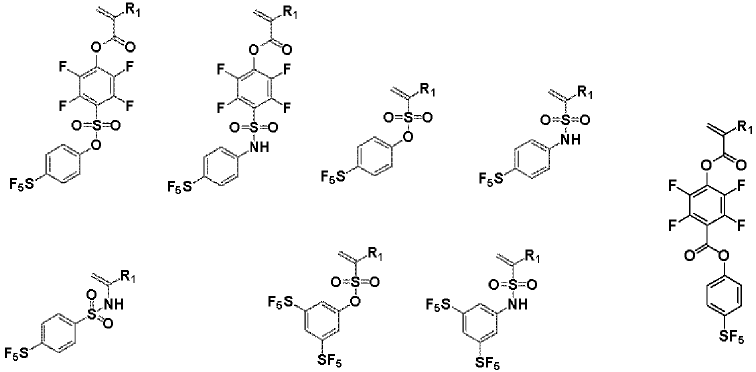

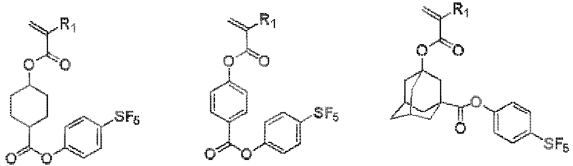

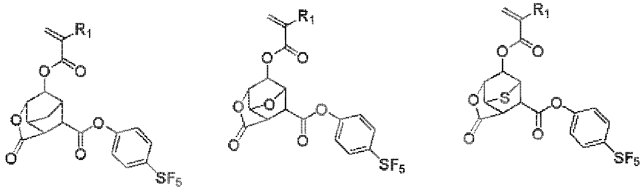

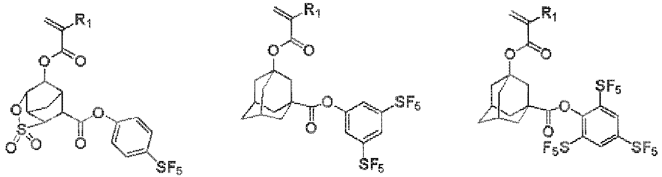

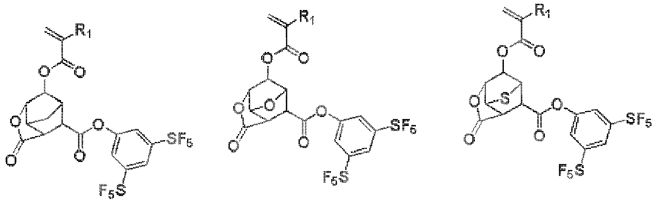

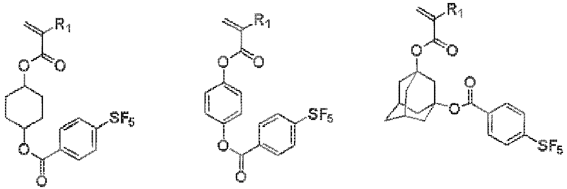

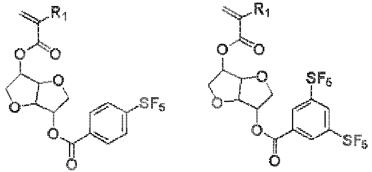

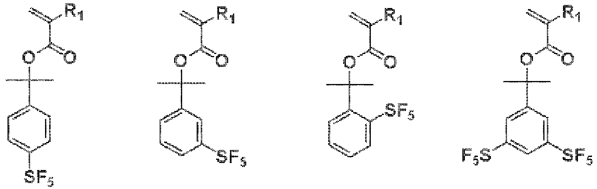

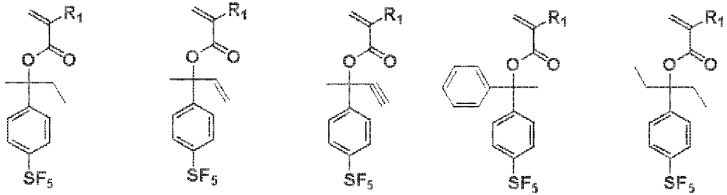

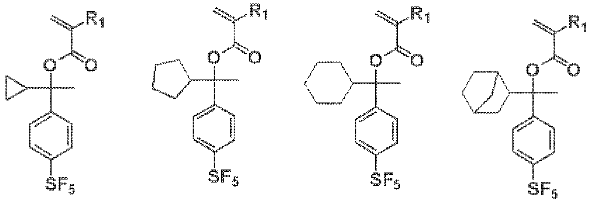

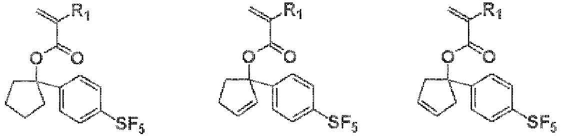

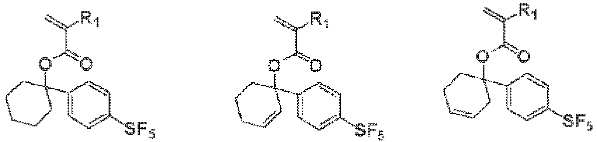

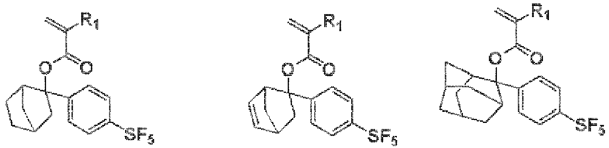

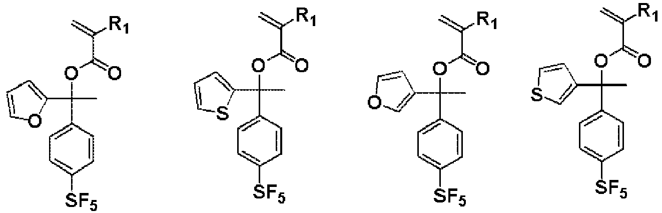

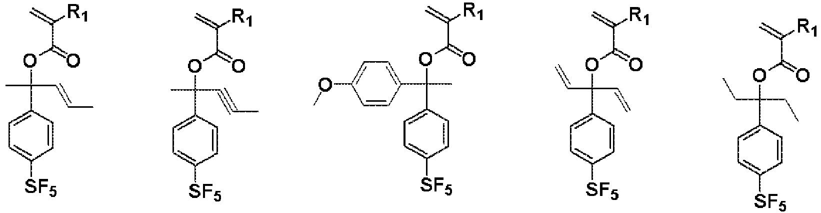

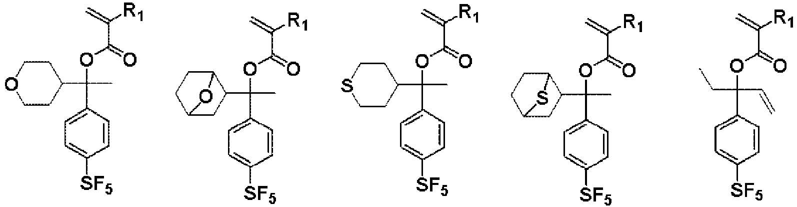

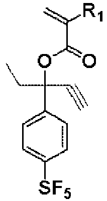

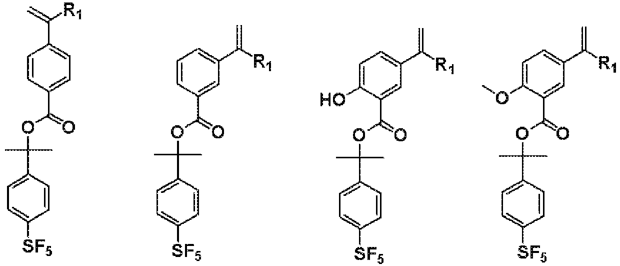

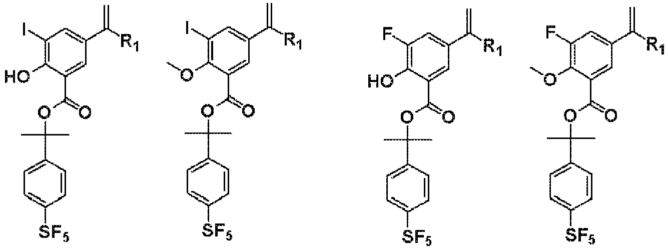

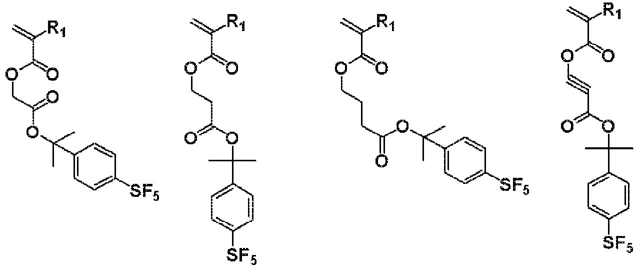

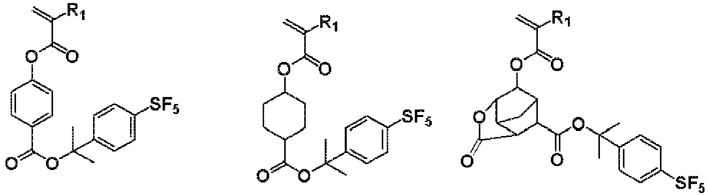

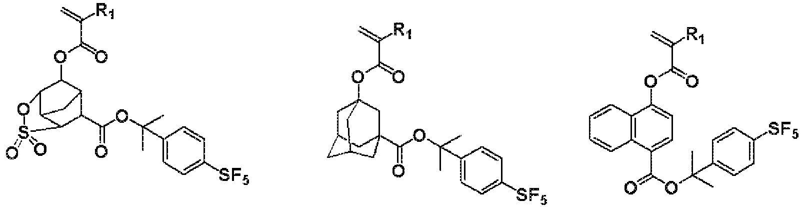

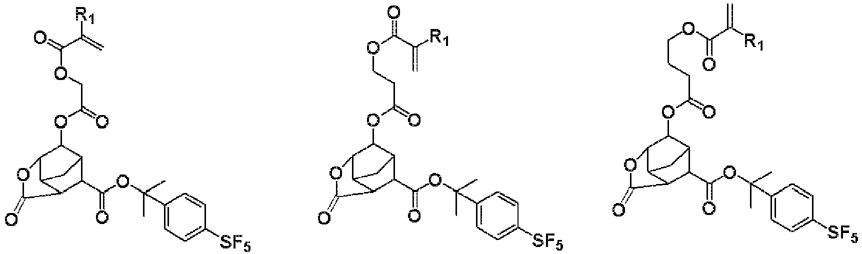

The polymer (a1) preferably includes further a repeating unit (b1) having a hydrophilic group. By including the repeating unit (a1) having a highly water-repellent fluorine atom and the repeating unit (b1) having a hydrophilic group, the effect of improving film-formability can be sufficiently imparted.

The repeating unit (b1) has a hydrophilic group selected from the group consisting of an ether bond, an ester bond, a hydroxy group, a carboxy group, a sulfonamide bond, a sulfonimide bond, a sulfo group, a lactone ring, a sultone ring, a carbonate bond, a urethane bond, and an amide bond.

Specific examples of the monomer providing the repeating unit (b1) include those shown below, but are not limited thereto.

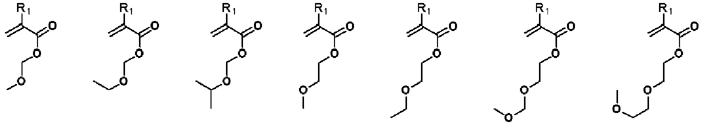

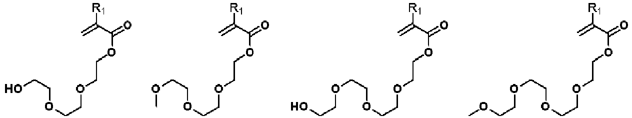

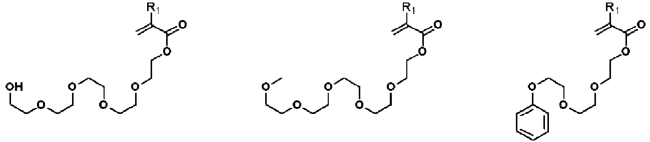

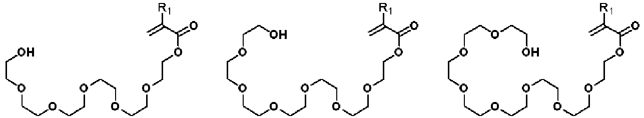

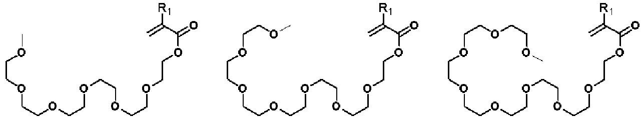

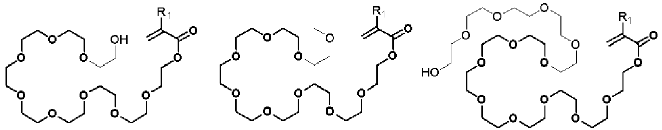

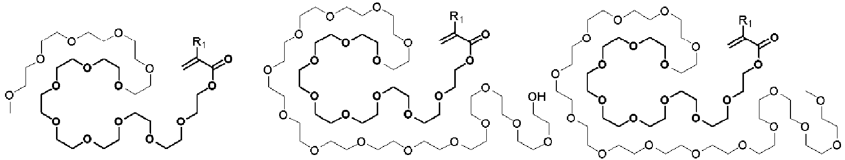

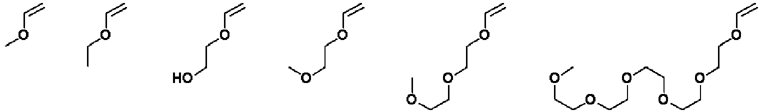

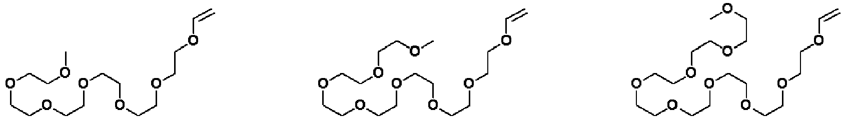

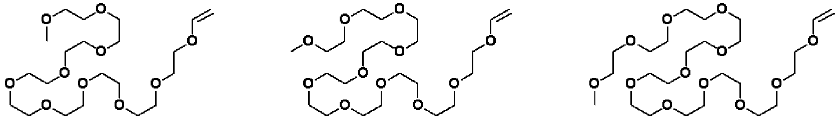

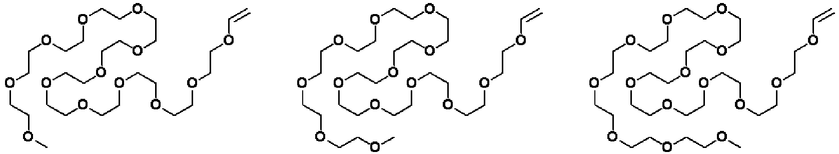

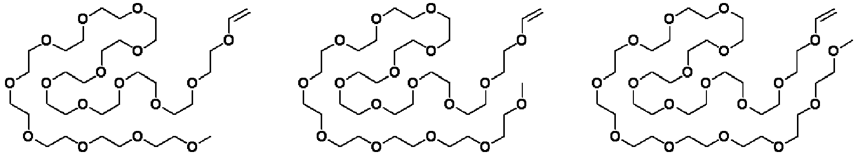

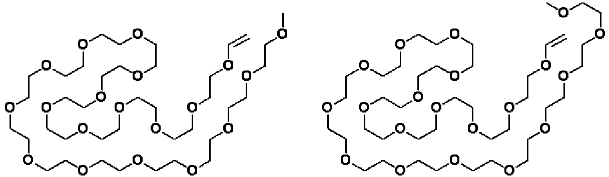

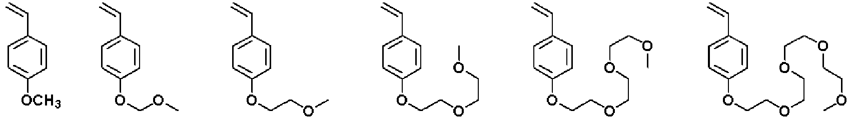

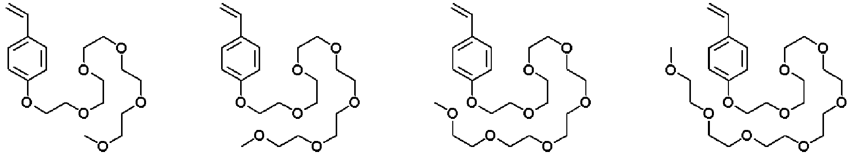

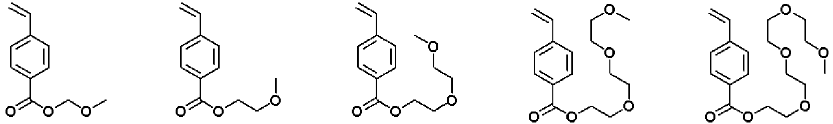

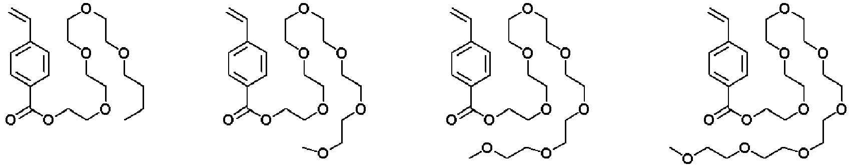

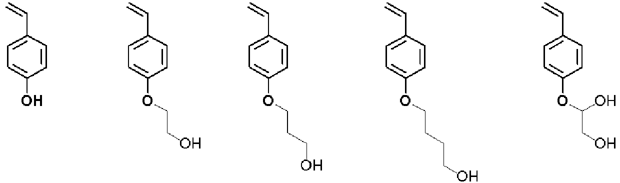

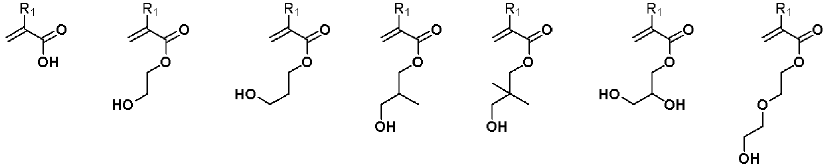

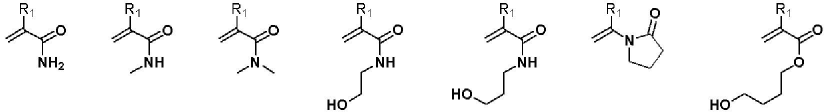

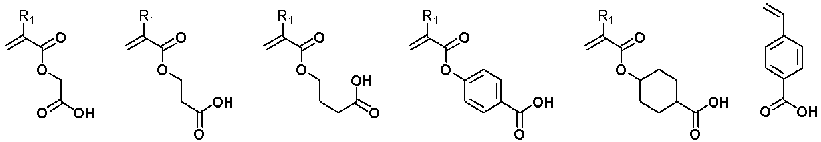

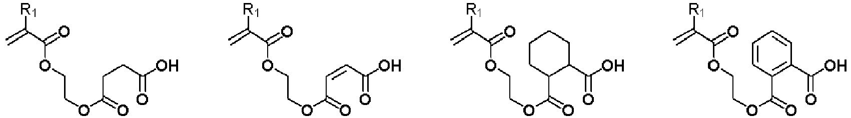

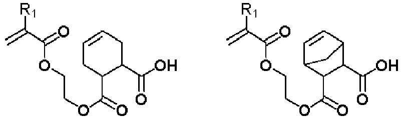

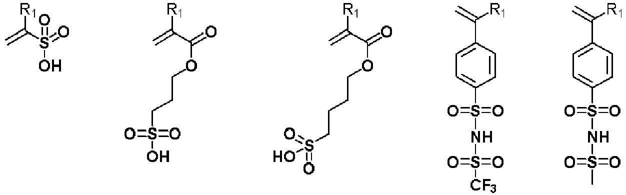

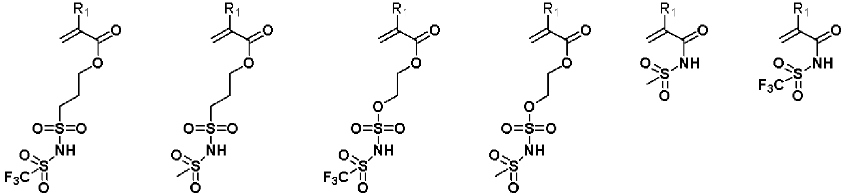

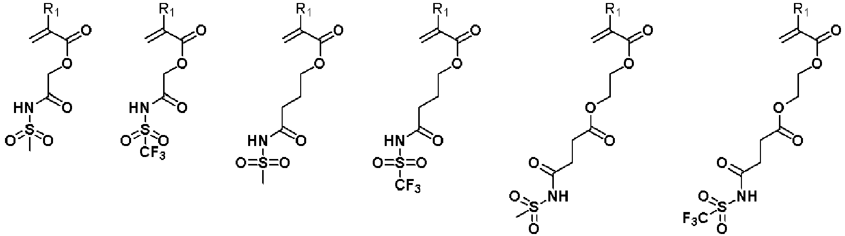

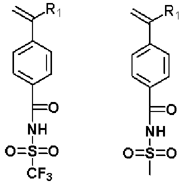

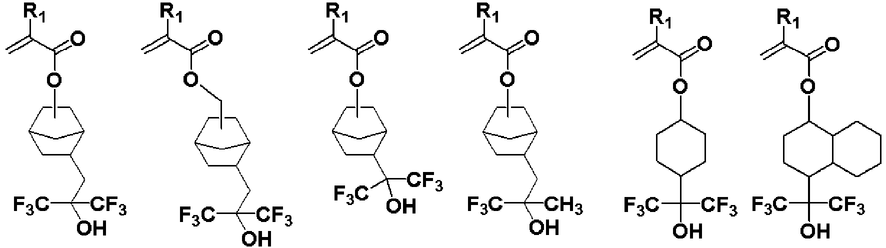

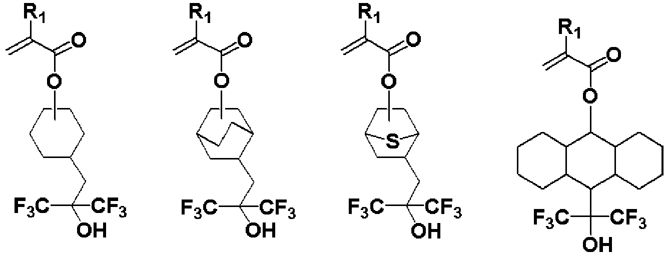

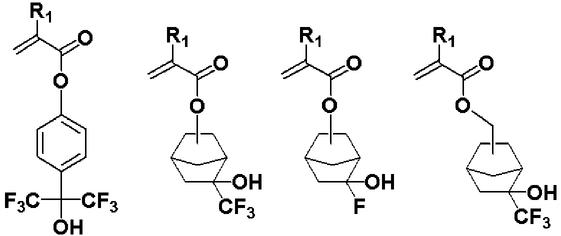

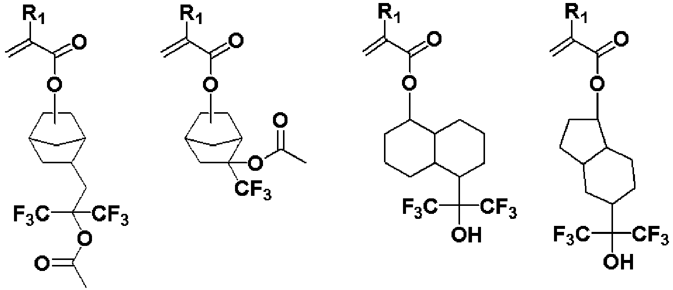

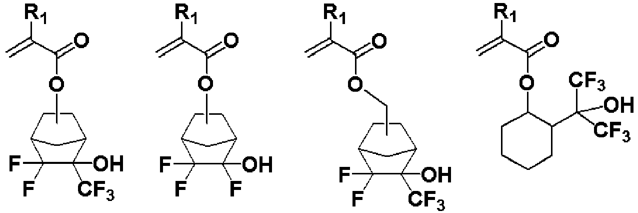

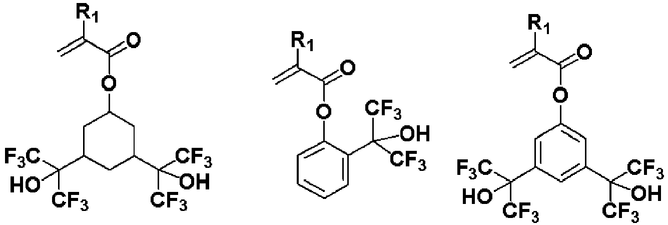

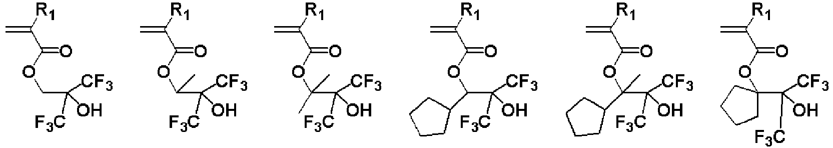

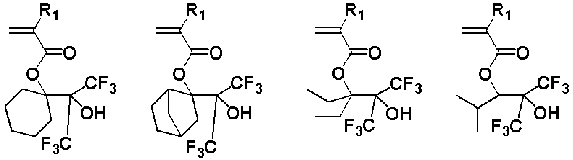

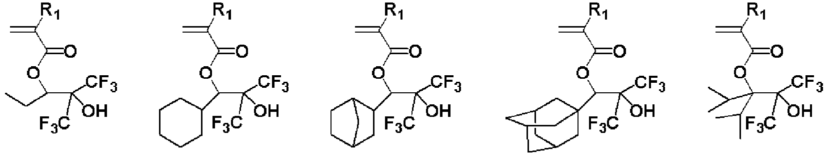

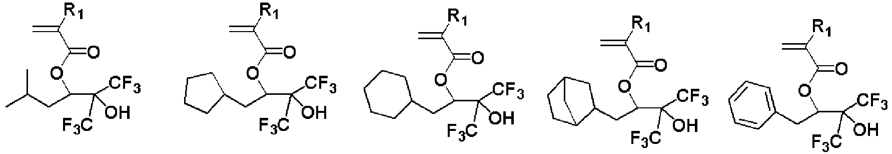

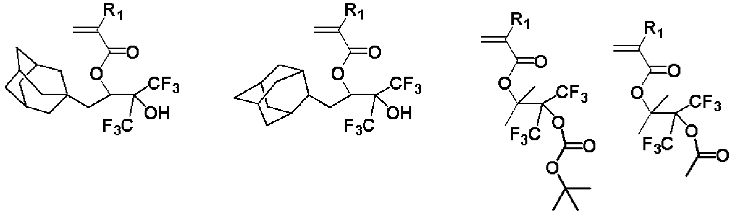

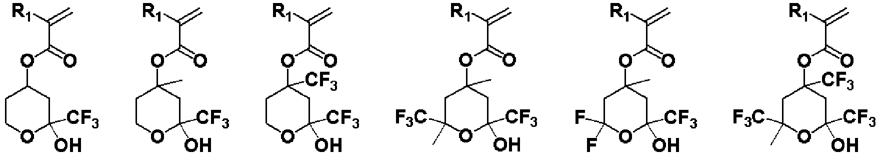

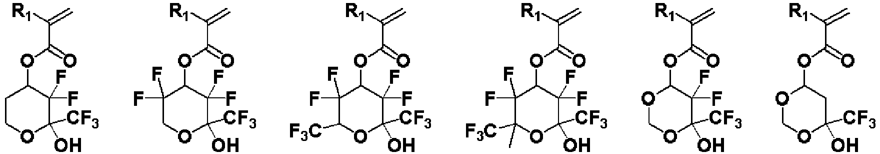

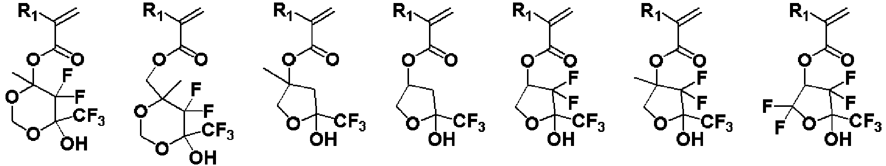

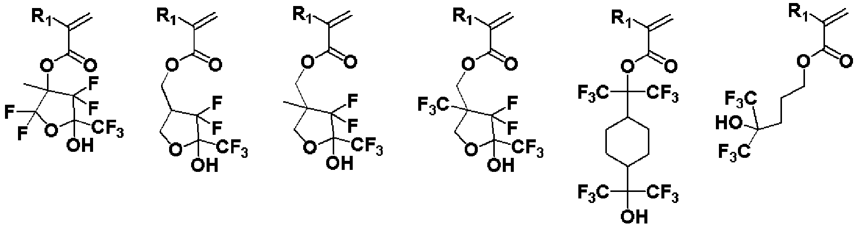

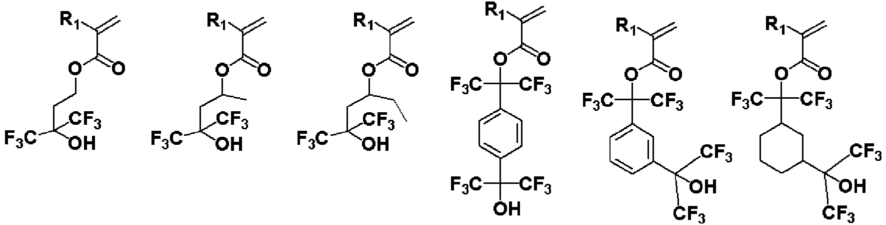

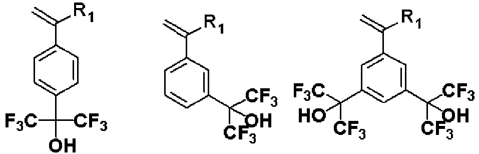

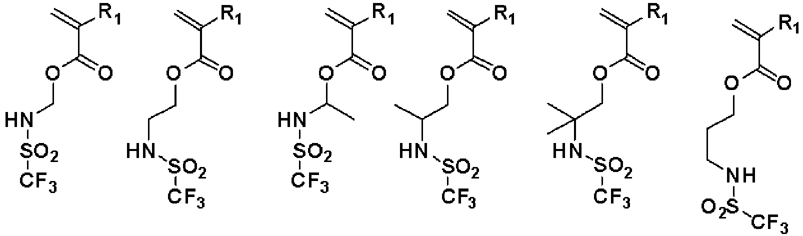

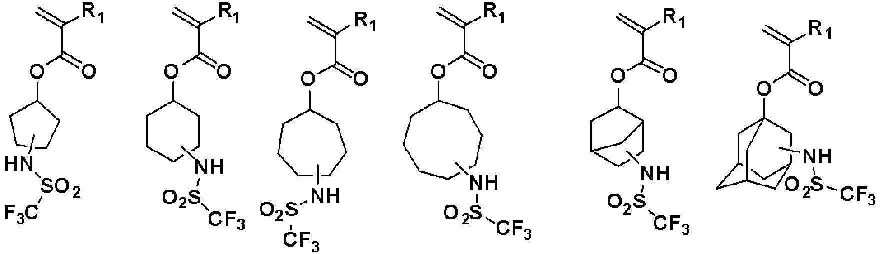

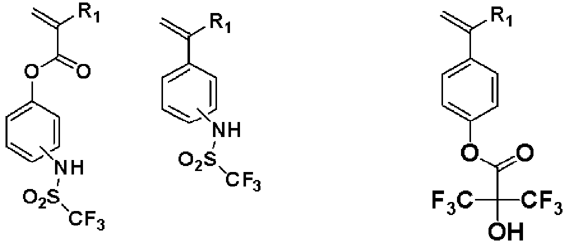

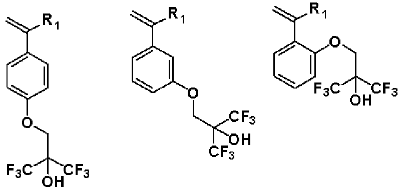

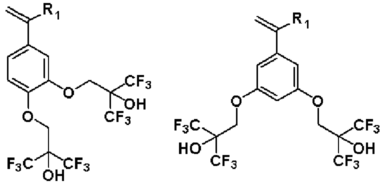

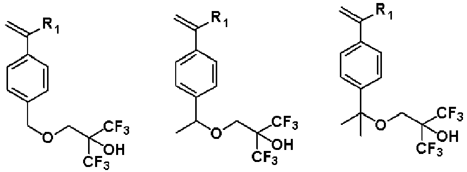

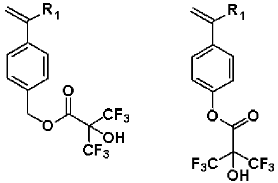

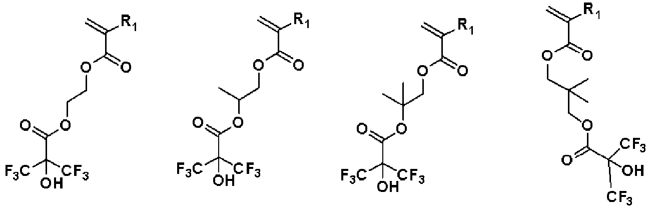

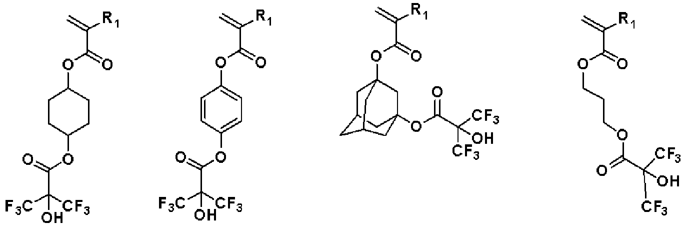

In the formulae, R₁ is a hydrogen atom or a methyl group.

The polymer (a1) preferably includes further a repeating unit (c1) having a fluorinated aromatic group, a repeating unit (c2) having a trifluoromethoxy group, a difluoromethoxy group, a trifluoromethylthio group, or a difluoromethylthio group. By having the repeating unit (a1) with a highly water-repellent fluorine atom and the highly water-repellent repeating units (c1) and/or (c2), it is possible to enhance water-repellency and alkali solubility further.

Specific examples of the monomer providing the repeating unit (c1) include those shown below, but are not limited thereto.

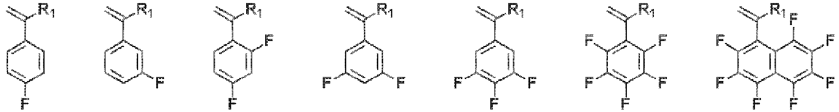

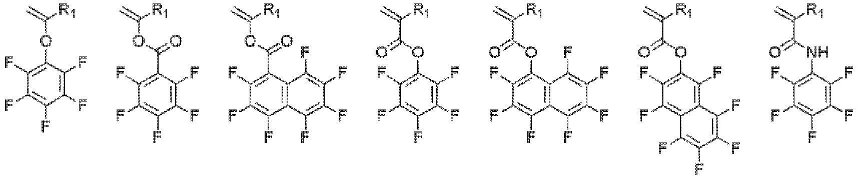

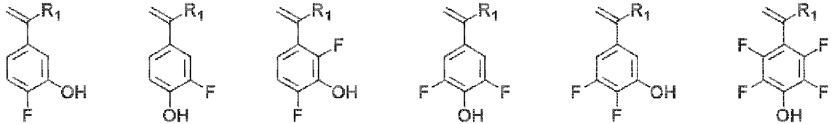

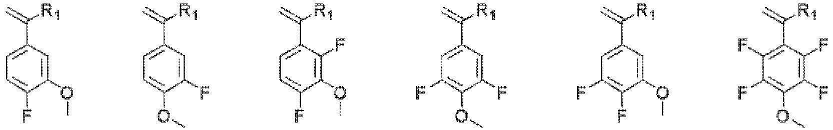

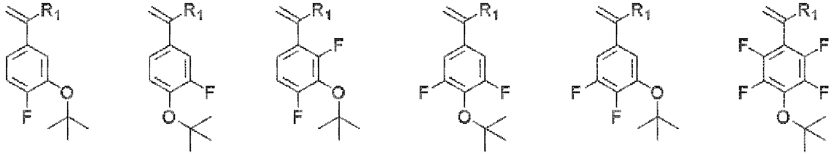

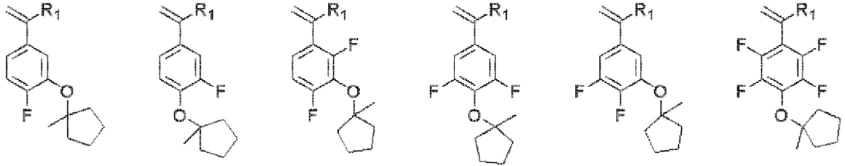

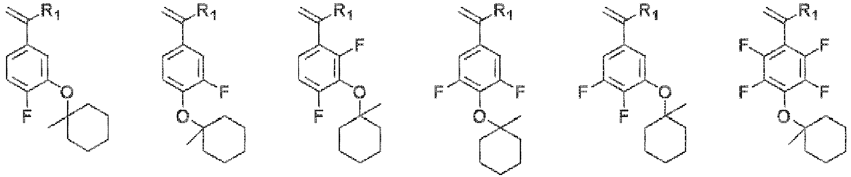

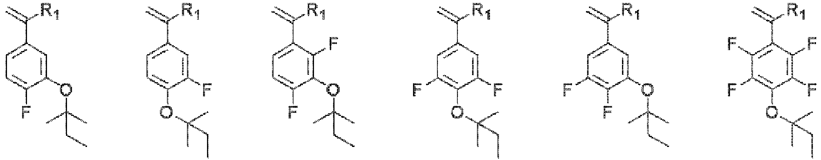

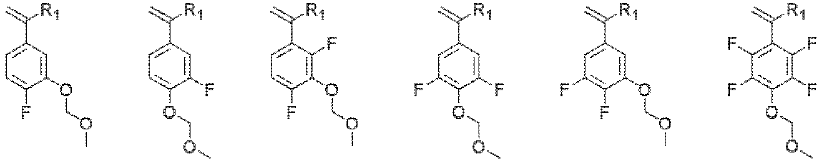

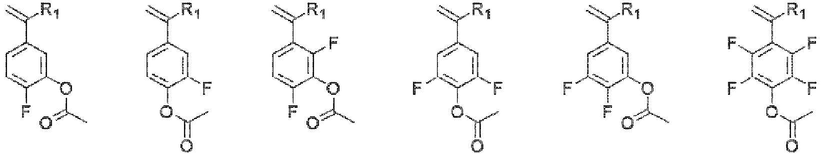

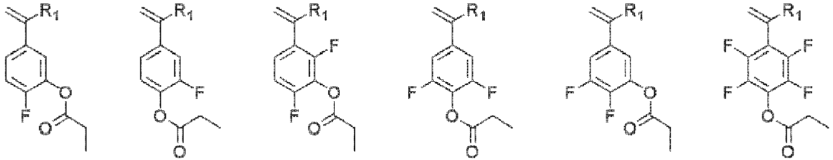

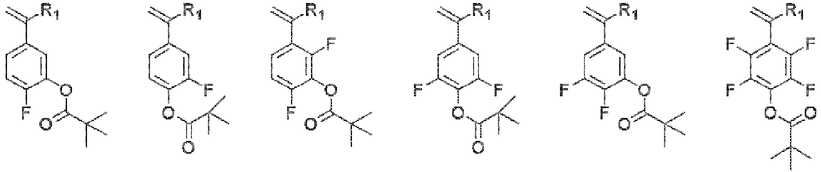

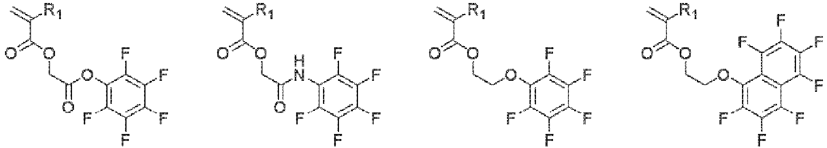

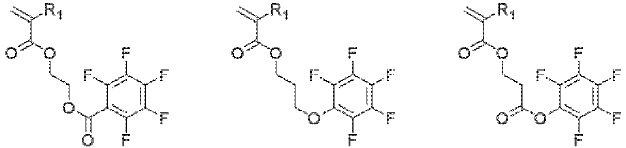

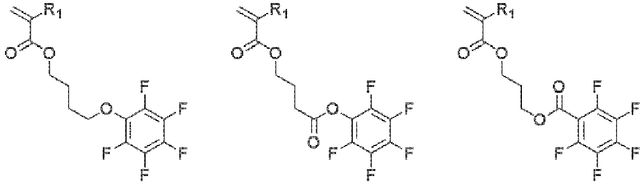

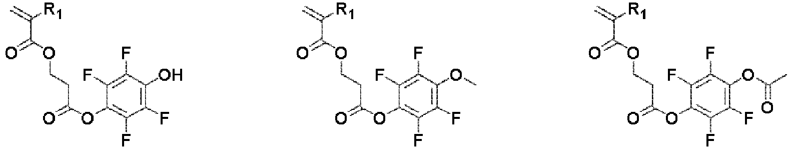

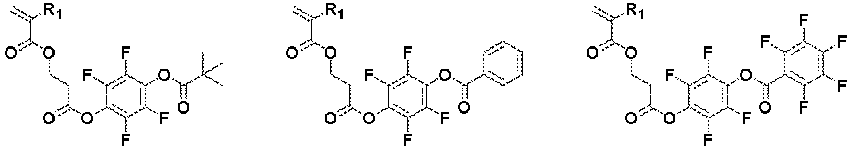

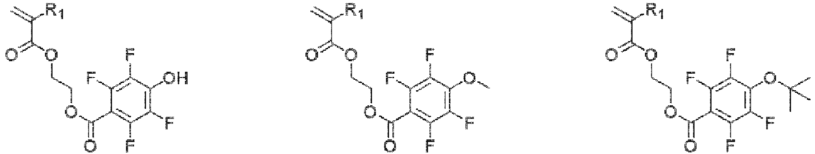

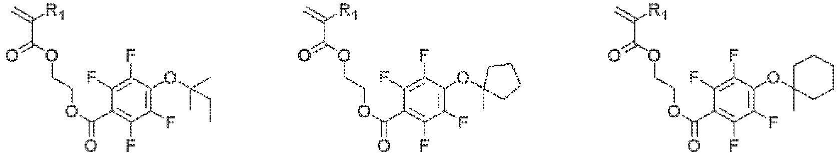

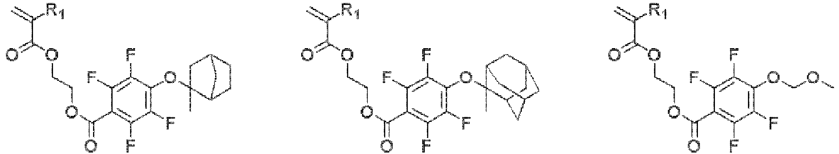

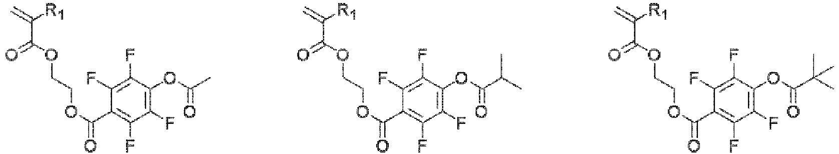

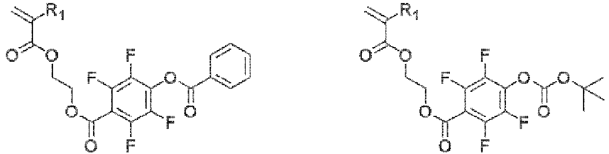

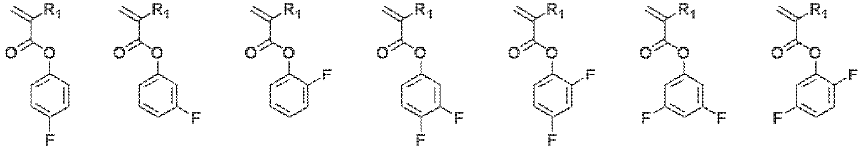

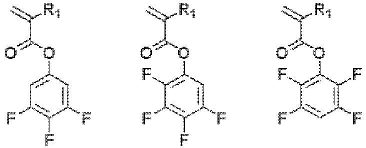

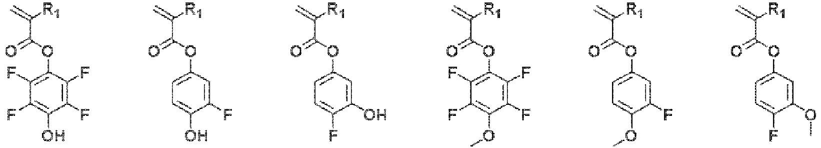

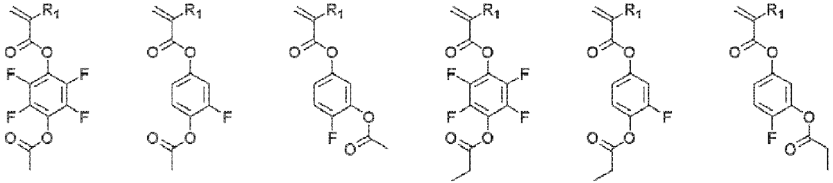

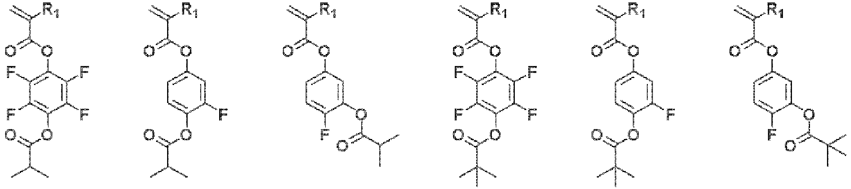

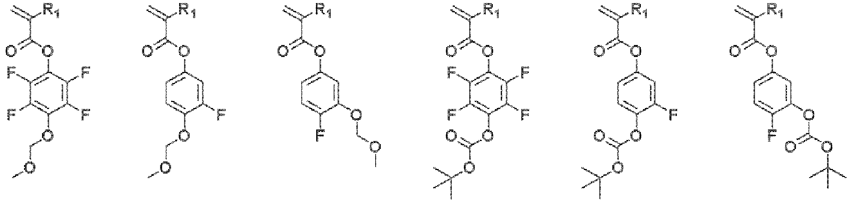

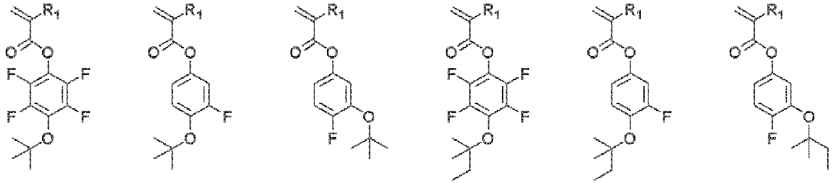

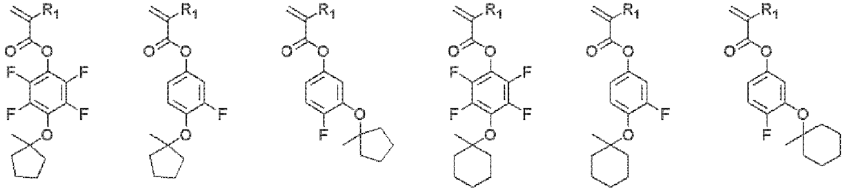

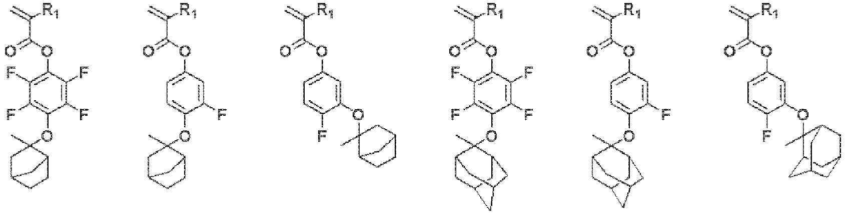

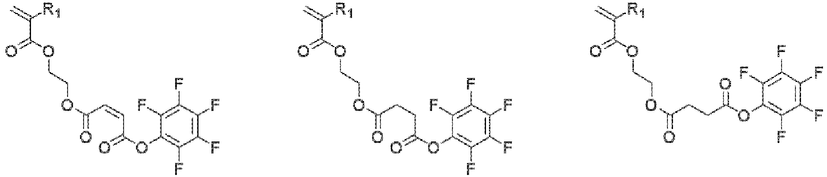

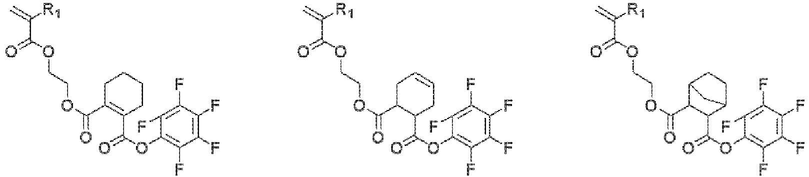

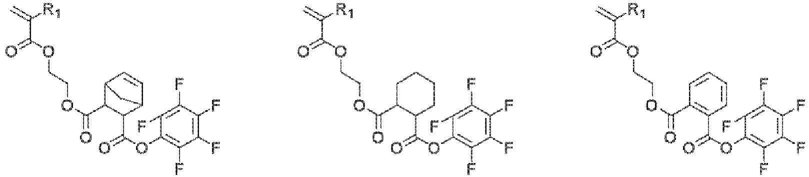

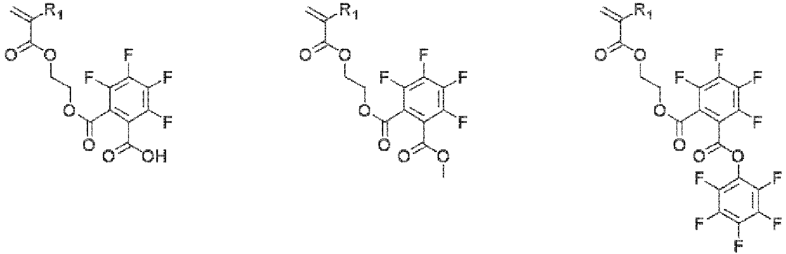

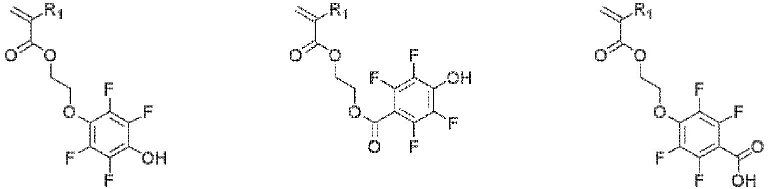

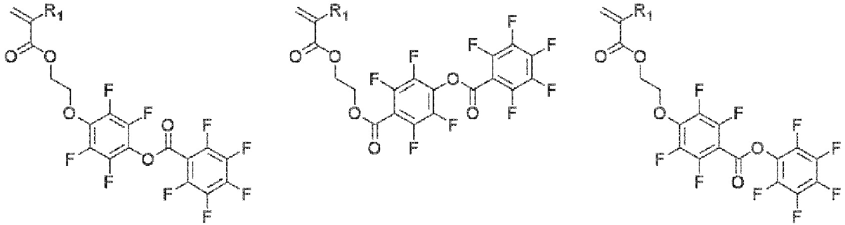

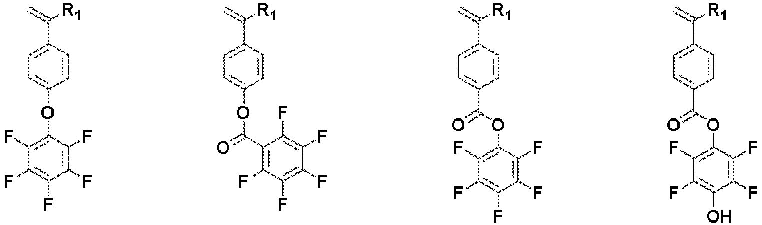

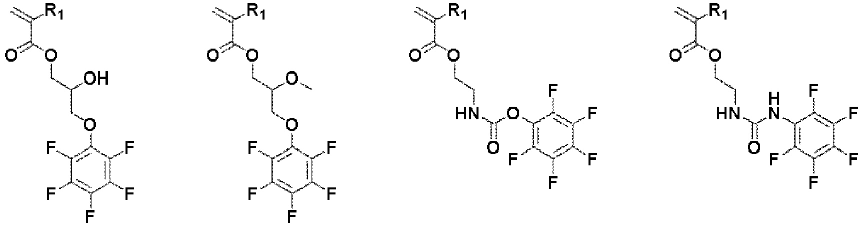

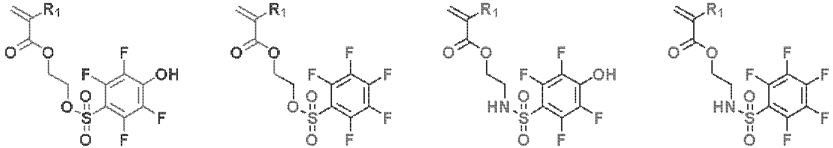

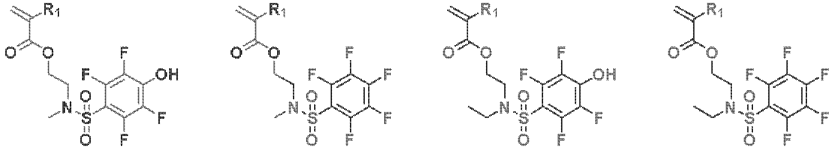

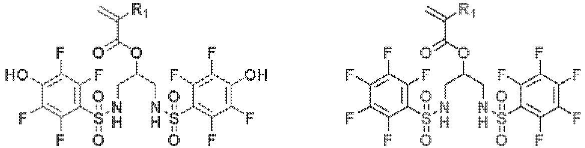

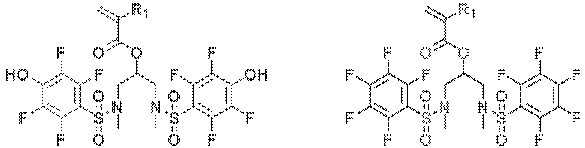

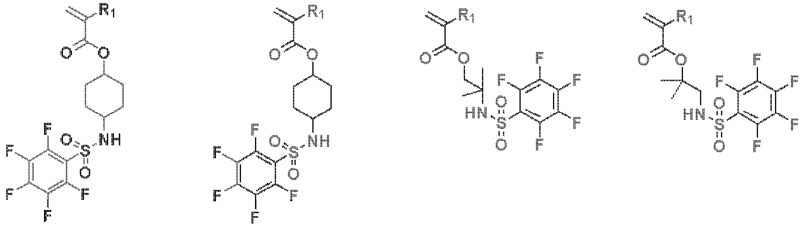

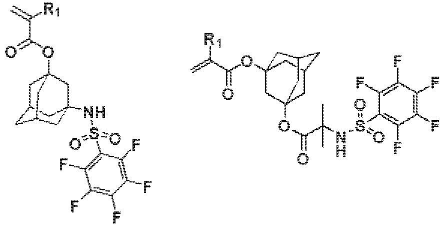

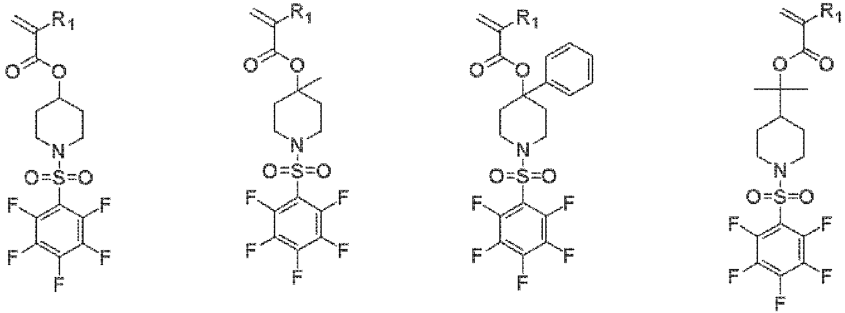

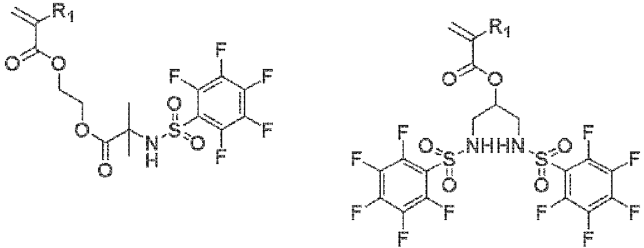

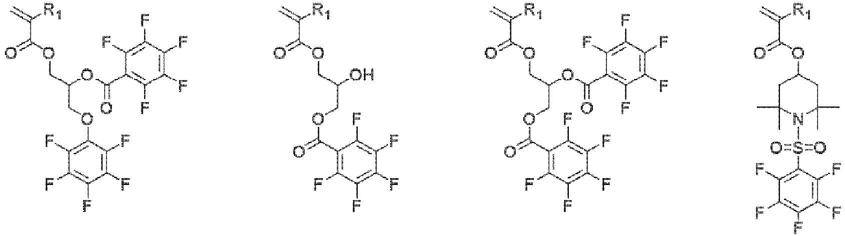

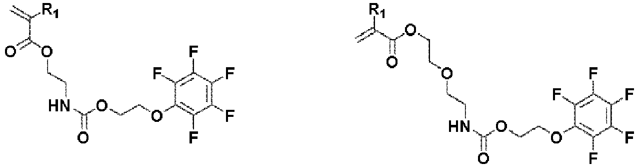

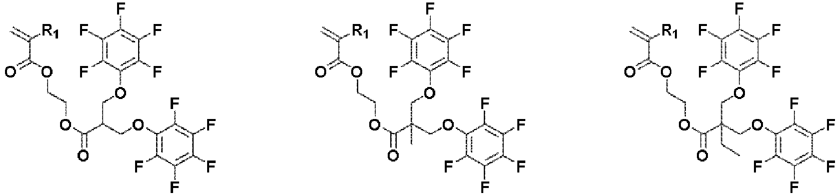

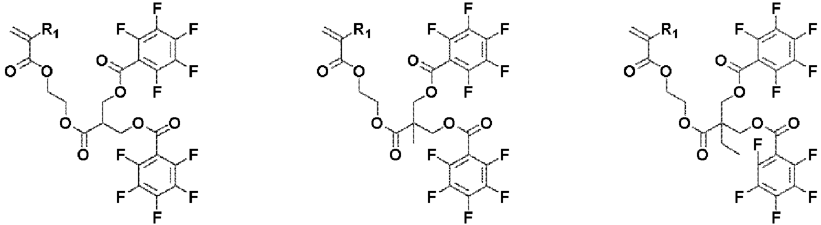

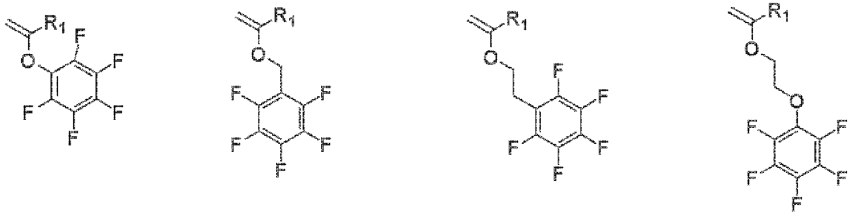

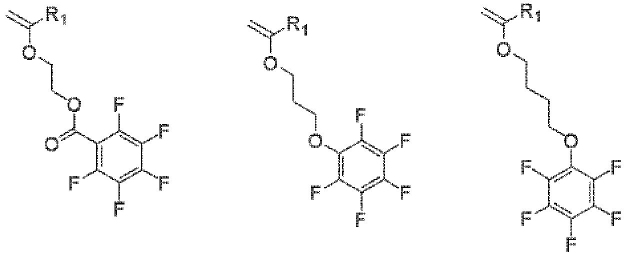

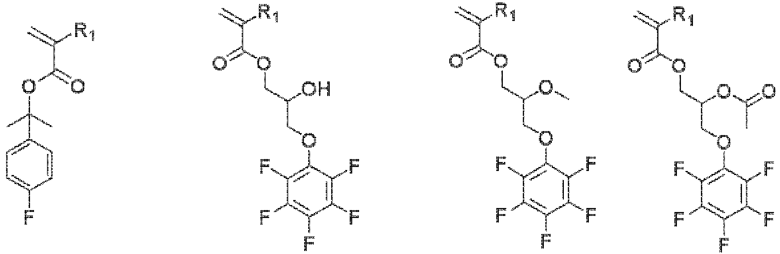

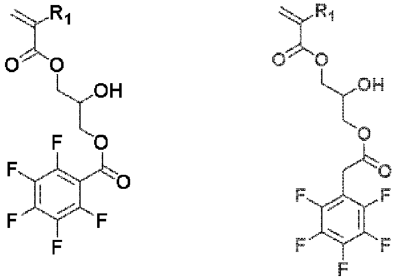

In the formula, R₁ is a hydrogen atom or a methyl group.

Monomers that provide the repeating unit c2 and have the trifluoromethoxy group, the difluoromethoxy group, the trifluoromethylthio group, or the difluoromethylthio group include those shown below, but are not limited thereto.

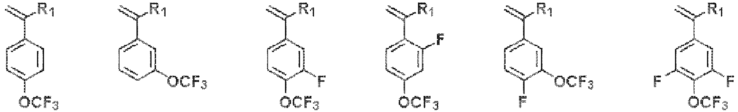

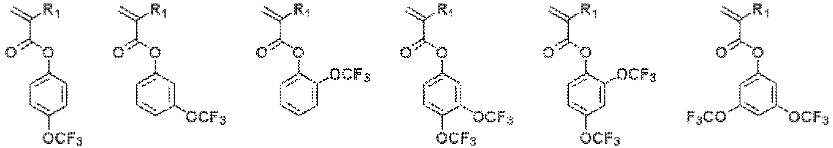

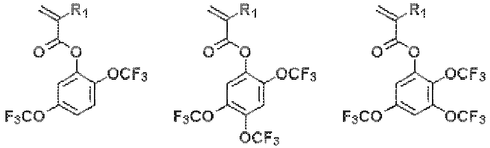

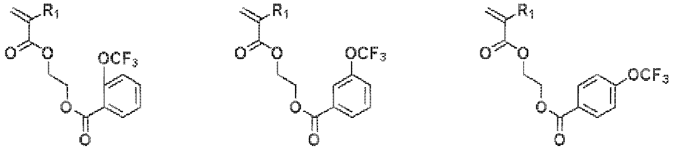

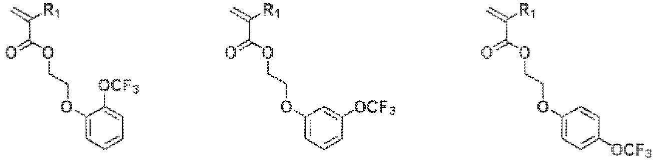

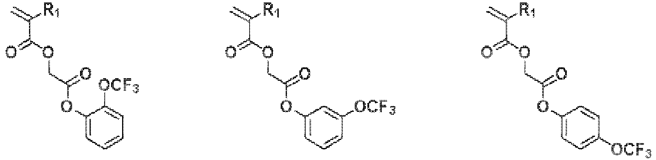

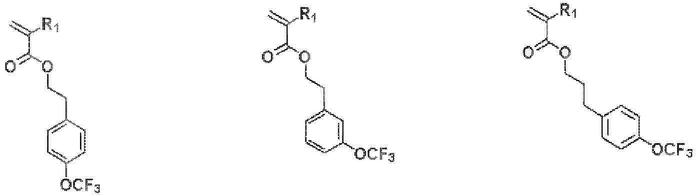

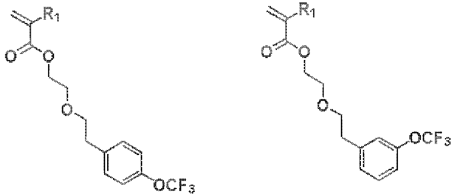

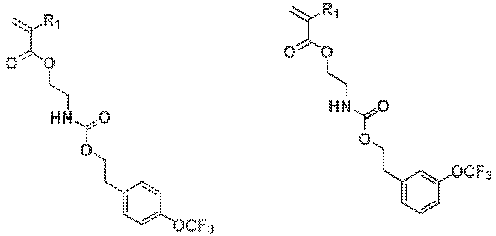

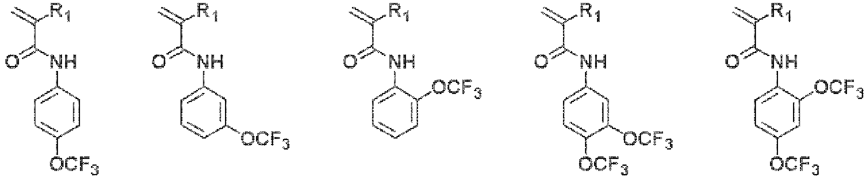

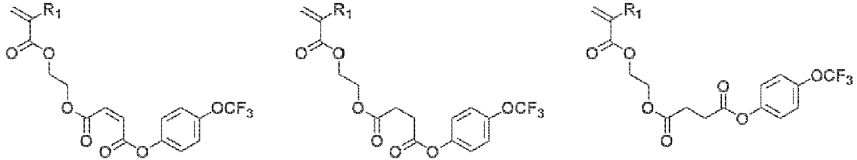

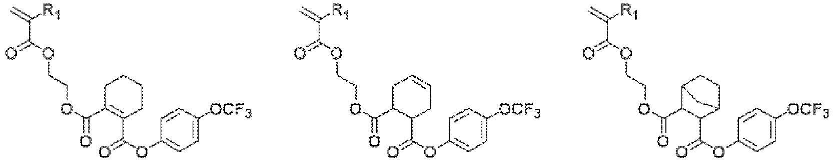

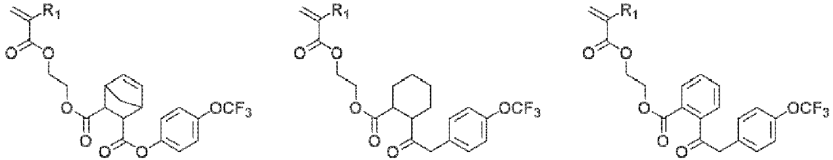

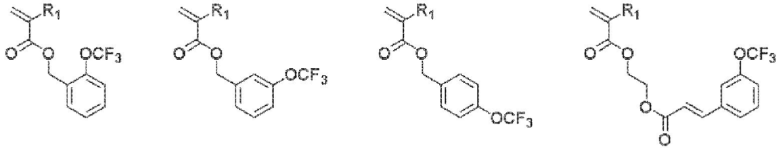

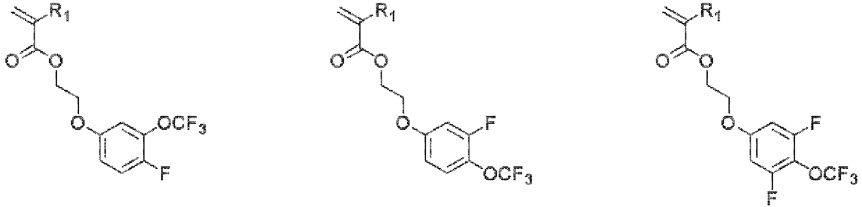

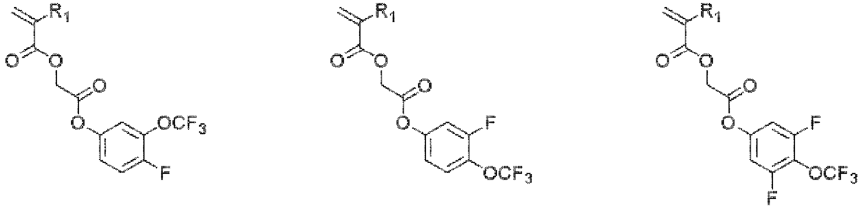

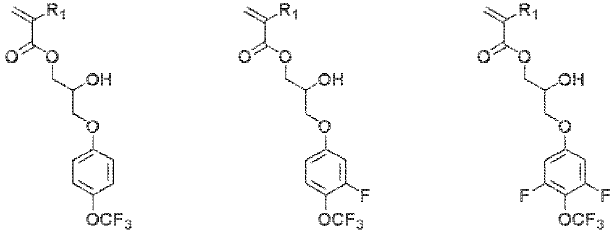

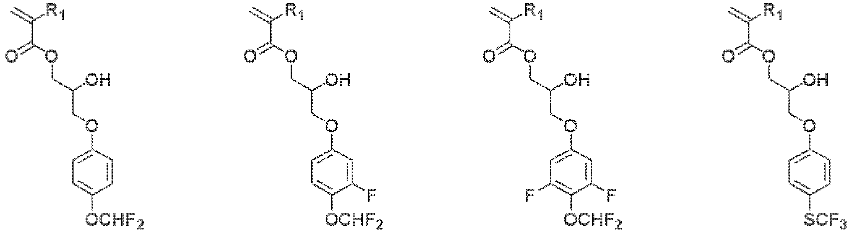

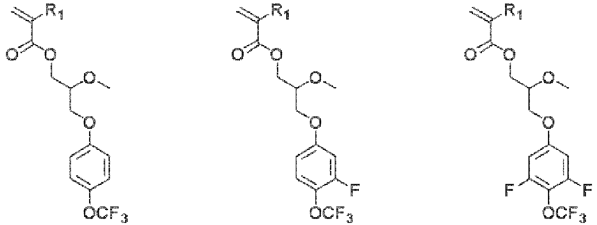

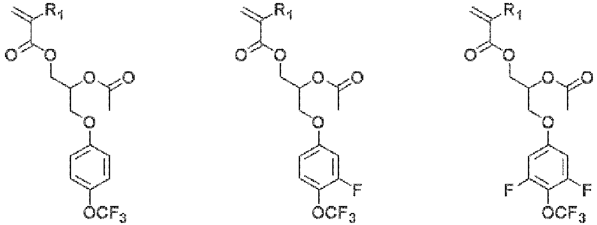

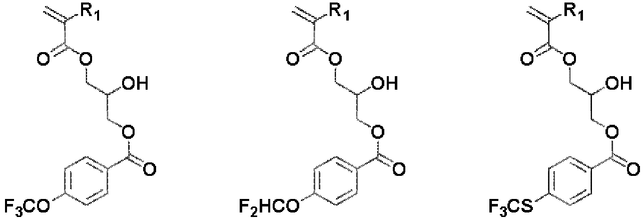

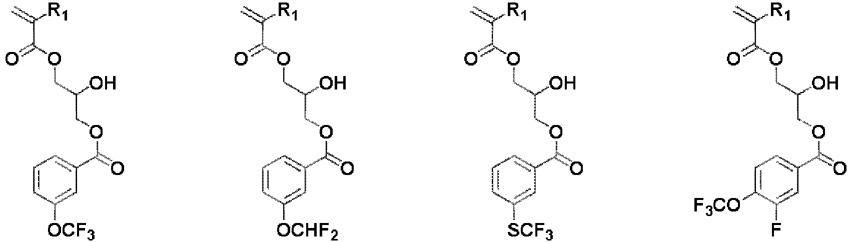

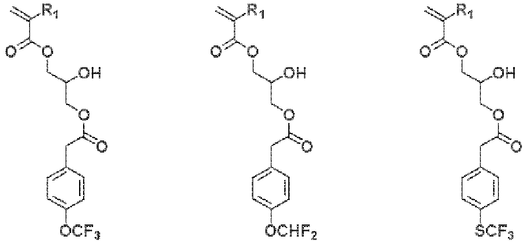

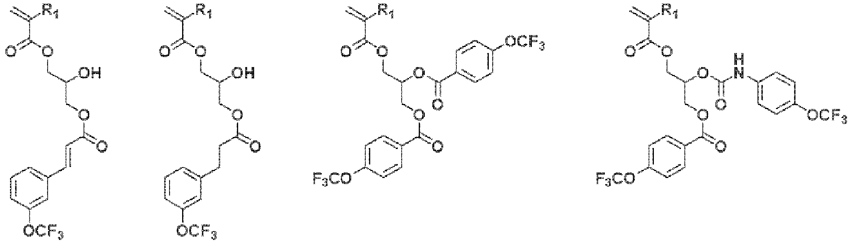

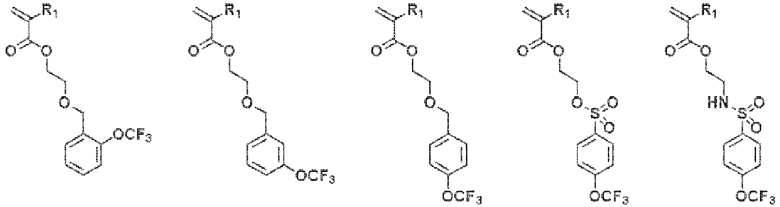

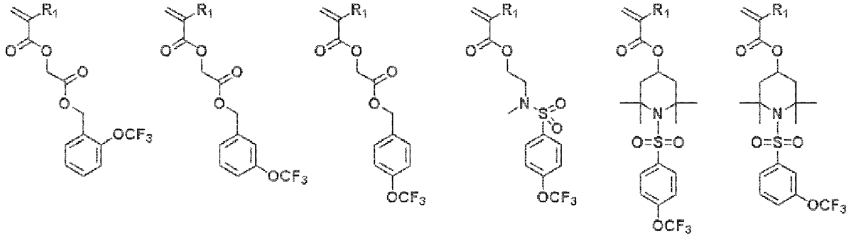

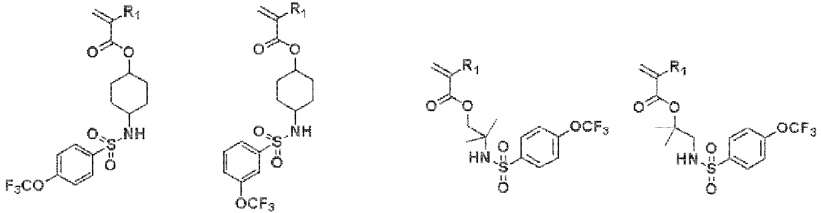

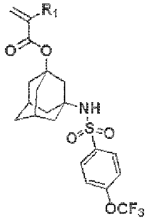

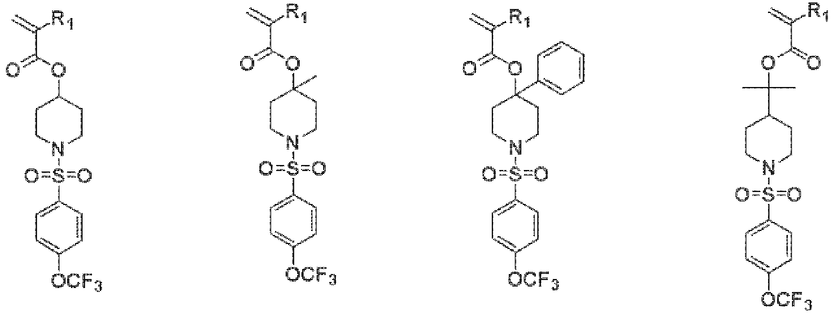

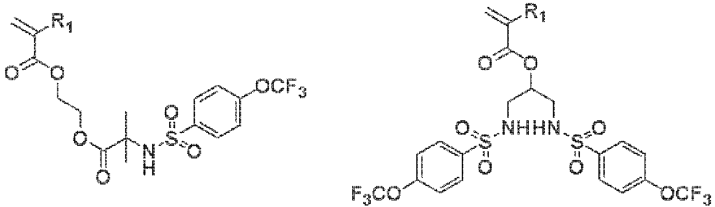

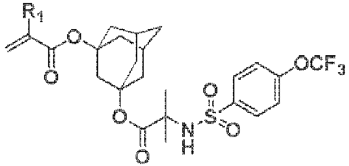

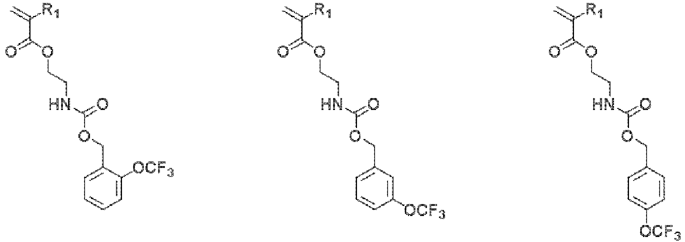

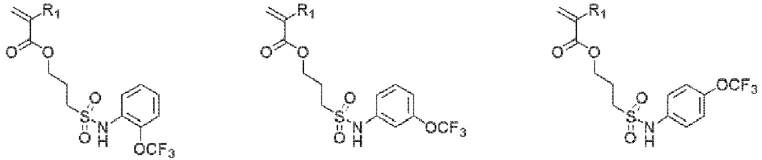

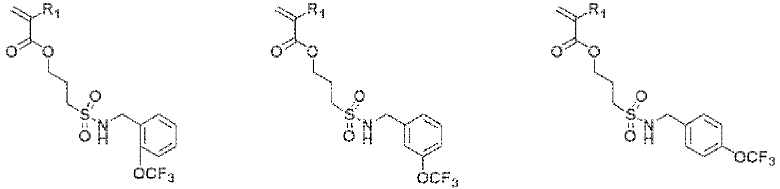

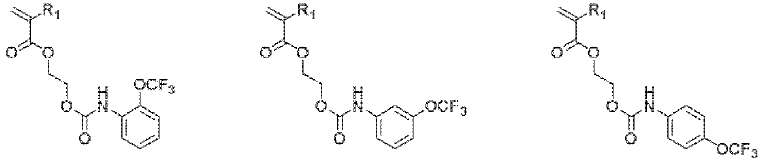

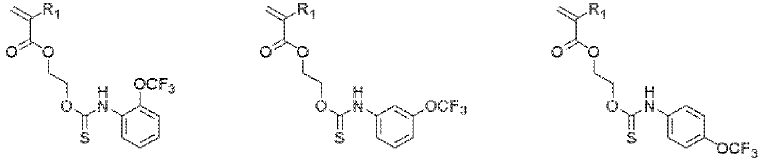

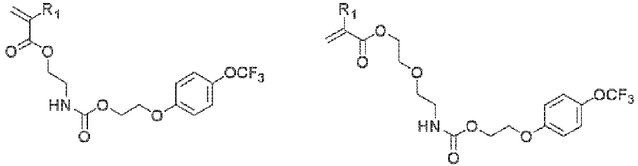

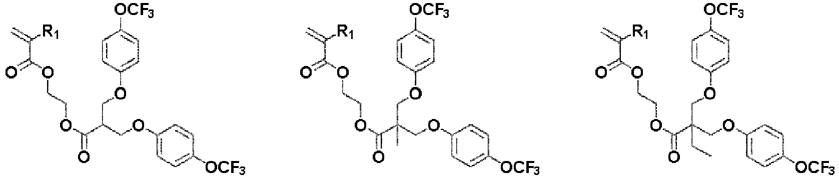

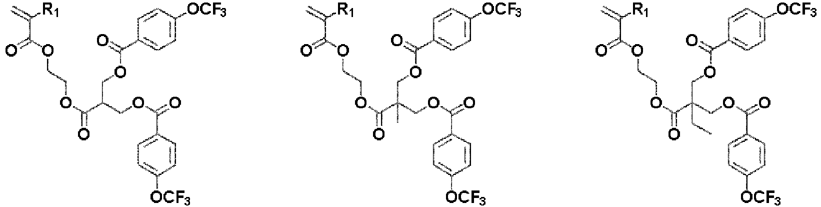

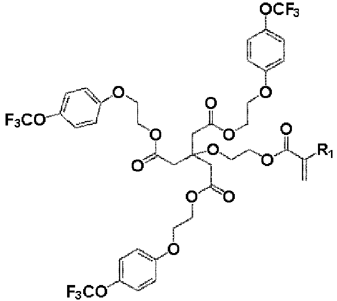

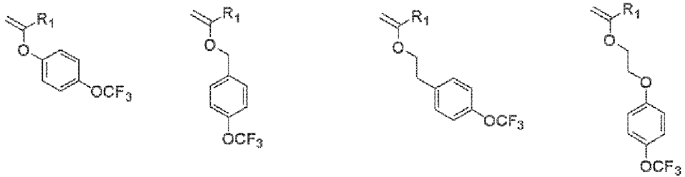

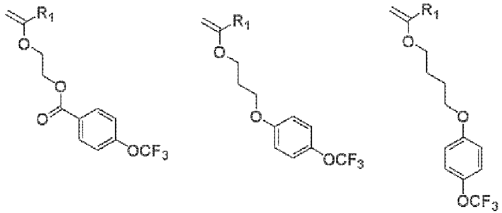

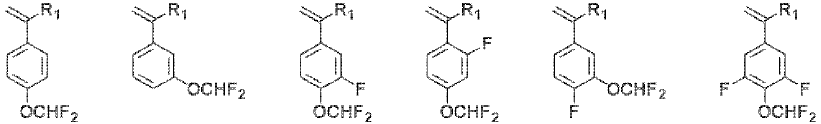

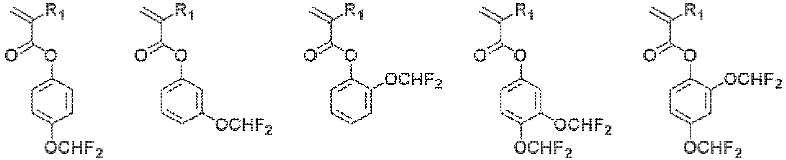

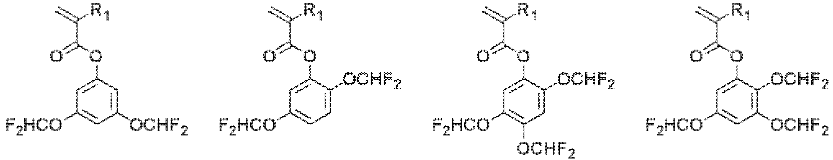

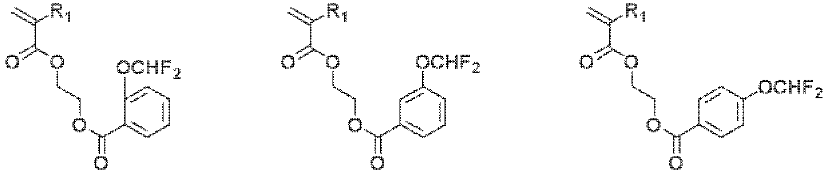

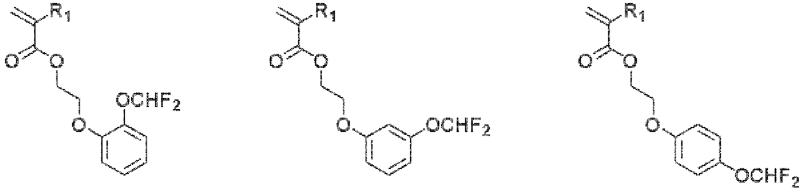

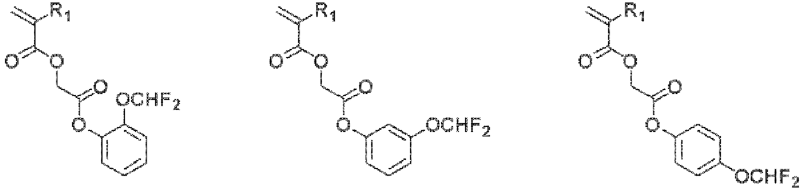

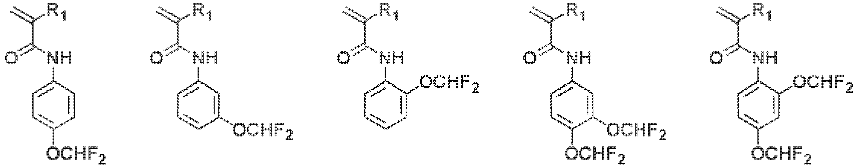

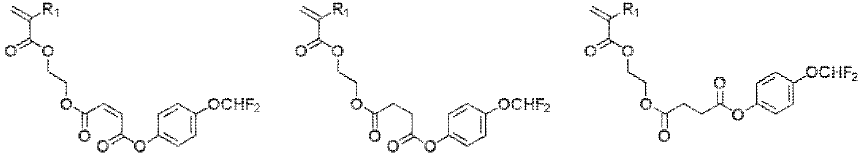

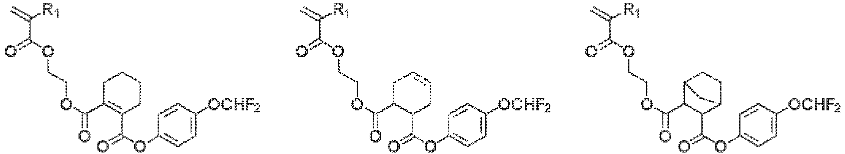

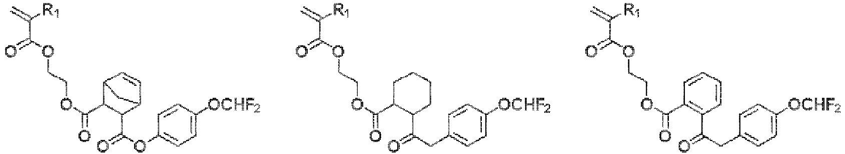

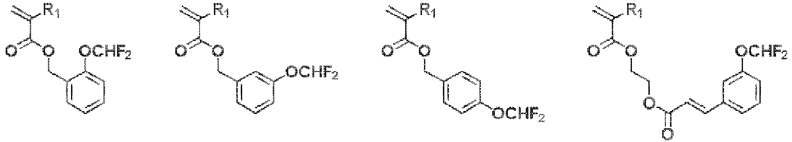

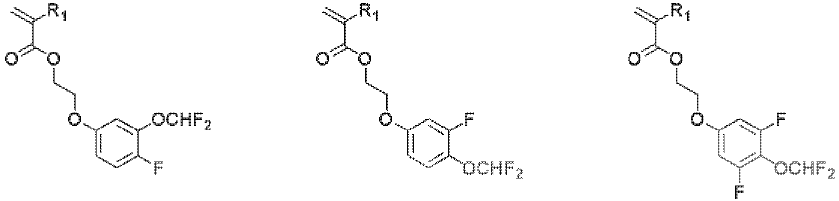

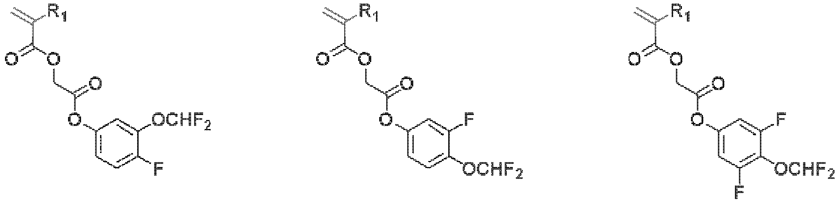

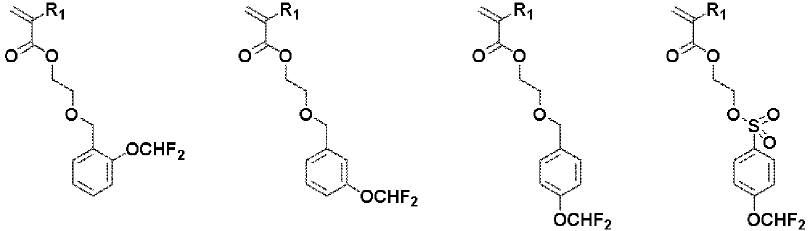

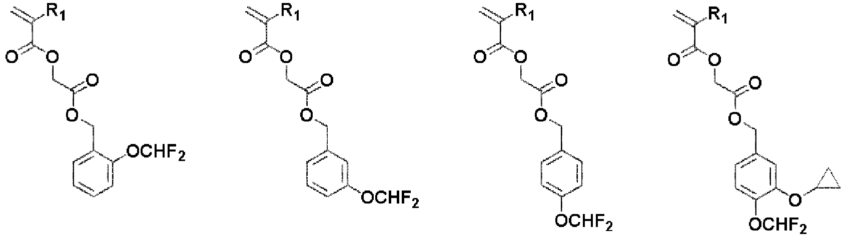

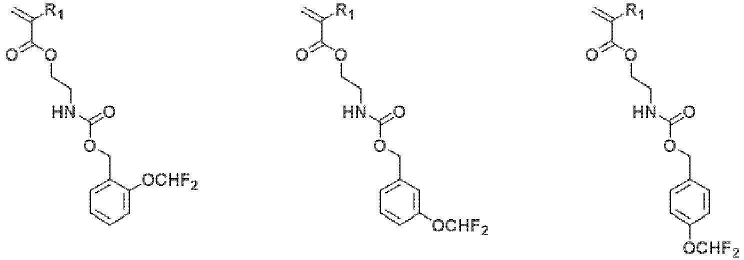

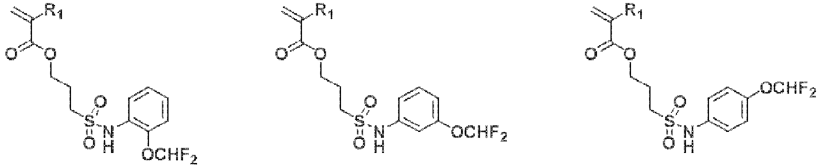

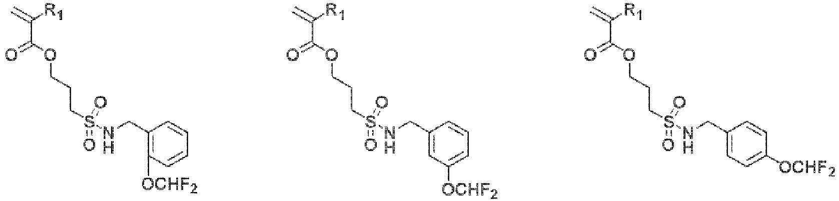

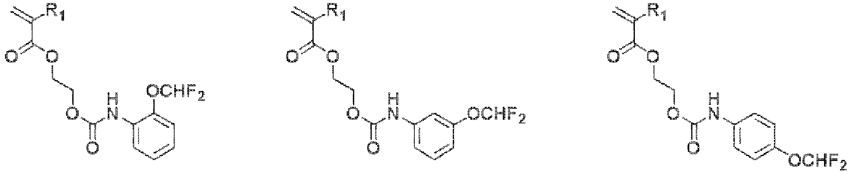

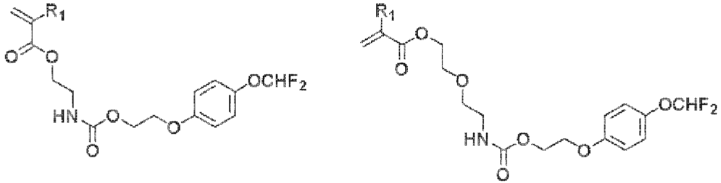

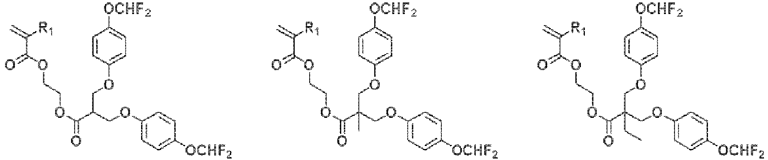

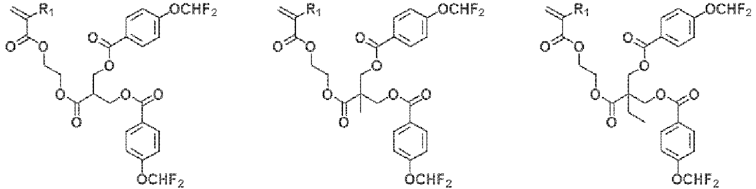

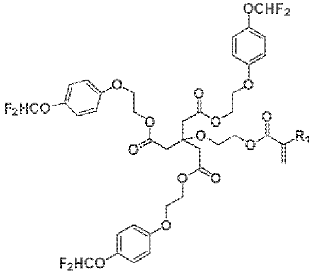

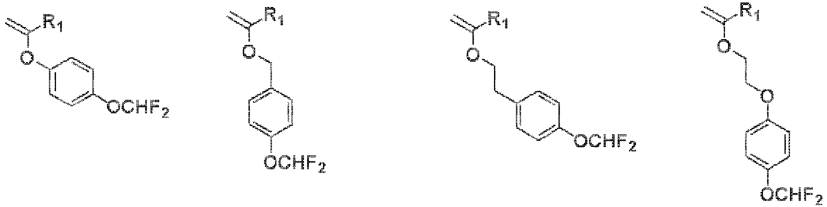

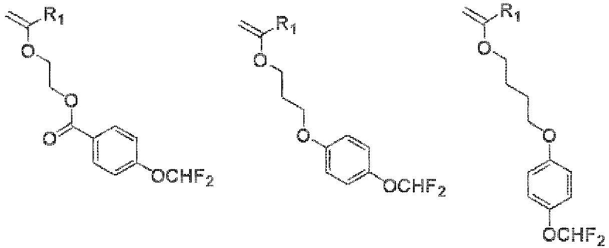

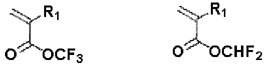

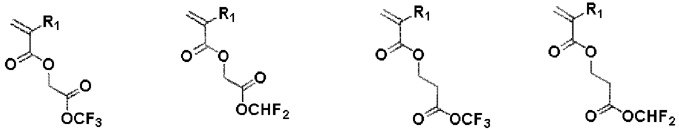

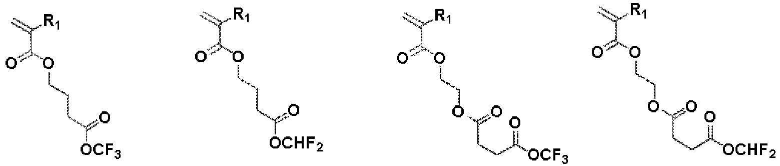

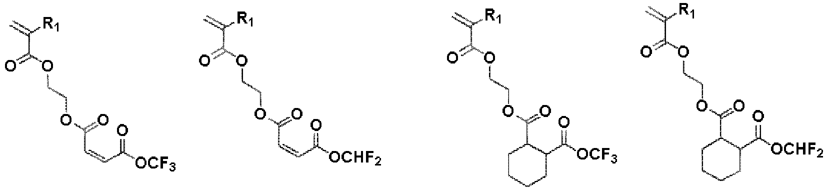

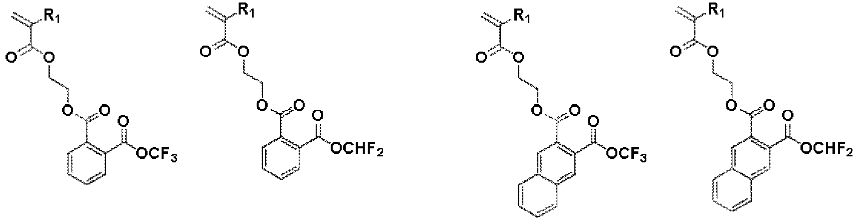

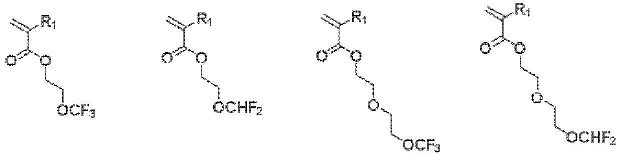

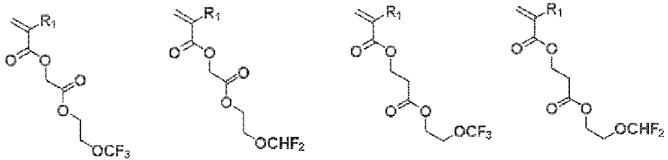

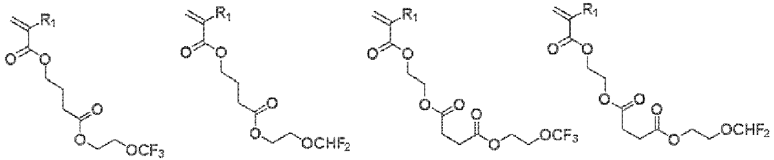

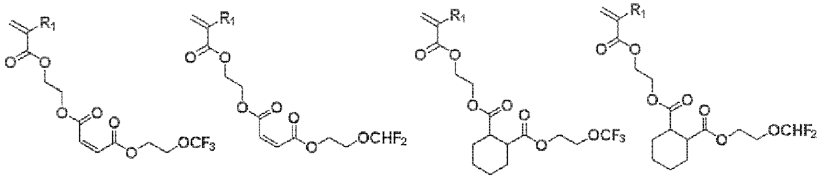

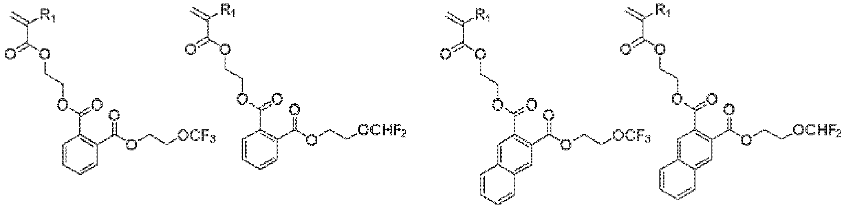

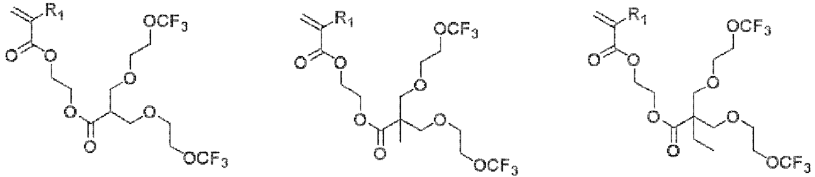

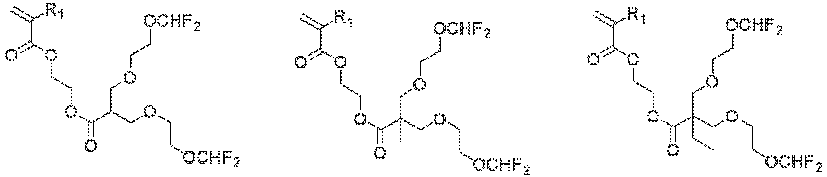

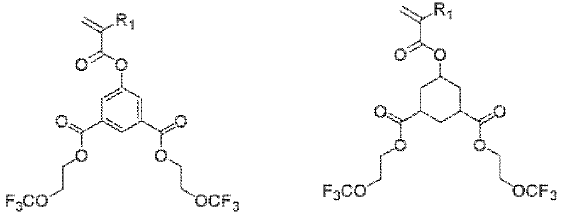

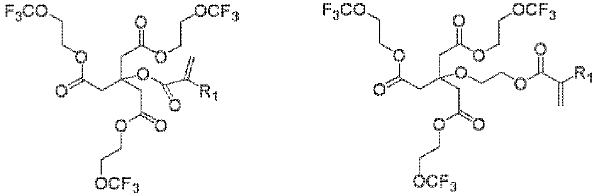

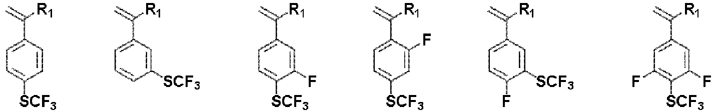

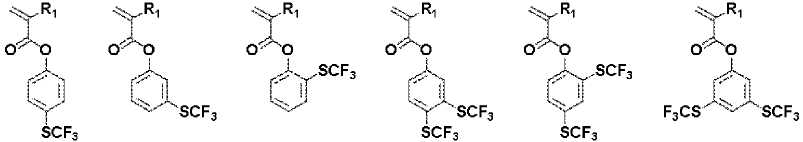

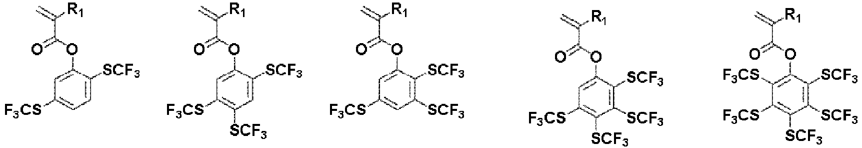

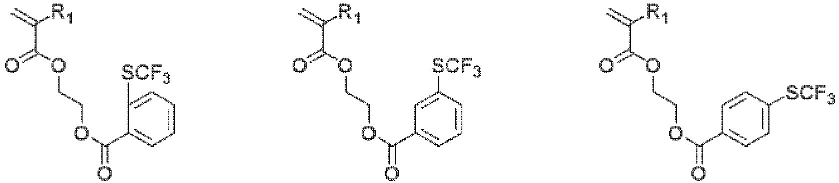

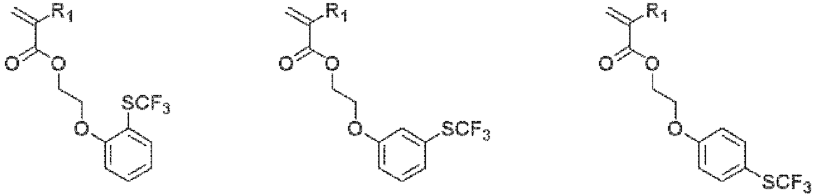

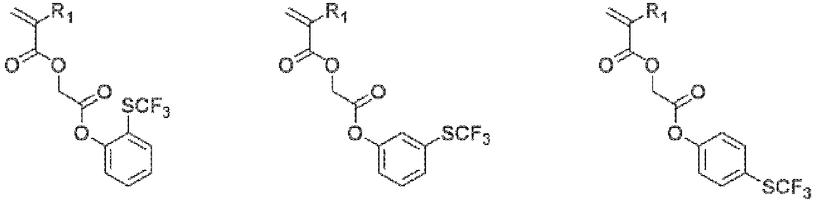

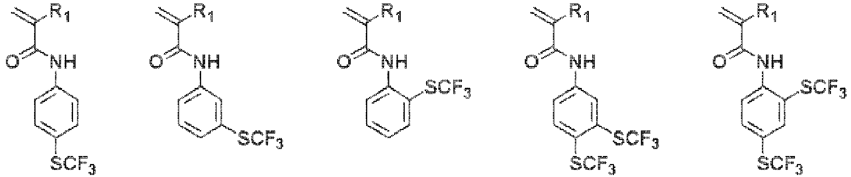

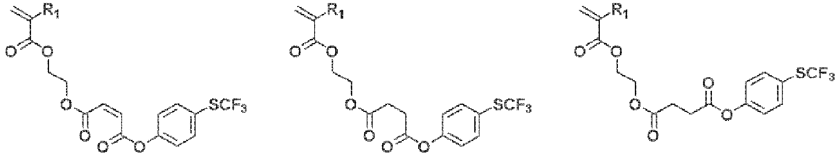

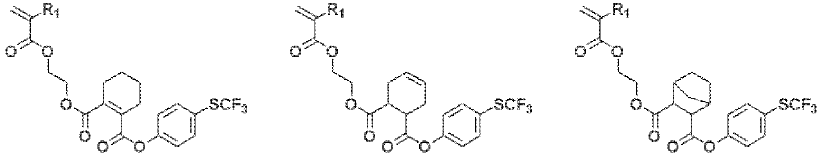

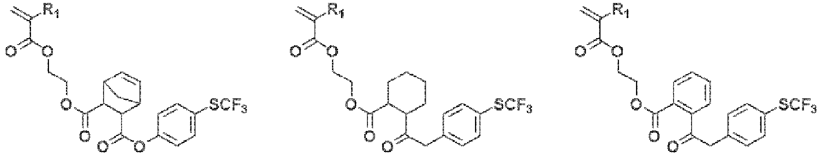

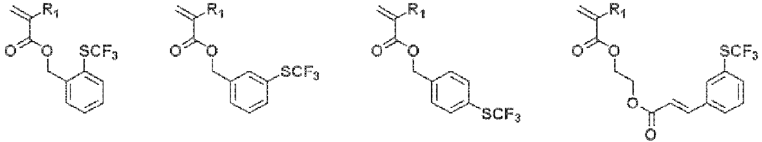

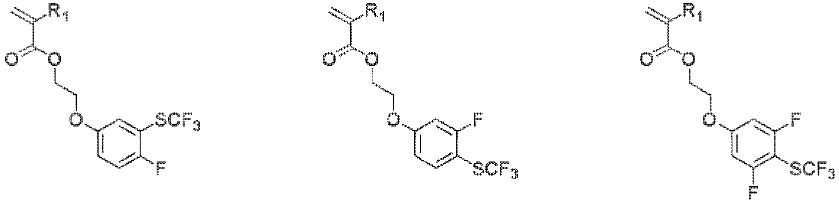

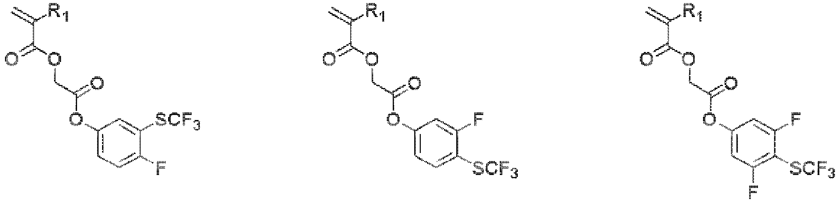

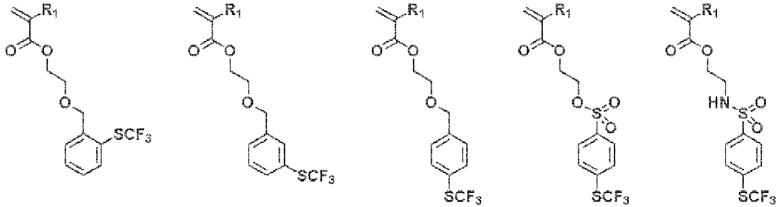

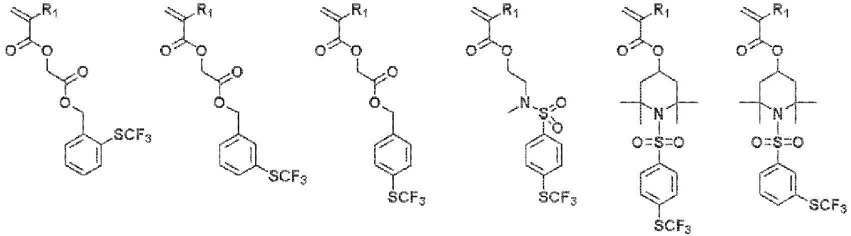

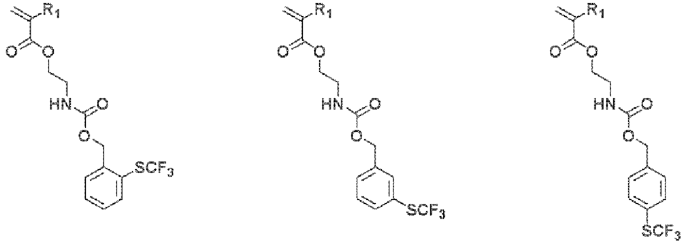

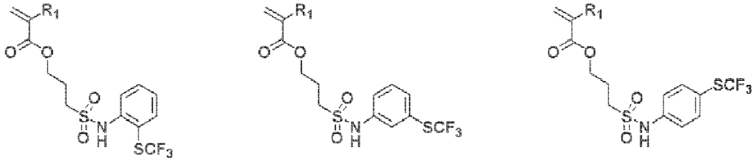

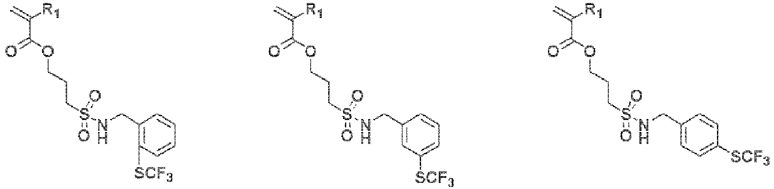

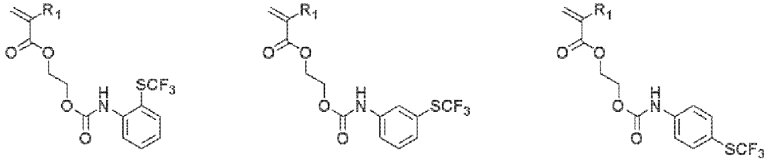

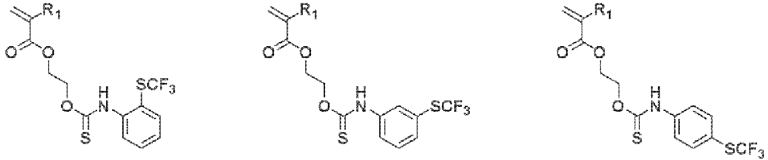

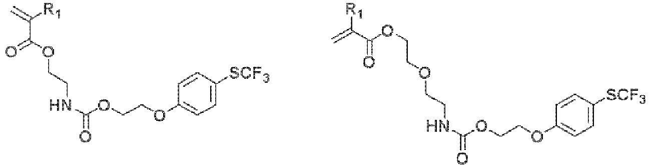

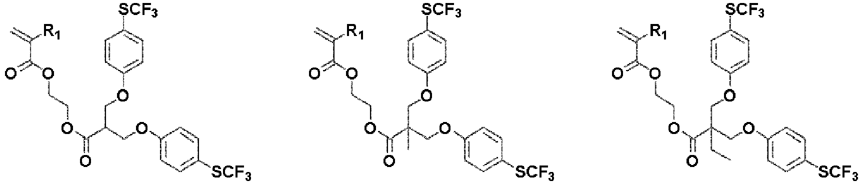

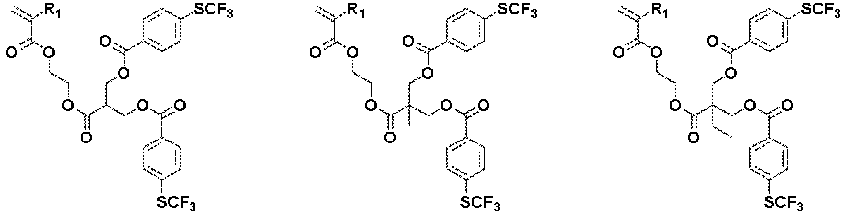

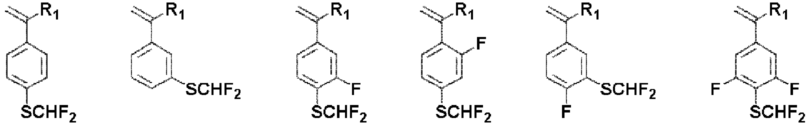

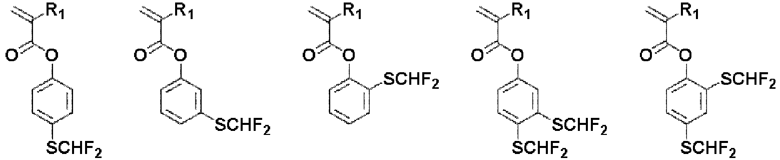

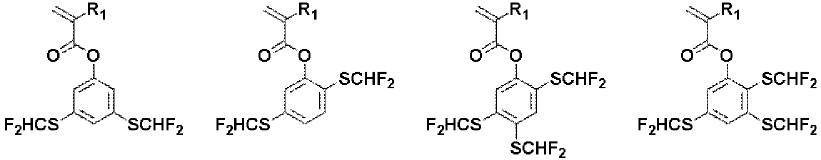

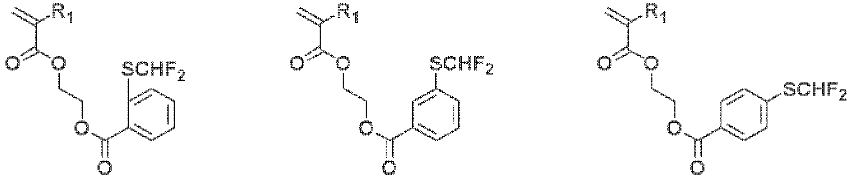

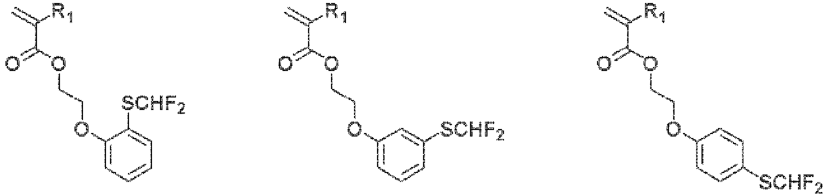

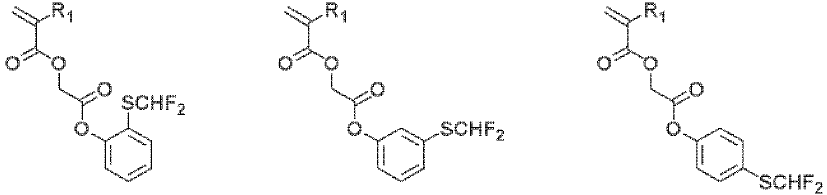

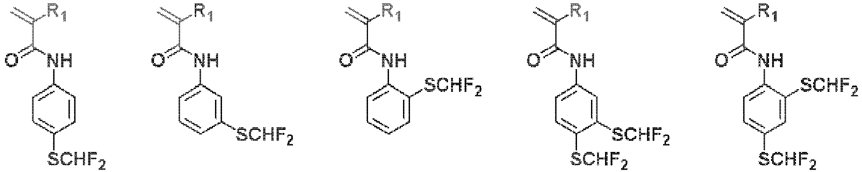

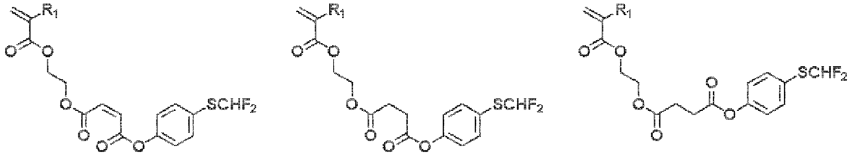

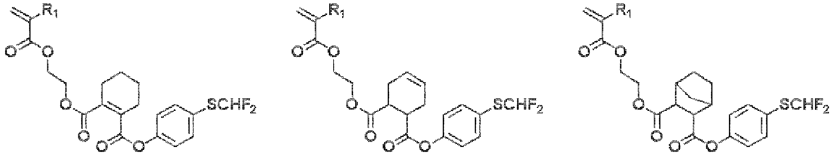

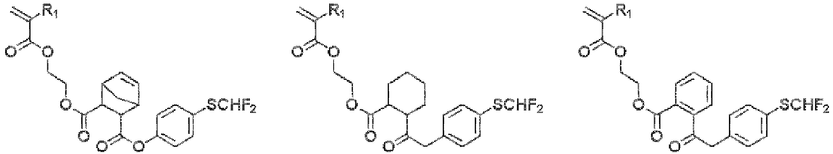

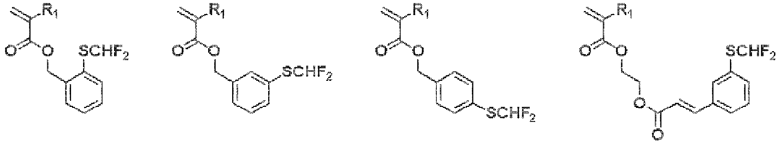

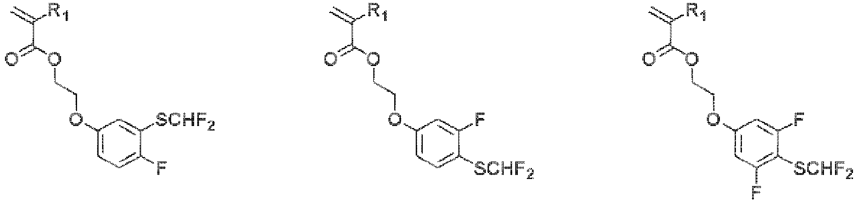

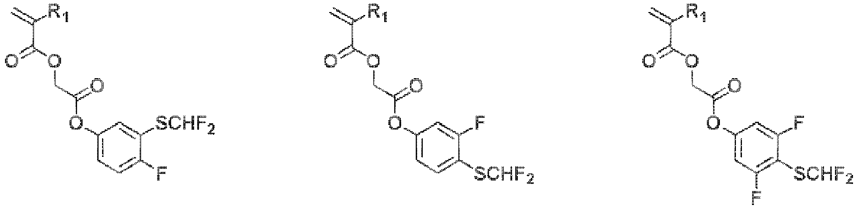

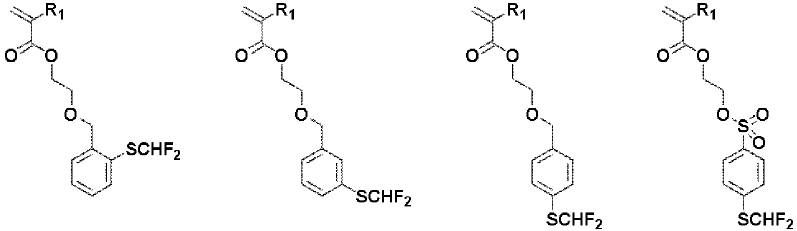

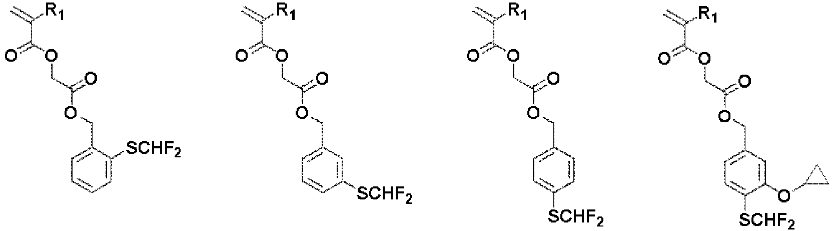

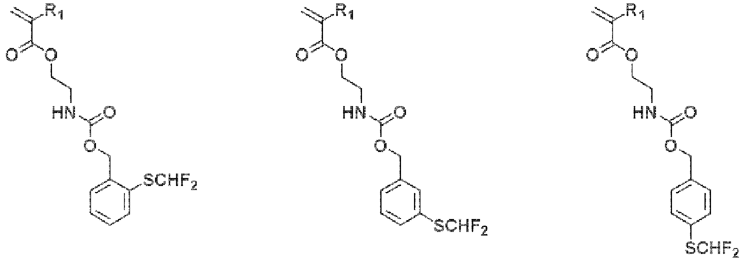

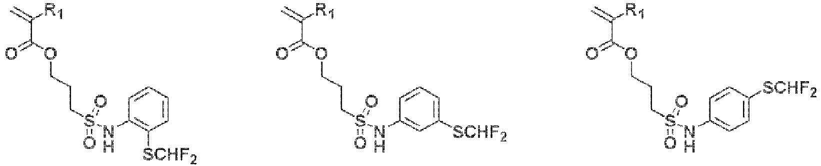

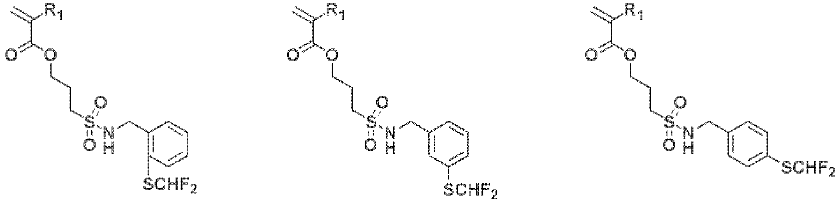

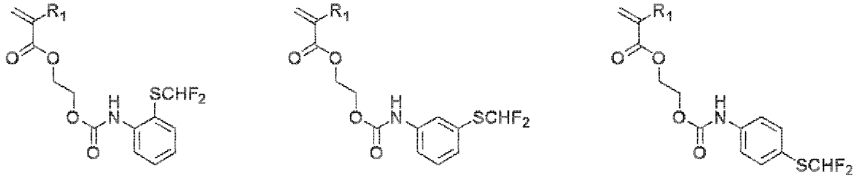

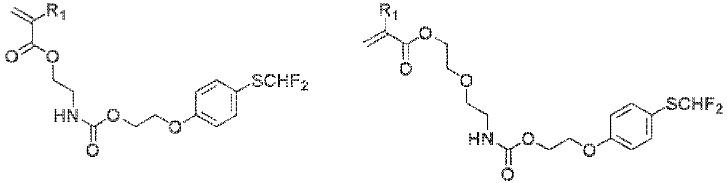

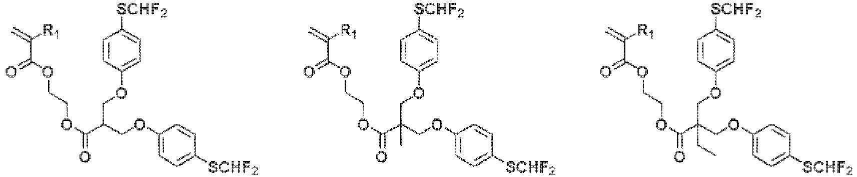

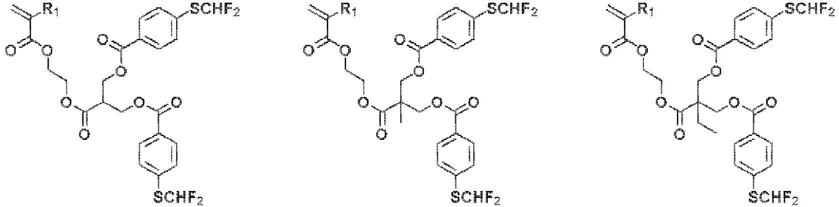

In the formula, R₁ is a hydrogen atom or a methyl group.

The polymer (a1) can also be further copolymerized with repeating units X (repeating units "x1" and "x2") used in polymers for resists shown below. The repeating unit X will be shown in detail in the description concerning resists.

In the polymer (a1), the content ratios of repeating units (a1), (b1), (c1), and (c2) are preferably 0<a1≤1.0, 0≤b1<1.0, 0≤c1<1.0, 0≤c2<1.0, more preferably 0.1≤a1≤0. 9 and 0.1≤b1≤0.9, 0.1≤c1≤0.9, 0.1≤c2≤0.9, and further preferably 0.2≤a1≤0.8, 0.2≤b1≤0.8, 0.2≤c1≤0.8, and 0.2≤c2≤0.8. However, they satisfy a1+b1+c1+c2=1.0.

The polymer (a1) has preferably a weight-average molecular weight (Mw) of 1,000 to 500,000, more preferably 2,000 to 30,000, in terms of polystyrene when determined by gel permeation chromatography (GPC) using THF as an eluent. Furthermore, if the molecular weight distribution (Mw/Mn) of the polymer (a1) is wide, the presence of polymers with low molecular weight or high molecular weight may pose a risk where a foreign matter may be observed on a pattern after exposure to light or where the shape of the pattern may deteriorate. As pattern rules become finer, the influence of Mw and Mw/Mn tends to increase. Therefore, in order to obtain a resist material that can be used suitably in the case of fine pattern dimensions, Mw/Mn of the polymer (a1) should have narrow dispersion preferably in a range of 1.0 to 4.0, particularly preferably 1.0 to 3.0. There is a concern that the surface-active effect may decrease due to low molecular weight components and that the hump characteristics may deteriorate by decrease in solubility due to inclusion of ultra-high molecular weight substances. Accordingly, the dispersity is desired to be narrow in such range that these components are not contained in large amounts.

The polymer (a1) may contain two or more polymers having different composition ratios, Mw, and Mw/Mn. Furthermore, polymers having different repeating units (a1), (b1), (c1), and (c2) may be blended.

The polymer (a1) is produced by polymerizing monomers that provide a repeating unit selected from the group consisting of the repeating units (a1), (b1), (c1), and (c2) by a method, such as radical polymerization, anionic polymerization, or cationic polymerization.

When the polymer (a1) contains two or more types of repeating units, it may be a random copolymer or a block copolymer, but a block copolymer is characterized to be more effective as a surfactant. In particular, it is preferably a block copolymer having repeating units (a1) and (b1), (c1), and (c2). The block copolymer may be a diblock copolymer consisting of two units, a triblock copolymer consisting of three units, or a tetrablock copolymer consisting of four units.

When performing random copolymerization by the radical polymerization, a common method is to mix monomers to be copolymerized and a radical initiator, and then polymerize them by heating. When the first monomer is polymerized in the presence of a radical initiator and the second monomer is added later, one side of the polymer molecule has a polymerized block of the first monomer, and the other side has a block polymerized with the second monomer. However, in this case, repeating units derived from the first monomer and the second monomer are mixed in the intermediate portion of the polymer molecule, and its form is different from that of a block copolymer. To form a block copolymer by the radical polymerization, living radical polymerization is preferably used. In the living radical polymerization method called
"Reversible Addition Fragmentation chain Transfer (RAFT) polymerization", the radical at the polymer end is always living, so that it is possible to form a block copolymer having both the first repeating unit and the second repeating unit by initiating the polymerization with the first monomer and adding the second monomer when the first monomer is consumed. A tri-block copolymer can be formed by: initiating the polymerization with the first monomer; adding the second monomer when the first monomer is consumed; and then adding a third monomer. When the RAFT polymerization is performed, a polymer having narrow dispersion is formed.

Examples of organic solvents used during polymerization include toluene, benzene, tetrahydrofuran (THF), diethyl ether, and dioxane. Examples of the polymerization initiator include 2,2'-azobisisobutyronitrile (AIBN), 2,2'-azobis(2,4-dimethylvaleronitrile), dimethyl 2,2-azobis(2-methylpropionate), benzoyl peroxide, and lauroyl peroxide. The temperature during polymerization is preferably 50 to 80°C. The reaction time is preferably 2 to 100 hours, more preferably 5 to 20 hours.

For carrying out the RAFT polymerization, a chain transfer agent is necessary. Specific examples thereof include 2-cyano-2-propylbenzothioate, 4-cyano-4-phenylcarbonothioylthiopentanoic acid, 2-cyano-2-propyl dodecyl trithiocarbonate, 4-cyano-4-[(dodecylsulfanylthiocarbonyl)sulfanyl]pentanoic acid, 2-(dodecylthiocarbonothioylthio)-2-methylpropanoic acid, cyanomethyl dodecyl thiocarbonate, cyanomethyl N-methyl-N-(phenyl)carbamothioate, bis(thiobenzoyl)disulfide, and bis(dodecylsulfanylthiocarbonyl)disulfide. Among these, 2-cyano-2-propylbenzothioate is most preferable.

When the RAFT polymerization is performed by adding monomers all at once, a polymer with narrow dispersion can be synthesized.

When the polymer has at least one repeating unit selected from the group consisting of repeating units (a2) and (a3), the polymer is a so-called "novolac resin" (hereinafter also referred to as polymer (a2-a3)). The polymer (a2-a3) can be obtained by a polycondensation reaction between a phenol compound and an aldehyde compound. Incidentally, at least one of the phenol compound and the aldehyde compound has a pentafluorosulfanyl group as a substituent on an aromatic ring.

The phenol compound is a compound having a hydroxy group bonded to an aromatic ring. Examples thereof include phenol, cresol, naphthol, dihydroxynaphthalene, 9,9-bis(4-hydroxyphenyl)fluorene, 9,9-bis(6-hydroxynaphthyl)fluorene, 1,1,1-tris(4-hydroxyphenyl) ethane, 1,1-bis(4-hydroxyphenyl)-1-phenylethane, bis(4-hydroxyphenyl)cyclohexane, 2,2-bis(4-hydroxyphenyl)adamantane, 4,4',4"-trihydroxytriphenylmethane, and 9-hydroxy-9-phenylfluorene.

Examples of the aldehyde compound include formaldehyde, trioxane, paraformaldehyde, benzaldehyde, acetaldehyde, propylaldehyde, phenylacetaldehyde, α-phenylpropylaldehyde, β-phenylpropylaldehyde, o-hydroxybenzaldehyde, m-hydroxybenzaldehyde, p-hydroxybenzaldehyde, o-chlorobenzaldehyde, m-chlorobenzaldehyde, p-chlorobenzaldehyde, o-nitrobenzaldehyde, m-nitrobenzaldehyde, p-nitrobenzaldehyde, o-methylbenzaldehyde, m-methylbenzaldehyde, p-methylbenzaldehyde, p-ethylbenzaldehyde, p-n-butylbenzaldehyde, and furfural. Among these, formaldehyde can be particularly preferably used. One kind of these aldehyde compounds may be used or two or more kinds thereof may be used in combination.

At least one of the above phenol compounds and the above aldehyde compounds has a pentafluorosulfanyl group as a substituent on an aromatic compound. Examples of compounds having these groups include the following.

Examples of aldehyde compounds having a pentafluorosulfanyl group include the following.

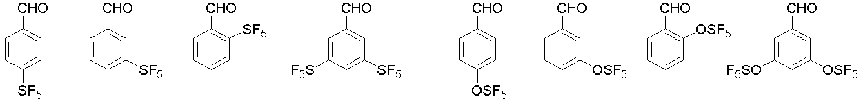

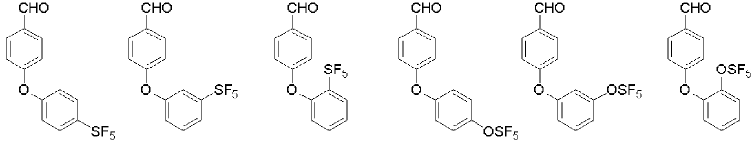

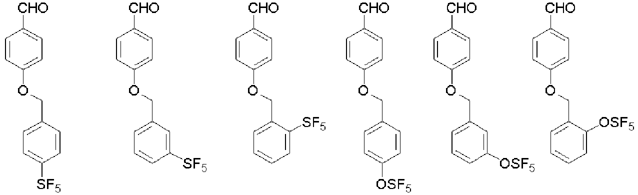

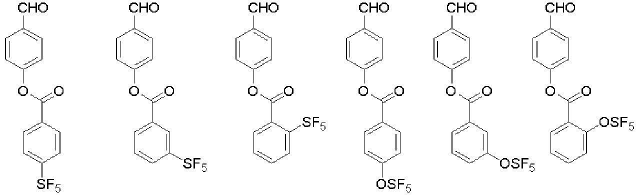

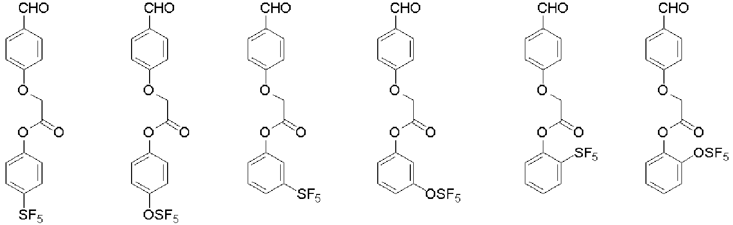

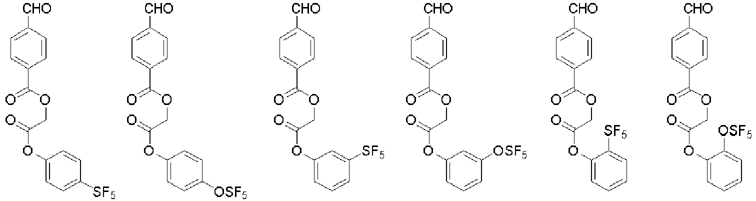

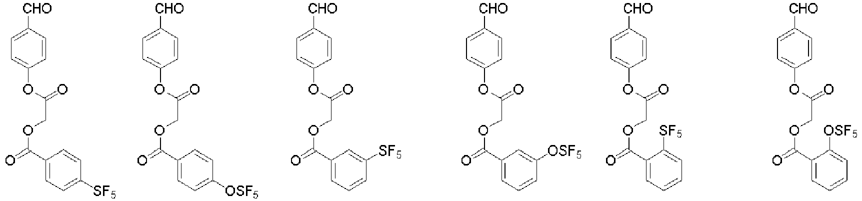

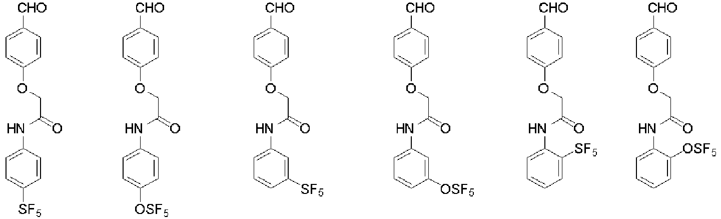

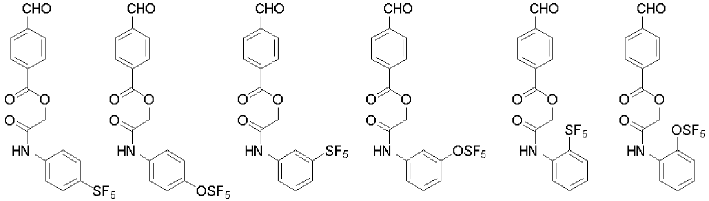

Examples of phenol compounds having a pentafluorosulfanyl group include the following.

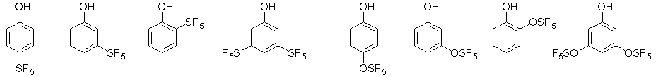

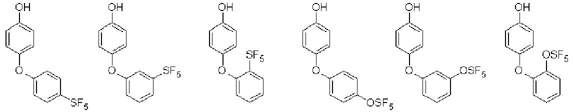

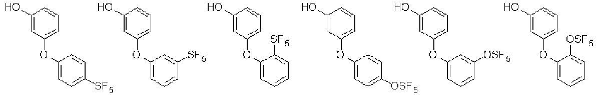

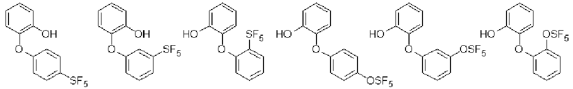

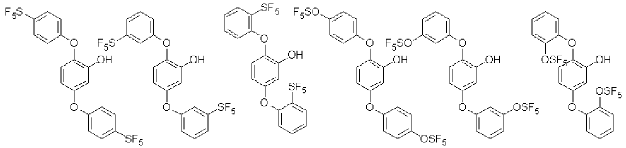

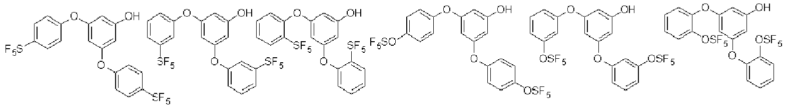

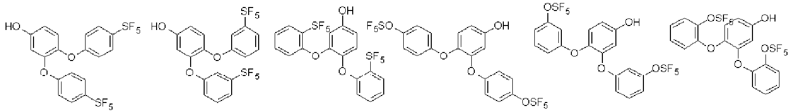

One kind of the phenol compound may be used or two or more kinds thereof may be used in combination. One kind of the aldehyde compound may be used or two or more kinds thereof may be used in combination. The amount of the aldehyde compound used is preferably 0.2 to 5 mol, more preferably 0.5 to 2 mol, relative to 1 mol of the phenol compound.

A catalyst may also be used in the polycondensation reaction between a phenol compound and an aldehyde compound. Examples of the catalyst include acidic catalysts, such as hydrochloric acid, nitric acid, sulfuric acid, formic acid, oxalic acid, acetic acid, methanesulfonic acid, camphorsulfonic acid, tosylic acid, and trifluoromethanesulfonic acid. The amount of these acidic catalysts used is preferably 1×10⁻⁵ to 5×10-¹ relative to 1 mol of the phenol compound.

As a reaction solvent in the polycondensation, water, methanol, ethanol, propanol, butanol, tetrahydrofuran, dioxane, or a mixed solvent thereof can be used. It is preferable to use these solvents in an amount range of 0 to 2000 parts by mass relative to 100 parts by mass of the raw materials. The reaction temperature may be appropriately selected depending on the reactivity of the raw materials, but is usually in a range of 10 to 200°C.

The polycondensation reaction includes a method in which the phenol compound, the aldehyde compound, and the catalyst are charged all at once and a method in which the phenol compound and the aldehyde compound are added dropwise in the presence of the catalyst. After the completion of the polycondensation reaction, in order to remove unreacted materials, catalysts, etc. present in the system, the temperature of the reaction vessel is raised to 130 to 230°C, and volatile components may be removed at about 1 to 50 mmHg.

Further, the substituents represented by R₃ in the polymer include the following.

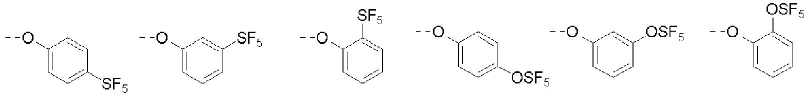

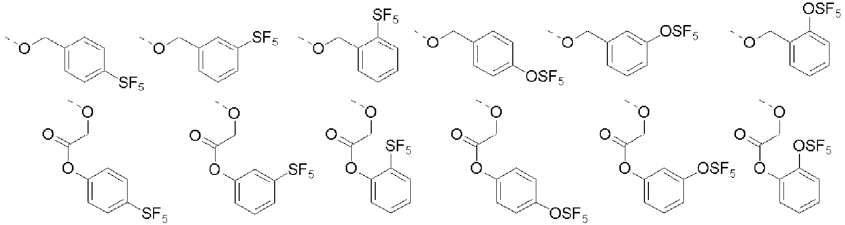

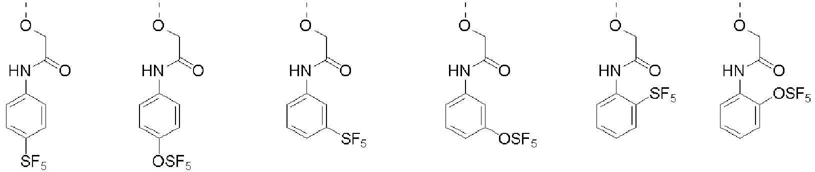

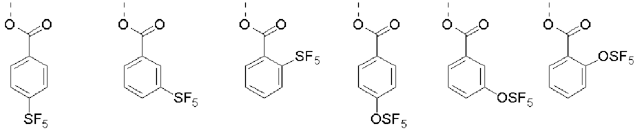

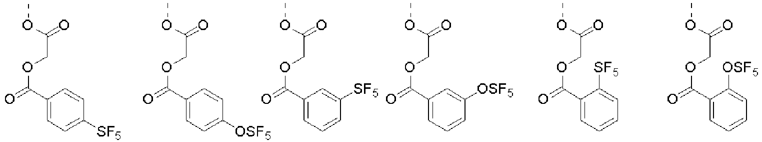

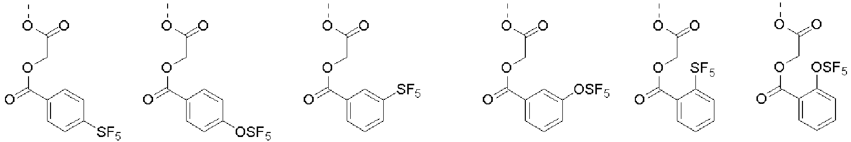

As another method for introducing the substituent R₃, there is a method, as follows, in which R₃ is introduced (modified) after obtaining an intermediate polymer from a phenol compound and an aldehyde compound by the method described above. For example, in the case of the repeating unit (a2), it can be synthesized by condensing a compound (phenol compound) represented by a circled Ar₂ having a phenolic hydroxyl group on an aromatic ring and a compound (aldehyde compound) represented by Ar₃ having a phenolic hydroxyl group on an aromatic ring and then having the phenolic hydroxyl group esterified or etherified. Similarly, in the case of the repeating unit (a3), a polymer (a2-a3) can be obtained by condensing the above phenol compound and an aldehyde compound corresponding to R₄, and then modifying the phenolic hydroxyl group.

### 1. In the case of (a2)

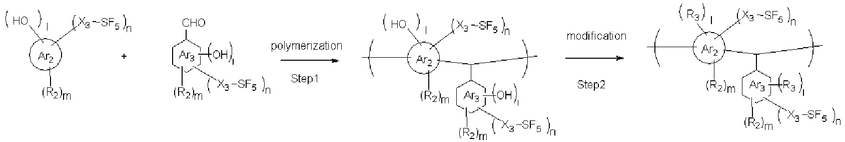

### 2. In the case of (a3)

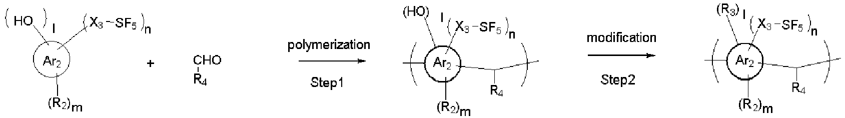

The aldehyde component having a phenolic hydroxyl group to be used in the above reaction can be exemplified as follows. Examples of the aldehyde having a substituent represented by R₄ include those listed as the aldehyde compound used to obtain the polymer (a2-a3).

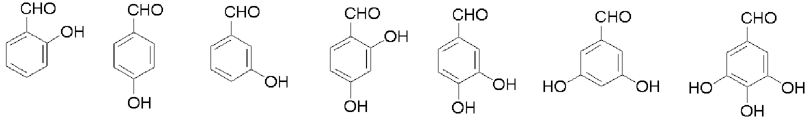

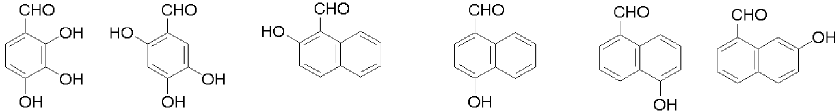

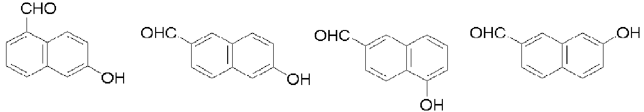

Example of the compound having a phenolic hydroxyl group to be used in the above reaction include the compounds listed as the phenol compound to be used to obtain the polymer (a2-a3). More specifically, the following can be exemplified.

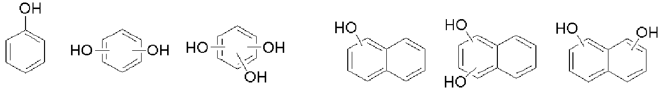

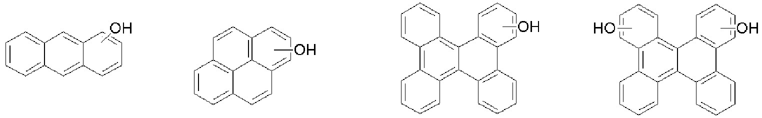

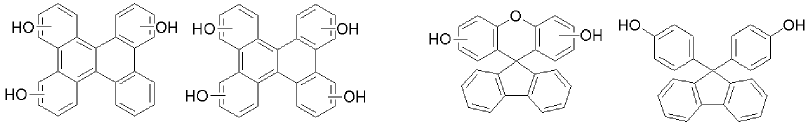

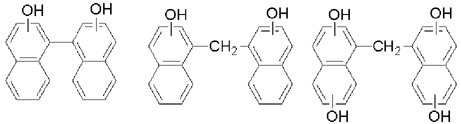

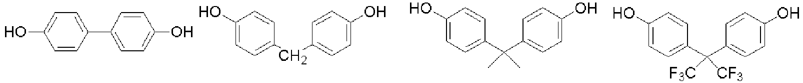

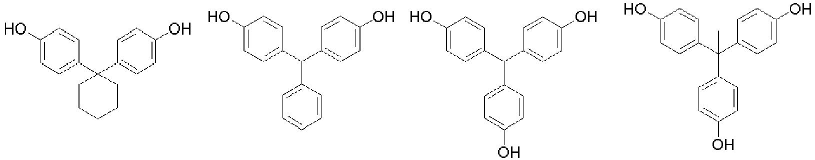

In the case of etherification in the above reaction, it can be synthesized by reaction with a halide, mesylate, or tosylate corresponding to R₃.

Etherification proceeds easily by publically known methods. Specifically, it can be obtained by the reaction with the halide, the mesylate, or the tosylate corresponding to a partial structure of R₃. The esterification is preferably carried out by; using ether solvents, such as diethyl ether, tetrahydrofuran, and dioxane, aromatic solvents, such as benzene, toluene, and xylene, acetonitrile, dimethyl sulfoxide, N,N-dimethylformamide, N-methylpyrrolidone, water, or the like, alone or as a mixed solvent; adding the polymer obtained in STEP 1, and the halide, the mesylate, or the tosylate corresponding to a partial structure of R₃, and basic catalysts, such as sodium hydrogen, sodium carbonate, potassium carbonate, calcium carbonate, cesium carbonate, sodium hydroxide, potassium hydroxide, sodium hydride, potassium phosphate, triethylamine, pyridine, and N-methylmorpholine sequentially or simultaneously; and then optionally cooling down, heating the mixture, or the like if necessary. The resulting reaction product can be purified by washing with water or the like and recovered as a powder or a solution.

When esterification is performed by the above reaction, it is possible to synthesize the compound by reacting with an acid chloride or acid anhydride corresponding to R₃.

The esterification can be easily carried out by a publicly known method, but specifically, it is preferable to use an acid chloride or an acid anhydride corresponding to the partial structure of R₃. When an acid chloride is used, the esterification is preferably carried out, without a solvent or in solvents, such as methylene chloride, acetonitrile, toluene, and hexane, by; adding the polymer obtained in STEP 1, an acid chloride, and a base, such as triethylamine, pyridine, and 4-dimethylaminopyridine sequentially or simultaneously; and then optionally cooling down, heating the mixture, or the like if necessary. Further, when an acid anhydride is used, the esterification is preferably carried out, in a solvent, such as toluene, by; adding the polymer obtained in STEP 1 and a base, such as triethylamine, pyridine, and 4-dimethylaminopyridine sequentially or simultaneously; and then optionally cooling down, heating the mixture, or the like if necessary. The resulting reaction product can be purified by washing with water or the like and recovered as a powder or a solution.

When the above manufacturing method is used, the modification ratio of the substituent R₃ in the repeating unit of the polymer can be controlled to an arbitrary ratio. When the ratio of R₃ having the modified hydroxyl group in the entire repeating unit is defined as c1 and the ratio of unmodified hydroxyl groups is defined as c2, it is preferably 0≤c1≤1.0 and 0≤c2≤1.0, more preferably 0.1≤c1≤0.9 and 0.1≤c2≤0.9, and further preferably 0.2≤c1≤0.8 and 0.2≤c2≤0.8, but they satisfy c1+c2=1.0.

The polymer (a2-a3) has preferably a polystyrene-equivalent weight-average molecular weight (Mw) of 1000 to 500000, more preferably 1500 to 30000, according to gel permeation chromatography (GPC) using THF as a solvent. Furthermore, when the molecular weight distribution (Mw/Mn) of the polymer (a2-a3) is broad, because of the presence of low-molecular-weight or high-molecular-weight polymers, there is a risk to produce a sublimate caused by low-molecular-weight or foreign matter during film formation caused by high-molecular-weight polymer, such as foreign matter on a pattern after exposure, and a risk to worsen a pattern profile. In order to ensure high-precision in-plane uniformity control of the film and film-formability on a substrate with a complex shape, the Mw/Mn of the polymer (a2-a3) has preferably narrow dispersion of 1.0 to 5.0, particularly 1.5 to 3.5.

The polymer (a2-a3) may contain two or more polymers having different composition ratios, Mw, or Mw/Mn.

When the polymer has the repeating unit (a4), the polymer is a ring-opening polymer of an oxetane compound (hereinafter, also referred to as polymer (a4)). The polymer (a4) can be obtained by ring-opening polymerization of the oxetane compound, and the substituent represented by R₃ of the oxetane compound has at least one pentafluorosulfanyl group as a substituent on an aromatic ring.

Examples of the oxetane compound include the following. R₃ is the same defined as above.

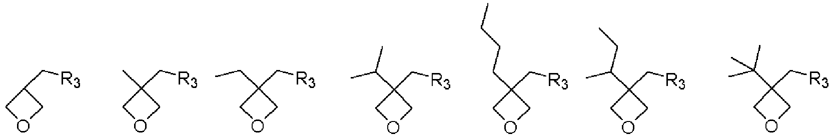

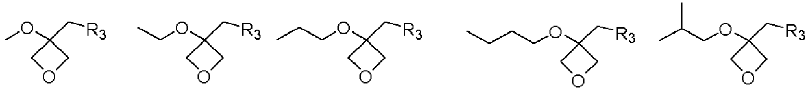

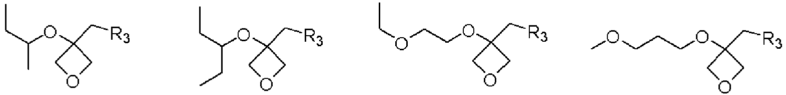

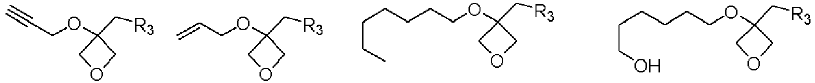

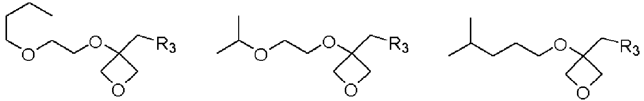

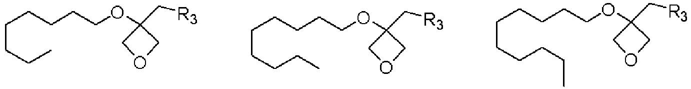

The polymer having the repeating unit (a4) can be synthesized by using an oxetane compound as a monomer and subjecting the oxetane compound to ring-opening polymerization using a hydroxyl group-containing compound as an initiator in the presence or absence of a catalyst and a solvent.

As the initiator, it is possible to use oxetane compounds having an alcohol group, such as 3-ethyl-3-(2-hydroxyethyl)oxymethyloxetane, 3-ethyl-3-(3-hydroxypropyl)oxymethyloxetane, 3-ethyl-3-(4-hydroxybutyl)oxymethyloxetane, 3-ethyl-3-(5-hydroxypentyl)oxymethyloxetane, 3-ethyl-3-(2-hydroxyethyloxyethyl)oxymethyloxetane, 3-ethyl-3-(2,3-dihydroxypropyl)oxymethyloxetane, 3-ethyl-3-hydroxy-poly(ethyleneoxy)methyloxetane, and 3-ethyl-3-hydroxy-poly(propyleneoxy)methyloxetane; alcohol compounds, such as methanol, ethanol, propanol and butanol; diol compounds, such as ethylene glycol, 1,4-butanediol, 1,6-hexanediol, 2,2-dimethyl-1,3-propanediol, diethylene glycol, triethylene glycol, and tetraethylene glycol; etc. The diol compounds are preferable from the viewpoint of molecular weight control. The amount of these initiator to be used is, for example, 0.1 to 100 mol, preferably 0.5 to 100 mol, and particularly preferably 1 to 100 mol, relative to 100 mol of the oxetane compound. By controlling the mole ratio of the oxetane compound to the initiator, the molecular weight of the obtained polymer can be controlled.

Examples of the catalyst include Lewis acid catalysts, such as boron trifluoride, phosphorus pentafluoride, antimony pentafluoride, zinc chloride, aluminum bromide, and other complexes. Among these, a preferable catalyst is BF₃·THF complex from the viewpoint of handling a material. The catalyst and initiator are preferably mixed in a solvent for 5 to 10 minutes before adding of the monomer, and the catalyst/initiator ratio is in the range of 1:1 to 1:5 mol/mol, and preferably in the range of 1:1 to 1:2 mol/mol.

The ring-opening reaction can be carried out without a solvent, but when a solvent is used, there is no particular limitation as long as the solvent is inert to the catalyst that is used for the reaction and the oxetane compound that is a monomer used in the reaction. The examples of the solvent include methylene chloride, carbon tetrachloride, chloroform, trichloroethane, chlorobenzene, ethyl bromide, dichloroethane, sulfur dioxide, hexane, petroleum ether, toluene, dioxane, and xylene. One kind of these solvents may be used or two or more kinds thereof may be used in combination. It is preferable to use the solvent(s) in a range of 0 to 2000 parts by mass relative to 100 parts by mass of the monomer.

The ring-opening polymerization can be easily carried out by a publicly known method, but it is preferable to mix a catalyst and an initiator in a solvent before adding oxetane compound(s) being one or more kind of monomers, and then slowly add the monomer over time while controlling heat generation to carry out the reaction. After the reaction is completed, the reaction is quenched using water or the like, and the catalyst is removed by liquid-liquid separation and washing or the like, and the polymer can be recovered by being crystallizing in a poor solvent, or by distilling unreacted monomers under reduced pressure. The reaction temperature at this time is preferably -20°C to 60°C, more preferably 0°C to 40°C. The charging ratio of the monomer to the catalyst (monomer/catalyst) is preferably within a range of 10:1 to 300:1, more preferably 50:1 to 100:1.

The polymer (a4) preferably has a polystyrene-equivalent weight-average molecular weight (Mw) of 1000 to 500000, more preferably 1500 to 30000, according to gel permeation chromatography (GPC) using THF as an eluent. Furthermore, when the molecular weight distribution (Mw/Mn) of the polymer (a4) is broad, there is a risk of deteriorated coating performance due to a sublimate caused by low-molecular-weight substance, foreign matter during film formation caused by high-molecular-weight substance, or the like. Because it makes influence of the Mw and Mw/Mn easy to become larger, the Mw/Mn of the polymer (a4) has preferably narrow dispersion of 1.0 to 5.0, particularly 1.5 to 3.5, when used as a high-precision film-forming material.

The polymer (a4) may contain two or more polymers having different composition ratios, Mw, or Mw/Mn.

A material used in semiconductor photolithography, including the polymers (a1), (a2-a3), and (a4), must have reduced metal impurities. It is because the presence of metal impurities in a material adversely affects the operation of devices manufactured using the material. To reduce metal impurities, washing with pure water or ion exchange resins is employed. In the case of a resist material, the amount of metal impurities is preferably 100 ppm or less, more preferably 100 ppb or less.

The inventive composition for forming a film preferably contains 0.0001 to 3 mass% of the polymer (a). The content of the polymer is preferably 0.0001 to 2 mass%, more preferably 0.0002 to 1 mass%. In this event, the polymer (a) serves as a surfactant for imparting film-formability. By using the polymer (a) as a surfactant in combination with an organic compound or the like, it is possible to impart higher film-formability thanks to the interactive effect between fluorine atoms resulting from SF₅ introduced into the repeating unit and the interactive effect between aromatic rings.

The composition for forming a film can be used as a component of a photosensitive or non-photosensitive resist material, a material for forming a topcoat formed on a resist film, a material for forming a resist underlayer film, etc. As the underlayer film material, it is possible to apply to not only an organic underlayer film using an organic compound or resin but also a material for forming a film having a metal element, such as silicon, titanium, zirconium, tin, and hafnium. For example, the resist material may be either a chemically-amplified resist or a non-chemically-amplified resist, and may be a hydrocarbon-based resist, or may be a metal-based resist having silicon, titanium, zirconium, hafnium, selenium, germanium, zinc, iron, cobalt, nickel, copper, tin, antimony, molybdenum, tungsten, indium, etc. Examples other than organic compounds or resins include silicon-containing resist underlayer film materials, such as those described in JP2007-302873A and JP2008-19423A, compositions for forming a coating-type BPSG film, such as those described in JP2016-074774A, titanium-containing resist underlayer film materials, such as those described in JP2014-199429A and JP2014-178602A, and materials for forming a metal oxide film, such as those described in JP2014-134581A. In addition to these, it is expected to be applied to various materials as a surfactant in forming a film.

### [Monomer]

Further, the present invention provides a monomer represented by the following general formula (a5).

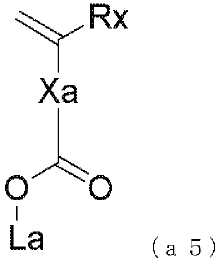

In the formula, Rₓ represents a hydrogen atom or a methyl group; Xₐ represents a single bond, a phenylene group, a naphthylene group, or a linking group having 1 to 12 carbon atoms and having an ester bond, an ether bond, or a lactone ring, and may have an oxygen atom, a nitrogen atom, a sulfur atom, and/or a halogen atom; and La represents an acid-labile group and has an aromatic group having at least one pentafluorosulfanyl group as a substituent.

The inventive monomer is preferably represented by the following general formula (a6).

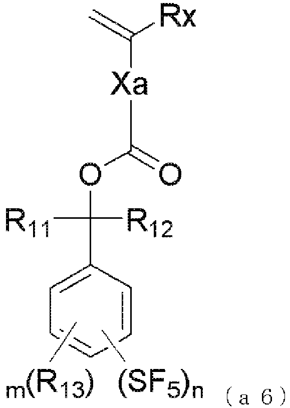

In the formula, Xₐ and Rₓ represent the same defined above; R₁₁ and R₁₂ each independently represent a linear, branched, or cyclic alkyl group having 1 to 12 carbon atoms, a linear, branched, or cyclic alkenyl group having 2 to 12 carbon atoms, a linear, branched, or cyclic alkynyl group having 2 to 12 carbon atoms, or an aryl group having 5 to 10 carbon atoms, each of which may have an oxygen atom and/or a sulfur atom, and R₁₁ and R₁₂ may bond to each other to form a ring; R₁₃ each independently represents a hydrogen atom, a halogen atom, an alkyl group having 1 to 4 carbon atoms, or an alkoxy group; "m" represents an integer of 0 to 4; and "n" represents an integer of 1 to 3.

Specific example of the monomer represented by the general formula (a6) include the following.

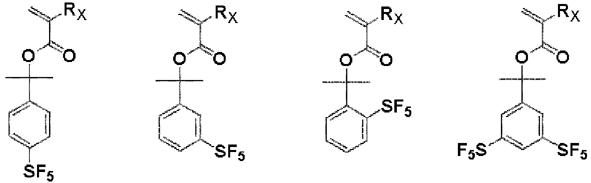

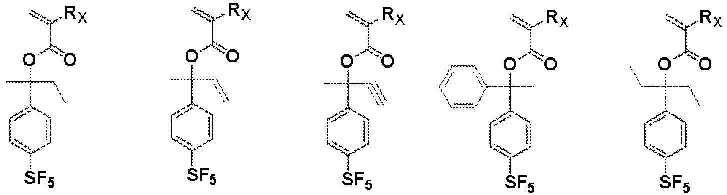

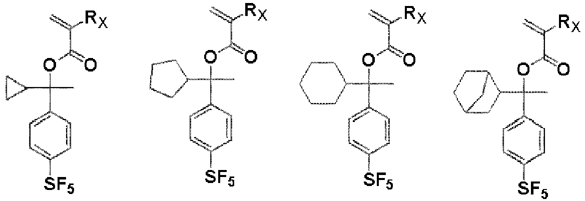

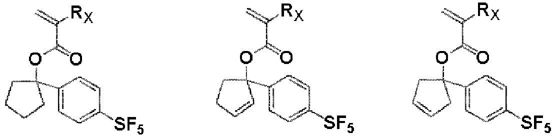

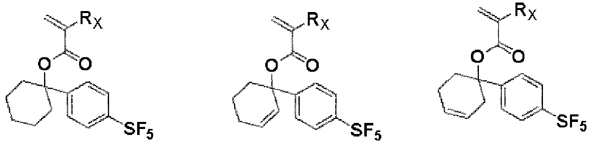

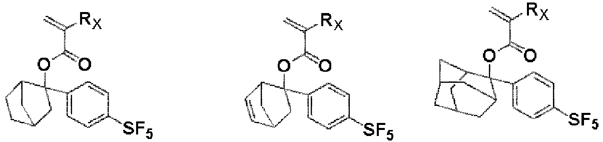

The above monomers can be expected to be used in various industries as materials for next-generation environment-conscious materials that comply with PFAS regulations.

### [Polymer]

Further, the present invention also provides a polymer having a repeating unit represented by the following general formula (a7) and having a weight-average molecular weight in a range of 100 to 500,000.

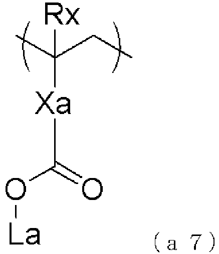

In the formula, Rₓ represents a hydrogen atom or a methyl group; Xₐ represents a single bond, a phenylene group, a naphthylene group, or a linking group having 1 to 12 carbon atoms and having an ester bond, an ether bond, or a lactone ring, and may have an oxygen atom, a nitrogen atom, a sulfur atom, and/or a halogen atom; La represents an acid-labile group and has an aromatic group having at least one pentafluorosulfanyl group as a substituent.

It is preferable that the inventive polymer has a repeating unit represented by the following general formula (a8) and has a weight-average molecular weight in a range of 100 to 500,000.

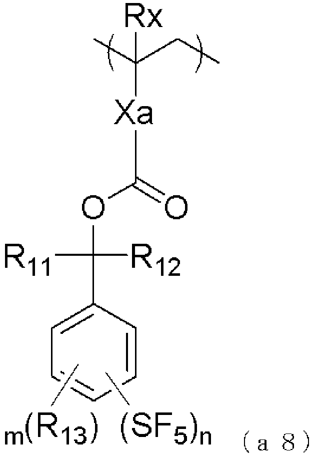

In the formula, Xₐ and Rₓ represent the same defined above; R₁₁ and R₁₂ each independently represent a linear, branched, or cyclic alkyl group having 1 to 12 carbon atoms, a linear, branched, or cyclic alkenyl group having 2 to 12 carbon atoms, a linear, branched, or cyclic alkynyl group having 2 to 12 carbon atoms, or an aryl group having 5 to 10 carbon atoms, each of which may have an oxygen atom and/or a sulfur atom, and R₁₁ and R₁₂ may bond to each other to form a ring; R₁₃ each independently represents a hydrogen atom, a halogen atom, an alkyl group having 1 to 4 carbon atoms, or an alkoxy group; "m" represents an integer of 0 to 4; and "n" represents an integer of 1 to 3.

The above polymers are promising candidates as polymers that do not fall under the PFAS regulations, and can replace conventional polymers having a fluorine substituent introduced.

### [Composition for Forming Organic Film]

The composition for forming an organic film includes a component (A) resin or compound for forming an organic film, a component (B) polymer (a), and a component (C) solvent.

In addition, as for the component (B) polymer (a), the component (A) resin or compound for forming an organic film, and the component (C) solvent in the inventive composition for forming an organic film, one kind thereof may be used or two or more kinds thereof may be used in combination. The polymer (a) in the present invention functions as a surfactant that provides excellent film-formability and high leveling performance. Its applications are not limited to organic underlayer films, but can be expandable to general coating materials for photolithography. Examples thereof include a photosensitive resist material and a material for forming a top coat to be formed on a resist film. Furthermore, it is applicable not only to material for forming an organic film but also to silicon-containing resist underlayer film, making it possible to use as a suitable surfactant to exhibit a highly versatile film-formability that can be applied to various materials for forming a film.

In addition, when using the polymer (a) as a surfactant in the present invention, one kind of them can be used alone or two or more kinds thereof can be used in combination. The amount of these polymers (a) to be added is such that the content of the polymer (a) **is** 0. 01 to 5 parts by mass when the component (A) resin or compound for forming an organic film is 100 parts by mass.

### [(A) Resin or Compound for Forming Organic Film (Organic Underlayer Film)]

The component (A) resin or compound for forming an organic film used in the inventive composition for forming an organic film is not particularly limited as long as the resin or compound has satisfactory film-formability in spin-coating and satisfactory curability. However, when used as an organic underlayer film material, a resin or compound having an aromatic skeleton is more preferable from the viewpoint of etching resistance, optical properties, heat resistance, etc.

Examples of the aromatic skeleton include benzene, naphthalene, anthracene, pyrene, indene, fluorene, furan, pyrrole, thiophene, phosphole, pyrazole, oxazole, isoxazole, thiazole, pyridine, pyrazine, pyrimidine, pyridazine, triazine, and carbazole. Among these, benzene, naphthalene, fluorene, and carbazole are particularly preferred.

The component (A) resin or compound for forming an organic film used in the present invention includes a resin having the following structure described in JP2012-001687A and JP2012-077295A.

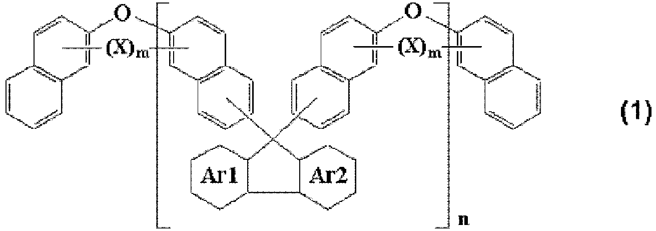

In the formula (1), the ring structures Ar1 and Ar2 represent a benzene ring or a naphthalene ring; X represents a single bond or an alkylene group having 1 to 20 carbon atoms; "m" represents 0 or 1; and "n" represents any natural number such that the molecular weight is 100,000 or less. Incidentally, the symbols in the formula are applied only in this formula.

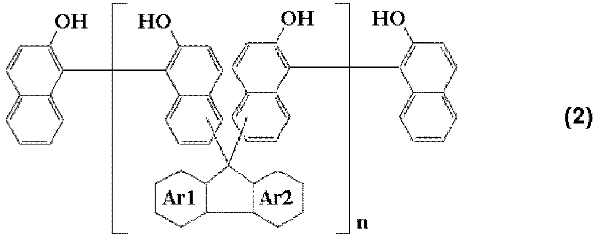

In the formula (2), the ring structures Ar1 and Ar2 represent a benzene ring or a naphthalene ring; and "n" represents any natural number such that a polystyrene-equivalent weight-average molecular weight is 100,000 or less according to gel permeation chromatography (GPC). Incidentally, the symbols in the formula are applied only in this formula.

The component (A) resin or compound for forming an organic film used in the present invention include further resins having the following structures described in JP2004-264710A, JP2005-043471A, JP2005-250434A, JP 2007-293294A, and JP2008-065303A.

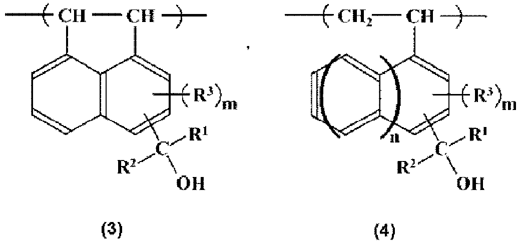

In the formulae (3) and (4), R¹ and R² represent a hydrogen atom, an alkyl group having 1 to 3 carbon atoms, or an aryl group, R³ represents an alkyl group having 1 to 3 carbon atoms, a vinyl group, an allyl group, or an optionally substituted aryl group; "n" represents 0 or 1; and "m" represents 0, 1, or 2. Incidentally, the symbols in the formulae are applied only in these formulae.

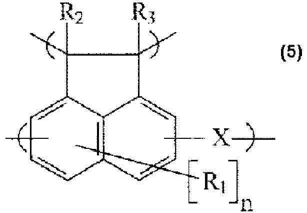

In the formula (5), R₁ represents a monovalent atom or group other than a hydrogen atom; "n" represents an integer of 0 to 4, and a plurality of R₁s may be the same or different from each other when "n" represents an integer of 2 to 4; R₂ and R₃ independently represent a monovalent atom or group; and X represents a divalent group. Incidentally, the symbols in the formula are applied only in this formula.

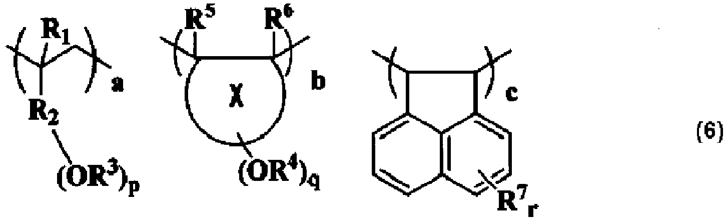

In the formula (6), R₁ represents a hydrogen atom or a methyl group; R₂ represents a single bond, a linear, branched, or cyclic alkylene group having 1 to 20 carbon atoms, or an arylene group having 6 to 10 carbon atoms, and may have one of ether, ester, lactone, and amide; R₃ and R₄ are each a hydrogen atom or a glycidyl group; X represents a polymer of a hydrocarbon having an indene skeleton, cycloolefin having 3 to 10 carbon atoms, or a maleimide, and may contain any one of an ether, an ester, lactone, and a carboxylic acid anhydride; R⁵ and R⁶ are each any one of a hydrogen atom, a fluorine atom, a methyl group, and a trifluoromethyl group; R⁷ is any one of a hydrogen atom, a linear, branched, or cyclic alkyl group having 1 to 6 carbon atoms, a hydroxy group, and an alkoxycarbonyl group; "p" and "q" are each integers of 1 to 4; "r" is an integer of 0 to 4; and "a", "b", and "c" each represent a number in a range satisfying 0.5≤a+b+c≤1, 0≤a≤0.8, 0≤b≤0.8, 0.1≤a+b≤0.8, and 0.1≤c≤0.8. Incidentally, the symbols in the formulae are applied only in theses formulae.

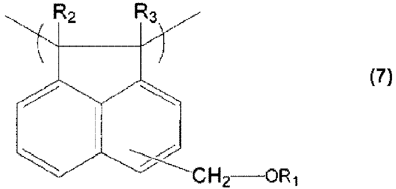

In the formula (7), R₁ represents a hydrogen atom or a monovalent organic group; and R₂ and R₃ each independently represent a monovalent atom or a monovalent organic group. Incidentally, the symbols in the formula are applied only in this formula.

Specific examples of the component (A) resin or compound for forming an organic film used in the present invention include resins having the following structures described in JP2004-205685A, JP2007-171895A, and JP2009-014816A.

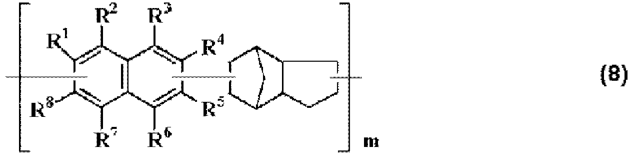

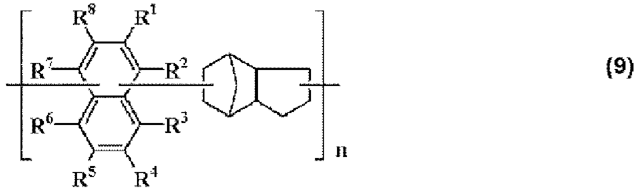

In the formulae (8) and (9), R¹ to R⁸ each independently represent a hydrogen atom, a hydroxyl group, a substitutable alkyl group having 1 to 6 carbon atoms, a substitutable alkoxy group having 1 to 6 carbon atoms, a substitutable alkoxycarboxyl group having 2 to 6 carbon atoms, a substitutable aryl group having 6 to 10 carbon atoms, a hydroxyalkyl group having 1 to 6 carbon atoms, an isocyanate group, or a glycidyl group; and "m" and "n" are positive integers. Incidentally, the symbols in the formulae are applied only in these formulae.

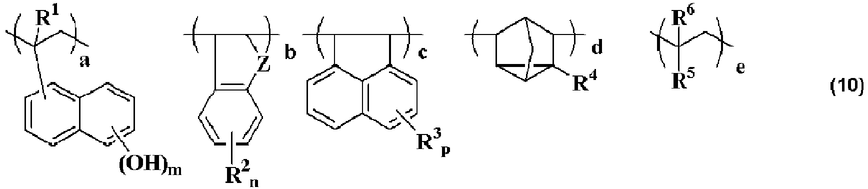

In the formula (10), R¹ and R⁶ represent a hydrogen atom or a methyl group; R², R³, and R⁴ represent a hydrogen atom, an alkyl group having 1 to 4 carbon atoms, an alkoxy group, a hydroxy group, an acetoxy group, an alkoxycarbonyl group, or an aryl group having 6 to 10 carbon atoms; R⁵ represents a condensed polycyclic hydrocarbon group having 13 to 30 carbon atoms, -O-R⁷, -C(=O)-O-R⁷, -O-C(=O)-R⁷, or -C(=O)-NR⁸-R⁷; "m" is an integer of 1 or 2; "n" is an integer of 0 to 4; "p" is an integer of 0 to 6; R⁷ is an organic group having 7 to 30 carbon atoms; R⁸ is a hydrogen atom or a hydrocarbon group having 1 to 6 carbon atoms; Z is any one of a methylene group, -O-, -S-, and -NH-; and "a", "b", "c", "d", and "e" represent numbers satisfying 0<a<1.0, 0≤b≤0.8, 0≤c≤0.8, 0≤d≤0.8, 0≤e≤0.8, and 0<b+c+d+e<1.0. Incidentally, the symbols in the formulae are applied only in these formulae.

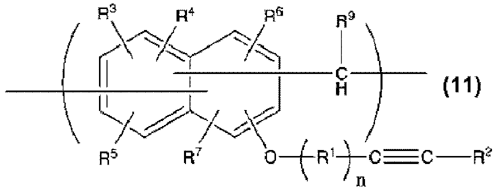

In the formula (11), **"n"** represents 0 or **1;** R¹ represents a methylene group that may be substituted, an alkylene group that may be substituted and has 2 to 20 carbon atoms, or an arylene group that may be substituted and has 6 to 20 carbon atoms; R² represents a hydrogen atom, an alkyl group that may be substituted and has 1 to 20 carbon atoms, or an aryl group that may be substituted and has 6 to 20 carbon atoms. R³ to R⁷ represent a hydroxyl group, an alkyl group that may be substituted and has 1 to 6 carbon atoms, an alkoxy group that may be substituted and has 1 to 6 carbon atoms, an alkoxycarbonyl group that may be substituted and has 2 to 10 carbon atoms, an aryl group that may be substituted and has 6 to 14 carbon atoms, or a glycidyl ether group that may be substituted and has 2 to 6 carbon atoms. R⁹ represents a hydrogen atom, a linear, branched or cyclic alkyl group having 1 to 10 carbon atoms, a linear, branched or cyclic alkyl ether group having 1 to 10 carbon atoms, or an aryl group having 6 to 10 carbon atoms. Incidentally, the symbols in the formula are applied only in this formula.

Examples of those represented by the formula (11) include the following resins.

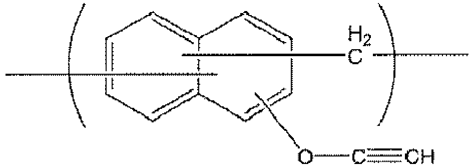

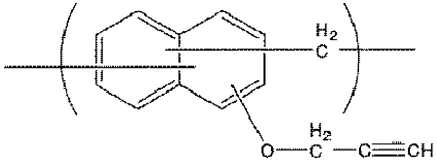

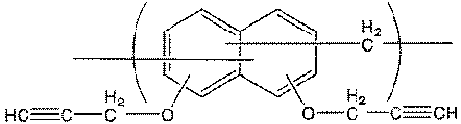

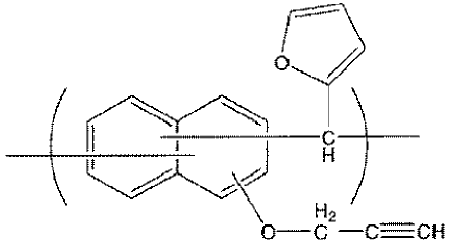

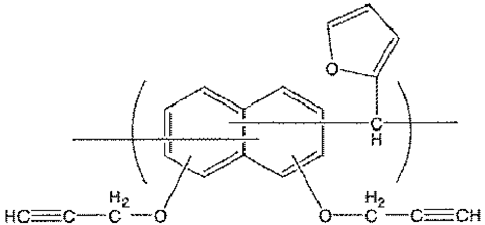

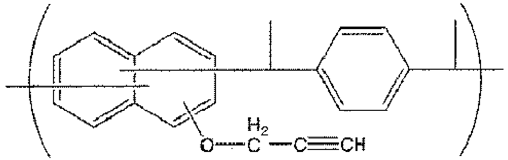

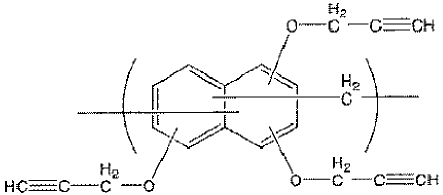

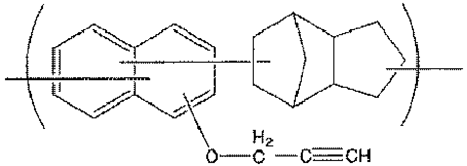

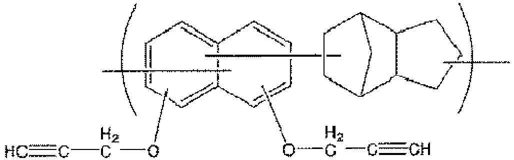

The component (A) resin or compound for forming an organic film used in the present invention include resins having the following structure described in JP2007-199653A, JP2008-274250A, and JP2010-122656A.

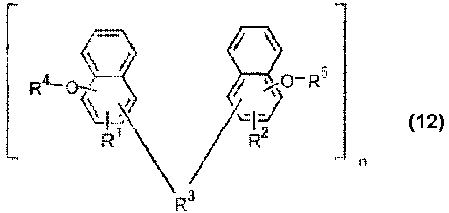

In the formula (12), R¹ and R² may be the same or different from each other and represent independently a hydrogen atom, a linear, branched, or cyclic alkyl group having 1 to 10 carbon atoms, an aryl group having 6 to 10 carbon atoms, or an alkenyl group having 2 to 10 carbon atoms; R³ represents a single bond or an alkylene group having a linear, branched or cyclic structure and having 1 to 30 carbon atoms, and may have a bridged cyclic hydrocarbon group, a double bond, a hetero atom or an aromatic groups having 6 to 30 carbon atoms; R⁴ and R⁵ each independently represent a hydrogen atom or a glycidyl group; and "n" is an integer of 1 to 4. Incidentally, the symbols in the formula are applied only in this formula.

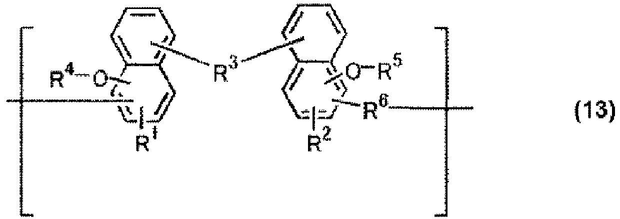

In the formula (13), R¹ and R² may be the same or different from each other and represent independently a hydrogen atom, a linear, branched, or cyclic alkyl group having 1 to 10 carbon atoms, an aryl group having 6 to 10 carbon atoms, or an alkenyl group having 2 to 10 carbon atoms; R³ represents a single bond or an alkylene group having a linear, branched or cyclic structure and having 1 to 30 carbon atoms, and may have a bridged cyclic hydrocarbon group, a double bond, a hetero atom or an aromatic groups having 6 to 30 carbon atoms; R⁴ and R⁵ each independently represent a hydrogen atom or a glycidyl group; and R⁶ is a single bond or a linear or branched alkylene group having 1 to 10 carbon atoms. Incidentally, the symbols in the formula are applied only in this formula.

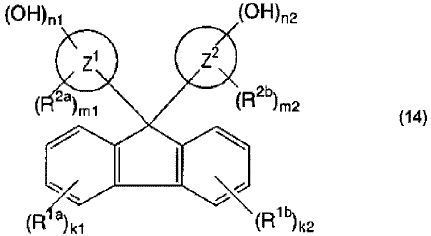

In the formula (14), ring Z¹ and ring Z² are a condensed polycyclic aromatic hydrocarbon ring; R^{1a}, R^{1b}, R^{2a}, and R^{2b} represent the same or different substituents; k1 and k2 are the same or different and represent 0 or an integer of 1 to 4; m1 and m2 each represent 0 or an integer of 1 or more; and n1 and n2 each represent 0 or an integer of 1 or more, and satisfy n1+n2≥1. Incidentally, the symbols in the formula are applied only in this formula.

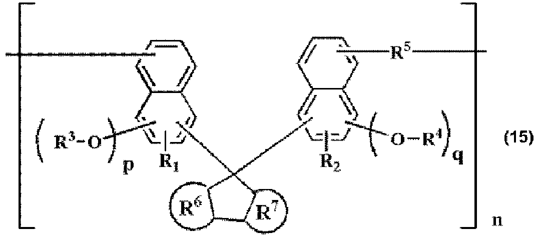

In the formula (15), R¹ and R² may be the same or different from each other and represent a hydrogen atom, a linear, branched, or cyclic alkyl group having 1 to 10 carbon atoms, an aryl group having 6 to 10 carbon atoms, or an alkenyl group having 2 to 10 carbon atoms; R³ and R⁴ represent a hydrogen atom or a glycidyl group; R⁵ is a single bond or a linear or branched alkylene group having 1 to 10 carbon atoms; R⁶ and R⁷ each represent a benzene ring or a naphthalene ring; "p" and "q" each represent 1 or 2; and "n" satisfies 0<n≤1. Incidentally, the symbols in the formula are applied only in this formula.

Examples of those represented by the formula (15) include the following resins.

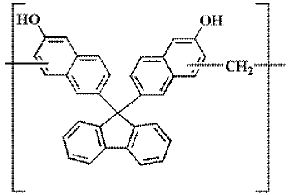

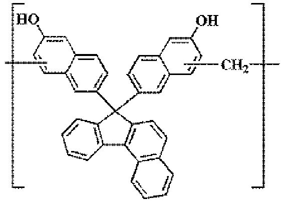

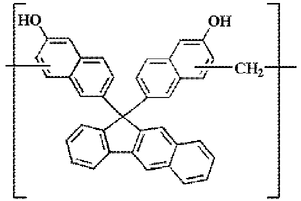

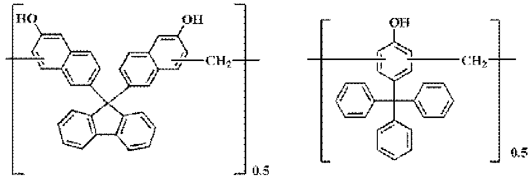

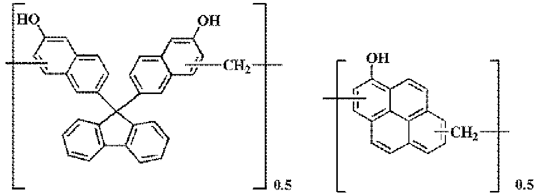

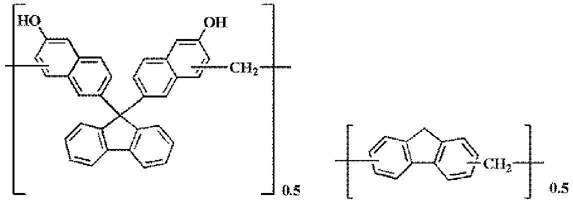

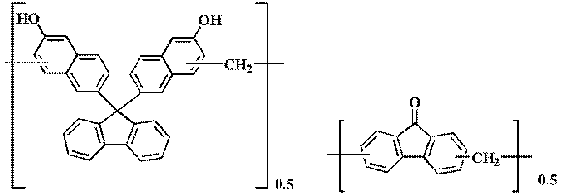

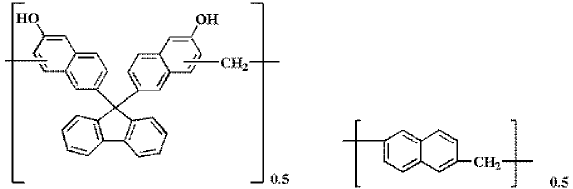

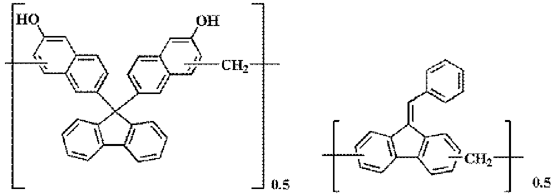

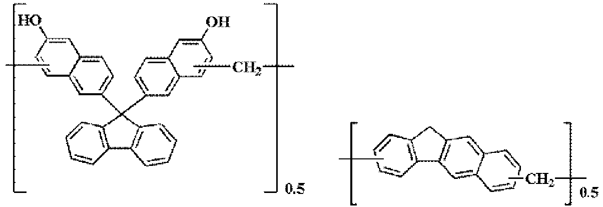

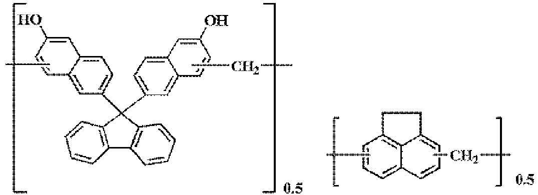

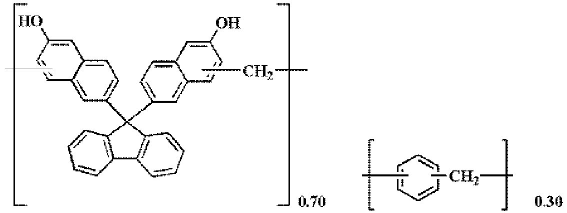

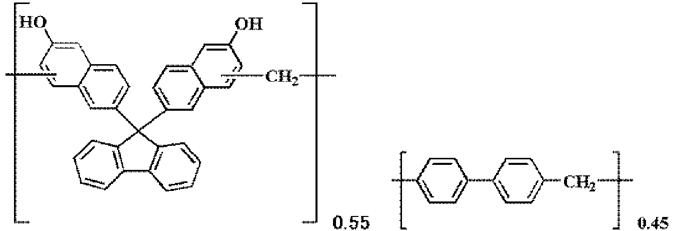

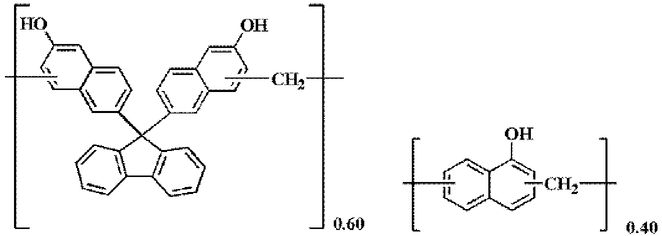

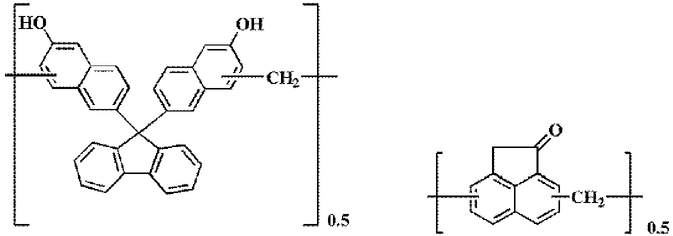

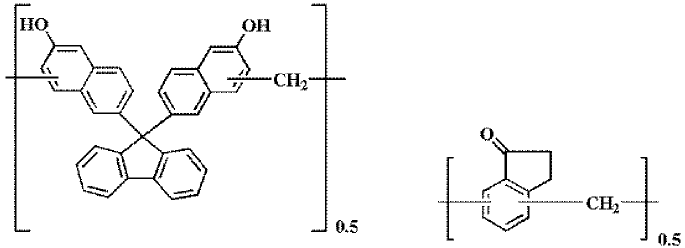

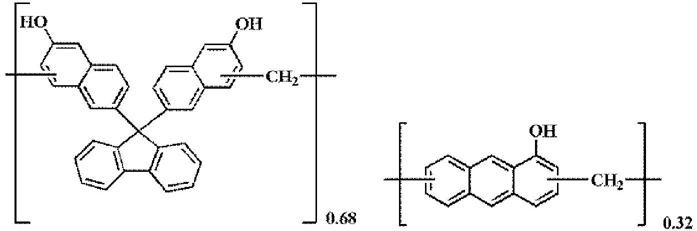

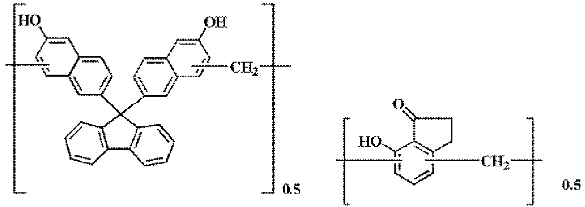

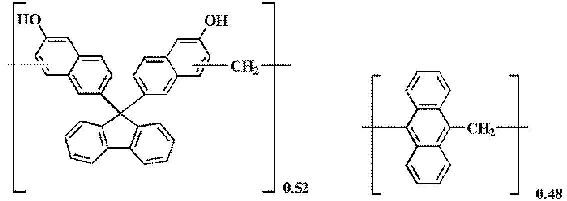

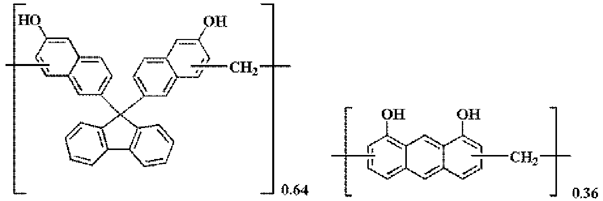

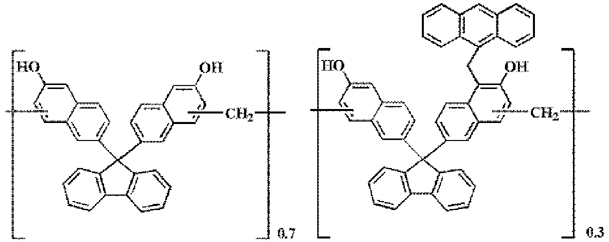

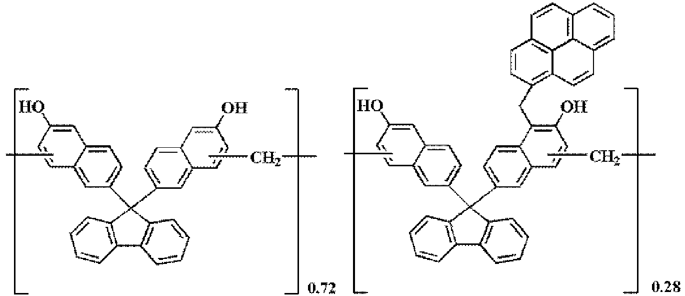

Examples of the component (A) resin or compound for forming an organic film used in the present invention include resins having the following structures described in JP2012-214720A.

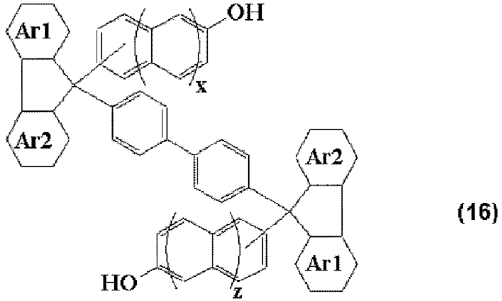

In the formula (16), the ring structures Ar1 and Ar2 represent a benzene ring or a naphthalene ring; and "x" and "z" each independently represent 0 or 1. Incidentally, the symbols in the formula are applied only in this formula.

Examples of the component (A) resin or compound for forming an organic film used in the present invention include resins described in JP2014-29435A.

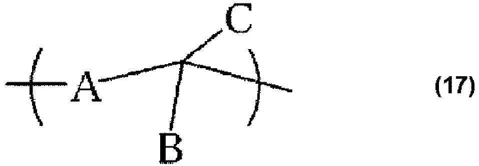

In the formula (17), A represents a structure having a carbazole; B represents a structure having an aromatic ring; C represents a structure having a hydrogen atom, an alkyl group, or an aromatic ring; B and C may mutually form a ring; A structure that combines the structures A, B, and C has 1 to 4 carboxyl groups, a salt thereof, or a carboxylic acid ester group. Incidentally, the symbols in the formula are applied only in this formula.

In addition, examples of the component (A) resin or compound for forming an organic film used in the present invention include the polymer that is described in WO2012/077640A1 and has a unit structure represented by the following formula (18) and a unit structure represented by the following formula (19), and has "3 to 97:97 to 3" as a mole ratio of the unit structure represented by the formula (18) and the unit structure represented by formula (19).

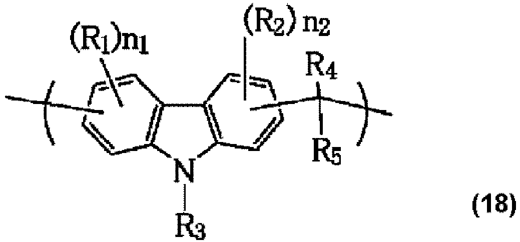

In the formula (18), R₁ and R₂ each independently represent a hydrogen atom, a halogen atom, a nitro group, an amino group, a hydroxy group, an alkyl group having 1 to 10 carbon atoms, an alkenyl group having 2 to 10 carbon atoms, an aryl group having 6 to 40 carbon atoms, or a combination of these groups which may contain an ether bond, a ketone bond, or an ester bond; R₃ represents a hydrogen atom, an alkyl group having 1 to 10 carbon atoms, an alkenyl group having 2 to 10 carbon atoms, an aryl group having 6 to 40 carbon atoms, or a combination of these groups that may contain an ether bond, a ketone bond, or an ester bond; R₄ represents a hydrogen atom, an aryl group that has 6 to 40 carbon atoms and may be substituted with a halogen atom, a nitro group, an amino group, or a hydroxy group, or a heterocyclic group; R₅ represents a hydrogen atom, an alkyl group that has 1 to 10 carbon atoms and may be substituted with a halogen atom, a nitro group, an amino group, or a hydroxy group, an aryl group having 6 to 40 carbon atoms, or a heterocyclic group; R₄ and R₅ may form a ring with each other; and n1 and n2 each represent an integer of 1 to 3. Incidentally, the symbols in the formula are applied only in this formula.

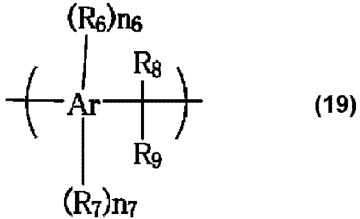

In the formula (19), Ar represents an aromatic ring group having 6 to 20 carbon atoms; R₆ represents a hydroxy group; R₇ represents a hydrogen atom, a halogen atom, a nitro group, an amino group, an alkyl group having 1 to 10 carbon atoms, an alkenyl group having 2 to 10 carbon atoms, an aryl group having 6 to 40 carbon atoms, or a combination of these groups that may have an ether bond, a ketone bond, or an ester bond. R₈ represents a hydrogen atom, an aryl group that has 6 to 40 carbon atoms and may be substituted with a halogen atom, a nitro group, an amino group, or a hydroxy group, or a heterocyclic group; R₉ represents a hydrogen atom, an alkyl group that has 1 to 10 carbon atoms and may be substituted with a halogen atom, a nitro group, an amino group, or a hydroxy group, an aryl group having 6 to 40 carbon atoms, or a heterocyclic group; R₈ and R₉ may form a ring with each other; n6 represents an integer of 1 to "p"; and n7 represents an integer of p-n6. Here, "p" represents the maximum number of substituents with which the aromatic ring group Ar can be substituted. Incidentally, the symbols in the formula are applied only in this formula.

Examples of the component (A) resin or compound for forming an organic film used in the present invention include polymers that have a unit structure represented by the following formula (20) described in WO2010/147155A1.

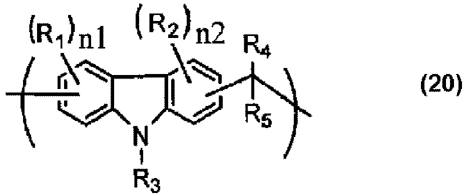

In the formula (20), R₁ and R₂ each represent a group selected from the group consist of a hydrogen atom, a halogen group, a nitro group, an amino group, a hydroxy group, an alkyl group having 1 to 10 carbon atoms, an alkenyl group having 2 to 10 carbon atoms, an aryl group having 6 to 40 carbon atoms, and a combination thereof, and the alkyl group, the alkenyl group, and the aryl group may have an ether bond, a ketone bond, or an ester bond; R₃ represent a group selected from the group consist of a hydrogen atom, an alkyl group having 1 to 10 carbon atoms, an alkenyl group having 2 to 10 carbon atoms, an aryl group having 6 to 40 carbon atoms, and a combination thereof, and the alkyl group, the alkenyl group, and the aryl group may have an ether bond, a ketone bond, or an ester bond; R₄ represents an aryl group that has 6 to 40 carbon atoms and may be substituted with a halogen group, a nitro group, an amino group, or a hydroxy group, or a heterocyclic group; R₅ represents a hydrogen atom, an alkyl group that has 1 to 10 carbon atoms and may be substituted with a halogen group, a nitro group, an amino group or a hydroxy group, an aryl group having 6 to 40 carbon atoms, or a heterocyclic group; R₄ and R₅ may form a ring together with the carbon atoms to which they are bonded; and "n1" and "n2" are each integers of 1 to 3. Incidentally, the symbols in the formula are applied only in this formula.

Examples of the component (A) resin or compound for forming an organic film used in the present invention include: novolac resins obtained by reacting one or more kinds among phenol sources, such as phenol, cresol, xylenol, catechol, resorcinol, hydroquinone, pyrogallol, hydroxyquinol, and phloroglucinol and one or more kinds among aldehyde sources, such as formaldehyde, paraformaldehyde, and trioxane using an acidic catalyst; and resins having a repeating unit structure represented by the following formula (21) described in WO2012/176767A1.

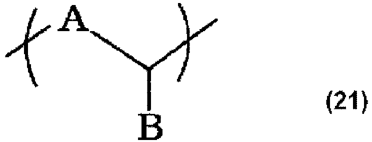

In the formula (21), A represents a hydroxy group-substituted phenylene group derived from polyhydroxybenzene; and B represents a monovalent condensed aromatic hydrocarbon ring group in which 2 to 6 benzene rings are condensed. Incidentally, the symbols in the formula are applied only in this formula.

Examples of the component (A) resin or compound for forming an organic film used in the present invention include: novolac resins having a fluorene or tetrahydrospirobindene structure described in JP2005-128509A, JP2006-259249A, JP2006-259482A, JP2006-293298A, and JP2007-316282A; and a resin having a repeating unit structure represented by the following formula (22-1) or (22-2).

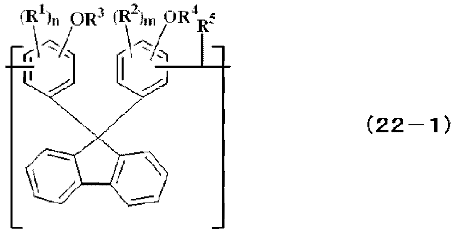

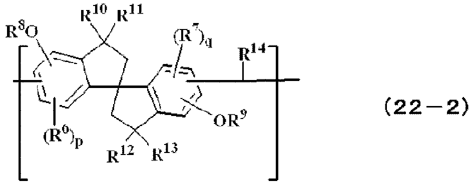

In the formulae (22-1) and (22-2), R¹, R², R⁶, and R⁷ independently represent a hydrogen atom, a linear, branched, or cyclic alkyl group having 1 to 10 carbon atoms, an aryl group having 6 to 10 carbon atoms, an allyl group, or a halogen atom; R³, R⁴, R⁸, and R⁹ independently represents a hydrogen atom, a linear, branched, or cyclic alkyl group having 1 to 6 carbon atoms, a linear, branched, or cyclic alkenyl group having 2 to 6 carbon atoms, an aryl group having 6 to 10 carbon atoms, or a glycidyl group; R⁵ and R¹⁴ independently represent a hydrogen atom, a linear, branched, or cyclic alkyl group having 1 to 10 carbon atoms, or an aryl group having 6 to 10 carbon atoms; "n", "m", "p", and "q" represent an integer of 1 to 3; R¹⁰ to R¹³ independently represent a hydrogen atom, a halogen atom, a hydroxy group, a linear, branched, or cyclic alkyl group having 1 to 6 carbon atoms, or a linear, branched, or cyclic alkoxy group having 1 to 6 carbon atoms. Incidentally, the symbols in the formula are applied only in this formula.

Examples of the component (A) resin or compound for forming an organic film used in the present invention include reaction products obtained by a method described in JP2012-145897A. More specifically, examples thereof include polymers obtained by condensing one or more compounds represented by the following general formula (23-1) and/or (23-2) with one or more compounds represented by the following general formula (24-1) and/or (24-2) and/or equivalents thereof.

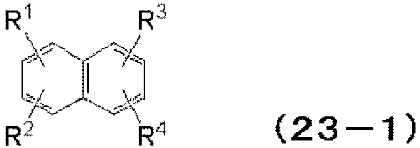

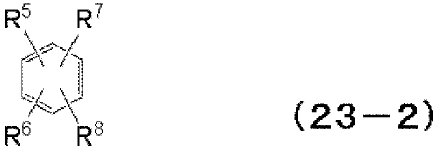

In the formulae (23-1) and (23-2), R¹ to R⁸ each independently represent a hydrogen atom, a halogen atom, a hydroxyl group, an isocyanato group, a glycidyloxy group, a carboxyl group, an amino group, an alkoxy group having 1 to 30 carbon atoms, an alkoxycarbonyl group having 1 to 30 carbon atoms, an alkanoyloxy group having 1 to 30 carbon atoms, and saturated or unsaturated organic group that has 1 to 30 carbon atoms and may be substituted. Furthermore, two substituents arbitrarily selected from R¹ to R⁴ or R⁵ to R⁸ in the molecule may be bonded to each other to further form a cyclic substituent. Incidentally, the symbols in the formula are applied only in this formula.

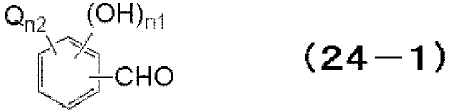

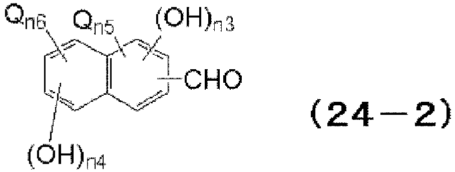

In the formulae (24-1) and (24-2), Q represents an organic group that has 1 to 30 carbon atoms and may be substituted, and two Qs selected arbitrarily within the molecule may be bonded to each other to form a cyclic substituent; "n1" to "n6" represent the numbers of substituents each, "n1" to "n6" are each independently 0, 1, or 2, and in the formula (24-1), hydroxybenzaldehyde is excluded. Meanwhile, in the formula (24-2), the relationships 0≤n3+n5≤3, 0≤n4+n6≤4, and 1≤n3+n4≤4 are satisfied. Incidentally, the symbols in the formula are applied only in this formula.

Further examples also include polymers obtained by condensing one or more compounds represented by the above general formula (23-1) and/or (23-2); one or more compounds represented by the above general formula (24-1) and/or (24-2) and/or an equivalent thereof; and one or more compounds represented by the following general formula (25) and/or an equivalent thereof.

Y-CHO (25)

In the formula (25), Y represents a hydrogen atom or a monovalent organic group that has 30 or fewer carbon atoms and may have a substituent, and thus the formula (25) is different from the general formula (24-1) and the general formula (24-2). Incidentally, the symbols in the formula are applied only in this formula.

Further examples of the component (A) resin or compound for forming an organic film used in the present invention include compounds having the following structure disclosed in JP2017-119671A.

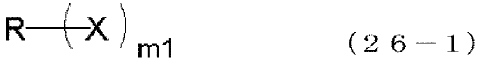

In the formula (26-1), R represents a single bond or an organic group having 1 to 50 carbon atoms; X represents a group represented by the following general formulae (26-2); and "m1" represents an integer that satisfies 2≤m1≤10. Incidentally, the symbols in the formula are applied only in this formula.

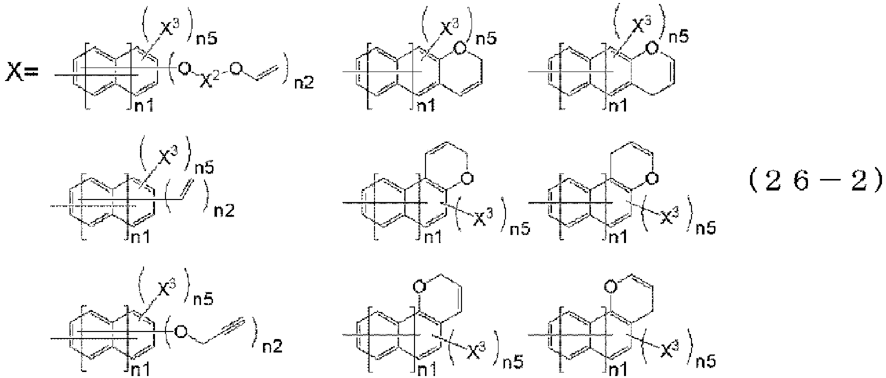

In the formulae, X² represents a divalent organic group having 1 to 10 carbon atoms; "n1" represents 0 or 1; "n2" represents 1 or 2; X³ represents a group represented by the following general formula (26-3); and "n5" represents 0, 1, or 2. Incidentally, the symbols in the formula are applied only in this formula.

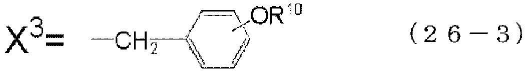

In the formula, R¹⁰ represents a hydrogen atom or a saturated or unsaturated hydrocarbon group having 1 to 10 carbon atoms, and a hydrogen atom on the benzene ring in the formula may be substituted with a methyl group or a methoxy group. Incidentally, the symbols in the formula are applied only in this formula.

Examples of compounds having the above structure include the following compounds.

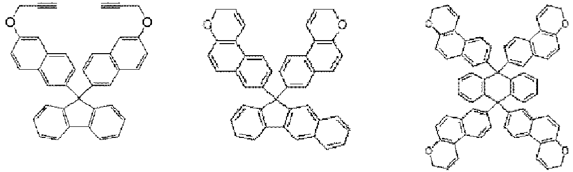

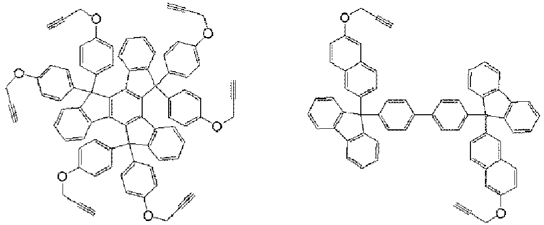

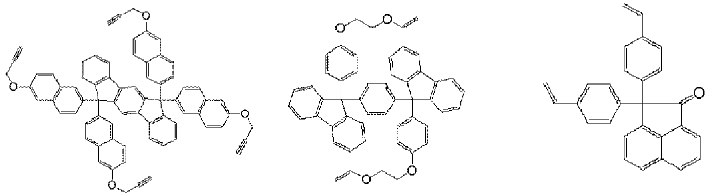

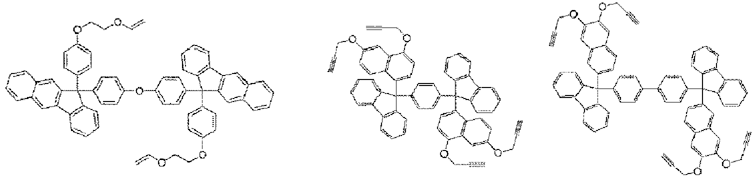

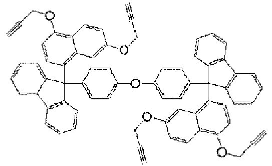

Examples of the component (A) resin or compound for forming an organic film used in the present invention include polymers having a repeating unit represented by the following general formula (27-1) disclosed in JP2019-044022A.

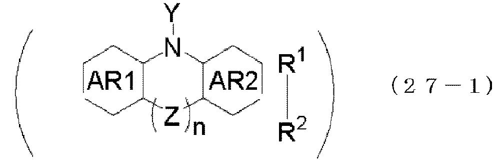

In the formula (27-1), AR1 and AR2 each represent a benzene ring or naphthalene ring optionally having a substituent. R¹ and R² each independently represent a hydrogen atom or an organic group having 1 to 30 carbon atoms, and when R¹ and R² each represent an organic group, R¹ and R² are optionally bonded to each other within a molecule to form a cyclic organic group. "n" represents 0 or 1; when n = 0, AR1 and AR2 do not form a bridged structure between the aromatic rings of AR1 and AR2 via Z; when n = 1, AR1 and AR2 form a bridged structure between the aromatic rings of AR1 and AR2 via Z; and Z represents a single bond or one of the following formulae (27-2). Y represents a group represented by the following formula (27-3). Note that the definitions of the symbols in the formula apply only in this formula.

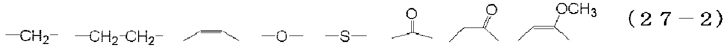

----R³-c≡c-R⁴ (27-3)

In the formula, R³ represents a single bond or a divalent organic group having 1 to 20 carbon atoms; R⁴ represents a hydrogen atom or a monovalent organic group having 1 to 20 carbon atoms; and a broken line represents an attachment point. Incidentally, the symbols in the formula are applied only in this formula.

Examples of the polymers having a repeating unit represented by the above general formula (27-1) include the following polymers.

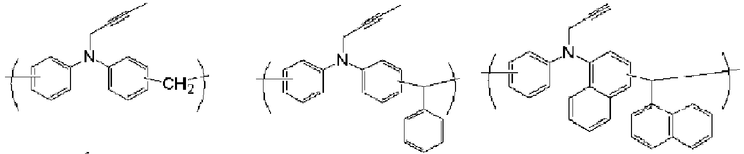

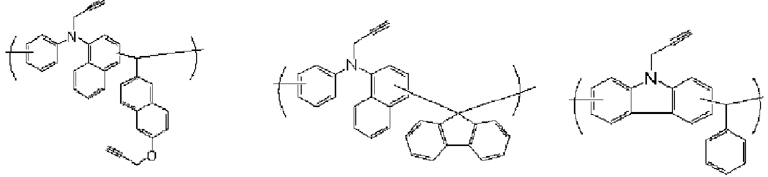

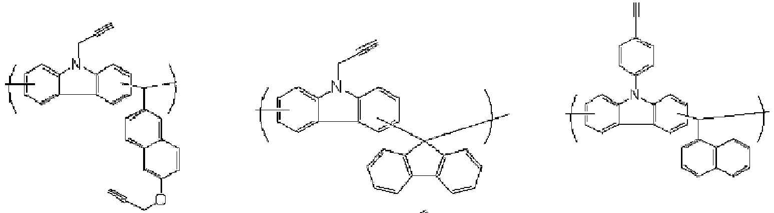

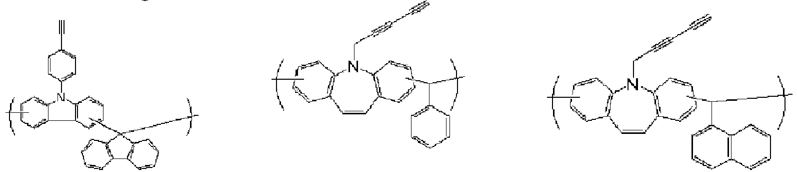

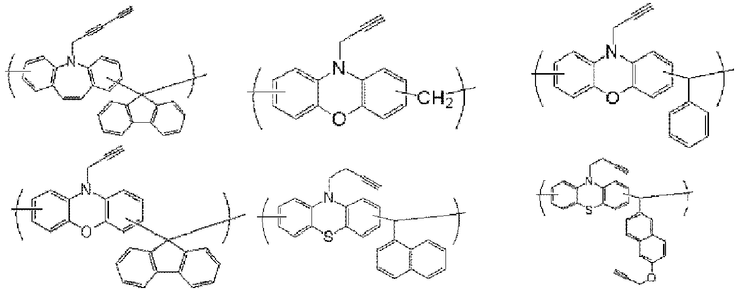

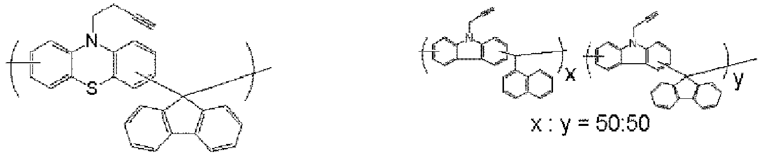

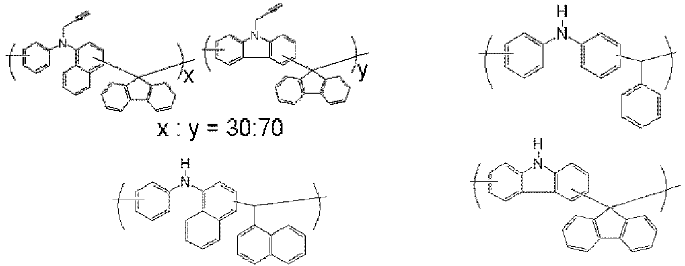

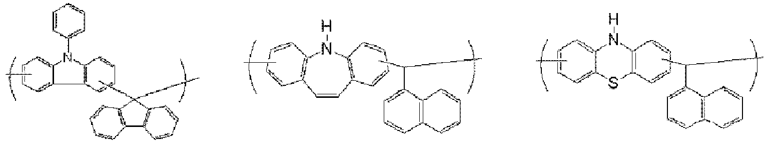

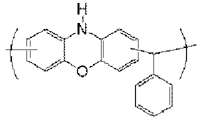

The component (A) resin or compound for forming an organic film may be synthesized by a publicly known method, and a commercially available product may be used.

The blending amount of the component (A) resin or compound for forming an organic film is not particularly limited as long as sufficient film-formability can be achieved by spin-coating with the composition for forming an organic film, but the component (A) resin or compound for forming an organic film is preferably contained in an amount of 10 to 40 parts by mass, more preferably 10 to 30 parts by mass, and further preferably 10 to 25 parts by mass, based on 100 parts by mass of the composition for forming an organic film. For example, when a hole or trench, having a very high aspect ratio, of a 3D NAND memory architecture is to be filled with the composition for forming an organic film, the contained amount of the resin for forming an organic film needs to be large. However, on the other hand, such a composition for forming an organic film is highly viscous, and in-plane uniformity after spin-coating and filling property are degraded. Even when the component (A) resin for forming an organic film is contained in such a proportion, the inventive composition for forming an organic film makes it possible to form an organic film having excellent in-plane uniformity and excellent filling property, and can therefore be applied suitably.

Meanwhile, the component (B) polymer (a) is preferably contained in an amount of 0.01 parts by mass to 5 parts by mass based on 100 parts by mass of the component (A) resin or compound for forming an organic film. A composition for forming an organic film containing the polymer in such an amount makes it possible to form an organic film with more excellent in-plane uniformity.

The component (C) solvent usable for the inventive composition for forming an organic film is not particularly limited as long as the solvent can dissolve the component (A) resin or compound for forming an organic film and the component (B) polymer (a), and the solvent can preferably also dissolve an acid generator, a crosslinking agent, a surfactant, etc., which will be described later. Specifically, it is possible to use a solvent having a boiling point lower than 180°C, such as the solvents disclosed in paragraphs [0091] and [0092] of JP2007-199653A. Among them, preferable solvents are propylene glycol monomethyl ether acetate, propylene glycol monomethyl ether, 2-heptanone, cyclopentanone, cyclohexanone, and mixtures of two or more thereof.

The component (C) solvent is preferably contained in an amount of 200 to 10,000 parts by mass, more preferably 300 to 5,000 parts by mass based on 100 parts by mass of the component (A) resin or compound for forming an organic film. When the amount is in such a range, the concentration can be adjusted in accordance with the desired film thickness.

Furthermore, the inventive material for forming an organic film may also contain a high-boiling-point solvent having a boiling point of 180°C or higher as an organic solvent in addition to the solvent having a boiling point of lower than 180°C (a mixture of a solvent having a boiling point of lower than 180°C and a solvent having a boiling point of 180°C or higher). The high-boiling-point organic solvent is not particularly limited to hydrocarbons, alcohols, ketones, esters, ethers, or chlorine-based solvents as long as the solvent is capable of dissolving compounds for forming an organic film. Specific examples include 1-octanol, 2-ethylhexanol, 1-nonanol, 1-decanol, 1-undecanol, ethylene glycol, 1,2-propylene glycol, 1,3-butylene glycol, 2,4-pentanediol, 2-methyl-2,4-pentanediol, 2,5-hexanediol, 2,4-heptanediol, 2-ethyl-1,3-hexanediol, diethylene glycol, dipropylene glycol, triethylene glycol, tripropylene glycol, glycerin, n-nonyl acetate, ethylene glycol monohexyl ether, ethylene glycol mono-2-ethylhexyl ether, ethylene glycol monophenyl ether, ethylene glycol monobenzyl ether, diethylene glycol monoethyl ether, diethylene glycol monoisopropyl ether, diethylene glycol mono-n-butyl ether, diethylene glycol monoisobutyl ether, diethylene glycol monohexyl ether, diethylene glycol monophenyl ether, diethylene glycol monobenzyl ether, diethylene glycol diethyl ether, diethylene glycol dibutyl ether, diethylene glycol butyl methyl ether, triethylene glycol dimethyl ether, triethylene glycol monomethyl ether, triethylene glycol-n-butyl ether, triethylene glycol butyl methyl ether, triethylene glycol diacetate, tetraethylene glycol dimethyl ether, dipropylene glycol monomethyl ether, dipropylene glycol mono-n-propyl ether, dipropylene glycol mono-n-butyl ether, tripropylene glycol dimethyl ether, tripropylene glycol monomethyl ether, tripropylene glycol mono-n-propyl ether, tripropylene glycol mono-n-butyl ether, ethylene glycol monoethyl ether acetate, ethylene glycol monobutyl ether acetate, diethylene glycol monomethyl ether acetate, diethylene glycol monoethyl ether acetate, diethylene glycol monobutyl ether acetate, triacetin, propylene glycol diacetate, dipropylene glycol monomethyl ether acetate, dipropylene glycol methyl-n-propyl ether, dipropylene glycol methyl ether acetate, 1,4-butanediol diacetate, 1,3-butylene glycol diacetate, 1,6-hexanediol diacetate, γ-butyrolactone, dihexyl malonate, diethyl succinate, dipropyl succinate, dibutyl succinate, dihexyl succinate, dimethyl adipate, diethyl adipate, and dibutyl adipate. One of the solvents may be used or a mixture of two or more kinds may be used.

The boiling point of the high-boiling-point solvent may be selected appropriately in accordance with temperature at which the material for forming an organic film is heated, and the boiling point of the high-boiling-point solvent to be added is preferably 180°C to 300°C, further preferably 200°C to 300°C. When the boiling point is as described, sufficient thermal flowability can be achieved, since there is no risk of excessive evaporation rate in baking (heating) due to the boiling point being too low. Moreover, a solvent having such a boiling point does not remain in the film without evaporating even after the baking due to the boiling point being high. Therefore, there is no risk of the solvent adversely affecting the physical properties, such as etching resistance, of the film.

Furthermore, when the high-boiling-point solvent is used, the blending amount of the high-boiling-point solvent is preferably 1 to 30 parts by mass relative to 100 parts by mass of the solvent having a boiling point of lower than 180°C. With such a blending amount, there is no risk that the solvent is too little to impart sufficient thermal flowability during baking or that the solvent is excessively blended to remain in the film and lead to degradation of physical properties, such as etching resistance, of the film.

By using the above-described composition for forming an organic film, the material for forming an organic film is provided with both high filling property and planarizing property by thermal flowability being imparted to the component (A) resin or compound for forming an organic film above by virtue of the high-boiling-point solvent being contained.

### [Other Components]

In addition, the inventive composition for forming an organic film can contain an acid generator or a crosslinking agent for further promoting the crosslinking reaction. Acid generators include those ((E) thermal acid generator) generate an acid by thermal decomposition and those ((D) photo-acid generator) generate an acid by light irradiation, and any acid generator can be contained. Specific examples of the acid generator include those disclosed in paragraphs [0061] to [0085] of JP2007-199653A. One kind of the acid generator may be used, or two or more kinds thereof may be used in combination. When the acid generator is contained, the contained amount is preferably 0.05 to 50 parts by mass, more preferably 0.1 to 10 parts by mass relative to 100 parts by mass of the component (A) resin or compound for forming an organic film. When the amount is as described, it is possible to promote the crosslinking reaction and form a dense film.

Specific examples of the crosslinking agent include those disclosed in paragraphs [0055] to [0060] of JP2007-199653A. One kind of the crosslinking agents may be used, or two or more kinds thereof may be used in combination. The crosslinking agent is preferably contained in an amount of 1 to 100 parts by mass, more preferably 5 to 50 parts by mass relative to 100 parts by mass of the component (A) resin or compound for forming an organic film. When the amount is as described, it is possible to enhance curability and further suppress intermixing with an upper layer film.

Further, the inventive composition for forming an organic film can also contain an additional surfactant other than the inventive compound (B), in order to further improve in-plane uniformity in spin-coating. Specific examples of the additional surfactant include those disclosed in paragraphs [0142] to [0147] of JP2009-269953A. One kind of the additional surfactants may be used, or two or more kinds thereof may be used in combination. When the additional surfactant is contained, the contained amount is preferably 0.01 to 10 parts by mass, more preferably 0.05 to 5 parts by mass relative to 100 parts by mass of the resin or compound for forming an organic film. When the amount is as described, it is possible to form an organic film excellent in in-plane uniformity.

The inventive composition for forming an organic film can further contain a basic compound for enhancing storage stability. The basic compound serves as a quencher to an acid in order to prevent a very small amount of acid generated by the acid generator from promoting the crosslinking reaction. Specific examples of such a basic compound include those disclosed in paragraphs [0086] to [0090] of JP2007-199653A. One kind of the basic compounds may be used, or two or more kinds thereof may be used in combination. When the basic compound is contained, the contained amount is preferably 0.05 to 50 parts by mass, more preferably 0.1 to 10 parts by mass relative to 100 parts by mass of the component (A) resin or compound for forming an organic film. When the amount is as described, it is possible to improve the storage stability of the composition for forming an organic film.

As described above, the inventive composition for forming an organic film is excellent in hump suppression at the time of an EBR process. Accordingly, the inventive composition for forming an organic film is extremely useful as a resist underlayer film material (material for an organic film) for multilayer resist processes, such as two-layer resist processes, three-layer resist processes using a silicon-containing resist middle layer film or a silicon-containing inorganic hard mask middle layer film, and four-layer resist processes using a silicon-containing resist middle layer film or a silicon-containing inorganic hard mask middle layer film and an organic antireflective film or an adhesive film.

### [Method for Forming Organic Film]

The present invention provides a method for forming an organic film to be used in a manufacturing process of a semiconductor device, the method comprising:
spin-coating a substrate to be processed with the above-mentioned inventive composition for forming a film; and
forming a cured film by heating the substrate coated with the composition for forming a film at a temperature of 100°C or higher and 600°C or lower for 10 to 600 seconds.

In this method for forming an organic film, firstly, the above-mentioned inventive composition for forming an organic film is applied onto a substrate to be processed by spin-coating. An excellent filling property can be obtained by using a spin-coating method. After removing the film on an edge portion in an EBR process, baking (heating) is performed in order to promote the crosslinking reaction. Incidentally, the solvent in the composition can be evaporated by this baking, and therefore, mixing can be prevented even when a resist upper layer film or a silicon-containing resist middle layer film is formed on the organic film.

The baking is performed at a temperature of 100°C or higher and 600°C or lower for 10 to 600 seconds, preferably at a temperature of 200°C or higher and 500°C or lower for 10 to 300 seconds. Considering influence on device damage and wafer deformation, the upper limit of the heating temperature in a wafer process of lithography is preferably 600°C or lower, more preferably 500°C or lower. By performing the heat treatment under such conditions, the crosslinking reaction can be promoted, and it is possible to form an organic film that does not mix with a film formed thereon.

### [Patterning Process]

In the following, patterning processes using the inventive composition for forming an organic film will be described.

### [Three-Layer Resist Process Using Silicon-Containing Resist Middle Layer Film]

The present invention provides a patterning process including:
forming an organic film on a body to be processed by using the composition for forming a film above;
forming a resist middle layer film on the organic film by using a silicon-containing resist middle layer film material;
forming a resist upper layer film on the resist middle layer film by using a resist upper layer film material containing a photoresist composition;
forming a circuit pattern in the resist upper layer film;
transferring the pattern to the resist middle layer film by etching while using the resist upper layer film having the formed circuit pattern as a mask;
transferring the pattern to the organic film by etching while using the resist middle layer film having the transferred pattern as a mask; and
further transferring the pattern in the body to be processed by etching while using the organic film having the transferred pattern as a mask.

The body to be processed is preferably a semiconductor device substrate or the semiconductor device substrate coated with any of a metal film, a metal carbide film, a metal oxide film, a metal nitride film, a metal oxycarbide film, and a metal oxynitride film. More specifically, the body to be processed is not particularly limited, but may be a substrate made of Si, α-Si, p-Si, SiO₂, SiN, SiON, W, TiN, Al, etc.; the substrate coated with the above-described metal film or the like as a layer to be processed; or the like.

Examples of the layer to be processed include various Low-k films made of Si, SiO₂, SiON, SiN, p-Si, α-Si, W, W-Si, Al, Cu, Al-Si, or the like; and stopper films thereof. Generally, the layer can be formed to have a thickness of 50 to 10,000 nm, particularly 100 to 5,000 nm. Incidentally, when the layer to be processed is formed, the substrate and the layer to be processed are formed from different materials.

Incidentally, as the metal constituting the body to be processed, it is preferable to use silicon, titanium, tungsten, hafnium, zirconium, chromium, germanium, copper, silver, gold, aluminum, indium, gallium, arsenic, palladium, iron, tantalum, iridium, molybdenum, or an alloy thereof.

When forming an organic film on the body to be processed by using the inventive composition for forming an organic film, the inventive method for forming an organic film described above can be adopted.

Subsequently, a resist middle layer film (silicon-containing resist middle layer film) is formed on the organic film by using silicon-containing resist middle layer film material. As the resist middle layer film material having a silicon atom, a polysiloxane-based middle layer film material is preferable. By imparting an antireflective effect to the silicon-containing resist middle layer film, reflection can be suppressed. Particularly, for 193-nm light exposure, when a material having many aromatic groups and having high etching selectivity relative to the substrate is used as a composition for forming an organic film, the k-value increases, and thus the substrate reflection is increased; in contrast, the reflection can be suppressed by imparting absorption to the silicon-containing resist middle layer film so as to have an appropriate k-value, and the substrate reflection can be reduced to 0.5% or less. As the silicon-containing resist middle layer film having an antireflective effect, anthracene is preferably used for exposure at 248 nm and 157 nm, and polysiloxane which has a pendant light-absorbing group having a phenyl group or a silicon-silicon bond and which crosslinks with acid or heat is preferably used for exposure at 193 nm.

Subsequently, a resist upper layer film is formed on the silicon-containing resist middle layer film by using a resist upper layer film material containing a photoresist composition. The resist upper layer film material may be a positive type or a negative type, and a photoresist composition that is normally used can be used. After the spin-coating with the resist upper layer film material, pre-baking is preferably performed at 60 to 180°C for 10 to 300 seconds. Then, light exposure, post-exposure baking (PEB), and development are performed according to a conventional method to obtain a resist upper layer film pattern. Incidentally, the thickness of the resist upper layer film is not particularly limited but is preferably 30 to 500 nm, particularly preferably 50 to 400 nm.

Subsequently, a circuit pattern (resist upper layer film pattern) is formed in the resist upper layer film. The circuit pattern is preferably formed by a lithography using light having a wavelength of 10 nm or more and 300 nm or less, a direct writing with electron beam, nanoimprinting, or a combination thereof.

Examples of exposure light include high-energy beams having a wavelength of 300 nm or less, and specific examples include deep ultraviolet ray, KrF excimer laser beam (248 nm), ArF excimer laser beam (193 nm), F₂ laser beam (157 nm), Kr₂ laser beam (146 nm), Ar₂ laser beam (126 nm), soft X-ray (EUV) at 3 to 20 nm, electron beam (EB), ion beam, X-ray.

Furthermore, in forming the circuit pattern, the circuit pattern is preferably developed with an alkaline development or an organic solvent.

Subsequently, the pattern is transferred to the silicon-containing resist middle layer film by etching while using the resist upper layer film having the formed circuit pattern as a mask. The etching of the silicon-containing resist middle layer film performed while using the resist upper layer film pattern as a mask is preferably performed by using a fluorocarbon-based gas. Thus, the silicon-containing resist middle layer film pattern is transferred.

Subsequently, the pattern is transferred to the organic film by etching while using the silicon-containing resist middle layer film having the transferred pattern as a mask. The silicon-containing resist middle layer film exhibits etching resistance to oxygen gas or hydrogen gas, and therefore, the etching of the organic film performed while using the silicon-containing resist middle layer film pattern as a mask is preferably performed by using an etching gas mainly containing oxygen gas or hydrogen gas. Thus, the organic film pattern is transferred.

Subsequently, the pattern is transferred in the body to be processed by etching while using the organic film having the pattern transferred as a mask. The subsequent etching of the body to be processed (layer to be processed) can be performed by a conventional method. For example, a body to be processed made of SiO₂, SiN, or silica-based low-dielectric insulating film is etched mainly with a fluorocarbon-based gas; and a body to be processed made of p-Si, Al, or W is etched mainly with a chlorine- or bromine-based gas. When a substrate is processed by etching with a fluorocarbon-based gas, the silicon-containing resist underlayer film pattern is removed when the substrate is processed. Meanwhile, when a substrate is processed by etching with a chlorine- or bromine-based gas, the silicon-containing resist underlayer film pattern needs to be removed by additional dry etching with a fluorocarbon-based gas after the processing of the substrate.

The organic film obtained by using the inventive composition for forming an organic film can be provided with excellent etching resistance when the body to be processed is etched as described above.

### [Four-Layer Resist Process Using Silicon-Containing Resist Middle Layer Film and Organic Antireflective Film or Adhesive Film]

The present invention also provides a patterning process including:
forming an organic film on a body to be processed by using the composition for forming a film above;
forming a resist middle layer film on the organic film by using a silicon-containing resist middle layer film material;
forming an organic antireflective film or an adhesive film on the resist middle layer film;
forming a resist upper layer film on the organic antireflective film or the adhesive film by using a resist upper layer film material containing a photoresist composition;
forming a circuit pattern in the resist upper layer film;
transferring the pattern to the organic antireflective film or the adhesive film and to the resist middle layer film by etching while using the resist upper layer film having the formed circuit pattern as a mask;
transferring the pattern to the organic film by etching while using the resist middle layer film having the transferred pattern as a mask; and
further transferring the pattern to the body to be processed by etching while using the organic film having the transferred pattern as a mask.

This method can be performed in the same manner as the three-layer resist process using the silicon-containing resist middle layer film above, except that the organic antireflective film (BARC) or the adhesive film is formed between the silicon-containing resist middle layer film and the resist upper layer film.

The organic antireflective film and the adhesive film can be formed by spin-coating by using a publicly known organic antireflective film material.

### [Three-Layer Resist Process Using Inorganic Hard Mask Middle Layer Film]

The present invention also provides a patterning process including:
forming an organic film on a body to be processed by using the composition for forming a film above;
forming an inorganic hard mask selected from the group consisting of a silicon oxide film, a silicon nitride film, and a silicon oxynitride film on the organic film;
forming a resist upper layer film on the inorganic hard mask by using a resist upper layer film material containing a photoresist composition;
forming a circuit pattern in the resist upper layer film;
transferring the pattern to the inorganic hard mask by etching while using the resist upper layer film having the formed circuit pattern as a mask;
transferring the pattern to the organic film by etching while using the inorganic hard mask having the transferred pattern as a mask; and
further transferring the pattern to the body to be processed by etching while using the organic film having the transferred pattern as a mask.

This method can be performed in the same manner as the three-layer resist process using the silicon-containing resist middle layer film above, except that the inorganic hard mask middle layer film is formed instead of the silicon-containing resist middle layer film on the organic film.

The inorganic hard mask middle layer film selected from the group consisting of a silicon oxide film, a silicon nitride film, and a silicon oxynitride film (SiON film) can be formed by a CVD method, an ALD method, etc. Examples of methods for forming the silicon nitride film are disclosed in JP2002-334869A, WO2004/066377A1, etc. The inorganic hard mask middle layer film preferably has a thickness of 5 to 200 nm, more preferably 10 to 100 nm. As the inorganic hard mask middle layer film, a SiON film, which is highly effective as an antireflective film, is the most preferably used.

### [Four-Layer Resist Process Using Inorganic Hard Mask Middle Layer Film and Organic Antireflective Film or Adhesive Film]

The present invention also provides a patterning process including:
forming an organic film on a body to be processed by using the composition for forming a film above;
forming an inorganic hard mask selected from the group consisting of a silicon oxide film, a silicon nitride film, and a silicon oxynitride film on the organic film;
forming an organic antireflective film or an adhesive film on the inorganic hard mask;
forming a resist upper layer film on the organic antireflective film or the adhesive film by using a resist upper layer film material containing a photoresist composition;
forming a circuit pattern in the resist upper layer film;
transferring the pattern to the organic antireflective film or the adhesive film and to the inorganic hard mask by etching while using the resist upper layer film having the formed circuit pattern as a mask;
transferring the pattern to the organic film by etching while using the inorganic hard mask having the transferred pattern as a mask; and
further transferring the pattern to the body to be processed by etching while using the organic film having the transferred pattern as a mask.

This method can be performed in the same manner as the above-described three-layer resist process using the inorganic hard mask middle layer film, except that the organic antireflective film (BARC) or the adhesive film is formed between the inorganic hard mask middle layer film and the resist upper layer film.

Particularly, when a SiON film is used as the inorganic hard mask middle layer film, two antireflective films including the SiON film and the BARC make it possible to suppress the reflection even in liquid-immersion exposure at a high NA exceeding 1.0. Another merit of forming the BARC is having an effect of reducing trailing of the resist upper layer film pattern immediately above the SiON film.

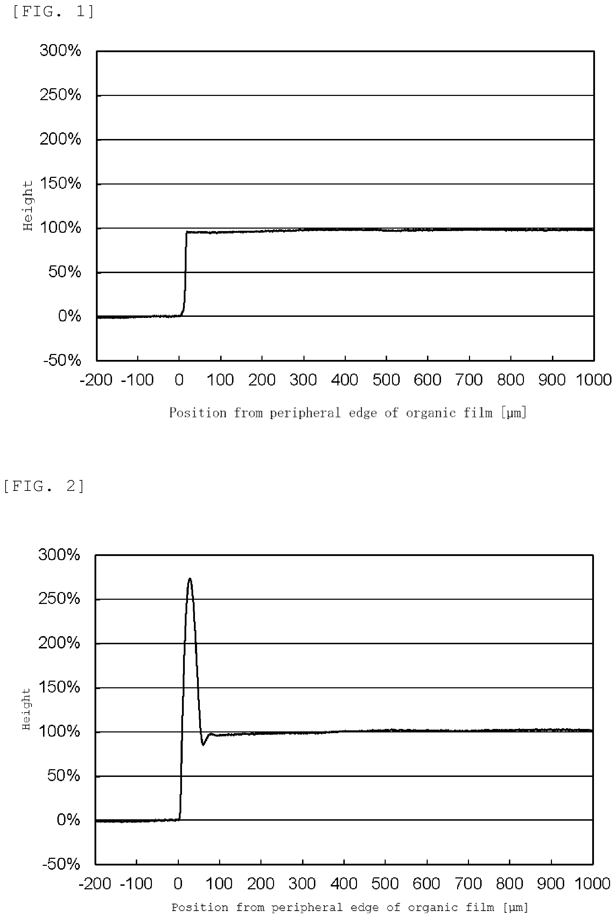

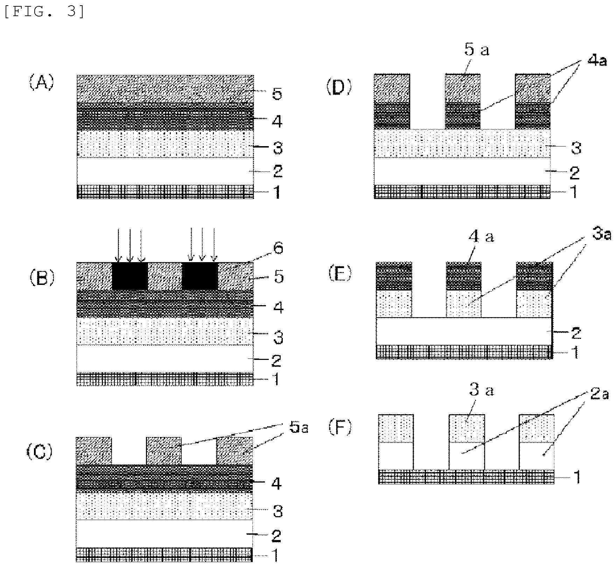

Here, an example of the inventive patterning processes according to a three-layer resist process is shown in FIG. 3 (A) to (F). In the case of a three-layer resist process, as shown in FIG. 3 (A), an organic film 3 is formed by using the inventive composition for forming an organic film on a layer 2 to be processed formed on a substrate 1; then, a silicon-containing resist middle layer film 4 is formed; and a resist upper layer film 5 is formed thereon. Next, as shown in FIG. 3 (B), an exposure portion 6 of the resist upper layer film 5 is exposed, and then PEB (post-exposure baking) is performed. Next, as shown in FIG. 3 (C), development is performed to form a resist upper layer film pattern 5a. Next, as shown in FIG. 3 (D), the silicon-containing resist middle layer film 4 is dry-etched by using a fluorocarbon-based gas while using the resist upper layer film pattern 5a as a mask, and thus, a silicon-containing resist middle layer film pattern 4a is formed. Next, as shown in FIG. 3 (E), the resist upper layer film pattern 5a is removed, then, the organic film 3 is etched with oxygen plasma while using the silicon-containing resist middle layer film pattern 4a as a mask, and thus, an organic film pattern 3a is formed. Furthermore, as shown in FIG. 3 (F), the silicon-containing resist middle layer film pattern 4a is removed, then the layer 2 to be processed is etched while using the organic film pattern 3a as a mask, and thus, a pattern 2a is formed. In the patterning process of this example, by hump being suppressed when the organic film is formed, during the dry etching of FIG. 1 (D), (E), and (F), it is possible to reduce defects that are generated due to hump of the organic film.

In a case where an inorganic hard mask middle layer film is formed, the silicon-containing resist middle layer film 4 may be changed to an inorganic hard mask middle layer film, and in a case where a BARC or an adhesive film is formed, the BARC or the adhesive film may be formed between the silicon-containing resist middle layer film 4 and the resist upper layer film 5. The etching of the BARC or the adhesive film may be performed continuously before the etching of the silicon-containing resist middle layer film 4; alternatively, after the BARC or the adhesive film alone is etched, the etching apparatus may be changed and so forth, and then the etching of the silicon-containing resist middle layer film 4 may be performed.

As described above, the inventive patterning processes make it possible to form a fine pattern in a body to be processed with high precision according to a multilayer resist process, and to suppress hump formation on an organic film, thus reducing defects derived from hump of the organic film.

### [Composition for Forming Organic Film (Resist Material)]

The inventive composition for forming an organic film can be used as a resist material.

The inventive composition for forming an organic film may further contain a conventional surfactant when the polymer (a) of the present invention is used as a surfactant. Examples of the conventional surfactants include those described in paragraphs [0165] and [0166] of JP2008-111103A. When the inventive composition for forming an organic film contains the conventional surfactant, its content is not particularly limited.

The inventive composition for forming an organic film may further contain a base polymer. The base polymer is not particularly limited as long as it can be used for a resist material.

Specifically, in the case of a positive-type resist material of a chemically-amplified resist, the base polymer includes a repeating unit having an acid-labile group. The repeating unit having the acid-labile group is preferably a repeating unit represented by the following formula (x1) (hereinafter also referred to as a repeating unit "x1") or a repeating unit represented by the following formula (x2) (hereinafter also referred to as a repeating unit "x2").

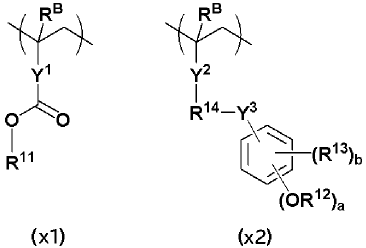

In formulae (x1) and (x2), R^{B} each independently represents a hydrogen atom or a methyl group; Y¹ represents a single bond, a phenylene group, a naphthylene group, or a linking group that has 1 to 12 carbon atoms and contains at least one selected from the group consisting of an ester bond, an ether bond, and a lactone ring; Y² represents a single bond or an ester bond; Y³ is a single bond, an ether bond or an ester bond; R¹¹ and R¹² each independently represent an acid-labile group, and in addition, when the base polymer contains both of the repeating units a1 and a2, R¹¹ and R¹² may be mutually the same or different from each other; R¹³ is a fluorine atom, a trifluoromethyl group, a cyano group, or a saturated hydrocarbyl group having 1 to 6 carbon atoms; R¹⁴ represents a single bond or an alkanediyl group having 1 to 6 carbon atoms, and a portion of -CH₂- of the alkanediyl group may be substituted with an ether bond or an ester bond; and "a" represents 1 or 2, "b" represents an integer of 0 to 4, and a+b fulfills 1≤a+b≤5.

Specific examples of monomers providing the repeating unit "x1" include those shown below but are not limited thereto. Incidentally, in the following formulae, R^{B} and R¹¹ represent the same defined as above.

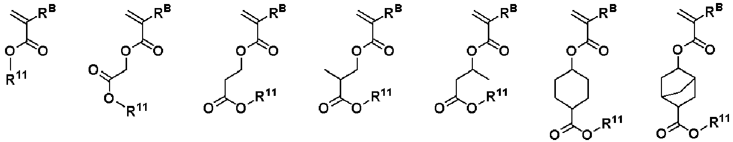

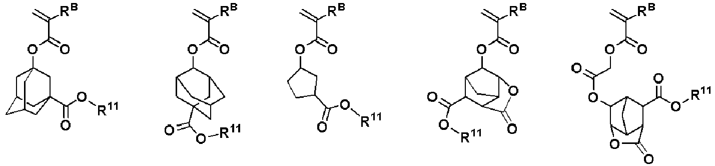

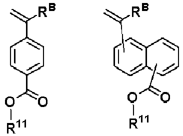

Specific examples of monomers providing the repeating unit "x2" include those shown below but are not limited thereto. Incidentally, in the following formulae, R^{B} and R¹² represent the same defined as above.

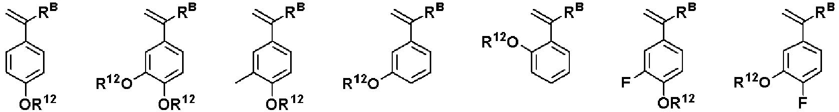

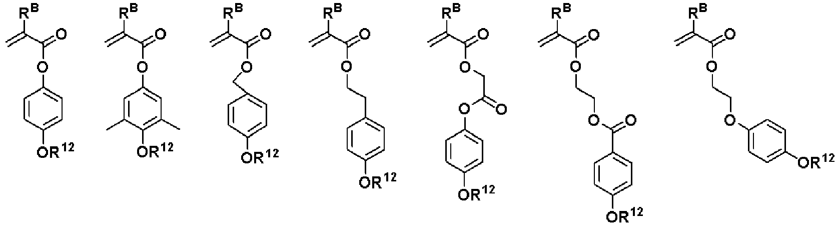

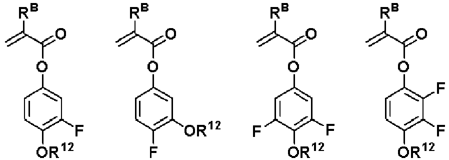

Examples of the acid-labile group represented by R¹¹ and R¹² include those described in JP2013-80033A and JP2013-83821A.

Specific examples of the acid-labile group include those represented by any of the following formulae (AL-1) to (AL-3).

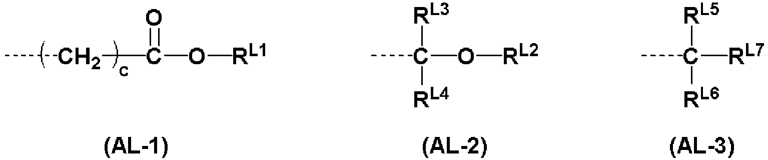

In the formulae, a broken line represents an attachment point.

In formulae (AL-1) and (AL-2), R^{L1} and R^{L2} are each independently a hydrocarbyl group having 1 to 40 carbon atoms, and may contain a heteroatom, such as an oxygen atom, a sulfur atom, a nitrogen atom, and a fluorine atom. The hydrocarbyl group may be saturated or unsaturated, and may be linear, branched, or cyclic. The hydrocarbyl group is preferably a saturated hydrocarbyl group having 1 to 40 carbon atoms, more preferably a saturated hydrocarbyl group having 1 to 20 carbon atoms.

In formula (AL-1), "c" is an integer of 0 to 10, preferably an integer of 1 to 5.

In formula (AL-2), R^{L3} and R^{L4} represent each independently a hydrogen atom or a hydrocarbyl group having 1 to 20 carbon atoms, and may contain a heteroatom, such as an oxygen atom, a sulfur atom, a nitrogen atom, and a fluorine atom. The hydrocarbyl group may be saturated or unsaturated, and may be linear, branched, or cyclic. The hydrocarbyl group is preferably a saturated hydrocarbyl group having 1 to 20 carbon atoms. Further, any two of R^{L2}, R^{L3}, and R^{L4} may be bonded to each other to form a ring having 3 to 20 carbon atoms together with the carbon atom, or together with the carbon atom and the oxygen atom, each bonded thereto. As the ring, a ring having 4 to 16 carbon atoms is preferable, and an alicyclic ring is particularly preferable.

In formula (AL-3), R^{L5}, R^{L6} and R^{L7} are each independently a hydrocarbyl group having 1 to 20 carbon atoms, and may contain a heteroatom, such as an oxygen atom, a sulfur atom, a nitrogen atom, or a fluorine atom. The hydrocarbyl group may be saturated or unsaturated, and may be linear, branched, or cyclic. The hydrocarbyl group is preferably a saturated hydrocarbyl group having 1 to 20 carbon atoms. In addition, any two of R^{L5}, R^{L6} and R^{L7} may be bonded to each other to form a ring having 3 to 20 carbon atoms together with the carbon atoms to which they are bonded. As the ring, a ring having 4 to 16 carbon atoms is preferable, and an alicyclic ring is particularly preferable.

The base polymer may include a repeating unit "y" having a phenolic hydroxy group as an adhesive group. Specific examples of monomers providing the repeating unit "y" include those shown below but are not limited thereto. Incidentally, in the following formula, R^{B} represents the same as defined above.

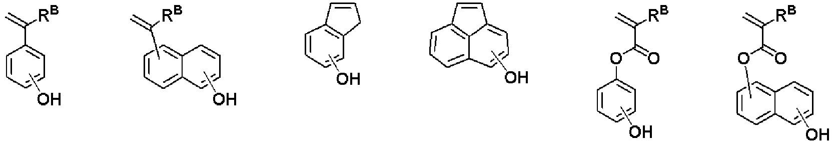

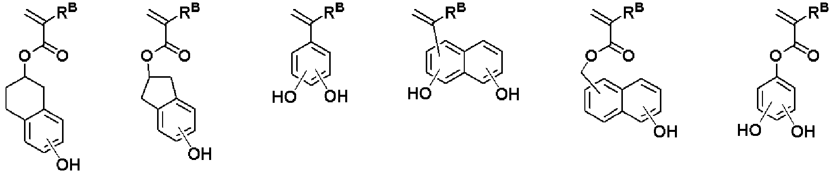

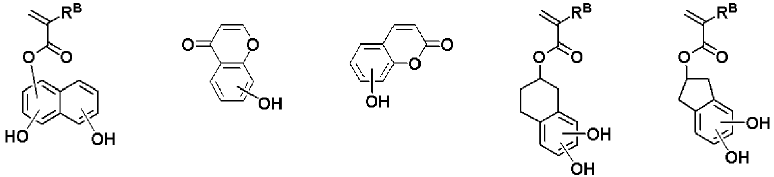

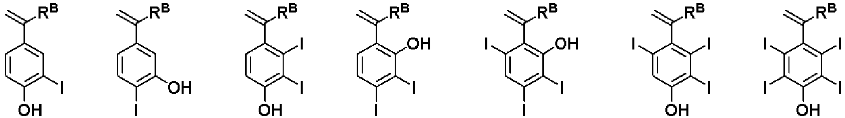

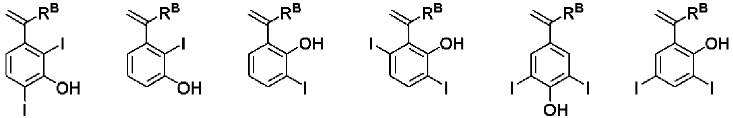

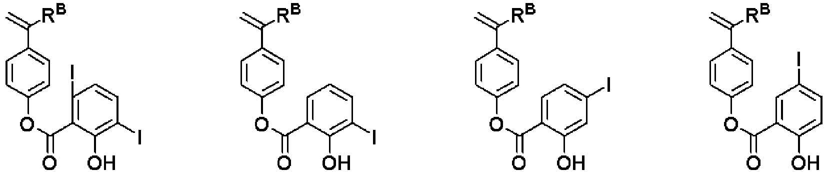

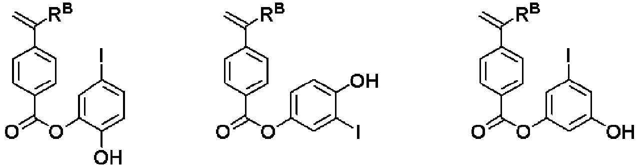

The base polymer may have a repeating unit "c" having a hydroxy group other than a phenolic hydroxy group, a lactone ring, a sultone ring, an ether bond, an ester bond, a sulfonic acid ester bond, a carbonyl group, a sulfonyl group, a cyano group, or a carboxy group as another adhesive group. Specific examples of monomers providing the repeating unit "c" include those shown below but are not limited thereto. Incidentally, in the following formulae, R^{B} is the same as defined above.

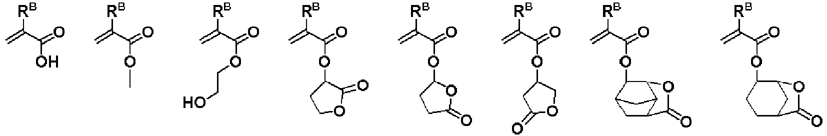

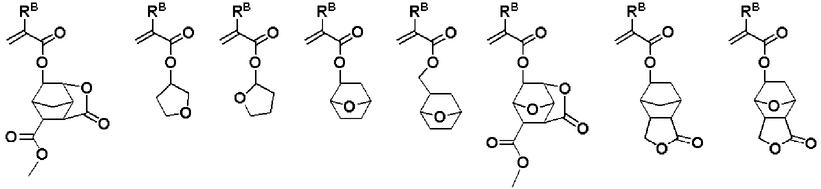

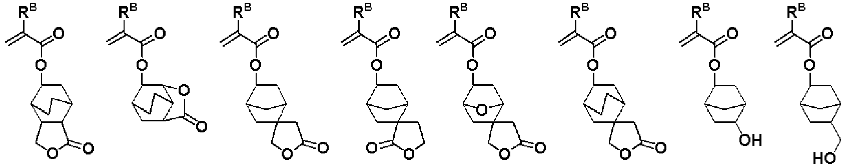

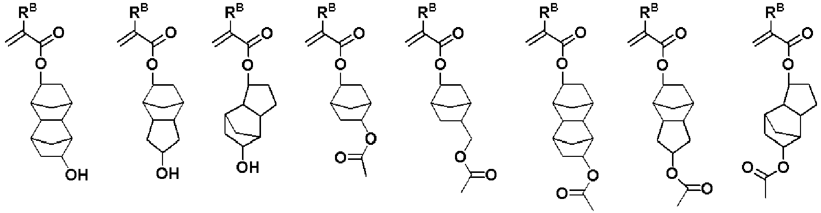

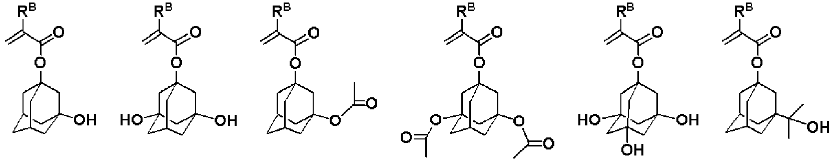

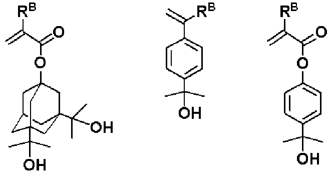

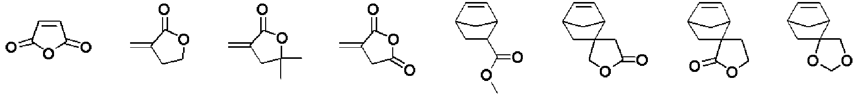

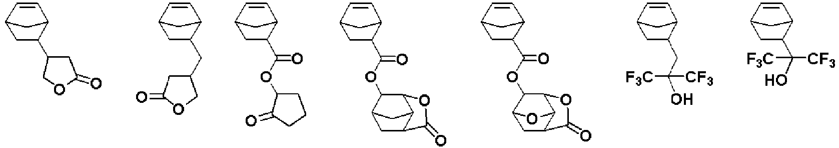

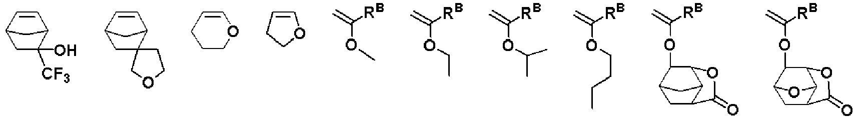

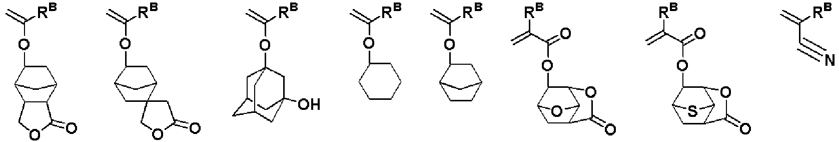

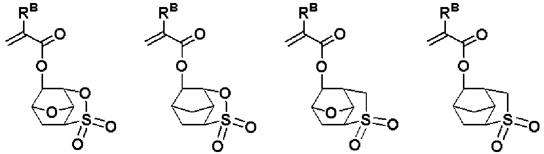

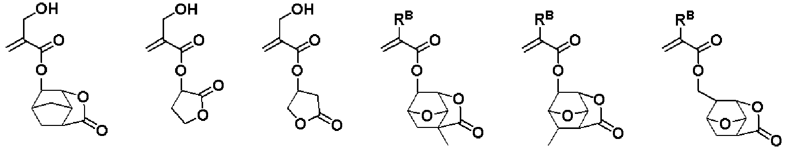

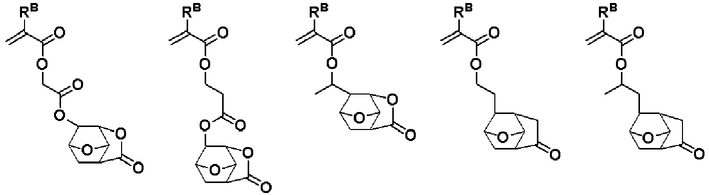

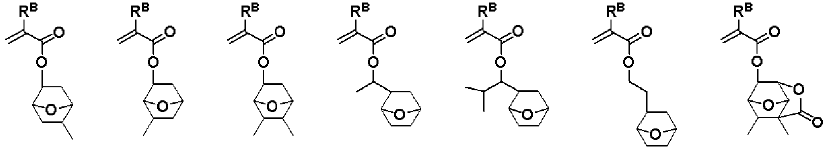

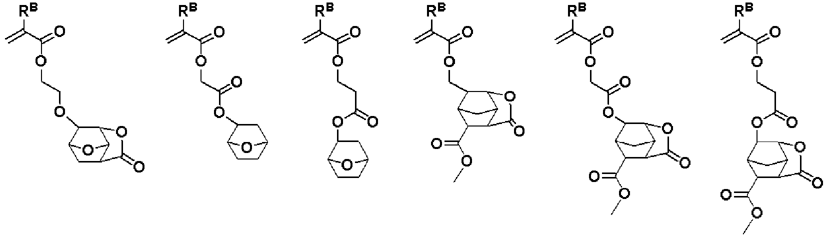

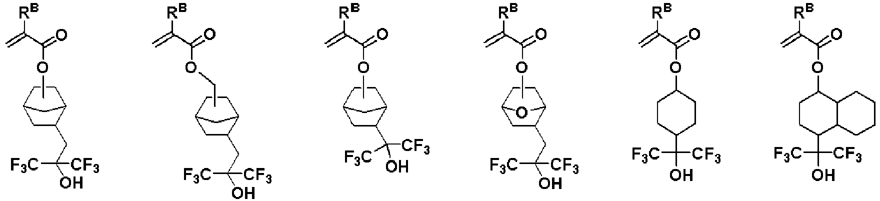

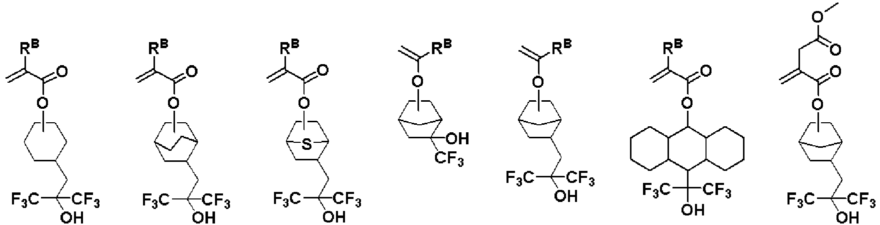

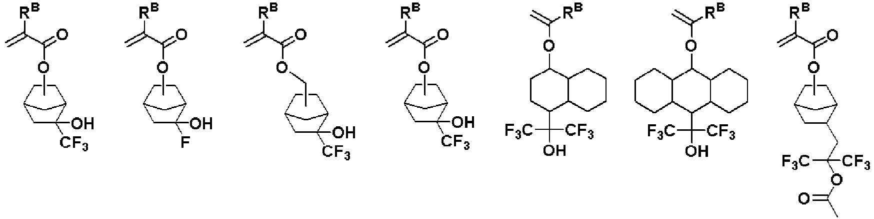

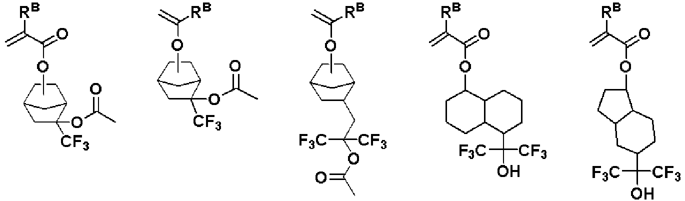

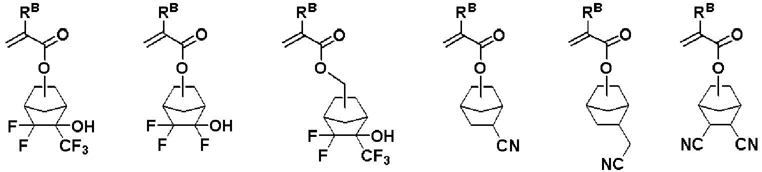

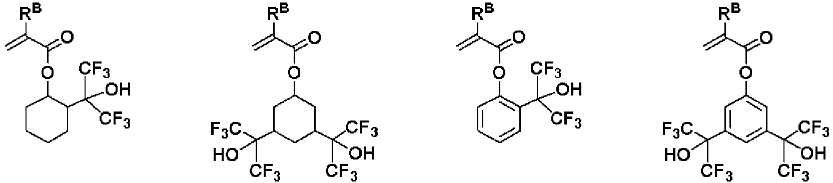

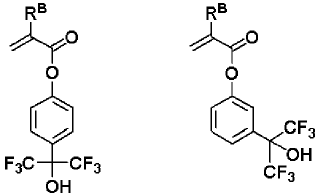

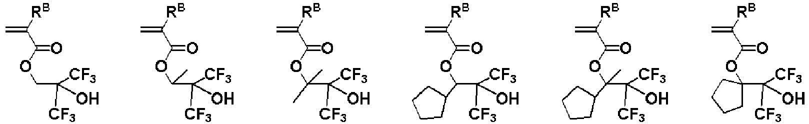

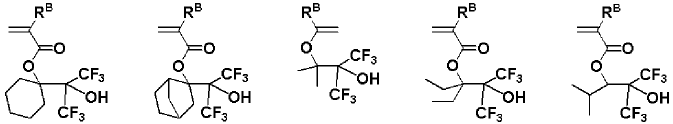

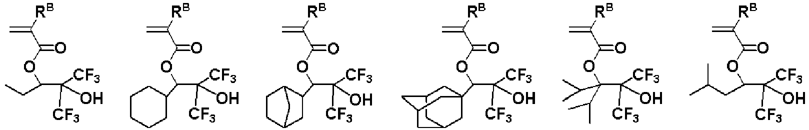

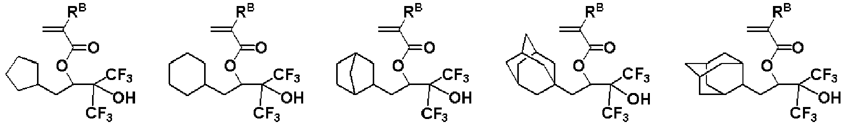

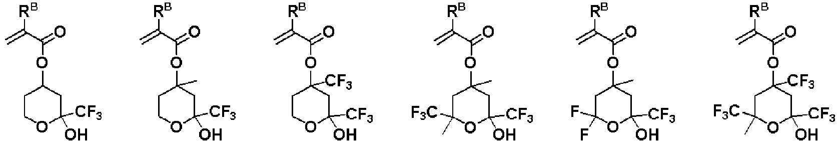

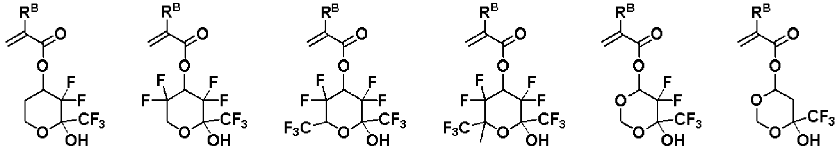

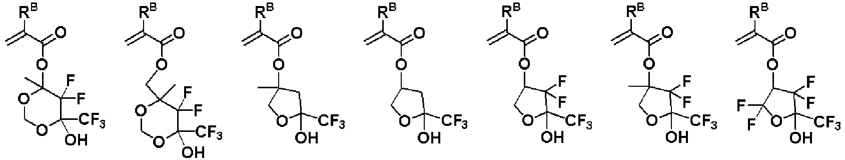

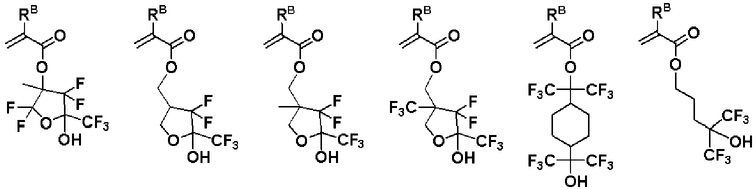

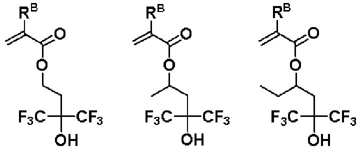

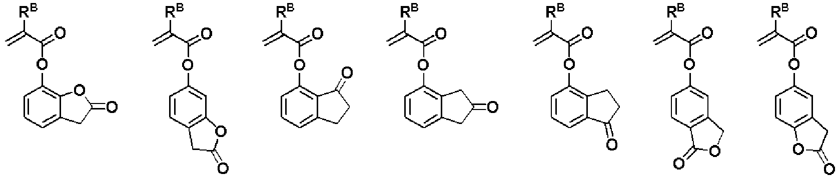

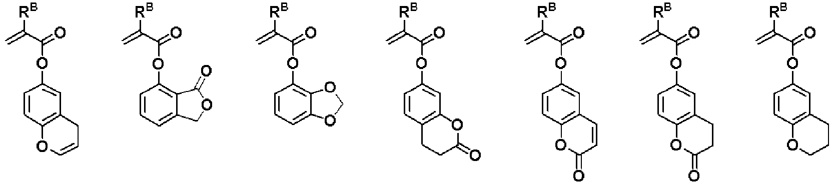

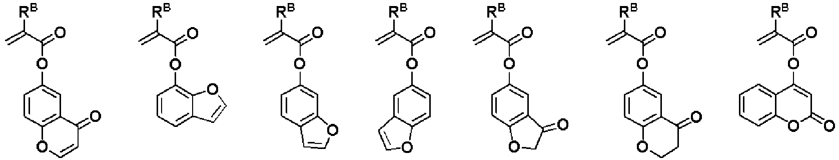

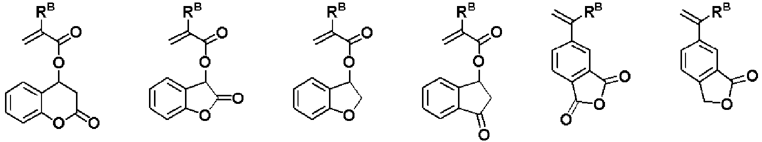

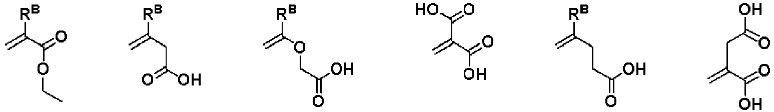

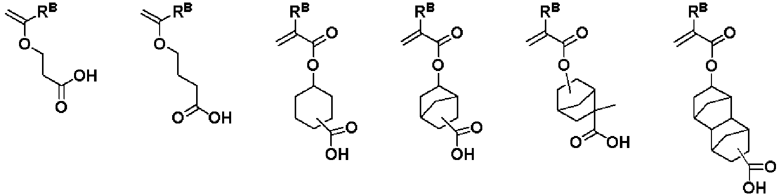

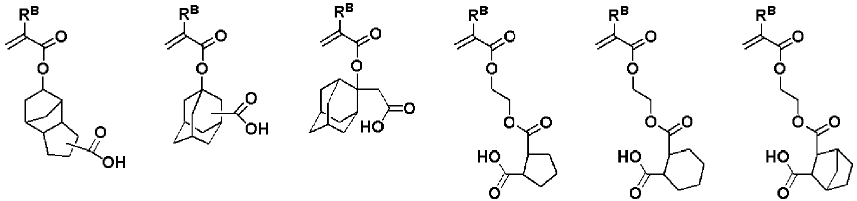

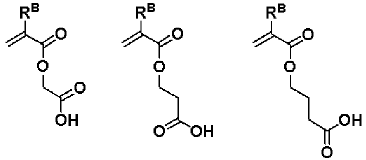

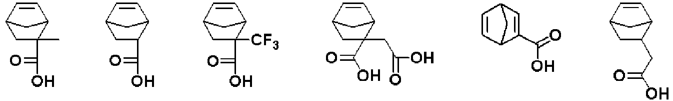

The base polymer may have a repeating unit "d" derived from indene, benzofuran, benzothiophene, acenaphthylene, chromone, coumarin, norbornadiene or a derivative thereof. Specific examples of monomers providing the repeating unit "d" include those shown below but are not limited thereto.

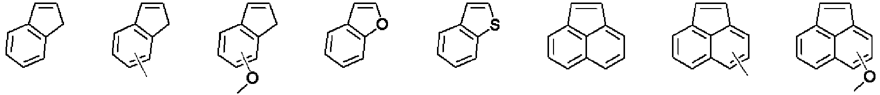

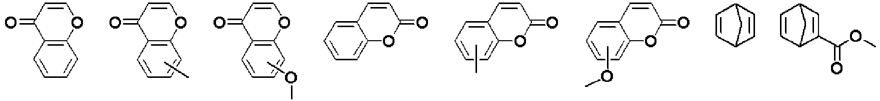

The base polymer may have a repeating unit "e" derived from styrene, vinylnaphthalene, vinylanthracene, vinylpyrene, methylene indane, vinylpyridine or vinylcarbazole.

The base polymer may have a repeating unit "f" derived from an onium salt having a polymerizable unsaturated bond. Specific examples of the preferable repeating unit "f" include a repeating unit represented by the following formula (f1) (hereinafter also referred to as repeating unit "f1"), a repeating unit represented by the following formula (f2) (hereinafter also referred to as repeating unit "f2"), and a repeating unit represented by the following formula (f3) (hereinafter also referred to as repeating unit "f3"). One kind of the repeating units "f1" to "f3" may be used or two or more kinds thereof may be used in combination.

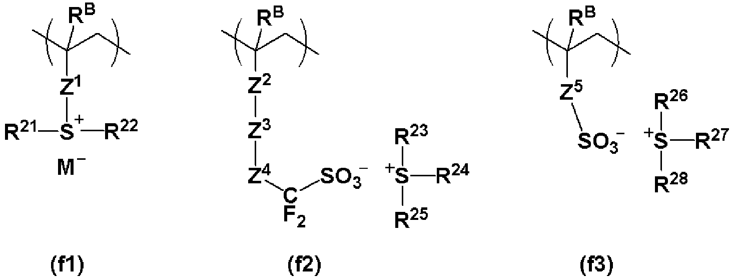

In formulae (f1) to (f3), R^{B} each independently represents a hydrogen atom or a methyl group; Z¹ represents a single bond, an aliphatic hydrocarbylene group having 1 to 6 carbon atoms, a phenylene group, a naphthylene group, or a group that is obtained by combining these and has 7 to 18 carbon atoms, -O-Z¹¹-, - C(=O)-O-Z¹¹-, or -C(=O)-NH-Z¹¹-; Z¹¹ is an aliphatic hydrocarbylene group having 1 to 6 carbon atoms, a phenylene group, a naphthylene group, or a group that is obtained by combining these and has 7 to 18 carbon atoms, and may contain a carbonyl group, an ester bond, an ether bond, or a hydroxy group; Z² is a single bond or an ester bond; Z³ is a single bond, -Z³¹-C(=O)-O-, -Z³¹-O-, or -Z³¹-O-C(=O)-; Z³¹ is an aliphatic hydrocarbylene group having 1 to 12 carbon atoms, a phenylene group, or a group that is obtained by combining these and has 7 to 18 carbon atoms, and may contain a carbonyl group, an ester bond, an ether bond, an iodine atom, or a bromine atom; Z⁴ is a methylene group, a 2,2,2-trifluoro-1,1-ethanediyl group or a carbonyl group; Z⁵ is a single bond, a methylene group, an ethylene group, a phenylene group, a fluorinated phenylene group, a phenylene group substituted with a trifluoromethyl group, -O-Z⁵¹-, - C(=O)-O-Z⁵¹-, or -C(=O)-NH-Z⁵¹-; Z⁵¹ is an aliphatic hydrocarbylene group having 1 to 6 carbon atoms, a phenylene group, a fluorinated phenylene group, or a phenylene group substituted with a trifluoromethyl group, and may contain a carbonyl group, an ester bond, an ether bond, a halogen atom, or a hydroxy group.

In formulae (f1) to (f3), R²¹ to R²⁸ each independently represent a halogen atom or a hydrocarbyl group that has 1 to 20 carbon atoms and may contain a hetero atom.

Specific examples of the halogen atom represented by R²¹ to R²⁸ include a fluorine atom, a chlorine atom, a bromine atom, and an iodine atom.

The hydrocarbyl group represented by R²¹ to R²⁸ may be saturated or unsaturated, and may be linear, branched, or cyclic. Specific examples of the hydrocarbyl group include: alkyl groups having 1 to 20 carbon atoms, such as a methyl group, an ethyl group, an n-propyl group, an isopropyl group, an n-butyl group, an isobutyl group, an sec-butyl group, a tert-butyl group, an n-pentyl group, an n-hexyl group, an n-octyl group, an n-nonyl group, an n-decyl group, an undecyl group, a dodecyl group, a tridecyl group, a tetradecyl group, a pentadecyl group, a heptadecyl group, an octadecyl group, a nonadecyl group, and an icosyl group; cyclic saturated hydrocarbyl groups having 3 to 20 carbon atoms, such as a cyclopropyl group, a cyclopentyl group, a cyclohexyl group, a cyclopropylmethyl group, a 4-methylcyclohexyl group, a cyclohexylmethyl group, a norbornyl group, and an adamantyl group; alkenyl groups having 2 to 20 carbon atoms, such as a vinyl group, a propenyl group, a butenyl group, and a hexenyl group; alkynyl groups having 2 to 20 carbon atoms, such as an ethynyl group, a propynyl group, and a butynyl group; cyclic unsaturated aliphatic hydrocarbyl groups having 3 to 20 carbon atoms, such as a cyclohexenyl group and a norbornenyl group; aryl groups having 6 to 20 carbon atoms, such as a phenyl group, a methylphenyl group, an ethylphenyl group, an n-propylphenyl group, an isopropylphenyl group, an n-butylphenyl group, an isobutylphenyl group, an sec-butylphenyl group, a tert-butylphenyl group, a naphthyl group, a methylnaphthyl group, an ethylnaphthyl group, an n-propylnaphthyl group, an isopropylnaphthyl group, an n-butylnaphthyl group, an isobutylnaphthyl group, an sec-butylnaphthyl group, and a tert-butylnaphthyl group; aralkyl groups having 7 to 20 carbon atoms, such as a benzyl group and a phenethyl group; and groups obtained by combination thereof.

In the hydrocarbyl group, a part or all of the hydrogen atoms may be substituted with a group having a heteroatom, such as an oxygen atom, a sulfur atom, a nitrogen atom, or a halogen atom, and a part of the -CH₂- may be substituted with a group having a heteroatom, such as an oxygen atom, a sulfur atom, or a nitrogen atom. The resulting hydrocarbyl group may have a hydroxy group, a fluorine atom, a chlorine atom, a bromine atom, an iodine atom, a cyano group, a nitro group, a carbonyl group, an ether bond, an ester bond, a sulfonic acid ester bond, a carbonate bond, a lactone ring, a sultone ring, a carboxylic anhydride (-C(=O)-O-C(=O)-), a haloalkyl group, etc.

In addition, R²³ and R²⁴ or R²⁶ and R²⁷ may be bonded to each other to form a ring together with a sulfur atom to which they are bonded. At this time, the ring preferably has the structure shown below.

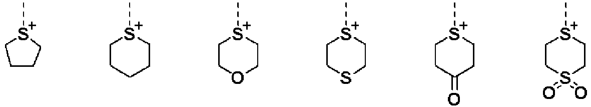

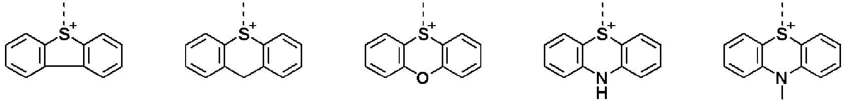

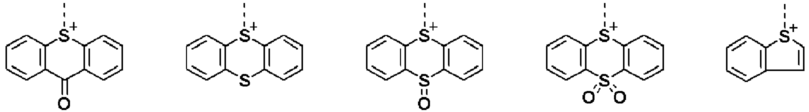

In the formulae, a broken line represents an attachment point.

In formula (f1), M⁻ represents a non-nucleophilic counter ion. Specific examples of the non-nucleophilic counter ions include: halide ions, such as a chloride ion and a bromide ion; fluoroalkylsulfonate ions, such as a triflate ion, a 1,1,1-trifluoroethanesulfonate ion, and a nonafluorobutanesulfonate ion; arylsulfonate ions, such as a tosylate ion, a benzenesulfonate ion, a **4-**fluorobenzenesulfonate ion, and 1,2,3,4,5-pentafluorobenzenesulfonate ion; alkylsulfonate ions, such as a mesylate ion and a butanesulfonate ion; imide ions, such as a bis(trifluoromethylsulfonyl)imide ion, a bis(perfluoroethylsulfonyl)imide ion, and a bis(perfluorobutylsulfonyl)imide ion; and methide ions, such as a tris(trifluoromethylsulfonyl)methide ion and a tris(perfluoroethylsulfonyl)methide ion.

Specific examples of the non-nucleophilic counter ion further include sulfonic acid ions represented by the following formula (f1-1) in which the α position is substituted with a fluorine atom, and sulfonic acid ions represented by the following formula (f1-2) in which the α position is substituted with a fluorine atom and the β position is substituted with a trifluoromethyl group.

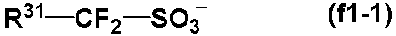

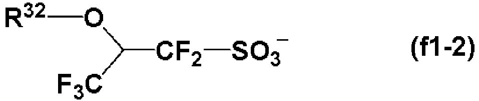

In formula (f1-1), R³¹ is a hydrogen atom or a hydrocarbyl group having 1 to 20 carbon atoms, and the hydrocarbyl group may have at least one selected from the group consisting of an ether bond, an ester bond, a carbonyl group, a lactone ring, and a fluorine atom. The hydrocarbyl group may be saturated or unsaturated, and may be linear, branched, or cyclic.

In formula (f1-2), R³² is a hydrogen atom, a hydrocarbyl group having 1 to 30 carbon atoms, or a hydrocarbyl carbonyl group having 6 to 20 carbon atoms, and the hydrocarbyl group and the hydrocarbyl carbonyl group may have an ether bond, an ester bond, a carbonyl group, or a lactone ring. The hydrocarbyl group and the hydrocarbyl moiety of the hydrocarbyl carbonyl group may be saturated or unsaturated, and may be linear, branched, or cyclic.

Specific examples of the cation of the monomer that provides the repeating unit f1 include those shown below but are not limited thereto. In addition, in the following formula, R^{B} is the same as defined above.

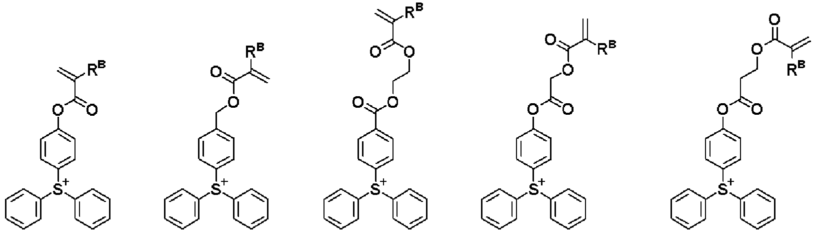

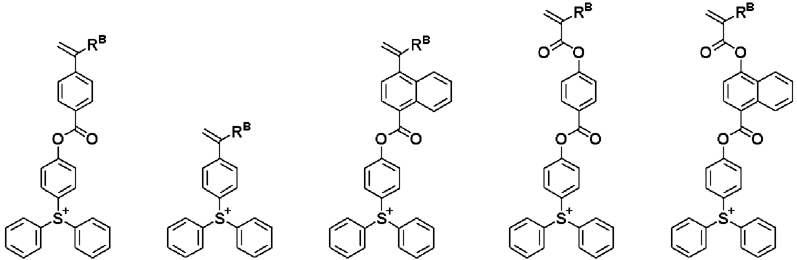

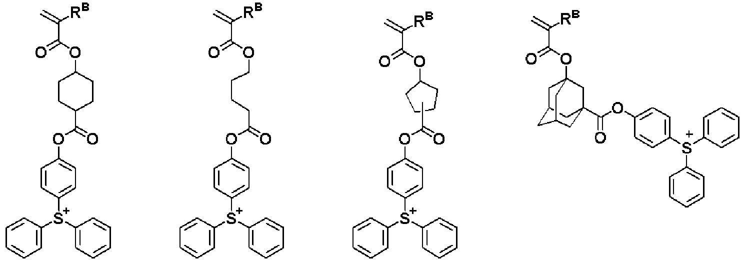

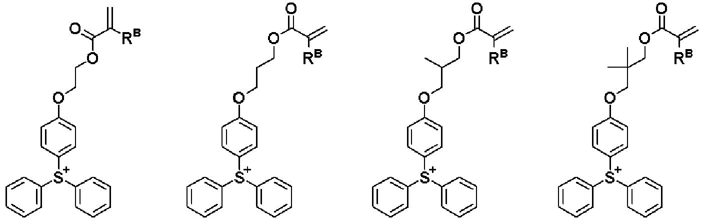

Examples of the anion of the monomer that provides the repeating unit f2 include those shown below but are not limited thereto. In addition, in the following formula, R^{B} is the same as defined above.

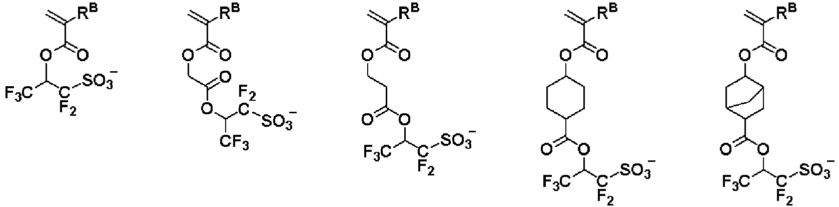

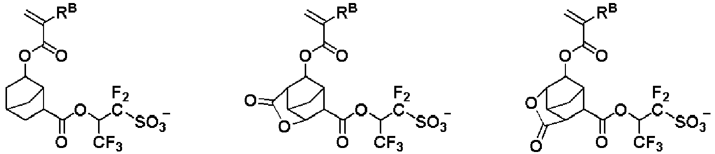

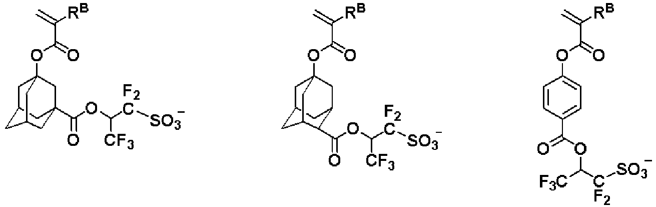

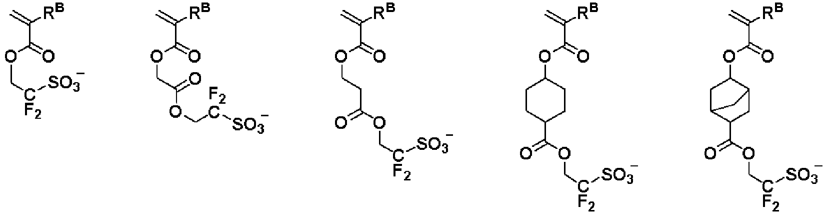

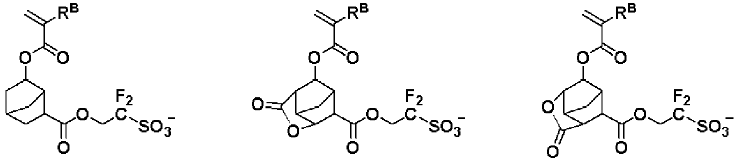

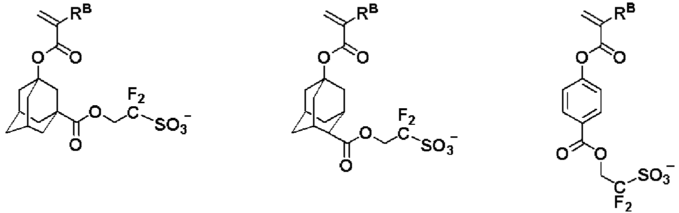

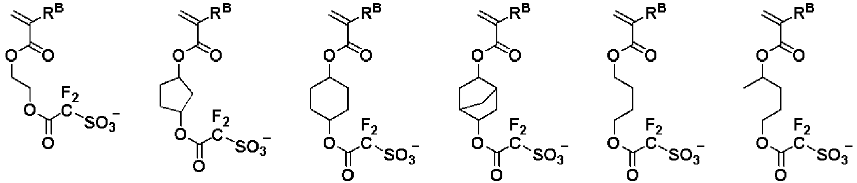

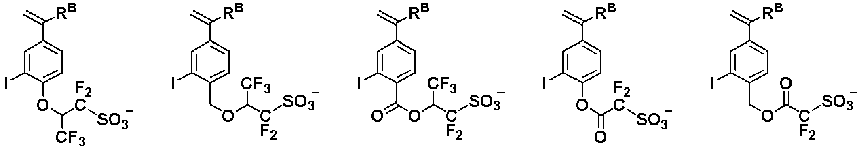

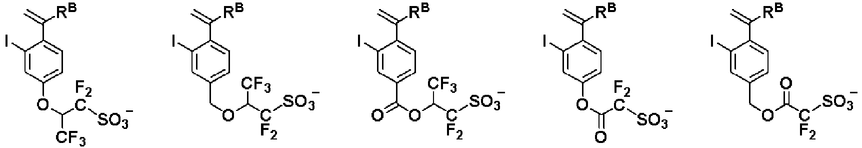

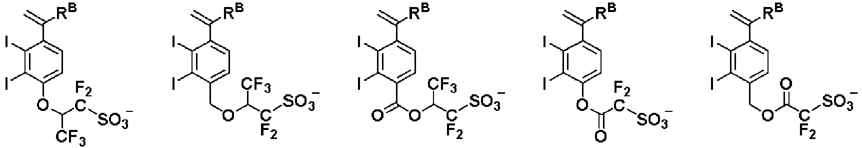

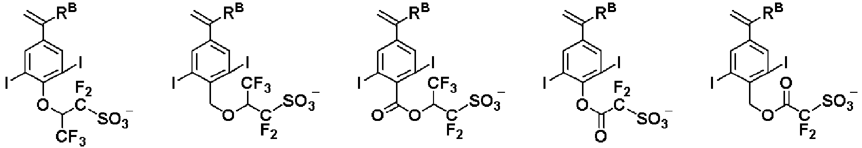

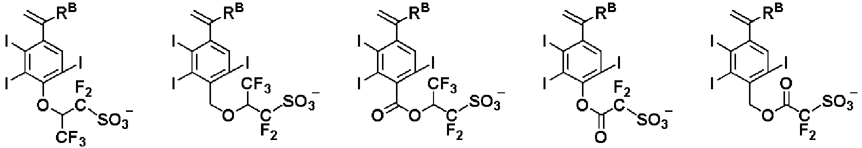

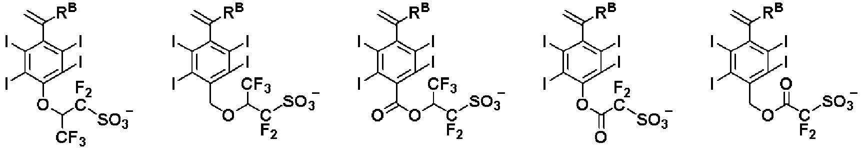

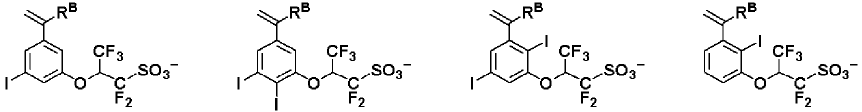

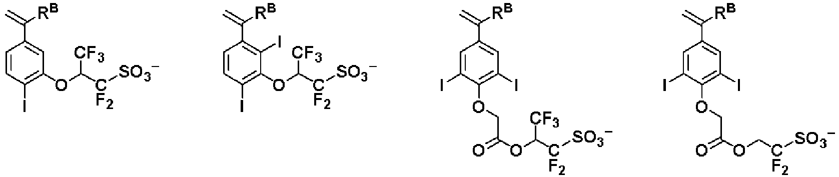

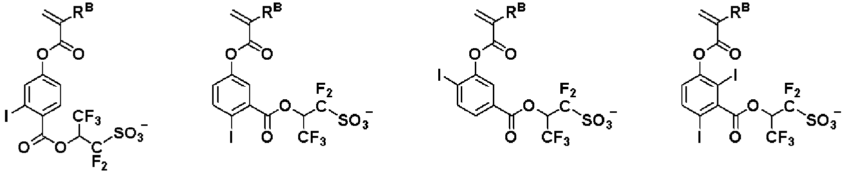

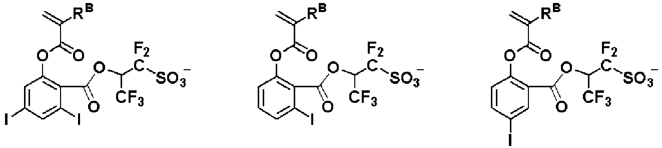

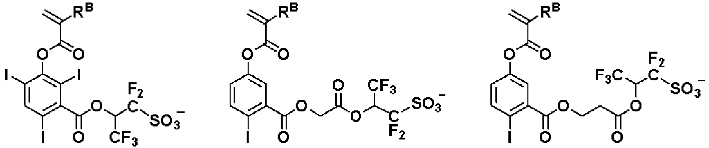

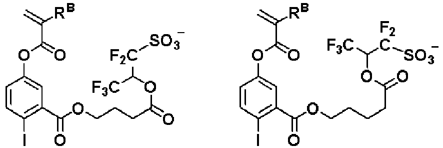

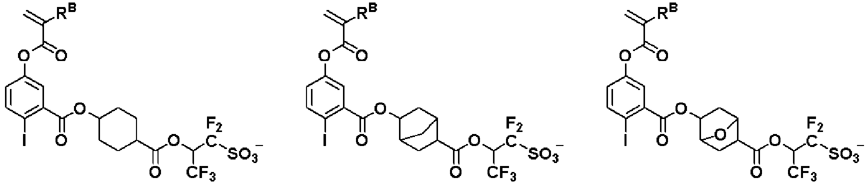

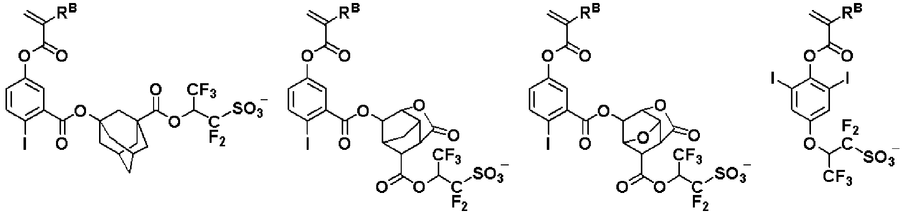

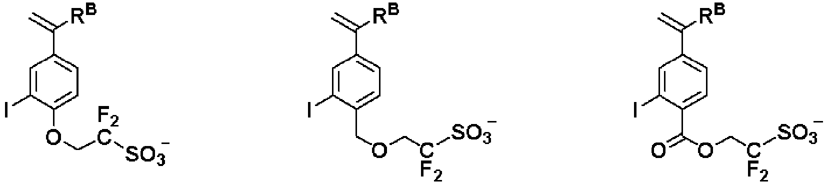

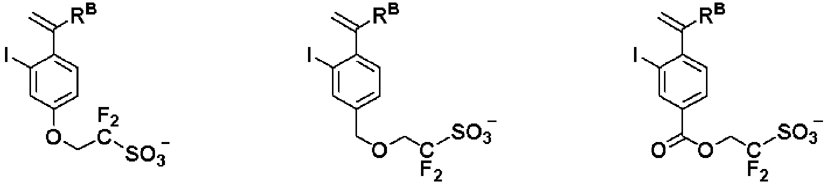

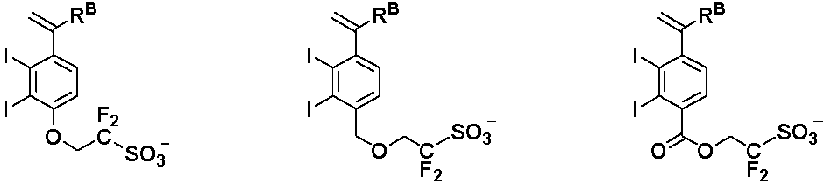

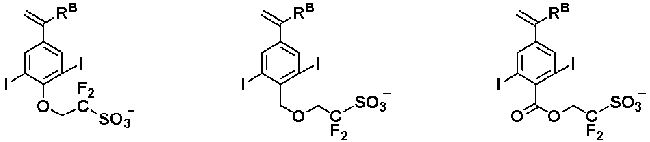

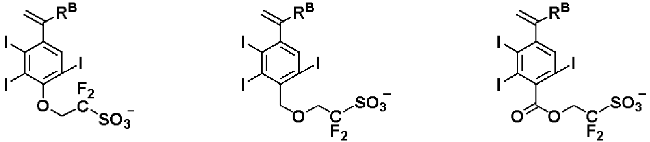

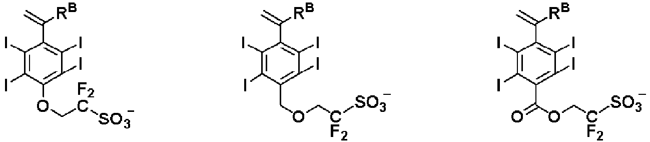

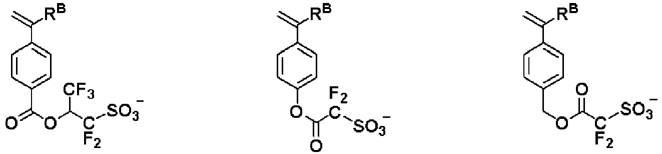

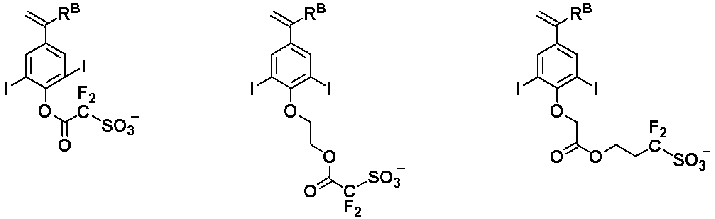

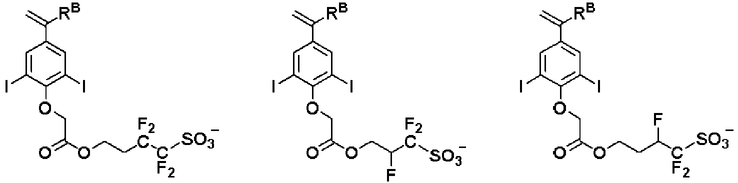

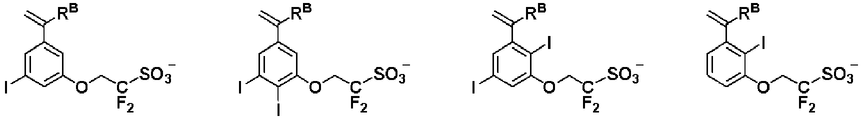

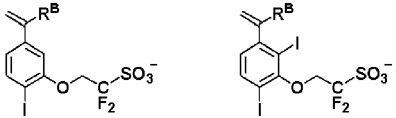

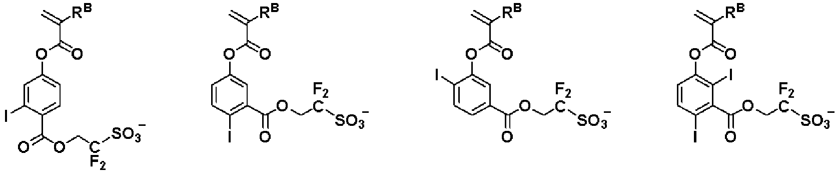

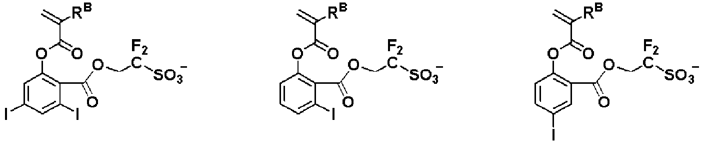

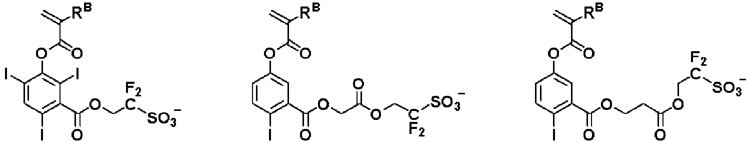

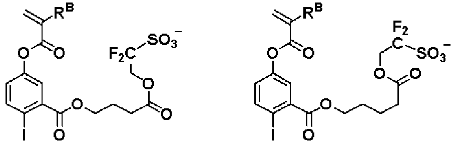

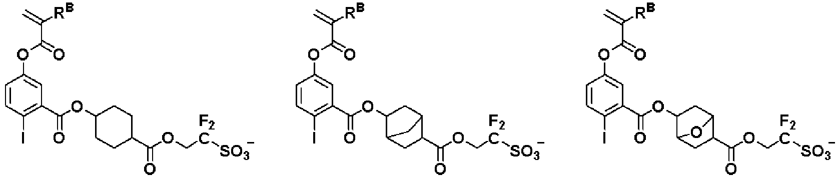

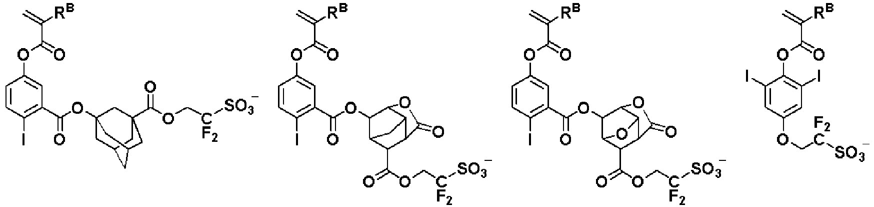

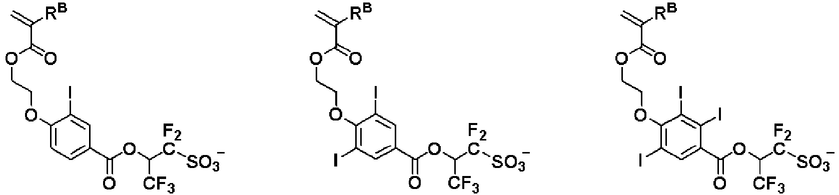

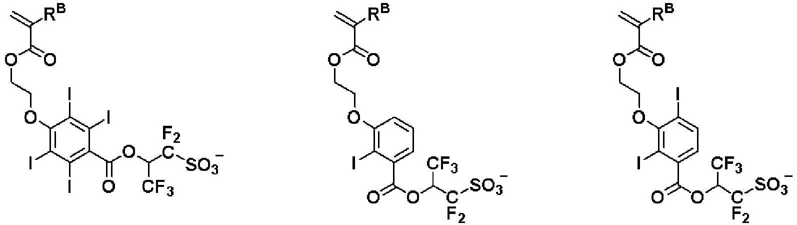

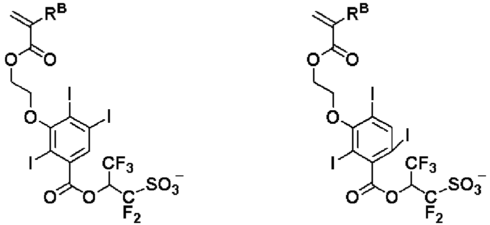

Examples of the anion of the monomer that provides the repeating unit f3 include those shown below but are not limited thereto. Incidentally, in the following formula, R^{B} is the same as defined above.

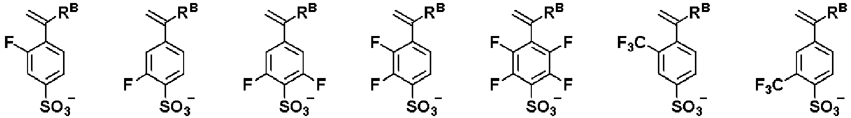

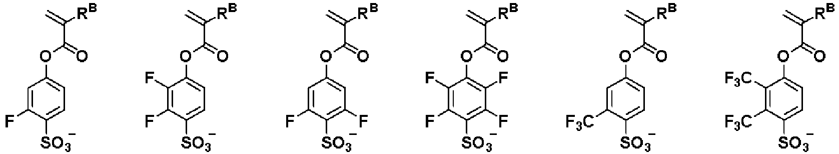

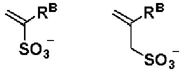

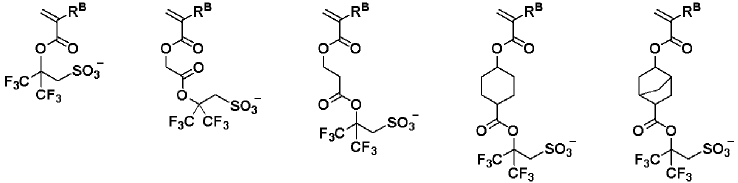

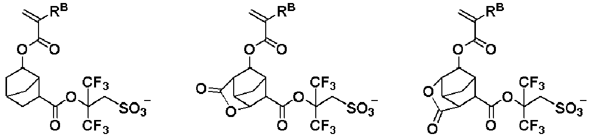

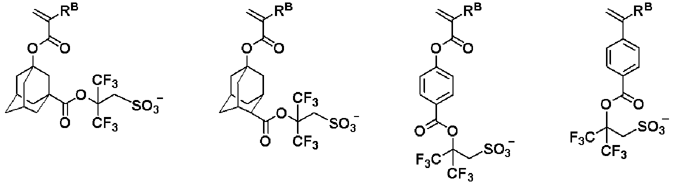

Specific examples of the cation of the repeating unit f2 or f3 include those similar to the sulfonium salts described in paragraphs [0063] to [0080] of JP2023-3926A and the iodonium salts described in paragraph [0082] of JP2023-3926A.

The repeating units f1 to f3 have the function as an acid generator. By binding an acid generator to the polymer main chain, acid diffusion can be reduced and resolution can be prevented from deteriorating due to blurring caused by acid diffusion. Further, LWR and CDU are improved by uniformly dispersing the acid generator. Incidentally, when a base polymer having repeating units f1 to f3 is used, the addition of an additive acid generator, which will be described later, may be omitted.

In the case of a negative-type resist material or a non-chemically-amplified resist material, the repeating units "x1" and "x2" are not necessarily required in the base polymer.

As the base polymer, in addition to the above-mentioned polymers, it is possible to use resins, such as novolak resin, polyimide, polyamide, polyvinylpyrrolidone, polyvinyl alcohol, polythiophene, and polyaniline.

### [Organic Solvent]

When the inventive composition for forming an organic film is used as a resist material, it is possible to contain an organic solvent. The organic solvent is not particularly limited as long as the organic solvent is capable of dissolving the components that were described above and will be described later. Examples of the organic solvent include those disclosed in paragraphs [0144] and [0145] of JP2008-111103A: ketones, such as cyclohexanone, cyclopentanone, methyl-2-n-pentyl ketone, and 2-heptanone; alcohols, such as 3-methoxybutanol, 3-methyl-3-methoxybutanol, 1-methoxy-2-propanol, 1-ethoxy-2-propanol, and diacetone alcohol; ethers, such as propylene glycol monomethyl ether, ethylene glycol monomethyl ether, propylene glycol monoethyl ether, ethylene glycol monoethyl ether, propylene glycol dimethyl ether, diethylene glycol dimethyl ether, and anisole; esters, such as propylene glycol monomethyl ether acetate, propylene glycol monoethyl ether acetate, ethyl lactate, ethyl pyruvate, butyl acetate, methyl 3-methoxypropionate, ethyl 3-ethoxypropionate, tert-butyl acetate, tert-butyl propionate, and propylene glycol mono-tert-butyl ether acetate; lactones, such as γ-butyrolactone; lactams, such as pyrrolidone, N-methylpyrrolidone, N-ethylpyrrolidone, and N-hydroxyethyl-2-pyrrolidone; water; and dimethyl sulfoxide.

In the inventive resist material, the contained amount of the organic solvent is preferably 100 to 10000 parts by mass, more preferably 200 to 8000 parts by mass relative to 100 parts by mass of the base polymer. One kind of the organic solvent may be used, or two or more kinds thereof may be used in mixture.

The inventive resist material may contain an additive-type acid generator. Examples of the additive-type acid generator include sulfonium salts or iodonium salts, sulfonium diazomethane, N-sulfonyloximide, and oxime-O-sulfonate described in paragraphs [0122] to [0142] of JP2008-111103A and paragraphs [0077] to [0148] of JP2023-62677A. When the inventive resist material contains the additive-type acid generator, its content is preferably 0.1 to 50 parts by mass, more preferably 1 to 40 parts by mass, relative to 100 parts by mass of the base polymer. One of the additive-type acid generators may be used or two or more kinds thereof may be used in combination.

The inventive resist material may contain a quencher. Incidentally, the quencher refers to a compound that can trap the acid generated from the acid generator in the resist material, thereby preventing it from diffusing into unexposed areas.

Examples of the quencher include conventional basic compounds, such as primary, secondary, and tertiary aliphatic amines, mixed amines, aromatic amines, heterocyclic amines, nitrogen-containing compounds having a carboxy group, nitrogen-containing compounds having a sulfonyl group, nitrogen-containing compounds having a hydroxy group, nitrogen-containing compounds having a hydroxyphenyl group, alcoholic nitrogen-containing compounds, amides, imides, and carbamates. Especially, preferable compounds are primary, secondary, and tertiary amine compounds described in paragraphs [0146] to [0164] of JP2008-111103A, particularly preferable compounds are amine compounds having a hydroxy group, an ether bond, an ester bond, a lactone ring, a cyano group, and a sulfonic acid ester bond or compounds having a carbamate group described in JP3790649A. By adding such a basic compound, it is possible to further suppress the acid diffusion rate in the resist film or to correct the shape, for example.

Further, examples of the quencher include onium salts such as sulfonium salts, iodonium salts, and ammonium salts with sulfonic acids and carboxylic acids whose α-positions are not fluorinated, which are described in JP2008-158339A. A sulfonic acid, imide acid, or methide acid fluorinated at the α-position is necessary to deprotect acid-labile group of the carboxylic acid ester, but a sulfonic acid or carboxylic acid that is not fluorinated at the α-position is released by salt exchange with an onium salt that is not fluorinated at the α-position. The Sulfonic acids and carboxylic acids that are not fluorinated at the α-position do not cause a deprotection reaction and therefore function as a quencher.

Examples of the quencher include polymer-type quenchers described in JP2008-239918A. This improves the rectangularity of the resist pattern by orienting it on the surface of the resist film. The polymer-type quenchers also have the effect of preventing film loss of a pattern and rounding of a pattern-top when a top coat for liquid-immersion exposure is applied.

When the inventive resist material contains the quencher, its content is preferably 0 to 5 parts by mass, more preferably 0 to 4 parts by mass, relative to 100 parts by mass of the base polymer. One of the quenchers may be used or two or more kinds thereof may be used in combination.

The inventive resist material may contain a crosslinking agent in the case of a negative resist. By incorporating a crosslinking agent, it is possible to further increase the difference in dissolution rate between the exposed areas and the unexposed areas, but there is also a risk of increased swelling in the developer. Specific examples of the crosslinking agent include those described in paragraphs [0170] to [0177] of JP2020-027297A. When the inventive resist material contains the crosslinking agent, its content is preferably 0 to 30 parts by mass, more preferably 0 to 20 parts by mass, relative to 100 parts by mass of the base polymer.

The resist material using the inventive composition for forming an organic film may contain a water repellency improver for improving the water repellency of the resist film surface. The water repellency improver can be used in liquid-immersion lithography without using a top coat.

The water repellency improver is preferably a polymer having a fluorinated alkyl group and a polymer having a 1,1,1,3,3,3-hexafluoro-2-propanol residue with a specific structure, and is more preferably those exemplified in JP2007-297590A, JP2008-111103A, etc. The water repellency improver needs to be dissolved in an alkaline developer or an organic solvent developer. The water repellency improver having the aforementioned specific 1,1,1,3,3,3-hexafluoro-2-propanol residue has good solubility in a developer. As a water repellency improver, a polymer having a repeating unit having an amino group or an amine salt prevents acid evaporation during post-exposure bake (PEB) and is highly effective in preventing opening defects of a hole pattern after development.

When the coating material or resist material for photolithography of the present invention contains the water repellency improver, its content is preferably 0 to 20 parts by mass, more preferably 0.5 to 10 parts by mass, relative to 100 parts by mass of the base polymer. One kind of the water repellency improvers may be used or two or more kinds thereof may be used in combination.

By adding the pentafluorosulfanyl group-containing resin material of the present invention to the resist composition, it is oriented on the resist film surface during spin coating, and the water repellency of the resist surface can be enhanced. This prevents the acid generated from the acid generator during the liquid-immersion exposure using water from dissolving into water, which causes a positive-type resist to have a **T-**top shape or a negative-type resist to have a rounded head, and can provide a highly water-repellent surface that does not leave water droplets on the resist film even during high-speed scanning of the stage during the liquid-immersion exposure. By introducing an acid-leaving unit that improves alkali solubility due to an acid in the inventive resin or a hydrolyzable unit of which hydrophilicity is improved by an alkali into the inventive resin, it is possible to further improve the resolution performance of the resist and prevent the occurrence of blob defects. For application as a water repellency improver for liquid-immersion exposure, the amount of the resin of the present invention to be added is 0.1 to 20 parts by mass, preferably 0.2 to 15 parts by mass relative to 100 parts by mass of the resin for a resist. It is possible to use both the inventive resin having a function as a surfactant and the inventive resin having a function as a water repellency improver for the liquid-immersion exposure in combination.

The surfactant to be contained when the inventive composition for forming an organic film is used as a resist material can also be used as the water repellency improver. In this case, in addition to a surfactant with a high water repellency improving function, a surfactant with a high surface-active effect can also be used in combination.

The inventive resist material may contain acetylene alcohols. Specific examples of the acetylene alcohols include those described in paragraphs [0179] to [0182] of JP2008-122932A. When the inventive resist material contains an acetylene alcohol, its content is preferably 0 to 5 parts by mass relative to 100 parts by mass of the base polymer. One kind of the acetylene alcohols may be used or two or more kinds thereof may be used in combination.

### [Patterning Process]

When the inventive resist material is used for manufacturing various integrated circuits, publicly known lithography techniques can be applied. For example, the patterning process includes a step of forming a resist film on a substrate by using the inventive composition for forming a film above, a step of exposing the resist film to a high-energy beam, and a step of developing the exposed resist film using a developer.

First, the inventive resist material is applied onto a substrate for manufacturing integrated circuits (Si, SiO₂, SiN, SiON, TiN, WSi, BPSG, SOG, organic antireflective film, etc.) or a substrate for manufacturing mask circuit (Cr, CrO, CrON, CrN, MoSi₂, SiO₂, Ta, MoSi laminated film, Ru, Ni, Co, W, Mo, V, alloys thereof, etc.) by an appropriate applying method, such as spin coating, roll coating, flow coating, dip coating, spray coating, doctor coating. This is prebaked on a hot plate, preferably at 60 to 150°C for 10 seconds to 30 minutes, more preferably at 80 to 150°C for 30 seconds to 20 minutes, to form a resist film.

Before applying the inventive resist material, the substrate can be treated by irradiating with light, EB, plasma, etc., or treated with CVD, ozone, hexamethyldisilazane (HMDS), etc.

The thickness of the resist film is preferably 1 nm to 200 µm, more preferably 2 nm to 100 µm.

A top coat may also be formed on the resist film. The top coat preferably has functions, such as environmental protection, light absorption, antistatic, reduction of resist pattern defect, and correction of resist pattern shape.

Then, the resist film is exposed using a high-energy beam. Specific examples of the high-energy beam include ultraviolet ray, deep ultraviolet ray, EB (electron beam), EUV having a wavelength of 3 to 15 nm, X-ray, soft X-ray, excimer laser beam, γ-ray, synchrotron radiation.

When ultraviolet ray, deep ultraviolet ray, EUV, X-ray, soft X-ray, excimer laser beam, γ-ray, synchrotron radiation, or the like is employed as the high-energy beam, the irradiation is performed directly or using a mask for forming a target pattern at an exposure dose of preferably about 1 to 200 mJ/cm², more preferably about 10 to 100 mJ/cm². When EB is employed as the high-energy beam, the exposure dose is preferably about 0.1 to 1000 µC/cm², more preferably about 0.5 to 900 µC/cm², and the writing is performed directly or while using a mask for forming a target pattern.

Incidentally, the inventive resist material is suitable for fine patterning with a g-line, an i-line, a KrF excimer laser beam, an ArF excimer laser beam, an EB, an EUV, X-ray, soft X-ray, γ-ray, or synchrotron radiation among the high-energy beams.

The exposure may be followed by post-exposure baking (PEB) on a hot plate, preferably at 40 to 150°C for 10 seconds to 30 minutes, more preferably 50 to 120°C for 30 seconds to 20 minutes.

After exposure or PEB, a target pattern is formed on the substrate by developing with a developer for 3 seconds to 3 minutes, preferably 5 seconds to 2 minutes, according to a conventional method, such as a dip method, a puddle method, and a spray method.

The developer may be an organic solvent developer or an alkaline aqueous developer.

Examples of the organic solvent developer include 2-octanone, 2-nonanone, 2-heptanone, 3-heptanone, 4-heptanone, 2-hexanone, 3-hexanone, diethyl ketone, methyl ethyl ketone, methyl isobutyl ketone, cyclopentanone, cyclohexanone, diisobutyl ketone, methyl cyclohexanone, acetophenone, methyl acetophenone, propyl acetate, butyl acetate, isobutyl acetate, pentyl acetate, butenyl acetate, isopentyl acetate, propyl formate, butyl formate, isobutyl formate, pentyl formate, isopentyl formate, methyl valerate, methyl pentenoate, methyl crotonate, chloro Ethyl tonate, methyl propionate, ethyl propionate, ethyl 3-ethoxypropionate, methyl lactate, ethyl lactate, propyl lactate, butyl lactate, isobutyl lactate, pentyl lactate, isopentyl lactate, methyl 2-hydroxyisobutyrate, ethyl 2-hydroxyisobutyrate, methyl benzoate, ethyl benzoate, phenyl acetate, benzyl acetate, methyl phenylacetate, benzyl formate, phenylethyl formate, methyl 3-phenylpropionate, benzyl propionate, ethyl phenylacetate, 2-phenylethyl acetate, diethylene glycol dimethyl ether, ethylene glycol dimethyl ether, xylene, toluene, anisole, methanol, ethanol, isopropanol, n-butyl alcohol, isobutyl alcohol, acetic anhydride, acetic acid.

Specific examples of the alkaline aqueous developer include aqueous solutions of potassium hydroxide, sodium hydroxide, or the like; and aqueous alkaline solutions of tetramethylammonium hydroxide, tetraethylammonium hydroxide, tetrapropylammonium hydroxide, tetrabutylammonium hydroxide, choline hydroxide, or the like.

One kind of the developers may be used, or two or more kinds thereof may be mixed to be used.

Rinsing is performed at the end of development. The rinsing liquid is preferably a solvent that can dissolve in the developer when mixed therewith and does not dissolve the resist film. Solvents used preferably include alcohols having 3 to 10 carbon atoms, ether compounds having 8 to 12 carbon atoms, and solvents of alkanes, alkenes, alkynes, which have 6 to 12 carbon atoms, aromatic solvents,and pure water.

Specific examples of the alcohol having 3 to 10 carbon atoms include n-propyl alcohol, isopropyl alcohol, 1-butyl alcohol, 2-butyl alcohol, isobutyl alcohol, tert-butyl alcohol, 1-pentanol, 2-pentanol, 3-pentanol, tert-pentyl alcohol, neopentyl alcohol, 2-methyl-1-butanol, 3-methyl-1-butanol, 3-methyl-3-pentanol, cyclopentanol, 1-hexanol, 2-hexanol, 3-hexanol, 2,3-dimethyl-2-butanol, 3,3-dimethyl-1-butanol, 3,3-dimethyl-2-butanol, 2-ethyl-1-butanol, 2-methyl-1-pentanol, 2-methyl-2-pentanol, 2-methyl-3-pentanol, 3-methyl-1-pentanol, 3-methyl-2-pentanol, 3-methyl-3-pentanol, 4-methyl-1-pentanol, 4-methyl-2-pentanol, 4-methyl-3-pentanol, cyclohexanol, and 1-octanol.

Specific examples of the ether compound having 8 to 12 carbon atoms include di-n-butyl ether, diisobutyl ether, di-sec-butyl ether, di-n-pentyl ether, diisopentyl ether, di-sec-pentyl ether, di-tert-pentyl ether, and di-n-hexyl ether.

Specific examples of the alkane having 6 to 12 carbon atoms include hexane, heptane, octane, nonane, decane, undecane, dodecane, methylcyclopentane, dimethylcyclopentane, cyclohexane, methylcyclohexane, dimethylcyclohexane, cycloheptane, cyclooctane, and cyclononane. Specific examples of the alkene having 6 to 12 carbon atoms include hexene, heptene, octene, cyclohexene, methylcyclohexene, dimethylcyclohexene, cycloheptene, and cyclooctene. Specific examples of the alkyne having 6 to 12 carbon atoms include hexyne, heptyne, and octyne.

Examples of aromatic solvents include toluene, xylene, ethylbenzene, isopropylbenzene, tert-butylbenzene, mesitylene.

By rinsing, it is possible to reduce collapse of a resist pattern and occurrence of a defect. Meanwhile, the rinsing is not necessarily essential, and without rinsing it is possible to reduce the amount of solvent used.

It is also possible to shrink the hole pattern or the trench pattern after development using thermal flow, RELACS technique or DSA technique. A shrink agent is applied onto the hole pattern; crosslinking of the shrink agent occurs on the surface of the resist film due to diffusion of acid catalyst from the resist film during baking; and the shrink agent adheres to the side walls of the hole pattern. The baking temperature is preferably 70 to 180°C, more preferably 80 to 170°C, and the baking time is preferably 10 to 300 seconds to remove excess shrink agent and reduce the hole pattern.

### EXAMPLE

Hereinafter, the present invention will be further specifically described with reference to Synthesis Examples, Comparative Synthesis Examples, Examples, and Comparative Examples. However, the present invention is not limited thereto. Incidentally, the molecular weight was measured specifically in the following manner. Weight-average molecular weight (Mw) and number-average molecular weight (Mn) in terms of polystyrene were measured by gel permeation chromatography (GPC) using tetrahydrofuran as an eluent (solvent), and dispersity (Mw/Mn) was calculated therefrom.

### [Synthesis of Polymer (B1) to (B18)]

Monomers used to synthesize polymers (B1) to (B18) for Examples are shown below.

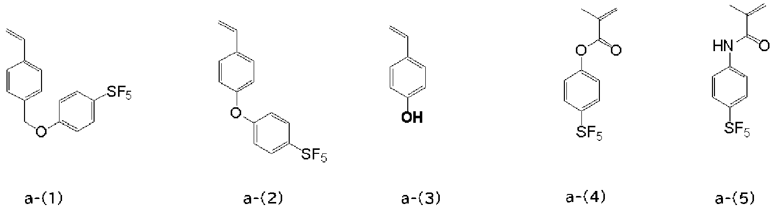

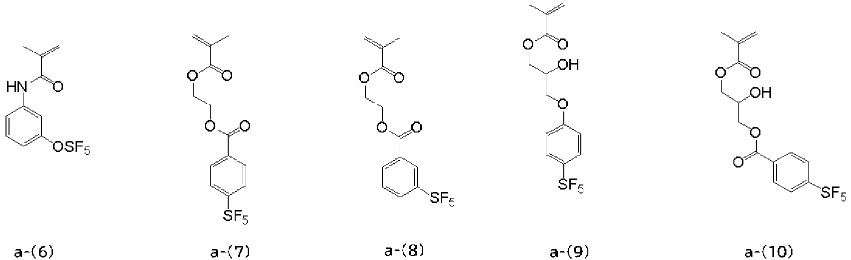

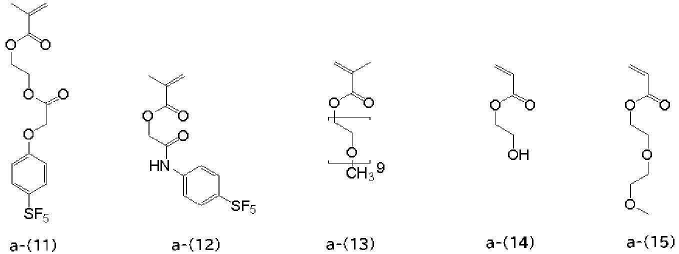

### [Synthesis Example 1] Synthesis of polymer (B1)

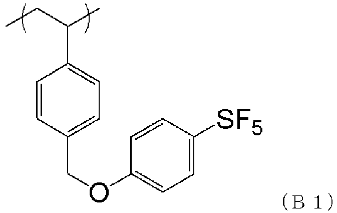

Under a nitrogen atmosphere, 1.0 g of propylene glycol monomethyl ether acetate (PGMEA) was heated and stirred at 80°C. A mixture of 5.04 g (15.0 mol) of a monomer a-(1), 0.173 g (0.75 mmol) of dimethyl 2,2-azobis(2-methylpropionate), and 15.0 g of PGMEA was added dropwise thereto over 4 hours. The mixture was further heated and stirred for 16 hours, and then cooled to room temperature to obtain a PGMEA solution of a target polymer (B1). As a result of analysis, the polymer (B1) had a weight-average molecular weight (Mw) of 12,900 and a dispersity (Mw/Mn) of 1.45.

### [Synthesis Example 2] Synthesis of polymer (B2)

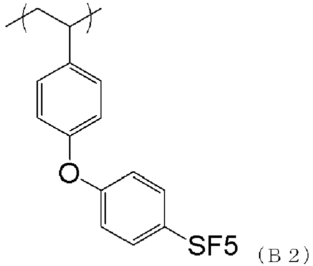

Under a nitrogen atmosphere, 10.0 g of PGMEA was heated and stirred at 80°C. A mixture of 4.83 g (15.0 mmol) of a monomer a-(2), 0.173 g (0.75 mmol) of dimethyl 2,2-azobis(2-methylpropionate), and 15.0 g of PGMEA was added dropwise thereto over 4 hours. The mixture was further heated and stirred for 16 hours, and then cooled to room temperature to obtain a PGMEA solution of a target polymer (B2). As a result of analysis, the polymer (B2) had a weight-average molecular weight (Mw) of 14200 and a dispersity (Mw/Mn) of 1.38.

### [Synthesis Example 3] Synthesis of polymer (B3)

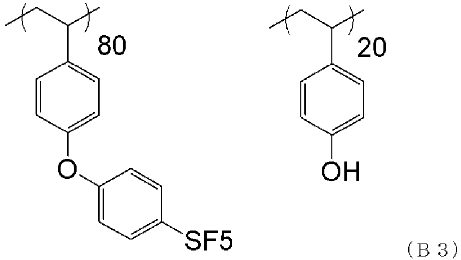

Under a nitrogen atmosphere, 10.0 g of PGMEA was heated and stirred at 80°C. A mixture of 3.87 g (12.0 mmol) of a monomer a-(2), 0.36 g (3.0 mmol) of a monomer a-(3), 0.173 g (0.75 mmol) of dimethyl 2,2-azobis(2-methylpropionate), and 15.0 g of PGMEA was added dropwise thereto over 4 hours. The mixture was further heated and stirred for 16 hours, and then cooled to room temperature to obtain a PGMEA solution of a target polymer (B3). As a result of analysis, the polymer (B3) had a weight-average molecular weight (Mw) of 12800 and a dispersity (Mw/Mn) of 1.58.

### [Synthesis Example 4] Synthesis of polymer (B4)

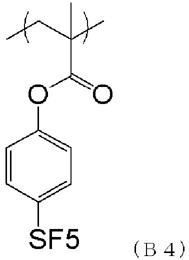

Under a nitrogen atmosphere, 10.0 g of PGMEA was heated and stirred at 80°C. A mixture of 4.32 g (15.0 mmol) of a monomer a-(4), 0.173 g (0.75 mmol) of dimethyl 2,2-azobis(2-methylpropionate), and 15.0 g of PGMEA was added dropwise thereto over 4 hours. The mixture was further heated and stirred for 16 hours, and then cooled to room temperature to obtain a PGMEA solution of a target polymer (B4). As a result of analysis, the polymer (B4) had a weight-average molecular weight (Mw) of 10800 and a dispersity (Mw/Mn) of 1.44.

### [Synthesis Example 5] Synthesis of polymer (B5)

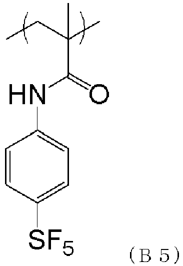

Under a nitrogen atmosphere, 10.0 g of PGMEA was heated and stirred at 80°C. A mixture of 4.31 g (15.0 mmol) of a monomer a-(5), 0.173 g (0.75 mmol) of dimethyl 2,2-azobis(2-methylpropionate), and 15.0 g of PGMEA was added dropwise thereto over 4 hours. The mixture was further heated and stirred for 16 hours and then cooled to room temperature to obtain a PGMEA solution of a target polymer (B5). As a result of analysis, the polymer (B5) had a weight-average molecular weight (Mw) of 12300 and a dispersity (Mw/Mn) of 1.34.

### [Synthesis Example 6] Synthesis of polymer (B6)

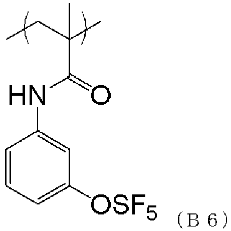

Under a nitrogen atmosphere, 10.0 g of PGMEA was heated and stirred at 80°C. A mixture of 4.55 g (15.0 mmol) of a monomer a-(6), 0.173 g (0.75 mmol) of dimethyl 2,2-azobis(2-methylpropionate), and 15.0 g of PGMEA was added dropwise thereto over 4 hours. The mixture was further heated and stirred for 16 hours and then cooled to room temperature to obtain a PGMEA solution of a target polymer (B6). As a result of analysis, the polymer (B6) had a weight-average molecular weight (Mw) of 11200 and a dispersity (Mw/Mn) of 1.41.

### [Synthesis Example 7] Synthesis of polymer (B7)

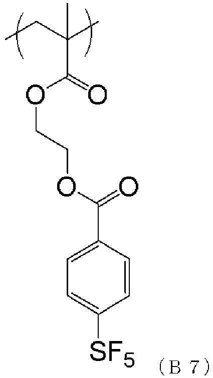

Under a nitrogen atmosphere, 10.0 g of PGMEA was heated and stirred at 80°C. A mixture of 5.40 g (15.0 mmol) of a monomer a-(7), 0.173 g (0.75 mmol) of dimethyl 2,2-azobis(2-methylpropionate), and 15.0 g of PGMEA was added dropwise thereto over 4 hours. The mixture was further heated and stirred for 16 hours and then cooled to room temperature to obtain a PGMEA solution of a target polymer (B7). As a result of analysis, the polymer (B7) had a weight-average molecular weight (Mw) of 16300 and a dispersity (Mw/Mn) of 1.56.

### [Synthesis Example 8] Synthesis of polymer (B8)

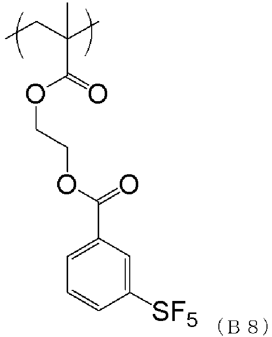

Under a nitrogen atmosphere, 10.0 g of PGMEA was heated and stirred at 80°C. A mixture of 5.40 g (15.0 mmol) of a monomer a-(8), 0.173 g (0.75 mmol) of dimethyl 2,2-azobis(2-methylpropionate), and 15.0 g of PGMEA was added dropwise thereto over 4 hours. The mixture was further heated and stirred for 16 hours and then cooled to room temperature to obtain a PGMEA solution of a target polymer (B8). As a result of analysis, the polymer (B8) had a weight-average molecular weight (Mw) of 14900 and a dispersity (Mw/Mn) of 1.54.

### [Synthesis Example 9] Synthesis of polymer (B9)

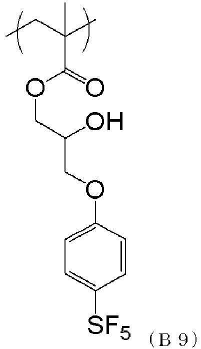

Under a nitrogen atmosphere, 10.0 g of PGMEA was heated and stirred at 80°C. A mixture of 5.44 g (15.0 mmol) of a monomer a-(9), 0.173 g (0.75 mmol) of dimethyl 2,2-azobis(2-methylpropionate), and 15.0 g of PGMEA was added dropwise thereto over 4 hours. The mixture was further heated and stirred for 16 hours and then cooled to room temperature to obtain a PGMEA solution of a target polymer (B9). As a result of analysis, the polymer (B9) had a weight-average molecular weight (Mw) of 17100 and a dispersity (Mw/Mn) of 1.42.

### [Synthesis Example 10] Synthesis of polymer (B10)

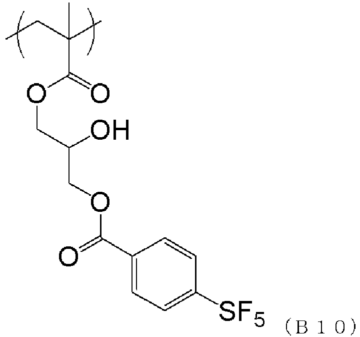

Under a nitrogen atmosphere, 10.0 g of PGMEA was heated and stirred at 80°C. A mixture of 5.85 g (15.0 mmol) of a monomer a-(B10), 0.173 g (0.75 mmol) of dimethyl 2,2-azobis(2-methylpropionate), and 15.0 g of PGMEA was added dropwise thereto over 4 hours. The mixture was further heated and stirred for 16 hours and then cooled to room temperature to obtain a PGMEA solution of a target polymer (B10). As a result of analysis, the polymer (B10) had a weight-average molecular weight (Mw) of 16100 and a dispersity (Mw/Mn) of 1.55.

### [Synthesis Example 11] Synthesis of polymer (B11)

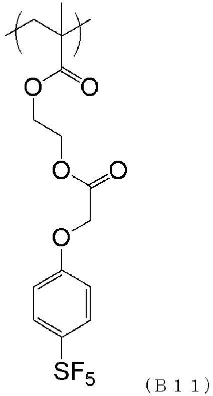

Under a nitrogen atmosphere, 10.0 g of PGMEA was heated and stirred at 80°C. A mixture of 5.85 g (15.0 mmol) of a monomer a-(11), 0.173 g (0.75 mmol) of dimethyl 2,2-azobis(2-methylpropionate), and 15.0 g of PGMEA was added dropwise thereto over 4 hours. The mixture was further heated and stirred for 16 hours and then cooled to room temperature to obtain a PGMEA solution of a target polymer (B11). As a result of analysis, the polymer (B11) had a weight-average molecular weight (Mw) of 18100 and a dispersity (Mw/Mn) of 1.65.

### [Synthesis Example 12] Synthesis of polymer (B12)

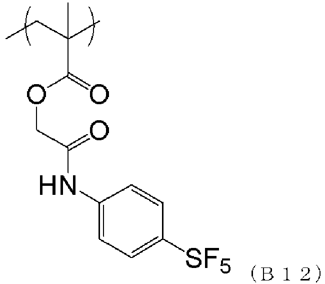

Under a nitrogen atmosphere, 10.0 g of PGMEA was heated and stirred at 80°C. A mixture of 5.18 g (15.0 mmol) of a monomer a-(12), 0.173 g (0.75 mmol) of dimethyl 2,2-azobis(2-methylpropionate), and 15.0 g of PGMEA was added dropwise thereto over 4 hours. The mixture was further heated and stirred for 16 hours and then cooled to room temperature to obtain a PGMEA solution of a target polymer (B12). As a result of analysis, the polymer (B12) had a weight-average molecular weight (Mw) of 16100 and a dispersity (Mw/Mn) of 1.55.

### [Synthesis Example 13] Synthesis of polymer (B13)

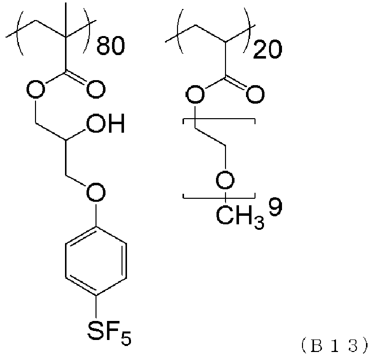

Under a nitrogen atmosphere, 10.0 g of PGMEA was heated and stirred at 80°C. A mixture of 4.35 g (12.0 mmol) of a monomer a-(9), 1.45 g (3.0 mmol) of a monomer a-(13), 0.173 g (0.75 mmol) of dimethyl 2,2-azobis(2-methylpropionate), and 15.0 g of PGMEA was added dropwise thereto over 4 hours. The mixture was further heated and stirred for 16 hours and then cooled to room temperature to obtain a PGMEA solution of a target polymer (B13). As a result of analysis, the polymer (B13) had a weight-average molecular weight (Mw) of 18800 and a dispersity (Mw/Mn) of 1.65.

### [Synthesis Example 14] Synthesis of polymer (B14)

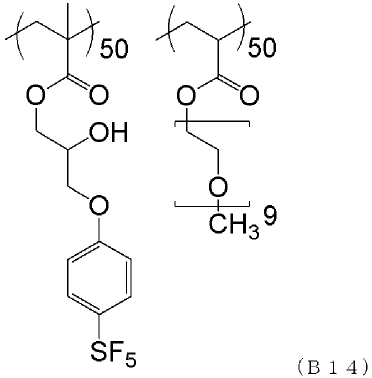

Under a nitrogen atmosphere, 10.0 g of PGMEA was heated and stirred at 80°C. A mixture of 2.72 g (7.5 mmol) of a monomer a-(9), 3.62 g (7.5 mmol) of a monomer a-(13), 0.173 g (0.75 mmol) of dimethyl 2,2-azobis(2-methylpropionate), and 15.0 g of PGMEA was added dropwise thereto over 4 hours. The mixture was further heated and stirred for 16 hours and then cooled to room temperature to obtain a PGMEA solution of a target polymer (B14). As a result of analysis, the polymer (B14) had a weight-average molecular weight (Mw) of 21200 and a dispersity (Mw/Mn) of 1.75.

### [Synthesis Example 15] Synthesis of polymer (B15)

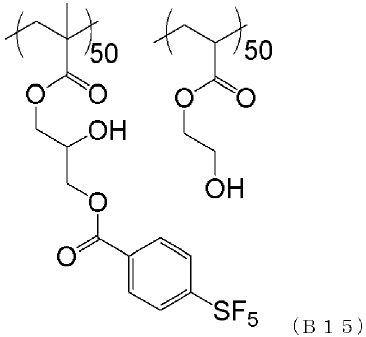

Under a nitrogen atmosphere, 10.0 g of PGMEA was heated and stirred at 80°C. A mixture of 2.92 g (7.5 mmol) of a monomer a-(10), 0.87 g (7.5 mmol) of a monomer a-(14), 0.173 g (0.75 mmol) of dimethyl 2,2-azobis(2-methylpropionate), and 15.0 g of PGMEA was added dropwise thereto over 4 hours. The mixture was further heated and stirred for 16 hours and then cooled to room temperature to obtain a PGMEA solution of a target polymer (B15). As a result of analysis, the polymer (B15) had a weight-average molecular weight (Mw) of 11200 and a dispersity (Mw/Mn) of 1.38.

### [Synthesis Example 16] Synthesis of polymer (B16)

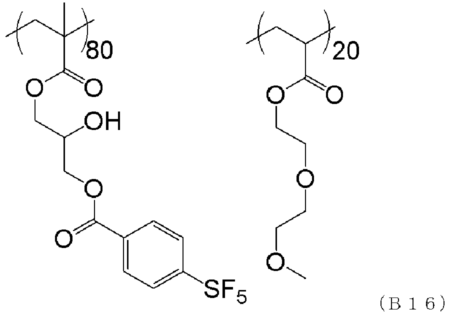

Under a nitrogen atmosphere, 10.0 g of PGMEA was heated and stirred at 80°C. A mixture of 4.68 g (12.0 mmol) of a monomer a-(10), 0.52 g (3.0 mmol) of a monomer a-(15), 0.173 g (0.75 mmol) of dimethyl 2,2-azobis(2-methylpropionate), and 15.0 g of PGMEA was added dropwise thereto over 4 hours. The mixture was further heated and stirred for 16 hours and then cooled to room temperature to obtain a PGMEA solution of a target polymer (B16). As a result of analysis, the polymer (B16) had a weight-average molecular weight (Mw) of 21200 and a dispersity (Mw/Mn) of 1.75.

### [Synthesis Example 17] Synthesis of polymer (B17)

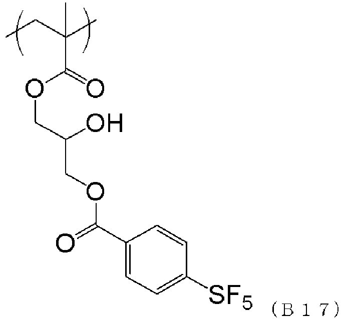

Under a nitrogen atmosphere, 10.0 g of PGMEA was heated and stirred at 80°C. A mixture of 5.85 g (15.0 mmol) of a monomer a-(10), 0.103 g (0.45 mmol) of dimethyl 2,2-azobis(2-methylpropionate), and 15.0 g of PGMEA was added dropwise thereto over 4 hours. The mixture was further heated and stirred for 16 hours and then cooled to room temperature to obtain a PGMEA solution of a target polymer (B17). As a result of analysis, the polymer (B17) had a weight-average molecular weight (Mw) of 28100 and a dispersity (Mw/Mn) of 1.89.

### [Synthesis Example 18] Synthesis of polymer (B18)

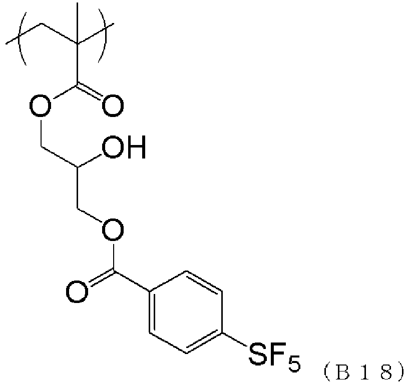

Under a nitrogen atmosphere, 10.0 g of PGMEA was heated and stirred at 80°C. A mixture of 5.85 g (15.0 mmol) of a monomer a-(10), 0.069 g (0.30 mmol) of dimethyl 2,2-azobis(2-methylpropionate), and 15.0 g of PGMEA was added dropwise thereto over 4 hours. The mixture was further heated and stirred for 16 hours and then cooled to room temperature to obtain a PGMEA solution of a target polymer (B18). As a result of analysis, the polymer (B18) had a weight-average molecular weight (Mw) of 32100 and a dispersity (Mw/Mn) of 2.03.

### [Synthesis of Polymer (B19) to (B29)]

Raw materials used to synthesize polymers (B19) to (B29) for Examples are shown below. Incidentally, formalin used in b-(9) was a 37% aqueous solution.

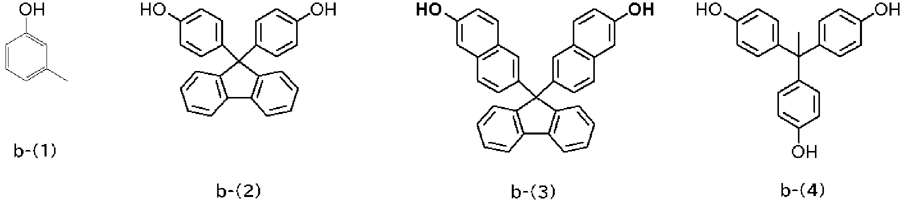

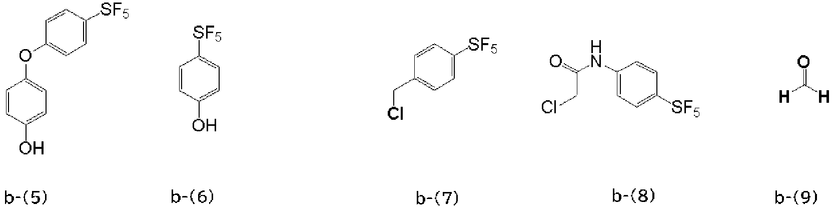

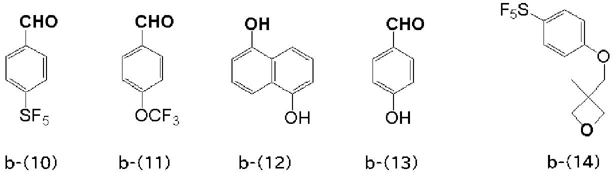

### [Synthesis Example 19] Synthesis of polymer

### (Intermediate 1)

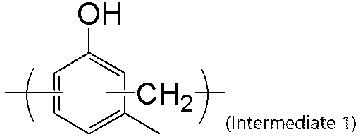

Under a nitrogen atmosphere, 216.8 g (2.00 mol) of a material b-(1), 146.0 g of a material b-(9) (corresponding to 1.80 mol as formaldehyde), 9.0 g of oxalic acid, and 100 g of dioxane were added and reacted for 24 hours at an internal temperature of 100°C. After the reaction was completed, the mixture was cooled to room temperature, 1000 ml of methyl isobutyl ketone (MIBK) was added, and washing was performed six times with 500 ml of pure water. The organic layer was collected, and the pressure was reduced to 2 mmHg at an internal temperature of 150°C, and moisture, solvent, and residual monomers were removed under reduced pressure, thereby obtaining a polymer (intermediate 1). As a result of analysis, the weight-average molecular weight (Mw) of the polymer (intermediate 1) was 8100, and the dispersity (Mw / Mn) was 3.51.

### [Synthesis Example 20] Synthesis of polymer (B19)

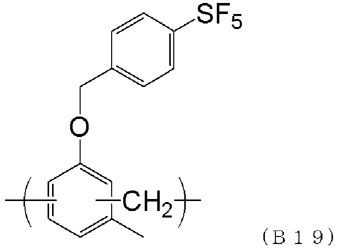

Under a nitrogen atmosphere, 10 g (corresponding to 83.2 mmol, in terms of a repeating unit) of a polymer (Intermediate 1), 13.80 g (99.9 mmol) of potassium carbonate, 100 g of DMF were added to prepare a uniform dispersion at an internal temperature 50°C. 25.23 g (99.9 mmol) of a material b-(7) was slowly added to the mixture, and the reaction was carried out at an internal temperature of 50°C for 24 hours. After adding 300 ml of MIBK and 300 g of pure water to the reaction solution to dissolve the precipitated salt, the separated aqueous layer was removed; 1500 g of hexane was added to the obtained organic layer; and the upper layer was removed. The lower layer precipitated like a rice cake was collected, redissolved in 300 ml of MIBK, and subjected to liquid-liquid separation and washing six times with 100 g of a 3% nitric acid aqueous solution and 100 g of pure water. And then, the resultant organic layer was evaporated to dryness under reduced pressure to obtain a polymer (B19). As a result of analysis, the weight-average molecular weight (Mw) of the polymer (B19) was 17400, and the dispersity (Mw / Mn) was 3.21.

### [Synthesis Example 21] Synthesis of polymer (B20)

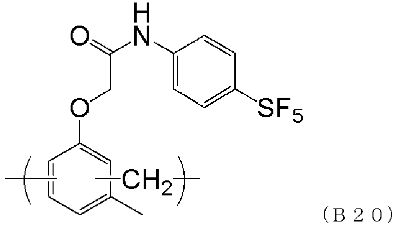

Under a nitrogen atmosphere, 10 g (corresponding to 83.2 mmol, in terms of a repeating unit) of a polymer (Intermediate 1), 13.80 g (99.9 mmol) of potassium carbonate, 100 g of DMF were added to prepare a uniform dispersion at an internal temperature 50°C. 29.53 g (99.9 mmol) of a material b-(8) was slowly added to the mixture, and the reaction was carried out at an internal temperature of 50°C for 24 hours. After adding 300 ml of MIBK and 300 g of pure water to the reaction solution to dissolve the precipitated salt, the separated aqueous layer was removed; 1500 g of hexane was added to the obtained organic layer; and the upper layer was removed. The lower layer precipitated like a rice cake was collected, redissolved in 300 ml of MIBK, and subjected to liquid-liquid separation and washing six times with 100 g of a 3% nitric acid aqueous solution and 100 g of pure water. And then, the resultant organic layer was evaporated to dryness under reduced pressure to obtain a polymer (B20). As a result of analysis, the weight-average molecular weight (Mw) of the polymer (B20) was 20400, and the dispersity (Mw / Mn) was 3.11. 5.

### [Synthesis Example 22] Synthesis of polymer (B21)

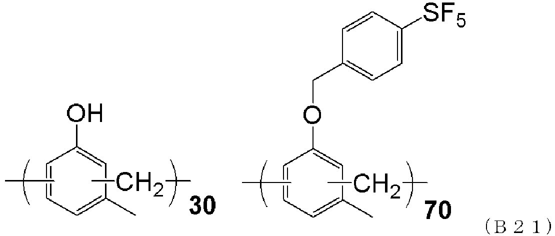

Under a nitrogen atmosphere, 10 g (corresponding to 83.2 mmol, in terms of a repeating unit) of a polymer (Intermediate 1), 11.50 g (83.2 mmol) of potassium carbonate, 100 g of DMF were added to prepare a uniform dispersion at an internal temperature 50°C. 14.72 g (58.3 mmol) of a material b-(7) was slowly added to the mixture, and the reaction was carried out at an internal temperature of 50°C for 24 hours. After adding 300 ml of MIBK and 300 g of pure water to the reaction solution to dissolve the precipitated salt, the separated aqueous layer was removed; 1500 g of hexane was added to the obtained organic layer; and the upper layer was removed. The lower layer precipitated like a rice cake was collected, redissolved in 300 ml of MIBK, and subjected to liquid-liquid separation and washing six times with 100 g of a 3% nitric acid aqueous solution and 100 g of pure water. And then, the resultant organic layer was evaporated to dryness under reduced pressure to obtain a polymer (B21). As a result of analysis, the weight-average molecular weight (Mw) of the polymer (B21) was 15400, and the dispersity (Mw / Mn) was 2.98.

### [Synthesis Example 23] Synthesis of polymer (B22)

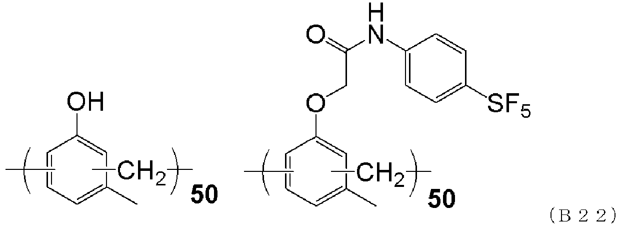

Under a nitrogen atmosphere, 10 g (corresponding to 83.2 mmol, in terms of a repeating unit) of a polymer (Intermediate 1), 11.50 g (83.2 mmol) of potassium carbonate, 100 g of DMF were added to prepare a uniform dispersion at an internal temperature 50°C. 12.30 g (41.6 mmol) of a material b-(8) was slowly added to the mixture, and the reaction was carried out at an internal temperature of 50°C for 24 hours. After adding 300 ml of MIBK and 300 g of pure water to the reaction solution to dissolve the precipitated salt, the separated aqueous layer was removed; 1500 g of hexane was added to the obtained organic layer; and the upper layer was removed. The lower layer precipitated like a rice cake was collected, redissolved in 300 ml of MIBK, and subjected to liquid-liquid separation and washing six times with 100 g of a 3% nitric acid aqueous solution and 100 g of pure water. And then, the resultant organic layer was evaporated to dryness under reduced pressure to obtain a polymer (B22). As a result of analysis, the weight-average molecular weight (Mw) of the polymer (B22) was 17900, and the dispersity (Mw / Mn) was 3.08.

### [Synthesis Example 24] Synthesis of polymer (B23)

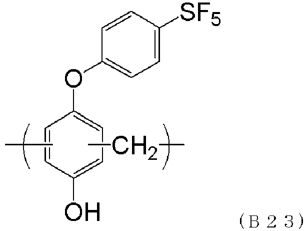

Under a nitrogen atmosphere, 5.00 g (16.0 mmol) of a material b-(5) and 1.17 g (corresponding to 14.4 mmol, in terms of formaldehyde) of a material b-(9), 0.5 g of oxalic acid, and 20 g of dioxane were added and reacted for 24 hours at an internal temperature of 100°C. After the reaction was completed, the mixture was cooled to room temperature, 60 g of hexane was added, and the upper layer was removed. The lower layer precipitated like a rice cake was collected, added with 100 ml of MIBK, and subjected to washing with 50 ml of pure water six times. The organic layer was collected, and the pressure was reduced to 2 mmHg at an internal temperature of 150°C, and moisture, solvent, and residual monomers were removed under reduced pressure, thereby obtaining a polymer (B23). As a result of analysis, the weight-average molecular weight (Mw) of the polymer (B23) was 10100, and the dispersity (Mw / Mn) was 2.91.

### [Synthesis Example 25] Synthesis of polymer (B24)

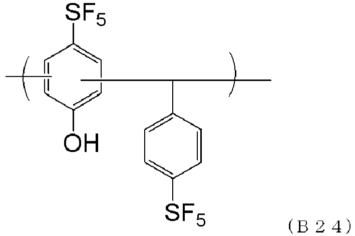

Under a nitrogen atmosphere, 5.00 g (22.7 mmol) of a material b-(6) and 1.17 g (20.4 mmol) of a material b-(10), 0.5 g of p-toluenesulfonic acid monohydrate, and 20 g of dioxane were added and reacted for 24 hours at an internal temperature of 100°C. After the reaction was completed, the mixture was cooled to room temperature, 60 g of hexane was added, and the upper layer was removed. The lower layer precipitated like a rice cake was collected, added with 100 ml of MIBK, and subjected to washing with 50 ml of pure water six times. The organic layer was collected, and the pressure was reduced to 2 mmHg at an internal temperature of 150°C, and moisture, solvent, and residual monomers were removed under reduced pressure, thereby obtaining a polymer (B24). As a result of analysis, the weight-average molecular weight (Mw) of the polymer (B24) was 6800, and the dispersity (Mw / Mn) was 2.12.

### [Synthesis Example 26] Synthesis of polymer (Intermediate 2)

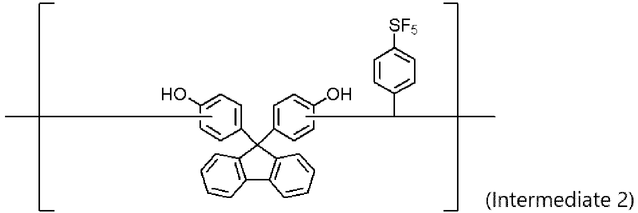

Under a nitrogen atmosphere, 5.00 g (14.3 mol) of a material b-(2), 2.98 g (12.8 mmol) of a material b-(10), 0.5 g of p-toluenesulfonic acid monohydrate, and 20 g of dioxane were added and reacted for 24 hours at an internal temperature of 100°C. After the reaction was completed, the mixture was cooled to room temperature, 60 g of hexane was added, and the upper layer was removed. The lower layer precipitated like a rice cake was collected, added with 100 ml of MIBK, and subjected to washing with 50 ml of pure water six times. The organic layer was collected, and the pressure was reduced to 2 mmHg at an internal temperature of 150°C, and moisture, solvent, and residual monomers were removed under reduced pressure, thereby obtaining a polymer (Intermediate 2). As a result of analysis, the weight-average molecular weight (Mw) of the polymer (Intermediate 2) was 3800, and the dispersity (Mw / Mn) was 2.52.

### [Synthesis Example 27] Synthesis of polymer (B25)

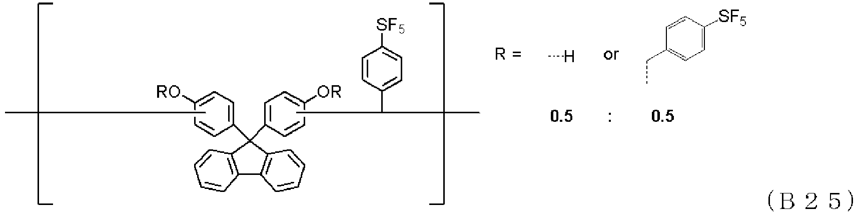

Under a nitrogen atmosphere, 5.0 g (corresponding to 8.86 mmol, in terms of a repeating unit) of a polymer (Intermediate 2), 2.45 g (17.7 mmol) of potassium carbonate, 30 g of DMF were added to prepare a uniform dispersion at an internal temperature 50°C. 2.24 g (8.86 mmol) of a material b-(7) was slowly added to the mixture, and the reaction was carried out at an internal temperature of 50°C for 24 hours. After adding 100 ml of MIBK and 100 g of pure water to the reaction solution to dissolve the precipitated salt, the separated aqueous layer was removed; 200 g of hexane was added to the obtained organic layer; and the upper layer was removed. The lower layer precipitated like a rice cake was collected, redissolved in 100 ml of MIBK, and subjected to liquid-liquid separation and washing six times with 50 g of a 3% nitric acid aqueous solution and 50 g of pure water. And then, the resultant organic layer was evaporated to dryness under reduced pressure to obtain a polymer (B25). As a result of analysis, the weight-average molecular weight (Mw) of the polymer (B25) was 5900, and the dispersity (Mw / Mn) was 2.58.

### [Synthesis Example 28] Synthesis of polymer (Intermediate 3)

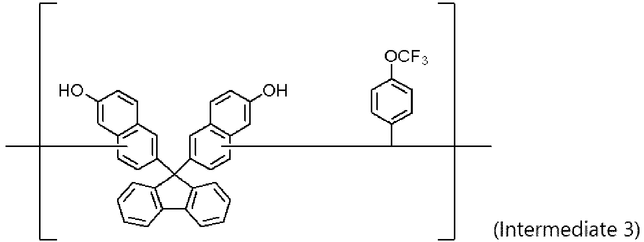

Under a nitrogen atmosphere, 5.00 g (11.1 mmol) of a material b-(3), 1.90 g (10.0 mmol) of a material b-(11), 0.5 g of p-toluenesulfonic acid monohydrate, and 20 g of dioxane were added and reacted for 24 hours at an internal temperature of 100°C. After the reaction was completed, the mixture was cooled to room temperature, 60 g of diisopropyl ether was added, and the upper layer was removed. The lower layer precipitated like a rice cake was collected, added with 100 ml of MIBK, and subjected to washing with 50 ml of pure water six times. The organic layer was collected, and the pressure was reduced to 2 mmHg at an internal temperature of 150°C, and moisture, solvent, and residual monomers were removed under reduced pressure, thereby obtaining a polymer (Intermediate 3). As a result of analysis, the weight-average molecular weight (Mw) of the polymer (Intermediate 3) was 4400, and the dispersity (Mw / Mn) was 2.34.

### [Synthesis Example 29] Synthesis of polymer (B26)

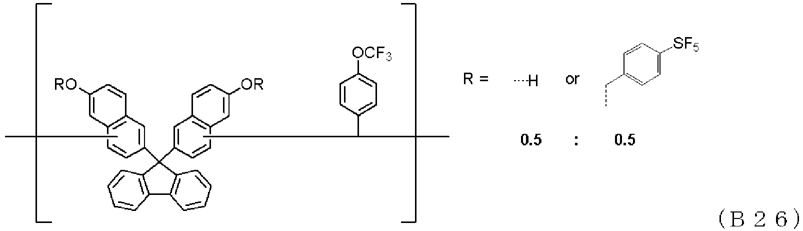

Under a nitrogen atmosphere, 5.0 g (corresponding to 8.03 mmol, in terms of a repeating unit) of a polymer (Intermediate 3), 2.22 g (16.1 mmol) of potassium carbonate, 30 g of DMF were added to prepare a uniform dispersion at an internal temperature 50°C. 2.03 g (8.03 mmol) of a material b-(7) was slowly added to the mixture, and the reaction was carried out at an internal temperature of 50°C for 24 hours. After adding 100 ml of MIBK and 100 g of pure water to the reaction solution to dissolve the precipitated salt, the separated aqueous layer was removed; 200 g of diisopropyl ether was added to the obtained organic layer; and the upper layer was removed. The lower layer precipitated like a rice cake was collected, redissolved in 100 ml of MIBK, and subjected to liquid-liquid separation and washing six times with 50 g of a 3% nitric acid aqueous solution and 50 g of pure water. And then, the resultant organic layer was evaporated to dryness under reduced pressure to obtain a polymer (B26). As a result of analysis, the weight-average molecular weight (Mw) of the polymer (B26) was 7300, and the dispersity (Mw / Mn) was 2.28.

### [Synthesis Example 30] Synthesis of polymer (Intermediate 4)

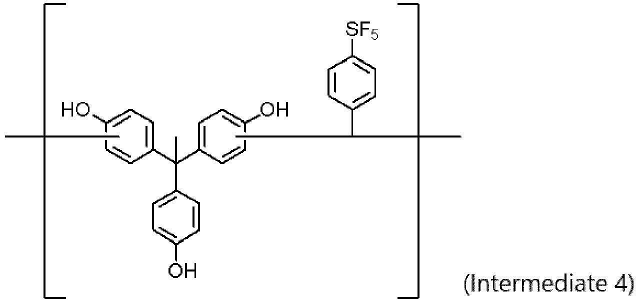

Under a nitrogen atmosphere, 5.00 g (16.3 mmol) of a material b-(4), 3.98 g (17.1 mmol) of a material b-(10), 4.70 g of methanesulfonic acid, 20 g of 1,2-dichloroethane were added and reacted at an internal temperature of 80°C for 24 hours. After the reaction was completed, the mixture was cooled to room temperature, 120 g of diisopropyl ether was added, and the upper layer was removed. The lower layer precipitated like a rice cake was collected, added with 100 ml of MIBK, and subjected to washing with 50 ml of pure water six times. The organic layer was collected, and the pressure was reduced to 2 mmHg at an internal temperature of 150°C, and moisture, solvent, and residual monomers were removed under reduced pressure, thereby obtaining a polymer (Intermediate 4). As a result of analysis, the polymer (Intermediate 4) had a weight-average molecular weight (Mw) of 7700 and a dispersity (Mw/Mn) of 3.64.

### [Synthesis Example 31] Synthesis of polymer (B27)

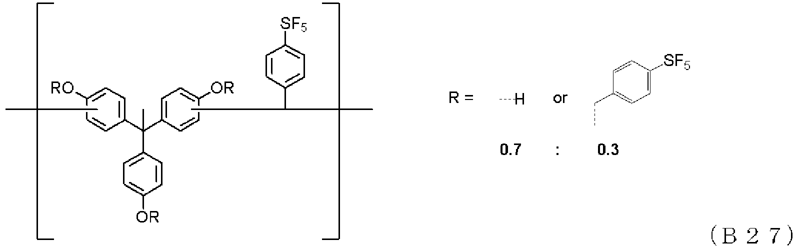

Under a nitrogen atmosphere, 5.0 g (corresponding to 9.60 mmol, in terms of a repeating unit) of a polymer (Intermediate 4), 3.98 g (28.8 mmol) of potassium carbonate, 30 g of DMF were added to prepare a uniform dispersion at an internal temperature 50°C. 2.18 g (8.65 mmol) of a material b-(7) was slowly added to the mixture, and the reaction was carried out at an internal temperature of 50°C for 24 hours. After adding 100 ml of MIBK and 100 g of pure water to the reaction solution to dissolve the precipitated salt, the separated aqueous layer was removed; 200 g of hexane was added to the obtained organic layer; and the upper layer was removed. The lower layer precipitated like a rice cake was collected, redissolved in 100 ml of MIBK, and subjected to liquid-liquid separation and washing six times with 50 g of a 3% nitric acid aqueous solution and 50 g of pure water. And then, the resultant organic layer was evaporated to dryness under reduced pressure to obtain a polymer (B27). As a result of analysis, the weight-average molecular weight (Mw) of the polymer (B27) was 9600, and the dispersity (Mw / Mn) was 3.48.

### [Synthesis Example 32] Synthesis of polymer (Intermediate 5)

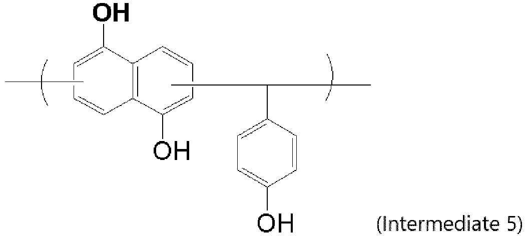

Under a nitrogen atmosphere, 5.00 g (31.2 mmol) of a material b-(12), 3.43 g (28.1 mmol) of a material b-(13), 0.50 g of p-toluenesulfonic acid monohydrate, and 30 g of propylene glycol monomethyl ether were added and reacted for 24 hours at an internal temperature of 120°C. After the reaction was completed, the mixture was cooled to room temperature, 80 g of diisopropyl ether was added, and the upper layer was removed. The lower layer precipitated like a rice cake was collected, added with 100 ml of MIBK, and subjected to washing with 50 ml of pure water six times. The organic layer was collected, and the pressure was reduced to 2 mmHg at an internal temperature of 150°C, and moisture, solvent, and residual monomers were removed under reduced pressure, thereby obtaining a polymer (Intermediate 5). As a result of analysis, the polymer (Intermediate 5) had a weight-average molecular weight (Mw) of 3700 and a dispersity (Mw/Mn) of 2.84.

### [Synthesis Example 33] Synthesis of polymer (B28)

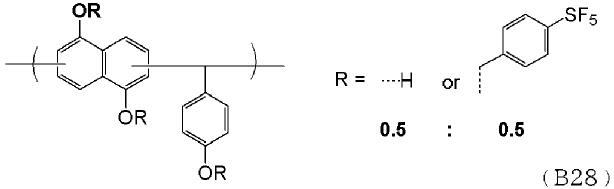

Under a nitrogen atmosphere, 5.0 g (corresponding to 18.92 mmol, in terms of a repeating unit) of a polymer (Intermediate 5), 7.84 g (56.8 mmol) of potassium carbonate, and 30 g of DMF were added to prepare a uniform dispersion at an internal temperature 50°C. 7.17 g (28.4 mmol) of a material b-(7) was slowly added to the mixture, and the reaction was carried out at an internal temperature of 50°C for 24 hours. After adding 100 ml of MIBK and 100 g of pure water to the reaction solution to dissolve the precipitated salt, the separated aqueous layer was removed; 200 g of hexane was added to the obtained organic layer; and the upper layer was removed. The lower layer precipitated like a rice cake was collected, redissolved in 100 ml of MIBK, and subjected to liquid-liquid separation and washing six times with 50 g of a 3% nitric acid aqueous solution and 50 g of pure water. And then, the resultant organic layer was evaporated to dryness under reduced pressure to obtain a polymer (B28). As a result of analysis, the weight-average molecular weight (Mw) of the polymer (B28) was 7300, and the dispersity (Mw / Mn) was 2.98.

### [Synthesis Example 34] Synthesis of polymer (B29)

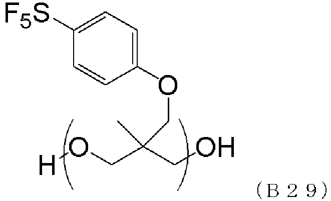

Under a nitrogen atmosphere, as an initiator, 0.142 g (13.69 mmol) of 2,2-dimethyl-1,3-propanediol, 0.192 g (13.69 mmol) of boron trifluoride tetrahydrofuran complex, and 30 g of methylene chloride were added to prepare a uniform solution in a water bath. 10.0 g (32.8 mmol) of a material b-(14) was slowly added dropwise to the solution. After completion of the adding dropwise, the solution was reacted at room temperature for 24 hours, and then the reaction was quenched by adding 30 g of 5% sodium bicarbonate solution in an ice bath. After removing the separated aqueous layer, washing with 30 g of pure water was performed 5 times, and the organic layer was evaporated to dryness under reduced pressure to obtain a polymer (B29). As a result of analysis, the weight-average molecular weight (Mw) of the polymer (B29) was 4300, and the dispersity (Mw / Mn) was 2.11.

### [Synthesis of Polymers (B30) to (B42)]

The monomers used in the synthesis of polymers (B30) to (B42) for Examples are shown below.

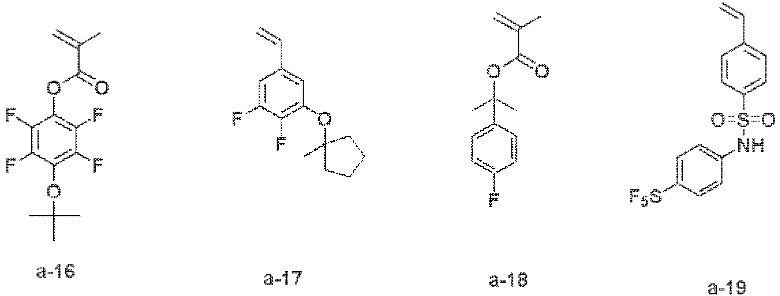

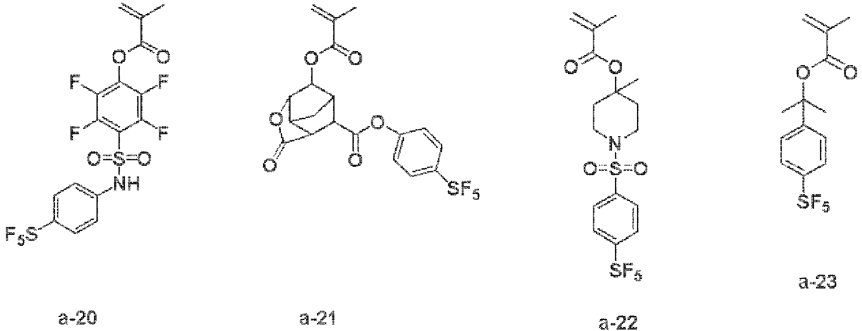

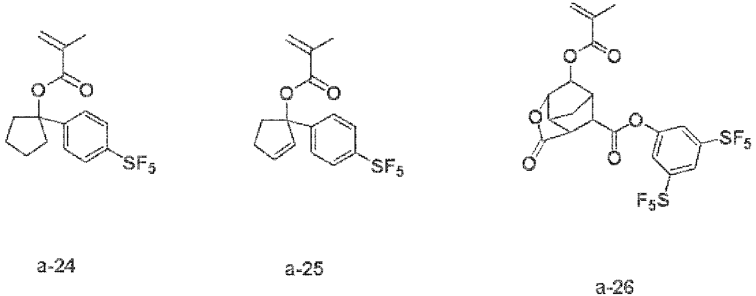

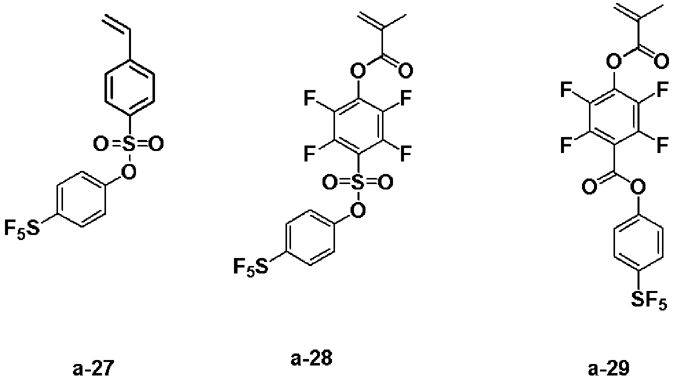

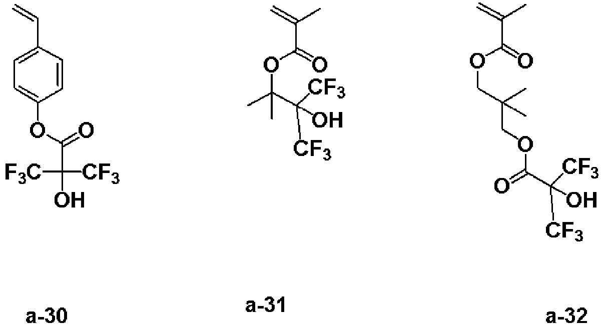

### [Synthesis Example 35] Synthesis of polymer (B30)

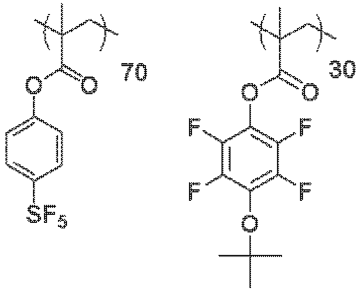

Under a nitrogen atmosphere, 10.0 g of PGMEA was heated and stirred at 80°C. A mixture of 3.02 g (10.5 mol) of a monomer a-(4), 1.38 g (4.5 mol) of a monomer a-(16), 0.069 g (0.30 mmol) of dimethyl 2,2-azobis(2-methylpropionate), and 15.0 g of PGMEA was added dropwise thereto over 4 hours. The mixture was further heated and stirred for 16 hours, and then cooled to room temperature to obtain a PGMEA solution of a target polymer (B30). As a result of analysis, the polymer (B30) had a weight-average molecular weight (Mw) of 10600 and a dispersity (Mw/Mn) of 1.83.

### [Synthesis Example 36] Synthesis of polymer (B31)

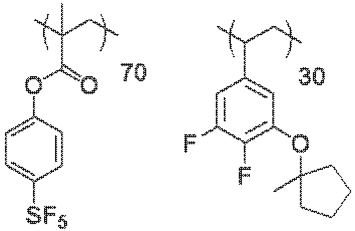

Under a nitrogen atmosphere, 10.0 g of PGMEA was heated and stirred at 80°C. A mixture of 3.02 g (10.5 mol) of a monomer a-(4), 1.07 g (4.5 mol) of a monomer a-(17), 0.069 g (0.30 mmol) of dimethyl 2,2-azobis(2-methylpropionate), and 15.0 g of PGMEA was added dropwise thereto over 4 hours. The mixture was further heated and stirred for 16 hours, and then cooled to room temperature to obtain a PGMEA solution of a target polymer (B31). As a result of analysis, the polymer (B31) had a weight-average molecular weight (Mw) of 9800 and a dispersity (Mw/Mn) of 1.77.

### [Synthesis Example 37] Synthesis of polymer (B32)

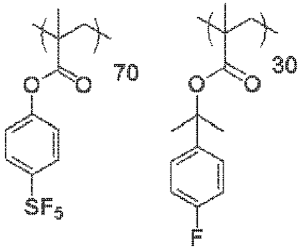

Under a nitrogen atmosphere, 10.0 g of PGMEA was heated and stirred at 80°C. A mixture of 3.02 g (10.5 mmol) of a monomer a-(4), 1.00 g (4.5 mmol) of a monomer a-(18), 0.069 g (0.30 mmol) of dimethyl 2,2-azobis(2-methylpropionate), and 15.0 g of PGMEA was added dropwise thereto over 4 hours. The mixture was further heated and stirred for 16 hours, and then cooled to room temperature to obtain a PGMEA solution of a target polymer (B32). As a result of analysis, the polymer (B32) had a weight-average molecular weight (Mw) of 10800 and a dispersity (Mw/Mn) of 1.87.

### [Synthesis Example 38] Synthesis of polymer (B33)

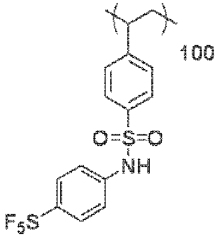

Under a nitrogen atmosphere, 10.0 g of PGMEA was heated and stirred at 80°C. A mixture of 5.78 g (15.0 mmol) of a monomer a-(19), 0.069 g (0.30 mmol) of dimethyl 2,2-azobis(2-methylpropionate), and 15.0 g of PGMEA was added dropwise thereto over 4 hours. The mixture was further heated and stirred for 16 hours, and then cooled to room temperature to obtain a PGMEA solution of a target polymer (B33). As a result of analysis, the polymer (B33) had a weight-average molecular weight (Mw) of 10800 and a dispersity (Mw/Mn) of 1.87.

### [Synthesis Example 39] Synthesis of polymer (B34)

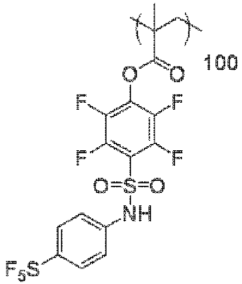

Under a nitrogen atmosphere, 10.0 g of PGMEA was heated and stirred at 80°C. A mixture of 7.70 g (15.0 mmol) of a monomer a-(20), 0.069 g (0.30 mmol) of dimethyl 2,2-azobis(2-methylpropionate), and 15.0 g of PGMEA was added dropwise thereto over 4 hours. The mixture was further heated and stirred for 16 hours, and then cooled to room temperature to obtain a PGMEA solution of a target polymer (B34). As a result of analysis, the polymer (B34) had a weight-average molecular weight (Mw) of 9200 and a dispersity (Mw/Mn) of 1.81.

### [Synthesis Example 40] Synthesis of polymer (B35)

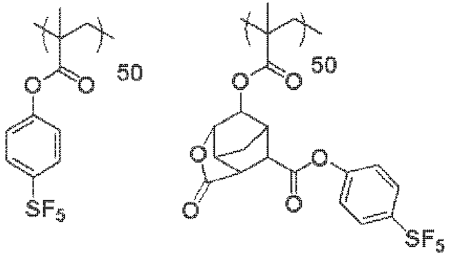

Under a nitrogen atmosphere, 10.0 g of PGMEA was heated and stirred at 80°C. A mixture of 2.16 g (7.5 mmol) of a monomer a-(4), 3.51 g (7.5 mmol) of a monomer a-(21), 0.069 g (0.30 mmol) of dimethyl 2,2-azobis(2-methylpropionate), and 15.0 g of PGMEA was added dropwise thereto over 4 hours. The mixture was further heated and stirred for 16 hours, and then cooled to room temperature to obtain a PGMEA solution of a target polymer (B35). As a result of analysis, the polymer (B35) had a weight-average molecular weight (Mw) of 9300 and a dispersity (Mw/Mn) of 1.85.

### [Synthesis Example 41] Synthesis of polymer (B36)

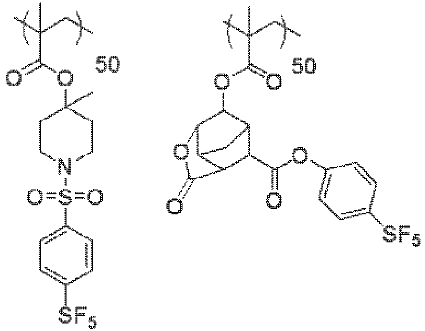

Under a nitrogen atmosphere, 10.0 g of PGMEA was heated and stirred at 80°C. A mixture of 3.37 g (7.5 mmol) of a monomer a-(22), 3.51 g (7.5 mmol) of a monomer a-(21), 0.069 g (0.30 mmol) of dimethyl 2,2-azobis(2-methylpropionate), and 15.0 g of PGMEA was added dropwise thereto over 4 hours. The mixture was further heated and stirred for 16 hours, and then cooled to room temperature to obtain a PGMEA solution of a target polymer (B36). As a result of analysis, the polymer (B36) had a weight-average molecular weight (Mw) of 7800 and a dispersity (Mw/Mn) of 1.65.

### [Synthesis Example 42] Synthesis of polymer (B37)

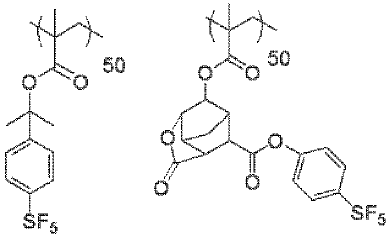

Under a nitrogen atmosphere, 10.0 g of PGMEA was heated and stirred at 80°C. A mixture of 2.48 g (7.5 mmol) of a monomer a-(23), 3.51 g (7.5 mmol) of a monomer a-(21), 0.069 g (0.30 mmol) of dimethyl 2,2-azobis(2-methylpropionate), and 15.0 g of PGMEA was added dropwise thereto over 4 hours. The mixture was further heated and stirred for 16 hours, and then cooled to room temperature to obtain a PGMEA solution of a target polymer (B37). As a result of analysis, the polymer (B37) had a weight-average molecular weight (Mw) of 7600 and a dispersity (Mw/Mn) of 1.69.

### [Synthesis Example 43] Synthesis of polymer (B38)

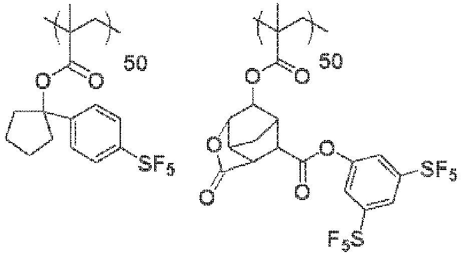

Under a nitrogen atmosphere, 10.0 g of PGMEA was heated and stirred at 80°C. A mixture of 2.52 g (7.5 mmol) of a monomer a-(24), 4.45 g (7.5 mmol) of a monomer a-(26), 0.069 g (0.30 mmol) of dimethyl 2,2-azobis(2-methylpropionate), and 15.0 g of PGMEA was added dropwise thereto over 4 hours. The mixture was further heated and stirred for 16 hours, and then cooled to room temperature to obtain a PGMEA solution of a target polymer (B38). As a result of analysis, the polymer (B38) had a weight-average molecular weight (Mw) of 7400 and a dispersity (Mw/Mn) of 1.61.

### [Synthesis Example 44] Synthesis of polymer (B39)

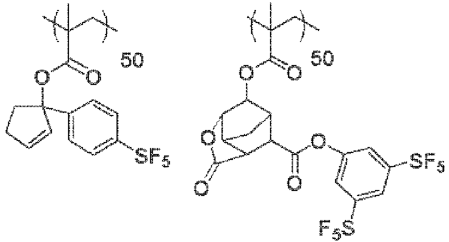

Under a nitrogen atmosphere, 10.0 g of PGMEA was heated and stirred at 80°C. A mixture of 2.66 g (7.5 mmol) of a monomer a-(25), 4.45 g (7.5 mmol) of a monomer a-(26), 0.069 g (0.30 mmol) of dimethyl 2,2-azobis(2-methylpropionate), and 15.0 g of PGMEA was added dropwise thereto over 4 hours. The mixture was further heated and stirred for 16 hours, and then cooled to room temperature to obtain a PGMEA solution of a target polymer (B39). As a result of analysis, the polymer (B39) had a weight-average molecular weight (Mw) of 7800 and a dispersity (Mw/Mn) of 1.60.

### [Synthesis Example 45] Synthesis of polymer (B40)

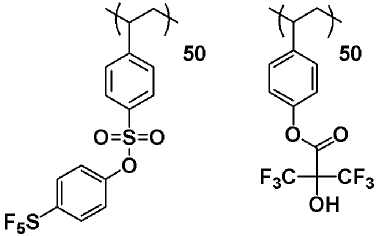

Under a nitrogen atmosphere, 10.0 g of PGMEA was heated and stirred at 80°C. A mixture of 3.87 g (7.5 mmol) of a monomer a-(27), 2.21 g (7.5 mmol) of a monomer a-(30), 0.069 g (0.30 mmol) of dimethyl 2,2-azobis(2-methylpropionate), and 15.0 g of PGMEA was added dropwise thereto over 4 hours. The mixture was further heated and stirred for 16 hours, and then cooled to room temperature to obtain a PGMEA solution of a target polymer (B40). As a result of analysis, the polymer (B40) had a weight-average molecular weight (Mw) of 6900 and a dispersity (Mw/Mn) of 1.50.

### [Synthesis Example 46] Synthesis of polymer (B41)

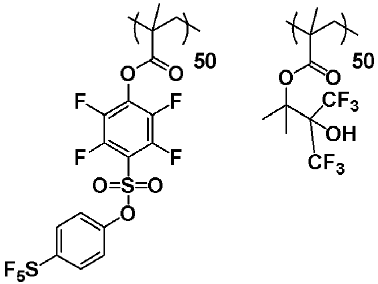

Under a nitrogen atmosphere, 10.0 g of PGMEA was heated and stirred at 80°C. A mixture of 2.90 g (7.5 mmol) of a monomer a-(28), 2.36 g (7.5 mmol) of a monomer a-(31), 0.069 g (0.30 mmol) of dimethyl 2,2-azobis(2-methylpropionate), and 15.0 g of PGMEA was added dropwise thereto over 4 hours. The mixture was further heated and stirred for 16 hours, and then cooled to room temperature to obtain a PGMEA solution of a target polymer (B41). As a result of analysis, the polymer (B41) had a weight-average molecular weight (Mw) of 8600 and a dispersity (Mw/Mn) of 1.78.

### [Synthesis Example 47] Synthesis of polymer (B42)

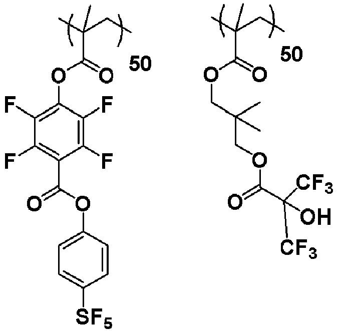

Under a nitrogen atmosphere, 10.0 g of PGMEA was heated and stirred at 80°C. A mixture of 3.60 g (7.5 mmol) of a monomer a-(29), 2.75 g (7.5 mmol) of a monomer a-(32), 0.069 g (0.30 mmol) of dimethyl 2,2-azobis(2-methylpropionate), and 15.0 g of PGMEA was added dropwise thereto over 4 hours. The mixture was further heated and stirred for 16 hours, and then cooled to room temperature to obtain a PGMEA solution of a target polymer (B42). As a result of analysis, the polymer (B42) had a weight-average molecular weight (Mw) of 8800 and a dispersity (Mw/Mn) of 1.82.

### [Synthesis of Comparative Polymers (R1) to (R7) and Comparative Compound (R8)]

Raw materials used to synthesize comparative polymers and compound (R1) to (R8) are shown below. The polymers (R1) to (R7) were obtained by using monomers (c1) to (c9). For synthesis of the compound (R8), (c10) was used.

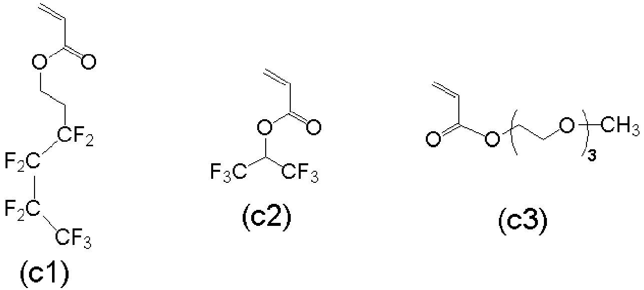

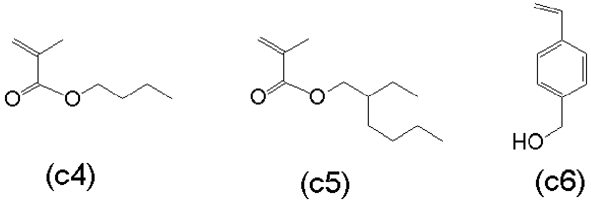

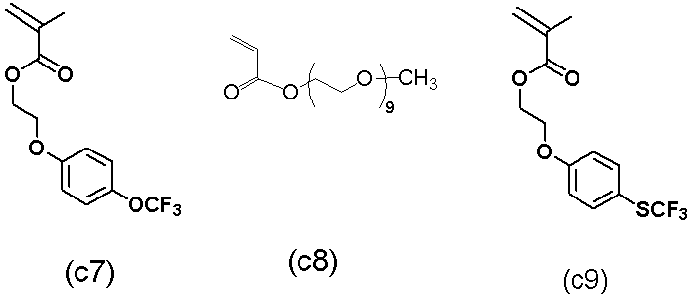

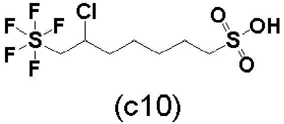

### [Comparative Synthesis Example 1] Synthesis of Comparative Polymer (R1)

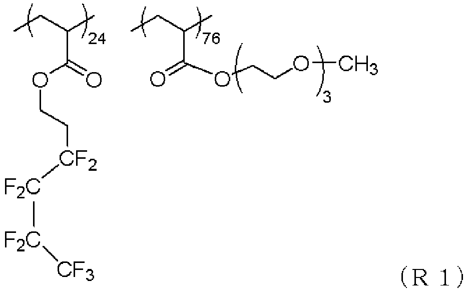

Under a nitrogen atmosphere, 10.0 g of PGMEA was heated and stirred at 80°C. A mixture of 3.44 g (10.8 mmol) of a monomer (c1), 7.46 g (34.2 mmol) of a monomer (c3), 0.473 g (2.05 mmol) of dimethyl 2,2-azobis(2-methylpropionate), and 15.0 g of PGMEA was added dropwise thereto over 4 hours. The mixture was further heated and stirred for 16 hours, and then cooled to room temperature to obtain a PGMEA solution of a target polymer (R1). As a result of analysis, the polymer (R1) had a weight-average molecular weight (Mw) of 9300 and a dispersity (Mw/Mn) of 1.23.

### [Comparative Synthesis Example 2] Synthesis of Comparative Polymer (R2)

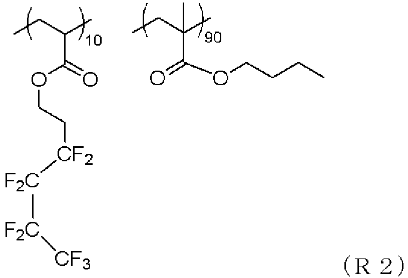

Under a nitrogen atmosphere, 10.0 g of PGMEA was heated and stirred at 80°C. A mixture of 1.43 g (4.5 mmol) of a monomer (c1), 5.76 g (34.2 mmol) of a monomer (c4), 0.473 g (2.05 mmol) of dimethyl 2,2-azobis(2-methylpropionate), and 15.0 g of PGMEA was added dropwise thereto over 4 hours. The mixture was further heated and stirred for 16 hours, and then cooled to room temperature to obtain a PGMEA solution of a target polymer (R2). As a result of analysis, the polymer (R2) had a weight-average molecular weight (Mw) of 6300 and a dispersity (Mw/Mn) of 1.50.

### [Comparative Synthesis Example 3] Synthesis of Comparative Polymer (R3)

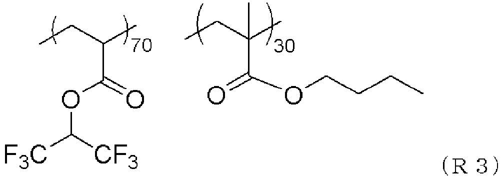

Under a nitrogen atmosphere, 10.0 g of PGMEA was heated and stirred at 80°C. A mixture of 7.00 g (31.5 mmol) of a monomer (c2), 2.95 g (13.5 mmol) of a monomer (c4), 0.473 g (2.05 mmol) of dimethyl 2,2-azobis(2-methylpropionate), and 20.0 g of PGMEA was added dropwise thereto over 4 hours. The mixture was further heated and stirred for 16 hours, and then cooled to room temperature to obtain a PGMEA solution of a target polymer (R3). As a result of analysis, the polymer (R3) had a weight-average molecular weight (Mw) of 6300 and a dispersity (Mw/Mn) of 1.38.

### [Comparative Synthesis Example 4] Synthesis of Comparative Polymer (R4)

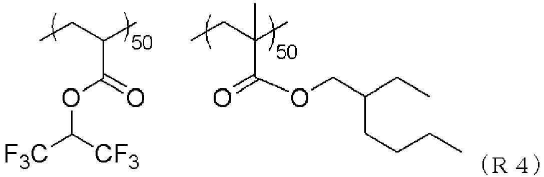

Under a nitrogen atmosphere, 10.0 g of PGMEA was heated and stirred at 80°C. A mixture of 5.0 g (22.5 mmol) of a monomer (c2), 4.46 g (22.5 mmol) of a monomer (c5), 0.473 g (2.05 mmol) of dimethyl 2,2-azobis(2-methylpropionate), and 15.0 g of PGMEA was added dropwise thereto over 4 hours. The mixture was further heated and stirred for 16 hours, and then cooled to room temperature to obtain a PGMEA solution of a target polymer (R4). As a result of analysis, the polymer (R4) had a weight-average molecular weight (Mw) of 8300 and a dispersity (Mw/Mn) of 1.33.

### [Comparative Synthesis Example 5] Synthesis of Comparative Polymer (R5)

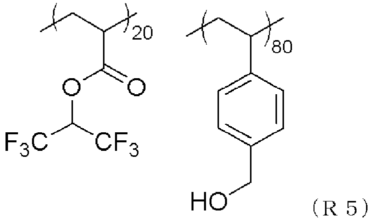

Under a nitrogen atmosphere, 10.0 g of PGMEA was heated and stirred at 80°C. A mixture of 2.0 g (9.0 mmol) of a monomer (c2), 4.83 g (36.0 mmol) of a monomer (c6), 0.473 g (2.05 mmol) of dimethyl 2,2-azobis(2-methylpropionate), and 15.0 g of PGMEA was added dropwise thereto over 4 hours. The mixture was further heated and stirred for 16 hours, and then cooled to room temperature to obtain a PGMEA solution of a target polymer (R5). As a result of analysis, the polymer (R5) had a weight-average molecular weight (Mw) of 4300 and a dispersity (Mw/Mn) of 1.43.

### [Comparative Synthesis Example 6] Synthesis of Comparative Polymer (R6)

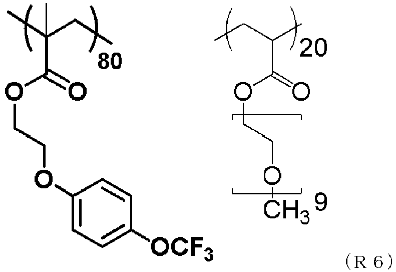

Under a nitrogen atmosphere, 10.0 g of PGMEA was heated and stirred at 80°C. A mixture of 4.0 g (13.7 mmol) of a monomer (c7), 1.66 g (3.5 mmol) of a monomer (c8), 0.198 g (0.86 mmol) of dimethyl 2,2-azobis(2-methylpropionate), and 15.0 g of PGMEA was added dropwise thereto over 4 hours. The mixture was further heated and stirred for 16 hours, and then cooled to room temperature to obtain a PGMEA solution of a target polymer (R6). As a result of analysis, the polymer (R6) had a weight-average molecular weight (Mw) of 12300 and a dispersity (Mw/Mn) of 1.57.

### [Comparative Synthesis Example 7] Synthesis of Comparative Polymer (R7)

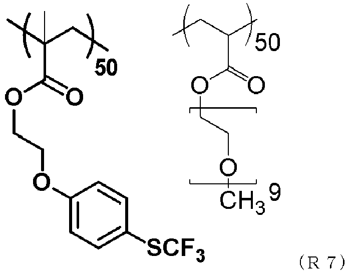

Under a nitrogen atmosphere, 10.0 g of PGMEA was heated and stirred at 80°C. A mixture of 4.73 g (9.8 mmol) of a monomer (c8), 4.00 g (9.8 mmol) of a monomer (c9), 0.225 g (0.98 mmol) of dimethyl 2,2-azobis(2-methylpropionate), and 15.0 g of PGMEA was added dropwise thereto over 4 hours. The mixture was further heated and stirred for 16 hours, and then cooled to room temperature to obtain a PGMEA solution of a target polymer (R7). As a result of analysis, the polymer (R7) had a weight-average molecular weight (Mw) of 16300 and a dispersity (Mw/Mn) of 1.62.

### [Comparative Synthesis Example 8] Synthesis of Comparative Compound (R8)

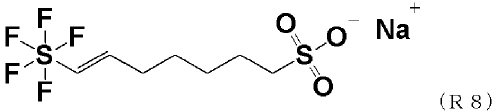

Under a nitrogen atmosphere, 10.0 g (29.35 mmol) of a material (c10) was uniformly dispersed in 400 ml of THF, and then 23.48 g (293.5 mmol in terms of sodium hydroxide) of a 50 wt% aqueous sodium hydroxide solution was added dropwise and the mixture was reacted under reflux for 10 hours. After completion of the reaction, 65 g of a 20% aqueous salt solution was added while cooling in an ice bath to make the liquid acidic, and then the organic layer was extracted with 200 ml of diisopropyl ether and washed with pure water five times. The organic layer was collected and evaporated to dryness under reduced pressure. Then, 100 ml of ethanol was added to disperse residue again; 28.2 g of 50% sodium hydroxide aqueous solution (corresponding to 352.2 mmol in terms of sodium hydroxide) was added, and the mixture was heated and stirred at 100° C. for 1 hour; and crystals were precipitated while stirring the mixture in an ice bath. The precipitated crystals were filtered off, washed twice with 100 ml of ethanol, and then collected and dried at 40°C to obtain a comparative compound (R8).

### [Resin or Compound for Forming Organic Film]

A1: Resin represented by the following formula (A1)
A2: Resin represented by the following formula (A2)
A3: Compound represented by the following formula (A3)
A4: Compound represented by the following formula (A4)
A5: Resin represented by the following formula (A5)
A6: Resin represented by the following formula (A6)

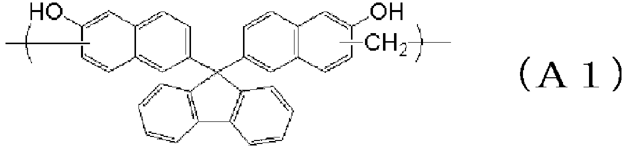

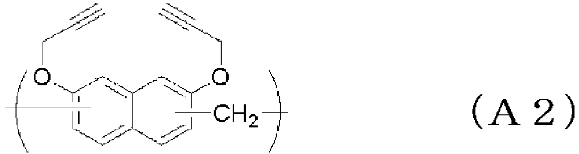

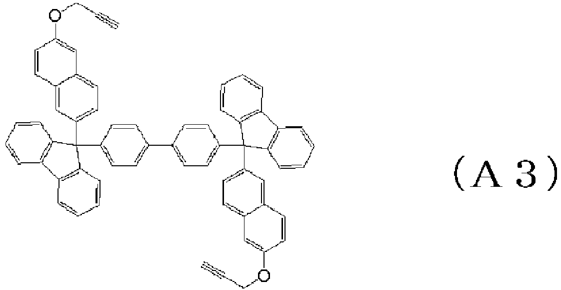

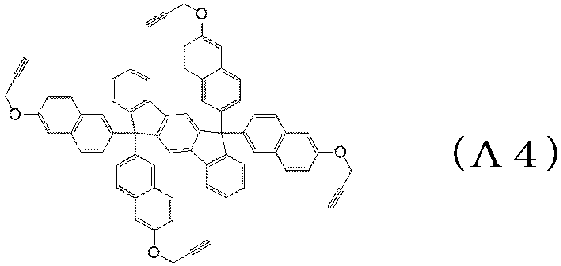

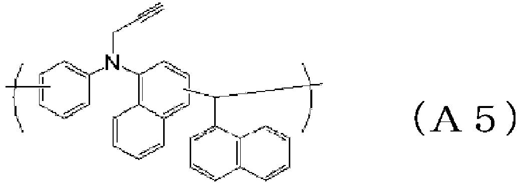

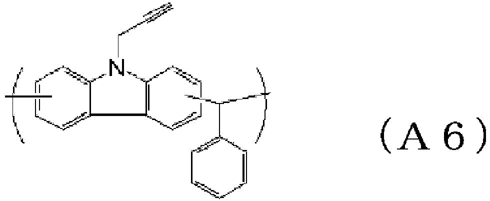

### [Solvents]

(C1): Propylene glycol monomethyl ether acetate
(C2): Propylene glycol monoethyl ether

### [Preparation of Compositions (UDL-1 to -138 and Comparative UDL-1 to -26) for Forming Organic Film]

Each of the polymers (A1) to (A29) and (R1) to (R7), the compound (R8) for Comparative Example, the resins (C1) to (C6) for forming an organic film, and the solvent, which are described above, were dissolved at the ratios shown in Tables 1-1 to 1-6. The mixture was filtered through a 0.1-µm filter made of a fluorinated resin to prepare each of the materials for forming an organic film (resist underlayer film materials: UDL-1 to -138 and comparative UDL-1 to -26).

**[Table 1-1]**

| Composition for forming organic film | Resin for forming organic film | | Polymer | | Solvent 1 | | Solvent 2 | |
|---|---|---|---|---|---|---|---|---|
| | Type | Part by mass | Type | Part by mass | Type | Part by mass | Type | Part by mass |
| UDL-1 | (A1) | 10.00 | (B1) | 0.10 | (C1) | 89.90 | | |
| UDL-2 | (A1) | 10.00 | (B2) | 0.10 | (C1) | 89.90 | | |
| UDL-3 | (A1) | 10.00 | (B3) | 0.10 | (C1) | 89.90 | | |
| UDL-4 | (A1) | 10.00 | (B4) | 0.10 | (C1) | 89.90 | | |
| UDL-5 | (A1) | 10.00 | (B5) | 0.10 | (C1) | 89.90 | | |
| UDL-6 | (A1) | 10.00 | (B6) | 0.10 | (C1) | 89.90 | | |
| UDL-7 | (A1) | 10.00 | (B7) | 0.10 | (C1) | 89.90 | | |
| UDL-8 | (A1) | 10.00 | (B8) | 0.10 | (C1) | 89.90 | | |
| UDL-9 | (A1) | 10.00 | (B9) | 0.10 | (C1) | 89.90 | | |
| UDL-10 | (A1) | 10.00 | (B10) | 0.10 | (C1) | 89.90 | | |
| UDL-11 | (A1) | 10.00 | (B11) | 0.10 | (C1) | 89.90 | | |
| UDL-12 | (A1) | 10.00 | (B12) | 0.10 | (C1) | 89.90 | | |
| UDL-13 | (A1) | 10.00 | (B13) | 0.10 | (C1) | 89.90 | | |
| UDL-14 | (A1) | 10.00 | (B14) | 0.10 | (C1) | 89.90 | | |
| UDL-15 | (A1) | 10.00 | (B15) | 0.10 | (C1) | 89.90 | | |
| UDL-16 | (A1) | 10.00 | (B16) | 0.10 | (C1) | 89.90 | | |
| UDL-17 | (A1) | 10.00 | (B17) | 0.10 | (C1) | 89.90 | | |
| UDL-18 | (A1) | 10.00 | (B18) | 0.10 | (C1) | 89.90 | | |
| UDL-19 | (A1) | 10.000 | (B19) | 0.10 | (C1) | 89.90 | | |
| UDL-20 | (A1) | 10.00 | (B20) | 0.10 | (C1) | 89.90 | | |
| UDL-21 | (A1) | 10.00 | (B21) | 0.10 | (C1) | 89.90 | | |
| UDL-22 | (A1) | 10.00 | (B22) | 0.10 | (C1) | 89.90 | | |
| UDL-23 | (A1) | 10.00 | (B23) | 0.10 | (C1) | 89.90 | | |
| UDL-24 | (A1) | 10.00 | (B24) | 0.10 | (C1) | 89.90 | | |
| UDL-25 | (A1) | 10.00 | (B25) | 0.10 | (C1) | 89.90 | | |
| UDL-26 | (A1) | 10.00 | (B26) | 0.10 | (C1) | 89.90 | | |
| UDL-27 | (A1) | 10.00 | (B27) | 0.10 | (C1) | 89.90 | | |
| UDL-28 | (A1) | 10.00 | (B28) | 0.10 | (C1) | 89.90 | | |
| UDL-29 | (A1) | 10.00 | (B29) | 0.10 | (C1) | 89.90 | | |
| UDL-30 | (A1) | 10.00 | (B3) | 0.10 | (C1) | 62.93 | (C2) | 26.97 |

**[Table 1-2]**

| Composition for forming organic film | Resin for forming organic film | | Polymer | | Solvent 1 | | Solvent 2 | |
|---|---|---|---|---|---|---|---|---|
| | Type | Part by mass | Type | Part by mass | Type | Part by mass | Type | Part by mass |
| UDL-31 | (A1) | 10.00 | (B9) | 0.10 | (C1) | 62.93 | (C2) | 26.97 |
| UDL-32 | (A1) | 10.00 | (B10) | 0.10 | (C1) | 62.93 | (C2) | 26.97 |
| UDL-33 | (A1) | 10.00 | (B11) | 0.10 | (C1) | 62.93 | (C2) | 26.97 |
| UDL-34 | (A1) | 10.00 | (B16) | 0.10 | (C1) | 62.93 | (C2) | 26.97 |
| UDL-35 | (A1) | 10.00 | (B18) | 0.10 | (C1) | 62.93 | (C2) | 26.97 |
| UDL-36 | (A1) | 10.00 | (B24) | 0.10 | (C1) | 62.93 | (C2) | 26.97 |
| UDL-37 | (A1) | 10.00 | (B29) | 0.10 | (C1) | 62.93 | (C2) | 26.97 |
| UDL-38 | (A1) | 10.00 | (B10) | 1.000 | (C1) | 89.00 | | |
| UDL-39 | (A1) | 10.00 | (B10) | 0.500 | (C1) | 89.50 | | |
| UDL-40 | (A1) | 10.00 | (B10) | 0.001 | (C1) | 90.00 | | |
| UDL-41 | (A2) | 10.00 | (B3) | 0.10 | (C1) | 89.90 | | |
| UDL-42 | (A2) | 10.00 | (B9) | 0.10 | (C1) | 89.90 | | |
| UDL-43 | (A2) | 10.00 | (B10) | 0.10 | (C1) | 89.90 | | |
| UDL-44 | (A2) | 10.00 | (B11) | 0.10 | (C1) | 89.90 | | |
| UDL-45 | (A2) | 10.00 | (B16) | 0.10 | (C1) | 89.90 | | |
| UDL-46 | (A2) | 10.00 | (B18) | 0.10 | (C1) | 89.90 | | |
| UDL-47 | (A2) | 10.00 | (B24) | 0.10 | (C1) | 89.90 | | |
| UDL-48 | (A2) | 10.00 | (B29) | 0.10 | (C1) | 89.90 | | |
| UDL-49 | (A3) | 10.00 | (B3) | 0.10 | (C1) | 89.90 | | |
| UDL-50 | (A3) | 10.00 | (B9) | 0.10 | (C1) | 89.90 | | |
| UDL-51 | (A3) | 10.00 | (B10) | 0.10 | (C1) | 89.90 | | |
| UDL-52 | (A3) | 10.00 | (B11) | 0.10 | (C1) | 89.90 | | |
| UDL-53 | (A3) | 10.00 | (B16) | 0.10 | (C1) | 89.90 | | |
| UDL-54 | (A3) | 10.00 | (B18) | 0.10 | (C1) | 89.90 | | |
| UDL-55 | (A3) | 10.00 | (B24) | 0.10 | (C1) | 89.90 | | |
| UDL-56 | (A3) | 10.00 | (B29) | 0.10 | (C1) | 89.90 | | |
| UDL-57 | (A4) | 10.00 | (B3) | 0.10 | (C1) | 89.90 | | |
| UDL-58 | (A4) | 10.00 | (B9) | 0.10 | (C1) | 89.90 | | |
| UDL-59 | (A4) | 10.00 | (B10) | 0.10 | (C1) | 89.90 | | |
| UDL-60 | (A4) | 10.00 | (B11) | 0.10 | (C1) | 89.90 | | |

**[Table 1-3]**

| Composition for forming organic film | Resin for forming organic film | | Polymer | | Solvent 1 | | Solvent 2 | |
|---|---|---|---|---|---|---|---|---|
| | Type | Part by mass | Type | Part by mass | Type | Part by mass | Type | Part by mass |
| UDL-61 | (A4) | 10.00 | (B16) | 0.10 | (C1) | 89.90 | | |
| UDL-62 | (A4) | 10.00 | (B18) | 0.10 | (C1) | 89.90 | | |
| UDL-63 | (A4) | 10.00 | (B24) | 0.10 | (C1) | 89.90 | | |
| UDL-64 | (A4) | 10.00 | (B29) | 0.10 | (C1) | 89.90 | | |
| UDL-65 | (A5) | 10.00 | (B3) | 0.10 | (C1) | 89.90 | | |
| UDL-66 | (A5) | 10.00 | (B9) | 0.10 | (C1) | 89.90 | | |
| UDL-67 | (A5) | 10.00 | (B10) | 0.10 | (C1) | 89.90 | | |
| UDL-68 | (A5) | 10.00 | (B11) | 0.10 | (C1) | 89.90 | | |
| UDL-69 | (A5) | 10.00 | (B16) | 0.10 | (C1) | 89.90 | | |
| UDL-70 | (A5) | 10.00 | (B18) | 0.10 | (C1) | 89.90 | | |
| UDL-71 | (A5) | 10.00 | (B24) | 0.10 | (C1) | 89.90 | | |
| UDL-72 | (A5) | 10.00 | (B29) | 0.10 | (C1) | 89.90 | | |
| UDL-73 | (A6) | 10.00 | (B3) | 0.10 | (C1) | 89.90 | | |
| UDL-74 | (A6) | 10.00 | (B9) | 0.10 | (C1) | 89.90 | | |
| UDL-75 | (A6) | 10.00 | (B10) | 0.10 | (C1) | 89.90 | | |
| UDL-76 | (A6) | 10.00 | (B11) | 0.10 | (C1) | 89.90 | | |
| UDL-77 | (A6) | 10.00 | (B16) | 0.10 | (C1) | 89.90 | | |
| UDL-78 | (A6) | 10.00 | (B18) | 0.10 | (C1) | 89.90 | | |
| UDL-79 | (A6) | 10.00 | (B24) | 0.10 | (C1) | 89.90 | | |
| UDL-80 | (A6) | 10.00 | (B29) | 0.10 | (C1) | 89.90 | | |
| UDL-81 | (A1) | 25. 00 | (B1) | 0.25 | (C1) | 74.75 | | |
| UDL-82 | (A1) | 25. 00 | (B2) | 0.25 | (C1) | 74.75 | | |
| UDL-83 | (A1) | 25. 00 | (B3) | 0.25 | (C1) | 74.75 | | |
| UDL-84 | (A1) | 25. 00 | (B4) | 0.25 | (C1) | 74.75 | | |
| UDL-85 | (A1) | 25. 00 | (B5) | 0.25 | (C1) | 74.75 | | |
| UDL-86 | (A1) | 25. 00 | (B6) | 0.25 | (C1) | 74.75 | | |
| UDL-87 | (A1) | 25.00 | (B7) | 0.25 | (C1) | 74.75 | | |
| UDL-88 | (A1) | 25. 00 | (B8) | 0.25 | (C1) | 74.75 | | |
| UDL-89 | (A1) | 25. 00 | (B9) | 0.25 | (C1) | 74.75 | | |
| UDL-90 | (A1) | 25. 00 | (B10) | 0.25 | (C1) | 74.75 | | |

**[Table 1-4]**

| Composition for forming organic film | Resin for forming organic film | | Polymer | | Solvent 1 | | Solvent 2 | |
|---|---|---|---|---|---|---|---|---|
| | Type | Part by mass | Type | Part by mass | Type | Part by mass | Type | Part by mass |
| UDL-91 | (A1) | 25. 00 | (B11) | 0.25 | (C1) | 74.75 | | |
| UDL-92 | (A1) | 25. 00 | (B12) | 0.25 | (C1) | 74.75 | | |
| UDL-93 | (A1) | 25. 00 | (B13) | 0.25 | (C1) | 74.75 | | |
| UDL-94 | (A1) | 25. 00 | (B14) | 0.25 | (C1) | 74.75 | | |
| UDL-95 | (A1) | 25. 00 | (B15) | 0.25 | (C1) | 74.75 | | |
| UDL-96 | (A1) | 25. 00 | (B16) | 0.25 | (C1) | 74.75 | | |
| UDL-97 | (A1) | 25. 00 | (B17) | 0.25 | (C1) | 74.75 | | |
| UDL-98 | (A1) | 25. 00 | (B18) | 0.25 | (C1) | 74.75 | | |
| UDL-99 | (A1) | 25. 00 | (B19) | 0.25 | (C1) | 74.75 | | |
| UDL-100 | (A1) | 25. 00 | (B20) | 0.25 | (C1) | 74.75 | | |
| UDL-101 | (A1) | 25.00 | (B21) | 0.25 | (C1) | 74.75 | | |
| UDL-102 | (A1) | 25.00 | (B22) | 0.25 | (C1) | 74.75 | | |
| UDL-103 | (A1) | 25.00 | (B23) | 0.25 | (C1) | 74.75 | | |
| UDL-104 | (A1) | 25. 00 | (B24) | 0.25 | (C1) | 74.75 | | |
| UDL-105 | (A1) | 25. 00 | (B25) | 0.25 | (C1) | 74.75 | | |
| UDL-106 | (A1) | 25. 00 | (B26) | 0.25 | (C1) | 74.75 | | |
| UDL-107 | (A1) | 25. 00 | (B27) | 0.25 | (C1) | 74.75 | | |
| UDL-108 | (A1) | 25. 00 | (B28) | 0.25 | (C1) | 74.75 | | |
| UDL-109 | (A1) | 25.00 | (B29) | 0.25 | (C1) | 74.75 | | |
| UDL-110 | (A1) | 1. 50 | (B1) | 0.02 | (C1) | 98.49 | | |
| UDL-111 | (A1) | 1. 50 | (B2) | 0.02 | (C1) | 98.49 | | |
| UDL-112 | (A1) | 1. 50 | (B3) | 0.02 | (C1) | 98.49 | | |
| UDL-113 | (A1) | 1. 50 | (B4) | 0.02 | (C1) | 98.49 | | |
| UDL-114 | (A1) | 1. 50 | (B5) | 0.02 | (C1) | 98.49 | | |
| UDL-115 | (A1) | 1. 50 | (B6) | 0.02 | (C1) | 98.49 | | |
| UDL-116 | (A1) | 1. 50 | (B7) | 0.02 | (C1) | 98.49 | | |
| UDL-117 | (A1) | 1. 50 | (B8) | 0.02 | (C1) | 98.49 | | |
| UDL-118 | (A1) | 1. 50 | (B9) | 0.02 | (C1) | 98.49 | | |
| UDL-119 | (A1) | 1. 50 | (B10) | 0.02 | (C1) | 98.49 | | |
| UDL-120 | (A1) | 1. 50 | (B11) | 0.02 | (C1) | 98.49 | | |

**[Table 1-5]**

| Composition for forming organic film | Resin for forming organic film | | Polymer | | Solvent 1 | | Solvent 2 | |
|---|---|---|---|---|---|---|---|---|
| | Type | Part by mass | Type | Part by mass | Type | Part by mass | Type | Part by mass |
| UDL-121 | (A1) | 1. 50 | (B12) | 0.02 | (C1) | 98.49 | | |
| UDL-122 | (A1) | 1. 50 | (B13) | 0.02 | (C1) | 98.49 | | |
| UDL-123 | (A1) | 1. 50 | (B14) | 0.02 | (C1) | 98.49 | | |
| UDL-124 | (A1) | 1. 50 | (B15) | 0.02 | (C1) | 98.49 | | |
| UDL-125 | (A1) | 1. 50 | (B16) | 0.02 | (C1) | 98.49 | | |
| UDL-126 | (A1) | 1. 50 | (B17) | 0.02 | (C1) | 98.49 | | |
| UDL-127 | (A1) | 1. 50 | (B18) | 0.02 | (C1) | 98.49 | | |
| UDL-128 | (A1) | 1. 50 | (B19) | 0.02 | (C1) | 98.49 | | |
| UDL-129 | (A1) | 1. 50 | (B20) | 0.02 | (C1) | 98.49 | | |
| UDL-130 | (A1) | 1. 50 | (B21) | 0.02 | (C1) | 98.49 | | |
| UDL-131 | (A1) | 1. 50 | (B22) | 0.02 | (C1) | 98.49 | | |
| UDL-132 | (A1) | 1. 50 | (B23) | 0.02 | (C1) | 98.49 | | |
| UDL-133 | (A1) | 1. 50 | (B24) | 0.02 | (C1) | 98.49 | | |
| UDL-134 | (A1) | 1. 50 | (B25) | 0.02 | (C1) | 98.49 | | |
| UDL-135 | (A1) | 1. 50 | (B26) | 0.02 | (C1) | 98.49 | | |
| UDL-136 | (A1) | 1. 50 | (B27) | 0.02 | (C1) | 98.49 | | |
| UDL-137 | (A1) | 1. 50 | (B28) | 0.02 | (C1) | 98.49 | | |
| UDL-138 | (A1) | 1. 50 | (B29) | 0.02 | (C1) | 98.49 | | |

**[Table 1-6]**

| Composition for forming organic film | Resin for forming organic film | | Polymer | | Solvent 1 | | Solvent 2 | |
|---|---|---|---|---|---|---|---|---|
| | Type | Part by mass | Type | Part by mass | Type | Part by mass | Type | Part by mass |
| Comparative UDL-1 | (A1) | 10.00 | (R1) | 0.10 | (C1) | 89.90 | | |
| Comparative UDL-2 | (A2) | 10.00 | (R1) | 0.10 | (C1) | 89.90 | | |
| Comparative UDL-3 | (A3) | 10.00 | (R1) | 0.10 | (C1) | 89.90 | | |
| Comparative UDL-4 | (A4) | 10.00 | (R1) | 0.10 | (C1) | 89.90 | | |
| Comparative UDL-5 | (A5) | 10.00 | (R1) | 0.10 | (C1) | 89.90 | | |
| Comparative UDL-6 | (A6) | 10.00 | (R1) | 0.10 | (C1) | 89.90 | | |
| Comparative UDL-7 | (A1) | 10.00 | (R2) | 0.10 | (C1) | 89.90 | | |
| Comparative UDL-8 | (A1) | 10.00 | (R3) | 0.10 | (C1) | 89.90 | | |
| Comparative UDL-9 | (A1) | 10.00 | (R4) | 0.10 | (C1) | 89.90 | | |
| Comparative UDL-10 | (A1) | 10.00 | (R5) | 0.10 | (C1) | 89.90 | | |
| Comparative UDL-11 | (A1) | 10.00 | (R6) | 0.10 | (C1) | 89.90 | | |
| Comparative UDL-12 | (A1) | 10.00 | (R7) | 0.10 | (C1) | 89.90 | | |
| Comparative UDL-13 | (A1) | 10.00 | (R8) | 0.10 | (C1) | 89.90 | | |
| Comparative UDL-14 | (A1) | 10.00 | | | (C1) | 90. 00 | | |
| Comparative UDL-15 | (A2) | 10.00 | | | (C1) | 90. 00 | | |
| Comparative UDL-16 | (A3) | 10.00 | | | (C1) | 90. 00 | | |
| Comparative UDL-17 | (A4) | 10.00 | | | (C1) | 90. 00 | | |
| Comparative UDL-18 | (A5) | 10.00 | | | (C1) | 90. 00 | | |
| Comparative UDL-19 | (A6) | 10.00 | | | (C1) | 90. 00 | | |
| Comparative UDL-20 | (A1) | 1.50 | (R1) | 0.02 | (C1) | 98.49 | | |
| Comparative UDL-21 | (A1) | 1.50 | (R2) | 0.02 | (C1) | 98.49 | | |
| Comparative UDL-22 | (A1) | 1.50 | (R3) | 0.02 | (C1) | 98.49 | | |
| Comparative UDL-23 | (A1) | 1.50 | (R4) | 0.02 | (C1) | 98.49 | | |
| Comparative UDL-24 | (A1) | 1.50 | (R5) | 0.02 | (C1) | 98.49 | | |
| Comparative UDL-25 | (A1) | 1.50 | (R6) | 0.02 | (C1) | 98.49 | | |
| Comparative UDL-26 | (A1) | 1.50 | (R7) | 0.02 | (C1) | 98.49 | | |

### [Preparation of Silicon Wafers Having Organic Cured Film Formed Thereon by Using Compositions (UDL-1 to -109 and Comparative UDL-1 to -19) for Forming Organic Film]

Using a coater/developer "CLEAN TRACK LITHIUS Pro AP" of Tokyo Electron Ltd., each of the compositions (UDL-1 to -109 and comparative UDL-1 to -19) for forming an organic film prepared above was respectively discharged in an amount of 2 ml onto a 300 mm silicon wafer at the center. Then, the wafer was rotated at a rotational rate at which the average film thickness was as shown in Tables 2-1 to 2-5 after baking to spread the composition and form a film. While rotating the silicon wafer at a rate of 1000 rpm, the remover-discharging nozzle was moved at a speed of 5 mm/s from the periphery of the silicon wafer to a position 3 mm from the center while discharging a remover (mixed solution of propylene glycol monomethyl ether acetate and propylene glycol monomethyl ether (30:70, mass ratio)) at a discharge rate of 2 ml/s, and the remover was further discharged at that position for 5 seconds at a discharge rate of 2 ml/s. After that, the discharging of the discharged liquid was terminated, and the silicon wafer was further rotated at a rate of 1000 rpm for 30 seconds. Next, the silicon wafer having a film formed by using the composition for forming an organic film above was heated at 350°C for 60 seconds. Thus, silicon wafers having an organic cured film were obtained.

### [Resistance to Solvent Evaluation: Examples 1-1 to 1-109 and Comparative Examples 1-1 to 1-19]

A film was formed on a silicon wafer by the above-described method by using each of the compositions (UDL-1 to -109 and comparative UDL-1 to -19) for forming an organic film, and the film thickness of the film was measured. Subsequently, a PGMEA solvent was dispensed thereon, left to stand for 30 seconds, spin-dried, and baked at 100°C for 60 seconds to evaporate the PGMEA, and then the film thickness was measured. The absolute value of the value determined by (X₁-X)/X × 100 was obtained as a film thickness change rate (%), where X is the film thickness before dispensing the PGMEA solvent and X₁ is the film thickness after the PGMEA solvent was dispensed. When the film thickness change rate was lower than 0.5%, the composition was evaluated as "Good", and when 0.5% or higher, "Poor".

### [In-Plane Uniformity Evaluation: Examples 1-1 to 1-109 and Comparative Examples 1-1 to 1-19]

A film was formed on a silicon wafer by the above-described method by using each of the compositions (UDL-1 to -109 and comparative UDL-1 to -19) for forming an organic film, and the film thickness of the organic cured film was measured at 225 concentric points within a radius of 145 mm from the wafer center by optical film thickness gauge. The value determined by (Xₘₐₓ-Xₘᵢₙ)/X_{average} was obtained as in-plane uniformity (%), where Xₘₐₓ is the maximum value of the film thickness, Xₘᵢₙ is the minimum value of the film thickness, and X_{average} is the average film thickness. When the in-plane uniformity was less than 2%, the composition was evaluated as A (good), when 2% or more to less than 3%, B, and when 3% or more, C (poor).

**[Table 2-1]**

| | Composition for forming organic film | Average film thickness | Resistance to solvent | In-plane uniformity |
|---|---|---|---|---|
| Example 1-1 | UDL-1 | 500 nm | Good | A |
| Example 1-2 | UDL-2 | 500 nm | Good | A |
| Example 1-3 | UDL-3 | 500 nm | Good | A |
| Example 1-4 | UDL-4 | 500 nm | Good | A |
| Example 1-5 | UDL-5 | 500 nm | Good | A |
| Example 1-6 | UDL-6 | 500 nm | Good | A |
| Example 1-7 | UDL-7 | 500 nm | Good | A |
| Example 1-8 | UDL-8 | 500 nm | Good | A |
| Example 1-9 | UDL-9 | 500 nm | Good | A |
| Example 1-10 | UDL-10 | 500 nm | Good | A |
| Example 1-11 | UDL-11 | 500 nm | Good | A |
| Example 1-12 | UDL-12 | 500 nm | Good | A |
| Example 1-13 | UDL-13 | 500 nm | Good | A |
| Example 1-14 | UDL-14 | 500 nm | Good | A |
| Example 1-15 | UDL-15 | 500 nm | Good | A |
| Example 1-16 | UDL-16 | 500 nm | Good | A |
| Example 1-17 | UDL-17 | 500 nm | Good | A |
| Example 1-18 | UDL-18 | 500 nm | Good | A |
| Example 1-19 | UDL-19 | 500 nm | Good | A |
| Example 1-20 | UDL-20 | 500 nm | Good | A |
| Example 1-21 | UDL-21 | 500 nm | Good | A |
| Example 1-22 | UDL-22 | 500 nm | Good | A |
| Example 1-23 | UDL-23 | 500 nm | Good | A |
| Example 1-24 | UDL-24 | 500 nm | Good | A |
| Example 1-25 | UDL-25 | 500 nm | Good | A |
| Example 1-26 | UDL-26 | 500 nm | Good | A |
| Example 1-27 | UDL-27 | 500 nm | Good | A |
| Example 1-28 | UDL-28 | 500 nm | Good | A |
| Example 1-29 | UDL-29 | 500 nm | Good | A |
| Example 1-30 | UDL-30 | 500 nm | Good | A |

**[Table 2-2]**

| | Composition for forming organic film | Average film thickness | Resistance to solvent | In-plane uniformity |
|---|---|---|---|---|
| Example 1-31 | UDL-31 | 500 nm | Good | A |
| Example 1-32 | UDL-32 | 500 nm | Good | A |
| Example 1-33 | UDL-33 | 500 nm | Good | A |
| Example 1-34 | UDL-34 | 500 nm | Good | A |
| Example 1-35 | UDL-35 | 500 nm | Good | A |
| Example 1-36 | UDL-36 | 500 nm | Good | A |
| Example 1-37 | UDL-37 | 500 nm | Good | A |
| Example 1-38 | UDL-38 | 500 nm | Good | B |
| Example 1-39 | UDL-39 | 500 nm | Good | A |
| Example 1-40 | UDL-40 | 500 nm | Good | B |
| Example 1-41 | UDL-41 | 500 nm | Good | A |
| Example 1-42 | UDL-42 | 500 nm | Good | A |
| Example 1-43 | UDL-43 | 500 nm | Good | A |
| Example 1-44 | UDL-44 | 500 nm | Good | A |
| Example 1-45 | UDL-45 | 500 nm | Good | A |
| Example 1-46 | UDL-46 | 500 nm | Good | A |
| Example 1-47 | UDL-47 | 500 nm | Good | A |
| Example 1-48 | UDL-48 | 500 nm | Good | A |
| Example 1-49 | UDL-49 | 500 nm | Good | A |
| Example 1-50 | UDL-50 | 500 nm | Good | A |
| Example 1-51 | UDL-51 | 500 nm | Good | A |
| Example 1-52 | UDL-52 | 500 nm | Good | A |
| Example 1-53 | UDL-53 | 500 nm | Good | A |
| Example 1-54 | UDL-54 | 500 nm | Good | A |
| Example 1-55 | UDL-55 | 500 nm | Good | A |
| Example 1-56 | UDL-56 | 500 nm | Good | A |
| Example 1-57 | UDL-57 | 500 nm | Good | A |
| Example 1-58 | UDL-58 | 500 nm | Good | A |
| Example 1-59 | UDL-59 | 500 nm | Good | A |
| Example 1-60 | UDL-60 | 500 nm | Good | A |

**[Table 2-3]**

| | Composition for forming organic film | Average film thickness | Resistance to solvent | In-plane uniformity |
|---|---|---|---|---|
| Example 1-61 | UDL-61 | 500 nm | Good | A |
| Example 1-62 | UDL-62 | 500 nm | Good | A |
| Example 1-63 | UDL-63 | 500 nm | Good | A |
| Example 1-64 | UDL-64 | 500 nm | Good | A |
| Example 1-65 | UDL-65 | 500 nm | Good | A |
| Example 1-66 | UDL-66 | 500 nm | Good | A |
| Example 1-67 | UDL-67 | 500 nm | Good | A |
| Example 1-68 | UDL-68 | 500 nm | Good | A |
| Example 1-69 | UDL-69 | 500 nm | Good | A |
| Example 1-70 | UDL-70 | 500 nm | Good | A |
| Example 1-71 | UDL-71 | 500 nm | Good | A |
| Example 1-72 | UDL-72 | 500 nm | Good | A |
| Example 1-73 | UDL-73 | 500 nm | Good | A |
| Example 1-74 | UDL-74 | 500 nm | Good | A |
| Example 1-75 | UDL-75 | 500 nm | Good | A |
| Example 1-76 | UDL-76 | 500 nm | Good | A |
| Example 1-77 | UDL-77 | 500 nm | Good | A |
| Example 1-78 | UDL-78 | 500 nm | Good | A |
| Example 1-79 | UDL-79 | 500 nm | Good | A |
| Example 1-80 | UDL-80 | 500 nm | Good | A |
| Example 1-81 | UDL-81 | 1000 nm | Good | A |
| Example 1-82 | UDL-82 | 1000 nm | Good | A |
| Example 1-83 | UDL-83 | 1000 nm | Good | A |
| Example 1-84 | UDL-84 | 1000 nm | Good | A |
| Example 1-85 | UDL-85 | 1000 nm | Good | A |
| Example 1-86 | UDL-86 | 1000 nm | Good | A |
| Example 1-87 | UDL-87 | 1000 nm | Good | A |
| Example 1-88 | UDL-88 | 1000 nm | Good | A |
| Example 1-89 | UDL-89 | 1000 nm | Good | A |
| Example 1-90 | UDL-90 | 1000 nm | Good | A |

**[Table 2-4]**

| | Composition for forming organic film | Average film thickness | Resistance to solvent | In-plane uniformity |
|---|---|---|---|---|
| Example 1-91 | UDL-91 | 1000 nm | Good | A |
| Example 1-92 | UDL-92 | 1000 nm | Good | A |
| Example 1-93 | UDL-93 | 1000 nm | Good | A |
| Example 1-94 | UDL-94 | 1000 nm | Good | A |
| Example 1-95 | UDL-95 | 1000 nm | Good | A |
| Example 1-96 | UDL-96 | 1000 nm | Good | A |
| Example 1-97 | UDL-97 | 1000 nm | Good | A |
| Example 1-98 | UDL-98 | 1000 nm | Good | B |
| Example 1-99 | UDL-99 | 1000 nm | Good | A |
| Example 1-100 | UDL-100 | 1000 nm | Good | A |
| Example 1-101 | UDL-101 | 1000 nm | Good | A |
| Example 1-102 | UDL-102 | 1000 nm | Good | A |
| Example 1-103 | UDL-103 | 1000 nm | Good | A |
| Example 1-104 | UDL-104 | 1000 nm | Good | A |
| Example 1-105 | UDL-105 | 1000 nm | Good | A |
| Example 1-106 | UDL-106 | 1000 nm | Good | A |
| Example 1-107 | UDL-107 | 1000 nm | Good | A |
| Example 1-108 | UDL-108 | 1000 nm | Good | A |
| Example 1-109 | UDL-109 | 1000 nm | Good | A |

**[Table 2-5]**

| | Composition for forming organic film | Average film thickness | Resistance to solvent | In-plane uniformity |
|---|---|---|---|---|
| Comparative Example 1-1 | Comparative UDL-1 | 500 nm | Good | A |
| Comparative Example 1-2 | Comparative UDL-2 | 500 nm | Good | A |
| Comparative Example 1-3 | Comparative UDL-3 | 500 nm | Good | A |
| Comparative Example 1-4 | Comparative UDL-4 | 500 nm | Good | A |
| Comparative Example 1-5 | Comparative UDL-5 | 500 nm | Good | A |
| Comparative Example 1-6 | Comparative UDL-6 | 500 nm | Good | A |
| Comparative Example 1-7 | Comparative UDL-7 | 500 nm | Good | A |
| Comparative Example 1-8 | Comparative UDL-8 | 500 nm | Good | A |
| Comparative Example 1-9 | Comparative UDL-9 | 500 nm | Good | A |
| Comparative Example 1-10 | Comparative UDL-10 | 500 nm | Good | A |
| Comparative Example 1-11 | Comparative UDL-11 | 500 nm | Good | A |
| Comparative Example 1-12 | Comparative UDL-12 | 500 nm | Good | A |
| Comparative Example 1-13 | Comparative UDL-13 | 500 nm | Good | C |
| Comparative Example 1-14 | Comparative UDL-14 | 500 nm | Good | C |
| Comparative Example 1-15 | Comparative UDL-15 | 500 nm | Good | C |
| Comparative Example 1-16 | Comparative UDL-16 | 500 nm | Good | C |
| Comparative Example 1-17 | Comparative UDL-17 | 500 nm | Good | C |
| Comparative Example 1-18 | Comparative UDL-18 | 500 nm | Good | C |
| Comparative Example 1-19 | Comparative UDL-19 | 500 nm | Good | C |

### [Hump Suppression Property Evaluation: Examples 2-1 to 2-109 and Comparative Examples 2-1 to 2-12]

A film was formed on a silicon wafer by the above-described method by using each of the compositions (UDL-1 to -109 and comparative UDL-1 to -12) for forming an organic film, and the variation in height was measured from the peripheral edge of the organic film towards the center of the silicon wafer to the position of 1000 µm by using Alpha-Step D-600 (a stylus profiler) manufactured by KLA TENCOR. The height at the silicon wafer was defined to be 0, and when the maximum height of the film was less than 110% of the film thickness as shown in FIG. 1, the composition was evaluated as A (good), when the maximum height was 110% or more and less than 150%, as B, and when a region where the height was 150% or more occurred as shown in FIG. 2, as C (poor).

### [Filling Property Evaluation-1: Examples 2-1 to 2-109 and Comparative Examples 2-1 to 2-12]

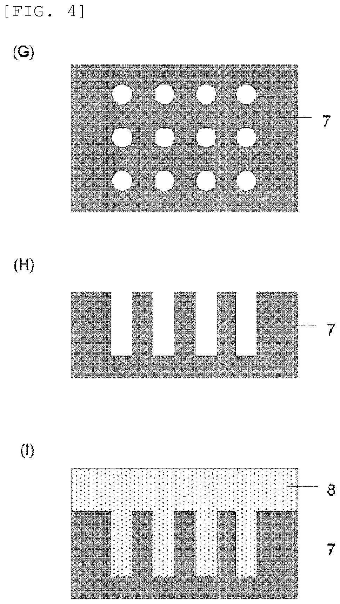

As shown in FIG. 4, a film was formed on an SiO₂ wafer substrate having a dense hole pattern (hole diameter: 0.2 µm, hole depth: 1.0 µm, distance between the centers of two adjacent holes: 0.4 µm) by using each of the compositions (UDL-1 to -109 and comparative UDL-1 to -12) for forming an organic film respectively according to the above-described method, thus a resist underlayer film 8 was formed. The substrate used was a base substrate (SiO₂ wafer substrate) 7 having the dense hole pattern shown in FIG. 4 (G) (downward view) and (H) (cross-sectional view). The cross-sectional shape of each of the obtained wafer substrates was observed using a scanning electron microscope (SEM), and it was checked whether or not the inside of the holes was filled with the resist underlayer film without a gap. When a resist underlayer film material having poor filling property is used, a gap is generated inside the hole. When a resist underlayer film material having a desirable filling property is used, the inside of the holes is filled with the resist underlayer film without a gap in this evaluation, as shown in FIG. 4 (I). When no gap was generated, the composition was evaluated as "Good", and when a gap was generated, as "Poor".

### [Filling Property Evaluation-2: Examples 2-81 to -109]

As shown in FIG. 4, a film was formed on an SiO₂ wafer substrate having a dense hole pattern (hole diameter: 0.2 µm, hole depth: 2.0 µm, distance between the centers of two adjacent holes: 0.4 µm) by using each of the compositions (UDL-81 to -109) for forming an organic film respectively according to the above-described method, thus a resist underlayer film 8 was formed. The substrate used was a base substrate (SiO₂ wafer substrate) 7 having the dense hole pattern shown in FIG. 4 (G) (downward view) and (H) (cross-sectional view). The cross-sectional shape of each of the obtained wafer substrates was observed using a scanning electron microscope (SEM), and it was checked whether or not the inside of the holes was filled with the resist underlayer film without a gap. When a resist underlayer film material having poor filling property is used, a gap is generated inside the hole. When a resist underlayer film material having a desirable filling property is used, the inside of the holes is filled with the resist underlayer film without a gap in this evaluation, as shown in FIG. 4 (I). When no gap was generated, the composition was evaluated as "Good", and when a gap was generated, as "Poor". Incidentally, in order to evaluate the superiority or inferiority of the filling property, this evaluation was performed under strict evaluation conditions in which a void is more likely to occur than the filling property evaluation-1.

### [Silicon-Containing Resist Middle Layer Film Coating Property Evaluation: Examples 2-1 to -109 and Comparative Examples 2-1 to -12]

An organic cured film was formed respectively on a silicon wafer substrate by using each of the compositions (UDL-1 to -109 and comparative UDL-1 to - 12) for forming an organic film by the above-described method, the silicon-containing resist middle layer film material (SOG1) described below was applied thereon, and baking was performed at 200°C for 60 seconds to form a silicon-containing resist middle layer film. Then, the condition of the coating film of the silicon-containing resist middle layer film was visually observed and evaluated. When the condition of the coating film was good, it was judged as good, and when a pinhole was generated, it was judged as poor. Incidentally, in order to evaluate the superiority or inferiority of the coatability of the silicon-containing resist middle layer film, this evaluation was performed under special strict evaluation conditions in which the film thickness of the silicon-containing resist middle layer film was 5 nm.

The silicon-containing resist middle layer film material (SOG1) was prepared by dissolving a polymer (SP1), a crosslinking catalyst, and an acid in an organic solvent and water at the ratios shown in Table 3, and filtering the solution with a 0.1-µm fluororesin filter.

**[Table 3]**

| | Polymer (part by mass) | Crosslinking catalyst (part by mass) | Acid (part by mass) | Organic solvent (part by mass) | Water (part by mass) |
|---|---|---|---|---|---|
| SOG1 | SP1 (100) | TMPANO₃ (1) | Maleic acid (1) | PGEE (44100) | Water (4900) |

| | | | | | |
|---|---|---|---|---|---|
| PGEE: Propylene glycol monoethyl ether | | | | | |

The polymer (SP1) is shown below. 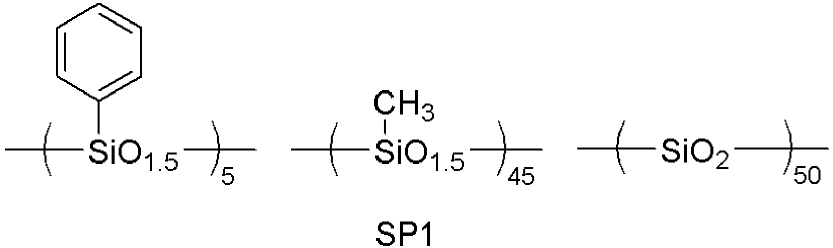
TMPANO3: Trimethylphenylammonium nitrate
PGEE: Propylene glycol ethyl ether

**[Table 4-1]**

| | Composition for forming organic film | Hump suppression property evaluation | Filling property evaluation 1 | Filling property evaluation 2 | Silicon-containing resist middle layer film coating property evaluation |
|---|---|---|---|---|---|
| Example 2-1 | UDL-1 | A | Good | - | Good |
| Example 2-2 | UDL-2 | A | Good | - | Good |
| Example 2-3 | UDL-3 | A | Good | - | Good |
| Example 2-4 | UDL-4 | A | Good | - | Good |
| Example 2-5 | UDL-5 | A | Good | - | Good |
| Example 2-6 | UDL-6 | A | Good | - | Good |
| Example 2-7 | UDL-7 | A | Good | - | Good |
| Example 2-8 | UDL-8 | A | Good | - | Good |
| Example 2-9 | UDL-9 | A | Good | - | Good |
| Example 2-10 | UDL-10 | A | Good | - | Good |
| Example 2-11 | UDL-11 | A | Good | - | Good |
| Example 2-12 | UDL-12 | A | Good | - | Good |
| Example 2-13 | UDL-13 | A | Good | - | Good |
| Example 2-14 | UDL-14 | A | Good | - | Good |
| Example 2-15 | UDL-15 | A | Good | - | Good |
| Example 2-16 | UDL-16 | A | Good | - | Good |
| Example 2-17 | UDL-17 | A | Good | - | Good |
| Example 2-18 | UDL-18 | A | Good | - | Good |
| Example 2-19 | UDL-19 | A | Good | - | Good |
| Example 2-20 | UDL-20 | A | Good | - | Good |
| Example 2-21 | UDL-21 | A | Good | - | Good |
| Example 2-22 | UDL-22 | A | Good | - | Good |
| Example 2-23 | UDL-23 | A | Good | - | Good |
| Example 2-24 | UDL-24 | A | Good | - | Good |
| Example 2-25 | UDL-25 | A | Good | - | Good |
| Example 2-26 | UDL-26 | A | Good | - | Good |
| Example 2-27 | UDL-27 | A | Good | - | Good |
| Example 2-28 | UDL-28 | A | Good | - | Good |
| Example 2-29 | UDL-29 | A | Good | - | Good |
| Example 2-30 | UDL-30 | A | Good | - | Good |

**[Table 4-2]**

| | Composition for forming organic film | Hump suppression property evaluation | Filling property evaluation 1 | Filling property evaluation 2 | Silicon-containing resist middle layer film coating property evaluation |
|---|---|---|---|---|---|
| Example 2-31 | UDL-31 | A | Good | - | Good |
| Example 2-32 | UDL-32 | A | Good | - | Good |
| Example 2-33 | UDL-33 | A | Good | - | Good |
| Example 2-34 | UDL-34 | A | Good | - | Good |
| Example 2-35 | UDL-35 | A | Good | - | Good |
| Example 2-36 | UDL-36 | A | Good | - | Good |
| Example 2-37 | UDL-37 | A | Good | - | Good |
| Example 2-38 | UDL-38 | A | Good | - | Good |
| Example 2-39 | UDL-39 | A | Good | - | Good |
| Example 2-40 | UDL-40 | A | Good | - | Good |
| Example 2-41 | UDL-41 | A | Good | - | Good |
| Example 2-42 | UDL-42 | A | Good | - | Good |
| Example 2-43 | UDL-43 | A | Good | - | Good |
| Example 2-44 | UDL-44 | A | Good | - | Good |
| Example 2-45 | UDL-45 | A | Good | - | Good |
| Example 2-46 | UDL-46 | A | Good | - | Good |
| Example 2-47 | UDL-47 | A | Good | - | Good |
| Example 2-48 | UDL-48 | A | Good | - | Good |
| Example 2-49 | UDL-49 | A | Good | - | Good |
| Example 2-50 | UDL-50 | A | Good | - | Good |
| Example 2-51 | UDL-51 | A | Good | - | Good |
| Example 2-52 | UDL-52 | A | Good | - | Good |
| Example 2-53 | UDL-53 | A | Good | - | Good |
| Example 2-54 | UDL-54 | A | Good | - | Good |
| Example 2-55 | UDL-55 | A | Good | - | Good |
| Example 2-56 | UDL-56 | A | Good | - | Good |
| Example 2-57 | UDL-57 | A | Good | - | Good |
| Example 2-58 | UDL-58 | A | Good | - | Good |
| Example 2-59 | UDL-59 | A | Good | - | Good |
| Example 2-60 | UDL-60 | A | Good | - | Good |

**[Table 4-3]**

| | Composition for forming organic film | Hump suppression property evaluation | Filling property evaluation 1 | Filling property evaluation 2 | Silicon-containing resist middle layer film coating property evaluation |
|---|---|---|---|---|---|
| Example 2-61 | UDL-61 | A | Good | - | Good |
| Example 2-62 | UDL-62 | A | Good | - | Good |
| Example 2-63 | UDL-63 | A | Good | - | Good |
| Example 2-64 | UDL-64 | A | Good | - | Good |
| Example 2-65 | UDL-65 | A | Good | - | Good |
| Example 2-66 | UDL-66 | A | Good | - | Good |
| Example 2-67 | UDL-67 | A | Good | - | Good |
| Example 2-68 | UDL-68 | A | Good | - | Good |
| Example 2-69 | UDL-69 | A | Good | - | Good |
| Example 2-70 | UDL-70 | A | Good | - | Good |
| Example 2-71 | UDL-71 | A | Good | - | Good |
| Example 2-72 | UDL-72 | A | Good | - | Good |
| Example 2-73 | UDL-73 | A | Good | - | Good |
| Example 2-74 | UDL-74 | A | Good | - | Good |
| Example 2-75 | UDL-75 | A | Good | - | Good |
| Example 2-76 | UDL-76 | A | Good | - | Good |
| Example 2-77 | UDL-77 | A | Good | - | Good |
| Example 2-78 | UDL-78 | A | Good | - | Good |
| Example 2-79 | UDL-79 | A | Good | - | Good |
| Example 2-80 | UDL-80 | A | Good | - | Good |
| Example 2-81 | UDL-81 | A | Good | Poor | Good |
| Example 2-82 | UDL-82 | A | Good | Poor | Good |
| Example 2-83 | UDL-83 | A | Good | Poor | Good |
| Example 2-84 | UDL-84 | A | Good | Poor | Good |
| Example 2-85 | UDL-85 | A | Good | Poor | Good |
| Example 2-86 | UDL-86 | A | Good | Poor | Good |
| Example 2-87 | UDL-87 | A | Good | Poor | Good |
| Example 2-88 | UDL-88 | A | Good | Poor | Good |
| Example 2-89 | UDL-89 | A | Good | Poor | Good |
| Example 2-90 | UDL-90 | A | Good | Poor | Good |

**[Table 4-4]**

| | Composition for forming organic film | Hump suppression property evaluation | Filling property evaluation 1 | Filling property evaluation 2 | Silicon-containing resist middle layer film coating property evaluation |
|---|---|---|---|---|---|
| Example 2-91 | UDL-91 | A | Good | Poor | Good |
| Example 2-92 | UDL-92 | A | Good | Poor | Good |
| Example 2-93 | UDL-93 | A | Good | Good | Good |
| Example 2-94 | UDL-94 | A | Good | Good | Good |
| Example 2-95 | UDL-95 | A | Good | Good | Good |
| Example 2-96 | UDL-96 | A | Good | Good | Good |
| Example 2-97 | UDL-97 | A | Good | Poor | Good |
| Example 2-98 | UDL-98 | A | Good | Poor | Good |
| Example 2-99 | UDL-99 | A | Good | Poor | Good |
| Example 2-100 | UDL-100 | A | Good | Poor | Good |
| Example 2-101 | UDL-101 | A | Good | Poor | Good |
| Example 2-102 | UDL-102 | A | Good | Poor | Good |
| Example 2-103 | UDL-103 | A | Good | Poor | Good |
| Example 2-104 | UDL-104 | A | Good | Poor | Good |
| Example 2-105 | UDL-105 | A | Good | Poor | Good |
| Example 2-106 | UDL-106 | A | Good | Poor | Good |
| Example 2-107 | UDL-107 | A | Good | Poor | Good |
| Example 2-108 | UDL-108 | A | Good | Poor | Good |
| Example 2-109 | UDL-109 | A | Good | Poor | Good |
| Comparative Example 2-1 | Comparative UDL-1 | C | Poor | - | Poor |
| Comparative Example 2-2 | Comparative UDL-2 | C | Poor | - | Poor |
| Comparative Example 2-3 | Comparative UDL-3 | C | Poor | - | Poor |
| Comparative Example 2-4 | Comparative UDL-4 | C | Poor | - | Poor |
| Comparative Example 2-5 | Comparative UDL-5 | C | Poor | - | Poor |
| Comparative Example 2-6 | Comparative UDL-6 | C | Poor | - | Poor |
| Comparative Example 2-7 | Comparative UDL-7 | C | Poor | - | Poor |
| Comparative Example 2-8 | Comparative UDL-8 | C | Poor | - | Poor |
| Comparative Example 2-9 | Comparative UDL-9 | C | Poor | - | Poor |
| Comparative Example 2-10 | Comparative UDL-10 | A | Poor | - | Poor |
| Comparative Example 2-11 | Comparative UDL-11 | C | Poor | - | Poor |
| Comparative Example 2-12 | Comparative UDL-12 | B | Poor | - | Poor |

As shown in Tables 2-1 to -5 and Tables 4-1 to -4, it was confirmed that the inventive composition (UDL-1 to -109) for forming an organic film is excellent in resistance to solvent, in-plane uniformity, hump suppression property, filling property, and coatability of a silicon-containing resist underlayer film.

### [Coatability Evaluation of Organic Thin Film: Examples 3-1 to -29 and Comparative Examples 3-1 to -7]

The composition (UDL-110 to -138 and comparative UDL-20 to -26)for forming an organic film was applied onto a silicon wafer substrate and baked at 200°C for 60 seconds to form a 20 nm organic film. Then, the state of the coating film of the organic film was observed using an optical microscope and evaluated. When the condition of the coating film was good, it was judged as "good", and when a pinhole was generated, as "poor". Incidentally, in order to evaluate the superiority or inferiority of the coatability of the organic film, this evaluation was performed under strict evaluation conditions in which the film thickness of the organic film was 20 nm.

**[Table 5]**

| | Composition for forming organic film | Organic thin film coating property evaluation |
|---|---|---|
| Example 3-1 | UDL-110 | Good |
| Example 3-2 | UDL-111 | Good |
| Example 3-3 | UDL-112 | Good |
| Example 3-4 | UDL-113 | Good |
| Example 3-5 | UDL-114 | Good |
| Example 3-6 | UDL-115 | Good |
| Example 3-7 | UDL-116 | Good |
| Example 3-8 | UDL-117 | Good |
| Example 3-9 | UDL-118 | Good |
| Example 3-10 | UDL-119 | Good |
| Example 3-11 | UDL-120 | Good |
| Example 3-12 | UDL-121 | Good |
| Example 3-13 | UDL-122 | Good |
| Example 3-14 | UDL-123 | Good |
| Example 3-15 | UDL-124 | Good |
| Example 3-16 | UDL-125 | Good |
| Example 3-17 | UDL-126 | Good |
| Example 3-18 | UDL-127 | Good |
| Example 3-19 | UDL-128 | Good |
| Example 3-20 | UDL-129 | Good |
| Example 3-21 | UDL-130 | Good |
| Example 3-22 | UDL-131 | Good |
| Example 3-23 | UDL-132 | Good |
| Example 3-24 | UDL-133 | Good |
| Example 3-25 | UDL-134 | Good |
| Example 3-26 | UDL-135 | Good |
| Example 3-27 | UDL-136 | Good |
| Example 3-28 | UDL-137 | Good |
| Example 3-29 | UDL-138 | Good |
| Comparative Example 3-1 | Comparative UDL-20 | Poor |
| Comparative Example 3-2 | Comparative UDL-21 | Poor |
| Comparative Example 3-3 | Comparative UDL-22 | Poor |
| Comparative Example 3-4 | Comparative UDL-23 | Poor |
| Comparative Example 3-5 | Comparative UDL-24 | Poor |
| Comparative Example 3-6 | Comparative UDL-25 | Poor |
| Comparative Example 3-7 | Comparative UDL-26 | Poor |

As shown in Table 5, it was confirmed that the inventive compositions (UDL-110 to -138) for forming an organic film were excellent in coatability.

### [Patterning Test: Examples 4-1 to -80]

An organic cured film was formed respectively on an SiO₂ wafer substrate by using each of the compositions (UDL-1 to -80) for forming an organic film according to the above-described method, the silicon-containing resist middle layer film material (SOG2) described below was applied thereon and baked at 200°C for 60 seconds to form a 35-nm thick silicon-containing resist middle layer film. The monolayer resist for ArF described below was applied thereon as a resist upper layer film material and baked at 105°C for 60 seconds to form a 100-nm thick photoresist film. The liquid immersion top coat composition (TC-1) described below was applied onto the photoresist film and baked at 90°C for 60 seconds to form a 50-nm thick top coat.

The silicon-containing resist middle layer film material (SOG2) was prepared by dissolving a polymer (SP1), a crosslinking catalyst, and an acid in an organic solvent and water at the ratios shown in Table 6, and filtering the solution with a 0.1-µm fluororesin filter.

**[Table 6]**

| | Polymer (part by mass) | Crosslinking catalyst (part by mass) | Acid (part by mass) | Organic solvent (part by mass) | Water (part by mass) |
|---|---|---|---|---|---|
| SOG2 | SP1 (100) | TMPANO₃ (1) | Maleic acid (1) | PGEE (4410) | Water (490) |

| | | | | | |
|---|---|---|---|---|---|
| PGEE: Propylene glycol monoethyl ether | | | | | |

The resist upper layer film material (monolayer resist for ArF) was prepared by dissolving a polymer (RP1), an acid generator (PAG1), and a basic compound (Amine1), each at the ratio shown in Table 7, in a solvent containing 0.1 mass% of FC-430 (manufactured by Sumitomo 3M Limited), and filtering the solution with a 0.1-µm fluororesin filter.

**[Table 7]**

| | Polymer (part by mass) | Acid generator (part by mass) | Basic compound (part by mass) | Solvent (part by mass) |
|---|---|---|---|---|
| Monolayer resist for ArF | RP1 (100) | PAG1 (6.6) | Aminel (0.8) | PGMEA (2500) |

| | | | | |
|---|---|---|---|---|
| PGMEA: Propylene glycol monomethyl ether acetate | | | | |

The polymer (RP1), the acid generator (PAG1), and the basic compound (Amine1) are shown below.

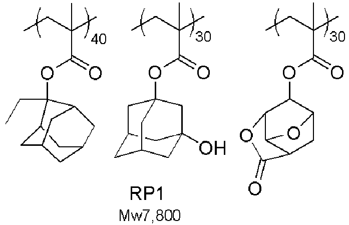

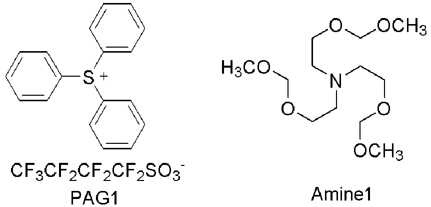

The liquid immersion top coat composition (TC-1) was prepared by dissolving a polymer (PP1) in an organic solvent at the ratios shown in Table 8, and filtering the solution with a 0.1-µm fluororesin filter.

**[Table 8]**

| | Polymer (part by mass) | Organic solvent (part by mass) |
|---|---|---|
| TC-1 | PP1 (100) | Diisoamyl ether (2700) |
| | | 2-Methyl-1-butanol (270) |

The polymer (PP1) is shown below.

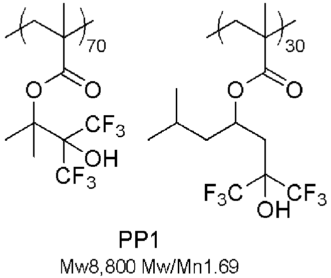

Then, the substrate was exposed to light with an ArF liquid immersion exposure apparatus (NSR-S610C manufactured by Nikon Corporation, NA 1.30, σ 0.98/0.65, 35 deg. s-polarized dipole illumination, 6% halftone phase shift mask), baked (PEB) at 100°C for 60 seconds, and developed with a 2.38 mass% aqueous solution of tetramethylammonium hydroxide (TMAH) for 30 seconds, thereby obtaining a 55 nm 1:1 positive line-and-space pattern (a resist upper layer film pattern).

Subsequently, using an etching apparatus Telius manufactured by Tokyo Electron Ltd., the silicon-containing resist underlayer film was dry-etched (the pattern was transferred) while using the resist upper layer film pattern as a mask to obtain a silicon-containing resist underlayer film pattern; the organic film was dry-etched (the pattern was transferred) while using the obtained silicon-containing resist underlayer film pattern as a mask to obtain an organic film pattern; and the SiO₂ wafer substrate (SiO₂ film) was dry-etched (the pattern was transferred) while using the obtained organic film pattern as a mask. The etching conditions were as follows.
Conditions in transferring resist upper layer film pattern to silicon-containing resist middle layer film
   Chamber pressure: 10.0 Pa
   RF-power: 1,500 W
   CF₄ gas flow rate: 75 mL/min
   O₂ gas flow rate: 15 mL/min
   Time: 15 sec
Conditions in transferring silicon-containing resist middle layer film pattern to organic film
   Chamber pressure: 2.0 Pa
   RF-power: 500 W
   Ar gas flow rate: 75 mL/min
   O₂ gas flow rate: 45 mL/min
   Time: 120 sec
Conditions in transferring organic film pattern to SiO₂ wafer substrate
   Chamber pressure: 2.0 Pa
   RF-power: 2,200 W
   C₅F₁₂ gas flow rate: 20 mL/min
   C₂F₆ gas flow rate: 10 mL/min
   Ar gas flow rate: 300 mL/min
   O₂ gas flow rate: 60 mL/min
   Time: 90 sec

The cross section of the obtained pattern was observed with an electron microscope (S-4700) manufactured by Hitachi, Ltd. The results are shown in Tables 9-1 to 9-3.

**[Table 9-1]**

| | Composition for forming organic film | Profile after etching for transferring to substrate |
|---|---|---|
| Example 4-1 | UDL-1 | Good |
| Example 4-2 | UDL-2 | Good |
| Example 4-3 | UDL-3 | Good |
| Example 4-4 | UDL-4 | Good |
| Example 4-5 | UDL-5 | Good |
| Example 4-6 | UDL-6 | Good |
| Example 4-7 | UDL-7 | Good |
| Example 4-8 | UDL-8 | Good |
| Example 4-9 | UDL-9 | Good |
| Example 4-10 | UDL-10 | Good |
| Example 4-11 | UDL-11 | Good |
| Example 4-12 | UDL-12 | Good |
| Example 4-13 | UDL-13 | Good |
| Example 4-14 | UDL-14 | Good |
| Example 4-15 | UDL-15 | Good |
| Example 4-16 | UDL-16 | Good |
| Example 4-17 | UDL-17 | Good |
| Example 4-18 | UDL-18 | Good |
| Example 4-19 | UDL-19 | Good |
| Example 4-20 | UDL-20 | Good |
| Example 4-21 | UDL-21 | Good |
| Example 4-22 | UDL-22 | Good |
| Example 4-23 | UDL-23 | Good |
| Example 4-24 | UDL-24 | Good |
| Example 4-25 | UDL-25 | Good |
| Example 4-26 | UDL-26 | Good |
| Example 4-27 | UDL-27 | Good |
| Example 4-28 | UDL-28 | Good |
| Example 4-29 | UDL-29 | Good |
| Example 4-30 | UDL-30 | Good |

**[Table 9-2]**

| | Composition for forming organic film | Profile after etching for transferring to substrate |
|---|---|---|
| Example 4-31 | UDL-31 | Good |
| Example 4-32 | UDL-32 | Good |
| Example 4-33 | UDL-33 | Good |
| Example 4-34 | UDL-34 | Good |
| Example 4-35 | UDL-35 | Good |
| Example 4-36 | UDL-36 | Good |
| Example 4-37 | UDL-37 | Good |
| Example 4-38 | UDL-38 | Good |
| Example 4-39 | UDL-39 | Good |
| Example 4-40 | UDL-40 | Good |
| Example 4-41 | UDL-41 | Good |
| Example 4-42 | UDL-42 | Good |
| Example 4-43 | UDL-43 | Good |
| Example 4-44 | UDL-44 | Good |
| Example 4-45 | UDL-45 | Good |
| Example 4-46 | UDL-46 | Good |
| Example 4-47 | UDL-47 | Good |
| Example 4-48 | UDL-48 | Good |
| Example 4-49 | UDL-49 | Good |
| Example 4-50 | UDL-50 | Good |
| Example 4-51 | UDL-51 | Good |
| Example 4-52 | UDL-52 | Good |
| Example 4-53 | UDL-53 | Good |
| Example 4-54 | UDL-54 | Good |
| Example 4-55 | UDL-55 | Good |
| Example 4-56 | UDL-56 | Good |
| Example 4-57 | UDL-57 | Good |
| Example 4-58 | UDL-58 | Good |
| Example 4-59 | UDL-59 | Good |
| Example 4-60 | UDL-60 | Good |

**[Table 9-3]**

| | Composition for forming organic film | Profile after etching for transferring to substrate |
|---|---|---|
| Example 4-61 | UDL-61 | Good |
| Example 4-62 | UDL-62 | Good |
| Example 4-63 | UDL-63 | Good |
| Example 4-64 | UDL-64 | Good |
| Example 4-65 | UDL-65 | Good |
| Example 4-66 | UDL-66 | Good |
| Example 4-67 | UDL-67 | Good |
| Example 4-68 | UDL-68 | Good |
| Example 4-69 | UDL-69 | Good |
| Example 4-70 | UDL-70 | Good |
| Example 4-71 | UDL-71 | Good |
| Example 4-72 | UDL-72 | Good |
| Example 4-73 | UDL-73 | Good |
| Example 4-74 | UDL-74 | Good |
| Example 4-75 | UDL-75 | Good |
| Example 4-76 | UDL-76 | Good |
| Example 4-77 | UDL-77 | Good |
| Example 4-78 | UDL-78 | Good |
| Example 4-79 | UDL-79 | Good |
| Example 4-80 | UDL-80 | Good |

As shown in Tables 9-1 to -3, in Examples 4-1 to - 80, where the inventive compositions (UDL-1 to -80) for forming an organic film were used, the resist upper layer film pattern was successfully transferred to the SiO₂ wafer substrate in the end in every case. Thus, it was confirmed that the inventive compositions for forming an organic film can be used suitably for fine processing using a multilayer resist method.

### [Preparation of Resist Upper Layer Film Material (PR1 to 29 and Comparative PR]

Each of resist upper layer film materials (PR1 to PR29 and comparative PR) was prepared by dissolving a polymer (P-1), an acid generator (PAG-2), a quencher (Q-1), and a polymer (B1 to B29) in a solvent at the ratios shown in Table 10 and filtering the resultant solution with a 0.1-um fluororesin filter.

**[Table 10]**

| Resist upper layer film material | Polymer (part by mass) | Additive (part by mass) | Polymer (part by mass) | Solvent (part by mass) |
|---|---|---|---|---|
| PR1 | P-1 (100) | PAG-2 (25.0) | (B1) | PGMEA(1800) |
| | | Q-1(6.6) | (0.1) | EL(1200) |
| PR2 | P-1 (100) | PAG-2 (25.0) | (B2) | PGMEA(1800) |
| | | Q-1(6.6) | (0.1) | EL(1200) |
| PR3 | P-1 (100) | PAG-2 (25.0) | (B3) | PGMEA(1800) |
| | | Q-1(6.6) | (0.1) | EL(1200) |
| PR4 | P-1 (100) | PAG-2(25.0) | (B4) | PGMEA(1800) |
| | | Q-1(6.6) | (0.1) | EL(1200) |
| PR5 | P-1 (100) | PAG-2 (25.0) | (B5) | PGMEA(1800) |
| | | Q-1(6.6) | (0.1) | EL(1200) |
| PR6 | P-1 (100) | PAG-2(25.0) | (B6) | PGMEA(1800) |
| | | Q-1(6.6) | (0.1) | EL(1200) |
| PR7 | P-1 (100) | PAG-2 (25.0) | (B7) | PGMEA(1800) |
| | | Q-1(6.6) | (0.1) | EL(1200) |
| PR8 | P-1 (100) | PAG-2(25.0) | (B8) | PGMEA(1800) |
| | | Q-1(6.6) | (0.1) | EL(1200) |
| PR9 | P-1 (100) | PAG-2 (25.0) | (B9) | PGMEA(1800) |
| | | Q-1(6.6) | (0.1) | EL(1200) |
| PR10 | P-1 (100) | PAG-2 (25.0) | (B10) | PGMEA(1800) |
| | | Q-1(6.6) | (0.1) | EL(1200) |
| PR11 | P-1 (100) | PAG-2 (25.0) | (B11) | PGMEA(1800) |
| | | Q-1(6.6) | (0.1) | EL(1200) |
| PR12 | P-1 (100) | PAG-2 (25.0) | (B12) | PGMEA(1800) |
| | | Q-1(6.6) | (0.1) | EL(1200) |
| PR13 | P-1 (100) | PAG-2 (25.0) | (B13) | PGMEA(1800) |
| | | Q-1(6.6) | (0.1) | EL(1200) |
| PR14 | P-1 (100) | PAG-2 (25.0) | (B14) | PGMEA(1800) |
| | | Q-1(6.6) | (0.1) | EL(1200) |
| PR15 | P-1 (100) | PAG-2 (25.0) | (B15) | PGMEA(1800) |
| | | Q-1(6.6) | (0.1) | EL(1200) |
| PR16 | P-1 (100) | PAG-2 (25.0) | (B16) | PGMEA(1800) |
| | | Q-1(6.6) | (0.1) | EL(1200) |
| PR17 | P-1 (100) | PAG-2 (25.0) | (B17) | PGMEA(1800) |
| | | Q-1(6.6) | (0.1) | EL(1200) |
| PR18 | P-1 (100) | PAG-2 (25.0) | (B18) | PGMEA(1800) |
| | | Q-1(6.6) | (0.1) | EL(1200) |
| PR19 | P-1 (100) | PAG-2 (25.0) | (B19) | PGMEA(1800) |
| | | Q-1(6.6) | (0.1) | EL(1200) |
| PR20 | P-1 (100) | PAG-2 (25.0) | (B20) | PGMEA(1800) |
| | | Q-1(6.6) | (0.1) | EL(1200) |
| PR21 | P-1 (100) | PAG-2 (25.0) | (B21) | PGMEA(1800) |
| | | Q-1(6.6) | (0.1) | EL(1200) |
| PR22 | P-1 (100) | PAG-2 (25.0) | (B22) | PGMEA(1800) |
| | | Q-1(6.6) | (0.1) | EL(1200) |
| PR23 | P-1 (100) | PAG-2 (25.0) | (B23) | PGMEA(1800) |
| | | Q-1(6.6) | (0.1) | EL(1200) |
| PR24 | P-1 (100) | PAG-2 (25.0) | (B24) | PGMEA(1800) |
| | | Q-1(6.6) | (0.1) | EL(1200) |
| PR25 | P-1 (100) | PAG-2 (25.0) | (B25) | PGMEA(1800) |
| | | Q-1(6.6) | (0.1) | EL(1200) |
| PR26 | P-1 (100) | PAG-2 (25.0) | (B26) | PGMEA(1800) |
| | | Q-1(6.6) | (0.1) | EL(1200) |
| PR27 | P-1 (100) | PAG-2 (25.0) | (B27) | PGMEA(1800) |
| | | Q-1(6.6) | (0.1) | EL(1200) |
| PR28 | P-1 (100) | PAG-2 (25.0) | (B28) | PGMEA(1800) |
| | | Q-1(6.6) | (0.1) | EL(1200) |
| PR29 | P-1 (100) | PAG-2 (25.0) | (B29) | PGMEA(1800) |
| | | Q-1(6.6) | (0.1) | EL(1200) |
| Comparative PR | P-1 (100) | PAG-2 (25.0) | - | PGMEA(1800) |
| | | Q-1(6.6) | | EL(1200) |

The polymer (P-1), the acid generator (PAG-2), the quencher (Q-1), and the solvent are shown below. 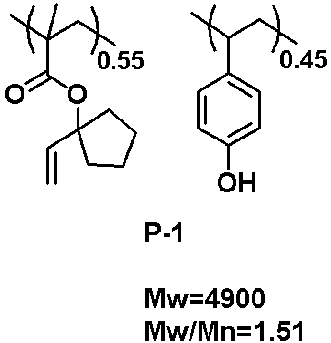 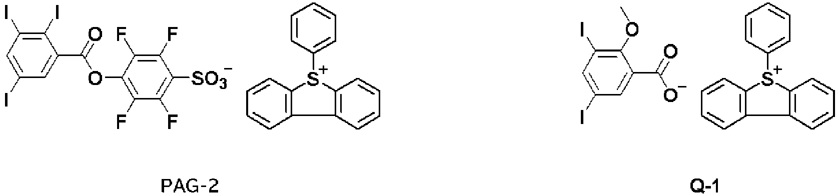
PGMEA: Propylene glycol monomethyl ether acetate
EL: Ethyl lactate

### [Preparation of Silicon wafer Having Resist Upper Layer Film Using Resist Upper Layer Film Material (PR1 to 29 and Comparative PR)]

Using a coater/developer "CLEAN TRACK LITHIUS Pro AP" of Tokyo Electron Ltd., each of the resist upper layer film materials (PR1 to PR29 and comparative PR) prepared above was respectively discharged in an amount of 2 ml onto the center of a silicon wafer. The wafer was rotated to spread the material at a rotational rate at which the average film thickness was 45 nm after baking. Next, the silicon wafer coated with the resist upper layer film above was heated at 110°C for 30 seconds. Thus, silicon wafers having a resist upper layer film were obtained.

### [In-Plane Uniformity Evaluation of Resist Upper Layer Film: Example 5-1 to -29 and Comparative Example 5-1]

The film thickness of the resist upper layer films (PR1 to PR29 and comparative PR) formed on the silicon wafer by the above-described method was measured at 225 concentric points within a radius of 145 mm from the wafer center by optical film thickness gauge. The value determined by (Xₘₐₓ-Xₘᵢₙ)/X_{average} was defined as in-plane uniformity (%), where Xₘₐₓ represents the maximum value of the film thickness, Xₘᵢₙ represents the minimum value of the film thickness, and X_{average} is the average film thickness. When the in-plane uniformity was less than 3%, the composition was evaluated as "good", when 3% or more, as "poor."

**[Table 11]**

| | Resist upper layer film material | In-plane uniformity |
|---|---|---|
| Example 5-1 | PR1 | Good |
| Example 5-2 | PR2 | Good |
| Example 5-3 | PR3 | Good |
| Example 5-4 | PR4 | Good |
| Example 5-5 | PR5 | Good |
| Example 5-6 | PR6 | Good |
| Example 5-7 | PR7 | Good |
| Example 5-8 | PR8 | Good |
| Example 5-9 | PR9 | Good |
| Example 5-10 | PR10 | Good |
| Example 5-11 | PR11 | Good |
| Example 5-12 | PR12 | Good |
| Example 5-13 | PR13 | Good |
| Example 5-14 | PR14 | Good |
| Example 5-15 | PR15 | Good |
| Example 5-16 | PR16 | Good |
| Example 5-17 | PR17 | Good |
| Example 5-18 | PR18 | Good |
| Example 5-19 | PR19 | Good |
| Example 5-20 | PR20 | Good |
| Example 5-21 | PR21 | Good |
| Example 5-22 | PR22 | Good |
| Example 5-23 | PR23 | Good |
| Example 5-24 | PR24 | Good |
| Example 5-25 | PR25 | Good |
| Example 5-26 | PR26 | Good |
| Example 5-27 | PR27 | Good |
| Example 5-28 | PR28 | Good |
| Example 5-29 | PR29 | Good |
| Comparative Example 5-1 | Comparative PR | Poor |

As shown in Table 11, the inventive resist upper layer film materials (PR1 to PR29) has excellent in-plane uniformity, and thus this indicates that the inventive polymer functions as a surfactant that imparts excellent leveling performance and can be used in various compositions for forming an organic film regardless of the type of resin used in combination.

### [EUV Lithography Evaluation: Examples 6-1 to -29]

Each of resist upper layer film materials (PR1 to PR29) shown in Table 10 was spin-coated respectively on a Si substrate on which a silicon-containing spin-on hard mask SHB-A940 (silicon content: 43 mass%) manufactured by Shin-Etsu Chemical Co., Ltd. was formed with a film thickness of 20 nm, and pre-baked at 110°C for 30 seconds on a hot plate to prepare a resist film with a film thickness of 45 nm. The resist film was exposed to light using an EUV scanner NXE3400 (NA 0.33, σ 0.9/0.7, dipole illumination) manufactured by ASML; PEB was performed on a hot plate at 80°C for 60 seconds; and development was performed with a 2.38 mass% TMAH aqueous solution for 30 seconds to form a line-and-space pattern with a 40 nm pitch and a 20 nm line width. The pattern was observed using a length measuring SEM (CG6300) manufactured by Hitachi High-Technologies Corporation, and it was evaluated as "good" when a line pattern was formed with a dimension of 20 nm ± 2.0 nm.

**[Table 12]**

| | Resist upper layer film material | In-plane uniformity |
|---|---|---|
| Example 6-1 | PR1 | Good |
| Example 6-2 | PR2 | Good |
| Example 6-3 | PR3 | Good |
| Example 6-4 | PR4 | Good |
| Example 6-5 | PR5 | Good |
| Example 6-6 | PR6 | Good |
| Example 6-7 | PR7 | Good |
| Example 6-8 | PR8 | Good |
| Example 6-9 | PR9 | Good |
| Example 6-10 | PR10 | Good |
| Example 6-11 | PR11 | Good |
| Example 6-12 | PR12 | Good |
| Example 6-13 | PR13 | Good |
| Example 6-14 | PR14 | Good |
| Example 6-15 | PR15 | Good |
| Example 6-16 | PR16 | Good |
| Example 6-17 | PR17 | Good |
| Example 6-18 | PR18 | Good |
| Example 6-19 | PR19 | Good |
| Example 6-20 | PR20 | Good |
| Example 6-21 | PR21 | Good |
| Example 6-22 | PR22 | Good |
| Example 6-23 | PR23 | Good |
| Example 6-24 | PR24 | Good |
| Example 6-25 | PR25 | Good |
| Example 6-26 | PR26 | Good |
| Example 6-27 | PR27 | Good |
| Example 6-28 | PR28 | Good |
| Example 6-29 | PR29 | Good |

As shown in Table 12, in Examples 6-1 to -29 using the inventive composition (PR1 to PR29) for forming an organic film, a resist upper layer film pattern of 20 nm was formed in each case, and it was confirmed that the resist upper layer film material, which is the inventive composition for forming an organic film, is suitably used for fine processing.

### [Preparation of Silicon-Containing Resist Middle Layer Film Material (SOG3 to SOG31)]

As a silicon-containing resist middle layer film material, each of the silicon-containing resist underlayer film materials (SOG3 to 31) was prepared by dissolving a polymer (SP1), a crosslinking catalyst, and an acid in an organic solvent and water at the ratios shown in Table 13, and filtering the solution with a 0.1-µm fluororesin filter.

**[Table 13]**

| Material for forming silicon-containing resist middle layer film | Polymer (part by mass) | Crosslinking catalyst (part by mass) | Acid (part by mass) | Compound (part by mass) | Organic solvent (part by mass) | Water (part by mass) |
|---|---|---|---|---|---|---|
| SOG3 | SP1 (100) | TMPANO3 (1.0) | Maleic acid (1.0) | (B1) | PGEE (44100) | Water (4900) |
| | | | | (1.0) | | |
| SOG4 | SP1 (100) | TMPANO3 (1.0) | Maleic acid (1.0) | (B2) | PGEE (44100) | Water (4900) |
| | | | | (1.0) | | |
| SOG5 | SP1 (100) | TMPANO3 (1.0) | Maleic acid (1.0) | (B3) | PGEE (44100) | Water (4900) |
| | | | | (1.0) | | |
| SOG6 | SP1 (100) | TMPANO3 (1.0) | Maleic acid (1.0) | (B4) | PGEE (44100) | Water (4900) |
| | | | | (1.0) | | |
| SOG7 | SP1 (100) | TMPANO3 (1.0) | Maleic acid (1.0) | (B5) | PGEE (44100) | Water (4900) |
| | | | | (1.0) | | |
| SOG8 | SP1 (100) | TMPANO3 (1.0) | Maleic acid (1.0) | (B6) | PGEE (44100) | Water (4900) |
| | | | | (1.0) | | |
| SOG9 | SP1 (100) | TMPANO3 (1.0) | Maleic acid (1.0) | (B7) | PGEE (44100) | Water (4900) |
| | | | | (1.0) | | |
| SOG10 | SP1 (100) | TMPANO3 (1.0) | Maleic acid (1.0) | (B8) | PGEE (44100) | Water (4900) |
| | | | | (1.0) | | |
| SOG11 | SP1 (100) | TMPANO3 (1.0) | Maleic acid (1.0) | (B9) | PGEE (44100) | Water (4900) |
| | | | | (1.0) | | |
| SOG12 | SP1 (100) | TMPANO3 (1.0) | Maleic acid (1.0) | (B10) | PGEE (44100) | Water (4900) |
| | | | | (1.0) | | |
| SOG13 | SP1 (100) | TMPANO3 (1.0) | Maleic acid (1.0) | (B11) | PGEE (44100) | Water (4900) |
| | | | | (1.0) | | |
| SOG14 | SP1 (100) | TMPANO3 (1.0) | Maleic acid (1.0) | (B12) | PGEE (44100) | Water (4900) |
| | | | | (1.0) | | |
| SOG15 | SP1 (100) | TMPANO3 (1.0) | Maleic acid (1.0) | (B13) | PGEE (44100) | Water (4900) |
| | | | | (1.0) | | |
| SOG16 | SP1 (100) | TMPANO3 (1.0) | Maleic acid (1.0) | (B14) | PGEE (44100) | Water (4900) |
| | | | | (1.0) | | |
| SOG17 | SP1 (100) | TMPANO3 (1.0) | Maleic acid (1.0) | (B15) | PGEE (44100) | Water (4900) |
| | | | | (1.0) | | |
| SOG18 | SP1 (100) | TMPANO3 (1.0) | Maleic acid (1.0) | (B16) | PGEE (44100) | Water (4900) |
| | | | | (1.0) | | |
| SOG19 | SP1 (100) | TMPANO3 (1.0) | Maleic acid (1.0) | (B17) | PGEE (44100) | Water (4900) |
| | | | | (1.0) | | |
| SOG20 | SP1 (100) | TMPANO3 (1.0) | Maleic acid (1.0) | (B18) | PGEE (44100) | Water (4900) |
| | | | | (1.0) | | |
| SOG21 | SP1 (100) | TMPANO3 (1.0) | Maleic acid (1.0) | (B19) | PGEE (44100) | Water (4900) |
| | | | | (1.0) | | |
| SOG22 | SP1 (100) | TMPANO3 (1.0) | Maleic acid (1.0) | (B20) | PGEE (44100) | Water (4900) |
| | | | | (1.0) | | |
| SOG23 | SP1 (100) | TMPANO3 (1.0) | Maleic acid (1.0) | (B21) | PGEE (44100) | Water (4900) |
| | | | | (1.0) | | |
| SOG24 | SP1 (100) | TMPANO3 (1.0) | Maleic acid (1.0) | (B22) | PGEE (44100) | Water (4900) |
| | | | | (1.0) | | |
| SOG25 | SP1 (100) | TMPANO3 (1.0) | Maleic acid (1.0) | (B23) | PGEE (44100) | Water (4900) |
| | | | | (1.0) | | |
| SOG26 | SP1 (100) | TMPANO3 (1.0) | Maleic acid (1.0) | (B24) | PGEE (44100) | Water (4900) |
| | | | | (1.0) | | |
| SOG27 | SP1 (100) | TMPANO3 (1.0) | Maleic acid (1.0) | (B25) | PGEE (44100) | Water (4900) |
| | | | | (1.0) | | |
| SOG28 | SP1 (100) | TMPANO3 (1.0) | Maleic acid (1.0) | (B26) | PGEE (44100) | Water (4900) |
| | | | | (1.0) | | |
| SOG29 | SP1 (100) | TMPANO3 (1.0) | Maleic acid (1.0) | (B27) | PGEE (44100) | Water (4900) |
| | | | | (1.0) | | |
| SOG30 | SP1 (100) | TMPANO3 (1.0) | Maleic acid (1.0) | (B28) | PGEE (44100) | Water (4900) |
| | | | | (1.0) | | |
| SOG31 | SP1 (100) | TMPANO3 (1.0) | Maleic acid (1.0) | (B29) | PGEE (44100) | Water (4900) |
| | | | | (1.0) | | |

| | | | | | | |
|---|---|---|---|---|---|---|
| PGEE: Propylene glycol monoethyl ether | | | | | | |

### [Coatability Evaluation of Silicon-Containing Resist Middle Layer Film: Examples 7-1 to -29 and Comparative Example 7-1]

An organic cured film was formed respectively on a silicon wafer substrate by using the compositions (comparative UDL-1) for forming an organic film by the above-described method; the silicon-containing resist middle layer film material (SOG1 and SOG3 to SOG31) described below was applied thereon; and baking was performed at 200°C for 60 seconds to form a silicon-containing resist middle layer film. Then, the condition of the coating film of the silicon-containing resist middle layer film was visually observed and evaluated. When the condition of the coating film was good, it was judged as "good," and when a pinhole was generated, as "poor." Incidentally, in order to evaluate the superiority or inferiority of the coatability of the silicon-containing resist middle layer film, this evaluation was performed under special strict evaluation conditions in which the film thickness of the silicon-containing resist middle layer film was 5 nm.

**[Table 14]**

| | Material for forming silicon-containing resist middle layer film | Silicon-containing resist middle layer film coating property evaluation |
|---|---|---|
| Example 7-1 | SOG3 | Good |
| Example 7-2 | SOG4 | Good |
| Example 7-3 | SOG5 | Good |
| Example 7-4 | SOG6 | Good |
| Example 7-5 | SOG7 | Good |
| Example 7-6 | SOG8 | Good |
| Example 7-7 | SOG9 | Good |
| Example 7-8 | SOG10 | Good |
| Example 7-9 | SOG11 | Good |
| Example 7-10 | SOG12 | Good |
| Example 7-11 | SOG13 | Good |
| Example 7-12 | SOG14 | Good |
| Example 7-13 | SOG15 | Good |
| Example 7-14 | SOG16 | Good |
| Example 7-15 | SOG17 | Good |
| Example 7-16 | SOG18 | Good |
| Example 7-17 | SOG19 | Good |
| Example 7-18 | SOG20 | Good |
| Example 7-19 | SOG21 | Good |
| Example 7-20 | SOG22 | Good |
| Example 7-21 | SOG23 | Good |
| Example 7-22 | SOG24 | Good |
| Example 7-23 | SOG25 | Good |
| Example 7-24 | SOG26 | Good |
| Example 7-25 | SOG27 | Good |
| Example 7-26 | SOG28 | Good |
| Example 7-27 | SOG29 | Good |
| Example 7-28 | SOG30 | Good |
| Example 7-29 | SOG31 | Good |
| Comparative Example 7-1 | SOG1 | Poor |

As shown in Table 14, the silicon-containing resist middle layer film materials (SOG3 to SOG31) do not generate a pinhole and have excellent film-formability. This indicates that the inventive polymer can be applied as a surfactant exhibiting high film-formability, not only for organic films, but also for various composition for forming a film.

### [Resist Patterning Evaluation in ArF Liquid-Immersion Lithography: Examples 8-1 to 8-14 and Comparative Examples 8-1 to 8-2]

Each of resist upper layer film materials (PR-30 to -43 and comparative PR-2) was prepared by dissolving a polymer (P-2), an acid generator (PAG-3), a quencher (Q-2), a polymer (B4 and B30 to B42) as a water-repellent material for resist surface, and a polymer (B9 to B18) as a surfactant in a solvent at the ratios shown in Table 15 and filtering the resultant solution with a 0.1-µm fluororesin filter.
PGMEA: Propylene glycol monomethyl ether acetate
GBL: Gamma butyrolactone

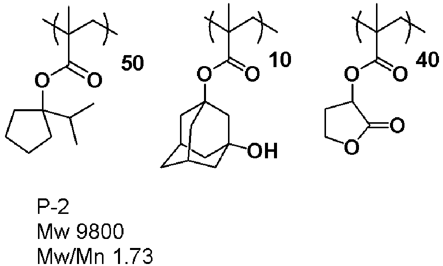

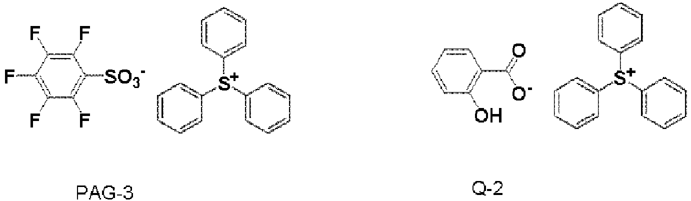

**[Table 15]**

| Resist material | Polymer (parts by mass) | Acid generator (parts by mass) | Quencher (parts by mass) | Water repellent (parts by mass) | Surfactant (parts by mass) | Organic solvent (parts by mass) |
|---|---|---|---|---|---|---|
| PR-30 | P-2 (100) | PAG-3 (10.0) | Q-2 (5.0) | B-4 (3.0) | B-9 (0.1) | PGMEA(2,000) |
| | | | | | | GBL(200) |
| PR-31 | P-2 (100) | PAG-3 (10.0) | Q-2 (5.0) | B-30 (4.0) | B-10 (0.1) | PGMEA(2,000) |
| | | | | | | GBL(200) |
| PR-32 | P-2 (100) | PAG-3 (10.0) | Q-2 (5.0) | B-31 (4.0) | B-11 (0.1) | PGMEA(2,000) |
| | | | | | | GBL(200) |
| PR-33 | P-2 (100) | PAG-3 (10.0) | Q-2 (5.0) | B-32 (4.0) | B-12 (0.1) | PGMEA(2,000) |
| | | | | | | GBL(200) |
| PR-34 | P-2 (100) | PAG-3 (10.0) | Q-2 (5.0) | B-33 (4.0) | B-13 (0.1) | PGMEA(2,000) |
| | | | | | | GBL(200) |
| PR-35 | P-2 (100) | PAG-3 (10.0) | Q-2 (5.0) | B-34 (4.0) | B-14 (0.1) | PGMEA(2,000) |
| | | | | | | GBL(200) |
| PR-36 | P-2 (100) | PAG-3 (10.0) | Q-2 (5.0) | B-35 (4.0) | B-15 (0.1) | PGMEA(2,000) |
| | | | | | | GBL(200) |
| PR-37 | P-2 (100) | PAG-3 (10.0) | Q-2 (5.0) | B-36 (4.0) | B-16 (0.1) | PGMEA(2,000) |
| | | | | | | GBL(200) |
| PR-38 | P-2 (100) | PAG-3 (10.0) | Q-2 (5.0) | B-37 (5.0) | B-17 (0.1) | PGMEA(2,000) |
| | | | | | | GBL(200) |
| PR-39 | P-2 (100) | PAG-3 (10.0) | Q-2 (5.0) | B-38 (4.0) | B-18 (0.1) | PGMEA(2,000) |
| | | | | | | GBL(200) |
| PR-40 | P-2 (100) | PAG-3 (10.0) | Q-2 (5.0) | B-39 (6.0) | B-18 (0.1) | PGMEA(2,000) |
| | | | | | | GBL(200) |
| PR-41 | P-2 (100) | PAG-3 (10.0) | Q-2 (5.0) | B-40 (3.0) | B-9 (0.1) | PGMEA(2,000) |
| | | | | | | GBL(200) |
| PR-42 | P-2 (100) | PAG-3 (10.0) | Q-2 (5.0) | B-41 (4.0) | B-9 (0.1) | PGMEA(2,000) |
| | | | | | | GBL(200) |
| PR-43 | P-2 (100) | PAG-3 (10.0) | Q-2 (5.0) | B-42 (4.0) | B-9 (0.1) | PGMEA(2,000) |
| | | | | | | GBL(200) |
| Comparative PR-2 | P-2 (100) | PAG-3 (10.0) | Q-2 (5.0) | - | - | PGMEA(2,000) |
| | | | | | | GBL(200) |

UDL-1 was applied onto a silicon substrate and baked at 350°C for 60 seconds to prepare an underlayer film with a film thickness of 500 nm. A silicon-containing resist middle layer film material (SOG2) was applied thereon and baked at 200°C for 60 seconds to form a silicon-containing resist middle layer film with a film thickness of 35 nm. On top of that, the following monolayer resist for ArF was applied thereon as a resist upper layer film material shown in Table 15, and baked at 105°C for 60 seconds to form a photoresist film with a film thickness of 100 nm. The contact angle of water on the photoresist film after baking was measured. The results are shown in Table 16.

Using an ArF excimer laser immersion lithography scanner (NSR-S610C manufacture by Nikon Corporation, NA 1.30, σ 0.90/0.72, opening 35 deg., cross-pole illumination, azimuthally polarized illumination, 6% halftone phase shift mask, dipole illumination), exposure of a line-and-space pattern (LS pattern) with an on-wafer size of 45 nm and a pitch of 90 nm was performed while varying the dose and focus, and then the wafer was baked (PEB) at 90°C for 60 seconds. After the PEB, in the case of Example 8-1 and Comparative Example 8-1, the wafer was puddle developed with n-butyl acetate for 30 seconds and spin-dried to form a negative pattern. After the PEB, in the case of Example 8-2 to - 14 and Comparative Example 8-2, the wafer was puddle developed in a 2.38 mass% TMAH aqueous solution for 30 seconds, rinsed with pure water and spin-dried to form a positive pattern. The exposure amount at which the line dimension was within 40 nm ± 4 nm using a length measuring SEM (CG-6300) manufactured by Hitachi High Technologies was defined as the optimum exposure amount (Eop). The developed wafer was cut into pieces, and the cross section of the LS pattern exposed at the optimum exposure amount was observed using the SEM. The results are shown in Table 16.

**[Table 16]**

| | Resist material | Eop (mJ/cm²) | Cross-sectional shape | Contact angle with water (degree) |
|---|---|---|---|---|
| Example 8-1 | PR-30 | 36.0 | Rectangle | 88 |
| Example 8-2 | PR-31 | 35.5 | Rectangle | 82 |
| Example 8-3 | PR-32 | 35.5 | Rectangle | 84 |
| Example 8-4 | PR-33 | 35.0 | Rectangle | 82 |
| Example 8-5 | PR-34 | 36.5 | Rectangle | 81 |
| Example 8-6 | PR-35 | 36.5 | Rectangle | 82 |
| Example 8-7 | PR-36 | 35.0 | Rectangle | 82 |
| Example 8-8 | PR-37 | 35.0 | Rectangle | 81 |
| Example 8-9 | PR-38 | 35.0 | Rectangle | 82 |
| Example 8-10 | PR-39 | 35.0 | Rectangle | 85 |
| Example 8-11 | PR-40 | 33.5 | Rectangle | 85 |
| Example 8-12 | PR-41 | 36.1 | Rectangle | 84 |
| Example 8-13 | PR-42 | 35.5 | Rectangle | 82 |
| Example 8-14 | PR-43 | 35.5 | Rectangle | 85 |
| Comparative Example 8-1 | Comparative PR-2 | 34.5 | Rounded pattern-top | 61 |
| Comparative Example 8-2 | Comparative PR-2 | 35.5 | T-shape pattern-top | 61 |

As shown in Table 16, it can be seen that the inventive composition for forming a film can form a good pattern even in liquid-immersion exposure.

From the above, the inventive composition for forming an organic film has excellent film-formability, high filling property, hump suppression property, and excellent coating property of silicon-containing resist middle layer films, and therefore, is extremely useful as a material for an organic film to be used in a multilayer resist process. In addition, the inventive patterning processes, using the inventive composition for forming an organic film, make it possible to fill holes and trenches having a very high aspect ratio without a gap, form a fine pattern with high precision, and form a hump-suppressed organic film. Further, when used in resist, it has excellent coating uniformity and good pattern profile, and when applied to liquid-immersion exposure, it improves the water repellency of the resist film surface, and therefore, make it possible to manufacture semiconductor devices and the like efficiently.

Furthermore, the inventive polymer exhibits high film-formability and can be widely used as a surfactant.

The present description includes the following embodiments.
[1]: A composition for forming a film comprising a polymer having an aromatic group having a pentafluorosulfanyl group as a substituent.
[2]: The composition for forming a film of the above [1], wherein the polymer is a polymer (a) having a repeating unit "a" having an aromatic group having a pentafluorosulfanyl group as a substituent.
[3]: The composition for forming a film of the above [2], wherein the repeating unit "a" is represented by any one of the following formulae (a1) to (a4), 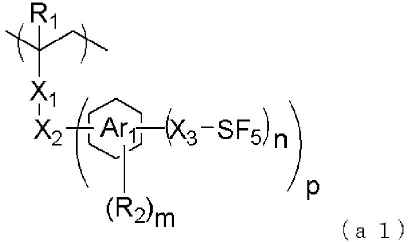
   wherein, "p" represents an integer of 1 to 3; "n" represents an integer of 0 to 5; "m" represents an integer of 0 to 4; R₁ represents a hydrogen atom or a methyl group; R₂ represents a hydrocarbyl group having 1 to 6 carbon atoms, a hydrocarbyloxy group having 1 to 12 carbon atoms, a hydrocarbyloxycarbonyl group having 2 to 6 carbon atoms, a hydrocarbylcarbonyloxy group having 2 to 12 carbon atoms, a hydroxy group, a carboxy group, a halogen atom, a trifluoromethoxy group, a cyano group, or a nitro group; X₁ represents a single bond, an ester bond, an ether bond, a sulfonic acid ester group, a sulfonamide group, an amide bond, or a phenylene group; X₂ represents a single bond or a hydrocarbylene group having 1 to 20 carbon atoms when "p" represents 1, and X₂ represents a (p+1)-valent hydrocarbon group having 1 to 20 carbon atoms when "p" represents 2 or 3, and the hydrocarbylene group and the (p+1)-valent hydrocarbon group may contain at least one atom selected from the group consisting of an oxygen atom, a sulfur atom, a nitrogen atom, and a halogen atom; X₃ represents a single bond or an ether bond; and Ar₁ each independently represents a (m+n+1)-valent group derived from benzene or naphthalene, provided that at least one of "n"s in the formula represents 1 or more, 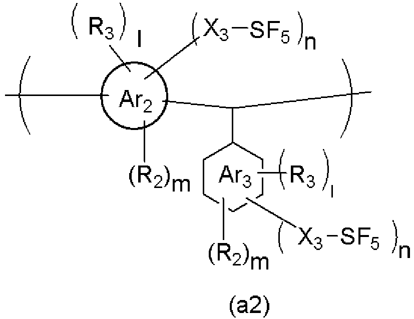
   wherein, "n" and "m" represent the same as defined above, "l" represents an integer of 0 to 3, and at least one of the two "n"s and the two "1"s in the formula represents 1 or more; R₂ and X₃ represent the same as defined above; and R₃ represents the following general formula (a2-1); Ar₂ each independently represents a (n+m+l+2)-valent aromatic hydrocarbon group having 6 to 30 carbon atoms; and Ar₃ represents a (n+m+l+1)-valent group derived from benzene or naphthalene, 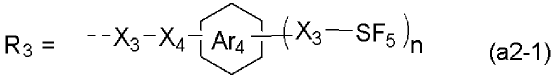
   wherein, X₃ and "n" represent the same as above; X₄ represents a single bond or a hydrocarbylene group having 1 to 12 carbon atoms, and the hydrocarbylene group may have at least one selected from the group consisting of a hydroxy group, a saturated hydrocarbyloxy group having 1 to 6 carbon atoms, an ether bond, an ester bond, and an amide bond; and Ar₄ represents a (n+1)-valent group derived from benzene or naphthalene, 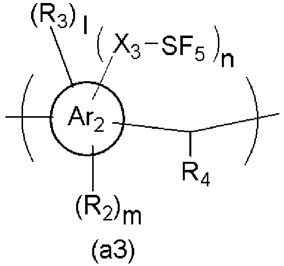
   wherein "n", "m", "l", Ar₂, R₂, R₃, and X₃ represent the same as defined above; R₄ represents a hydrogen atom or a hydrocarbyl group having 1 to 10 carbon atoms, and the hydrocarbyl group may have an oxygen atom but does not have a substituent having SF₅; and at least one of "n" and "l" in the formula represents 1 or more, 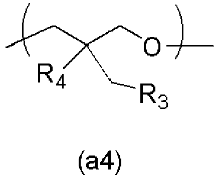
   wherein R₃ and R₄ represent the same as defined above.
[4]: The composition for forming a film of the above [3], wherein the repeating unit "a" is represented by the formula (a1).
[5]: The composition for forming a film of any one of the above [2] to [4], wherein the polymer (a) further comprises a repeating unit "b1" having a hydrophilic group selected from the group consisting of an ether bond, an ester bond, a hydroxy group, a carboxy group, a sulfonamide bond, a sulfonimide bond, a sulfo group, a lactone ring, a sultone ring, a carbonate bond, a urethane bond, and an amide bond.
[6]: The composition for forming a film of any one of the above [2] to [5], wherein the composition for forming a film is a composition for forming an organic film comprising (A) a resin or compound for forming an organic film; (B) the polymer (a); and (C) a solvent.
[7]: The composition for forming a film of any one of the above [6], wherein the component (B) is contained in an amount of 0.01 to 5 parts by mass based on 100 parts by mass of the component (A).
[8]: The composition for forming a film of any one of the above [6] or [7], further comprising (D) a photo-acid generator or (E) a thermal acid generator.
[9]: A method for forming an organic film to be used in a manufacturing process of a semiconductor device, the method comprising:
   spin-coating a substrate to be processed with the composition for forming a film of any one of the above [6] to [8]; and
   forming a cured film by heating the substrate coated with the composition for forming a film at a temperature of 100°C or higher and 600°C or lower for 10 to 600 seconds.
[10]: A patterning process comprising:
   forming an organic film on a body to be processed by using the composition for forming a film of any one of the above [6] to [8];
   forming a resist middle layer film on the organic film by using a silicon-containing resist middle layer film material;
   forming a resist upper layer film on the resist middle layer film by using a resist upper layer film material comprising a photoresist composition;
   forming a circuit pattern in the resist upper layer film;
   transferring the pattern to the resist middle layer film by etching while using the resist upper layer film having the formed circuit pattern as a mask;
   transferring the pattern to the organic film by etching while using the resist middle layer film having the transferred pattern as a mask; and
   further transferring the pattern in the body to be processed by etching while using the organic film having the transferred pattern as a mask.
[11]: A patterning process comprising:
   forming an organic film on a body to be processed by using the composition for forming a film of any one of the above [6] to [8];
   forming a resist middle layer film on the organic film by using a silicon-containing resist middle layer film material;
   forming an organic antireflective film or an adhesive film on the resist middle layer film;
   forming a resist upper layer film on the organic antireflective film or the adhesive film by using a resist upper layer film material comprising a photoresist composition;
   forming a circuit pattern in the resist upper layer film;
   transferring the pattern to the organic antireflective film or the adhesive film and to the resist middle layer film by etching while using the resist upper layer film having the formed circuit pattern as a mask;
   transferring the pattern to the organic film by etching while using the resist middle layer film having the transferred pattern as a mask; and
   further transferring the pattern to the body to be processed by etching while using the organic film having the transferred pattern as a mask.
[12]: A patterning process comprising:
   forming an organic film on a body to be processed by using the composition for forming a film of any one of the above [6] to [8];
   forming an inorganic hard mask selected from the group consisting of a silicon oxide film, a silicon nitride film, and a silicon oxynitride film on the organic film;
   forming a resist upper layer film on the inorganic hard mask by using a resist upper layer film material comprising a photoresist composition;
   forming a circuit pattern in the resist upper layer film;
   transferring the pattern to the inorganic hard mask by etching while using the resist upper layer film having the formed circuit pattern as a mask;
   transferring the pattern to the organic film by etching while using the inorganic hard mask having the transferred pattern as a mask; and
   further transferring the pattern to the body to be processed by etching while using the organic film having the transferred pattern as a mask.
[13]: A patterning process comprising:
   forming an organic film on a body to be processed by using the composition for forming a film of any one of the above [6] to [8];
   forming an inorganic hard mask selected from the group consisting of a silicon oxide film, a silicon nitride film, and a silicon oxynitride film on the organic film;
   forming an organic antireflective film or an adhesive film on the inorganic hard mask;
   forming a resist upper layer film on the organic antireflective film or the adhesive film by using a resist upper layer film material comprising a photoresist composition;
   forming a circuit pattern in the resist upper layer film;
   transferring the pattern to the organic antireflective film or the adhesive film and to the inorganic hard mask by etching while using the resist upper layer film having the formed circuit pattern as a mask;
   transferring the pattern to the organic film by etching while using the inorganic hard mask having the transferred pattern as a mask; and
   further transferring the pattern to the body to be processed by etching while using the organic film having the transferred pattern as a mask.
[14]: The patterning process of the above [12] or [13], wherein the inorganic hard mask is formed by a CVD method or an ALD method.
[15]: The patterning process of any one of the above [10] to [13], wherein the circuit pattern is formed by a lithography using light having a wavelength of 10 nm or more and 300 nm or less, a direct writing with an electron beam, nanoimprinting, or a combination thereof.
[16]: The patterning process of any one of the above [10] to [13], wherein the circuit pattern is developed with an alkaline development or an organic solvent.
[17]: The patterning process of any one of the above [10] to [13], wherein the body to be processed is a semiconductor device substrate or the semiconductor device substrate coated with any of a metal film, a metal carbide film, a metal oxide film, a metal nitride film, a metal oxycarbide film, and a metal oxynitride film.
[18]: The patterning process of the above [17], wherein the metal constituting the body to be processed is silicon, titanium, tungsten, hafnium, zirconium, chromium, germanium, copper, silver, gold, aluminum, indium, gallium, arsenic, palladium, iron, tantalum, iridium, molybdenum, or an alloy thereof.
[19]: A patterning process comprising:
   forming a resist film on a substrate by using the composition for forming a film according to any one of the above [6] to [8];
   exposing the resist film to a high-energy beam; and
   developing the exposed resist film by using a developer.
[20]: The patterning process according to the above [19], wherein the high-energy beam is a g-line, an i-line, a KrF excimer laser beam, an ArF excimer laser beam, an ultraviolet ray, an electron beam, or an extreme ultraviolet ray having a wavelength of 3 to 15 nm.
[21]: A monomer represented by the following general formula (a5), 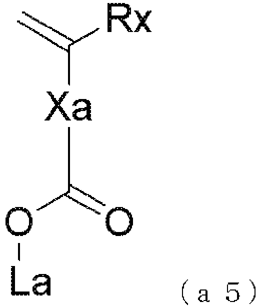 wherein Rₓ represents a hydrogen atom or a methyl group; Xₐ represents a single bond, a phenylene group, a naphthylene group, or a linking group having 1 to 12 carbon atoms and having an ester bond, an ether bond, or a lactone ring, and may have an oxygen atom, a nitrogen atom, a sulfur atom, and/or a halogen atom; and La represents an acid-labile group and has an aromatic group having at least one pentafluorosulfanyl group as a substituent.
[22]: The monomer according to the above [21] represented by the following general formula (a6), 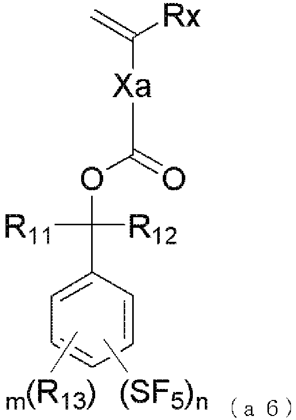 wherein Xₐ and Rₓ represent the same defined above; R₁₁ and R₁₂ each independently represent a linear, branched, or cyclic alkyl group having 1 to 12 carbon atoms, a linear, branched, or cyclic alkenyl group having 2 to 12 carbon atoms, a linear, branched, or cyclic alkynyl group having 2 to 12 carbon atoms, or an aryl group having 5 to 10 carbon atoms, each of which may have an oxygen atom and/or a sulfur atom, and R₁₁ and R₁₂ may bond to each other to form a ring; R₁₃ each independently represents a hydrogen atom, a halogen atom, an alkyl group having 1 to 4 carbon atoms, or an alkoxy group; "m" represents an integer of 0 to 4; and "n" represents an integer of 1 to 3.
[23]: A polymer having a repeating unit represented by the following general formula (a7) and having a weight-average molecular weight in a range of 100 to 500,000, 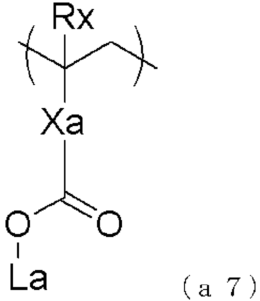 wherein, Rₓ represents a hydrogen atom or a methyl group; Xₐ represents a single bond, a phenylene group, a naphthylene group, or a linking group having 1 to 12 carbon atoms and having an ester bond, an ether bond, or a lactone ring, and may have an oxygen atom, a nitrogen atom, a sulfur atom, and/or a halogen atom; La represents an acid-labile group and has an aromatic group having at least one pentafluorosulfanyl group as a substituent.
[24]: The polymer of the above [23] having a repeating unit represented by the following general formula (a8) and having a weight-average molecular weight in a range of 100 to 500,000, 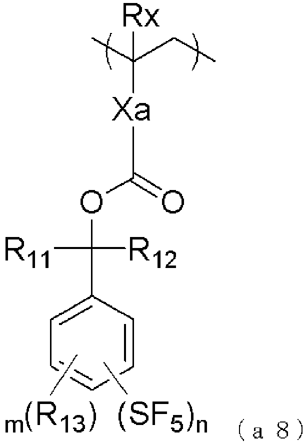 wherein Xₐ and Rₓ represent the same defined above; R₁₁ and R₁₂ each independently represent a linear, branched, or cyclic alkyl group having 1 to 12 carbon atoms, a linear, branched, or cyclic alkenyl group having 2 to 12 carbon atoms, a linear, branched, or cyclic alkynyl group having 2 to 12 carbon atoms, or an aryl group having 5 to 10 carbon atoms, each of which may have an oxygen atom and/or a sulfur atom, and R₁₁ and R₁₂ may bond to each other to form a ring; R₁₃ each independently represents a hydrogen atom, a halogen atom, an alkyl group having 1 to 4 carbon atoms, or an alkoxy group; "m" represents an integer of 0 to 4; and "n" represents an integer of 1 to 3.

It should be noted that the present invention is not limited to the above-described embodiments. The above embodiments are just examples, and any examples that have substantially the same feature and demonstrate the same functions and effects as those in the technical concept disclosed in claims of the present invention are included in the technical scope of the present invention.

## Claims

1. A composition for forming a film comprising a polymer having an aromatic group having a pentafluorosulfanyl group as a substituent.

2. The composition for forming a film according to claim 1, wherein the polymer is a polymer (a) having a repeating unit "a" having an aromatic group having a pentafluorosulfanyl group as a substituent.

3. The composition for forming a film according to claim 2, wherein the repeating unit "a" is represented by any one of the following formulae (a1) to (a4), preferably, wherein the repeating unit "a" is represented by the formula (a1), 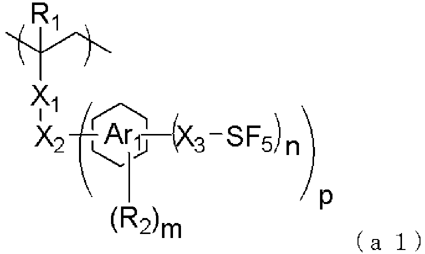
wherein, "p" represents an integer of 1 to 3; "n" represents an integer of 0 to 5; "m" represents an integer of 0 to 4; R₁ represents a hydrogen atom or a methyl group; R₂ represents a hydrocarbyl group having 1 to 6 carbon atoms, a hydrocarbyloxy group having 1 to 12 carbon atoms, a hydrocarbyloxycarbonyl group having 2 to 6 carbon atoms, a hydrocarbylcarbonyloxy group having 2 to 12 carbon atoms, a hydroxy group, a carboxy group, a halogen atom, a trifluoromethoxy group, a cyano group, or a nitro group; X₁ represents a single bond, an ester bond, an ether bond, a sulfonic acid ester group, a sulfonamide group, an amide bond, or a phenylene group; X₂ represents a single bond or a hydrocarbylene group having 1 to 20 carbon atoms when "p" represents 1, and X₂ represents a (p+1)-valent hydrocarbon group having 1 to 20 carbon atoms when "p" represents 2 or 3, and the hydrocarbylene group and the (p+1)-valent hydrocarbon group may contain at least one atom selected from the group consisting of an oxygen atom, a sulfur atom, a nitrogen atom, and a halogen atom; X₃ represents a single bond or an ether bond; and Ar₁ each independently represents a (m+n+1)-valent group derived from benzene or naphthalene, provided that at least one of "n"s in the formula represents 1 or more, 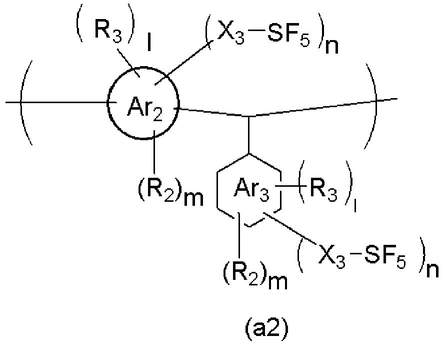
wherein, "n" and "m" represent the same as defined above, "l" represents an integer of 0 to 3, and at least one of the two "n"s and the two "1"s in the formula represents 1 or more; R₂ and X₃ represent the same as defined above; and R₃ represents the following general formula (a2-1); Ar₂ each independently represents a (n+m+l+2)-valent aromatic hydrocarbon group having 6 to 30 carbon atoms; and Ar₃ represents a (n+m+l+1)-valent group derived from benzene or naphthalene, 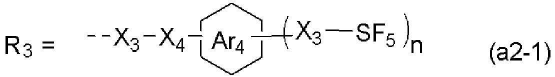
wherein, X₃ and "n" represent the same as above; X₄ represents a single bond or a hydrocarbylene group having 1 to 12 carbon atoms, and the hydrocarbylene group may have at least one selected from the group consisting of a hydroxy group, a saturated hydrocarbyloxy group having 1 to 6 carbon atoms, an ether bond, an ester bond, and an amide bond; and Ar₄ represents a (n+1)-valent group derived from benzene or naphthalene, 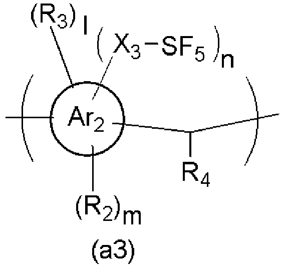
wherein "n", "m", "l", Ar₂, R₂, R₃, and X₃ represent the same as defined above; R₄ represents a hydrogen atom or a hydrocarbyl group having 1 to 10 carbon atoms, and the hydrocarbyl group may have an oxygen atom but does not have a substituent having SF₅; and at least one of "n" and "l" in the formula represents 1 or more, 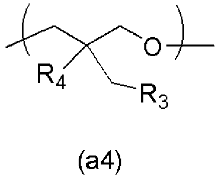
wherein R₃ and R₄ represent the same as defined above.

4. The composition for forming a film according to claim **2,** wherein the polymer (a) further comprises a repeating unit "b1" having a hydrophilic group selected from the group consisting of an ether bond, an ester bond, a hydroxy group, a carboxy group, a sulfonamide bond, a sulfonimide bond, a sulfo group, a lactone ring, a sultone ring, a carbonate bond, a urethane bond, and an amide bond.

5. The composition for forming a film according to claim 2, wherein the composition for forming a film is a composition for forming an organic film comprising (A) a resin or compound for forming an organic film; (B) the polymer (a); and (C) a solvent.

6. The composition for forming a film according to claim 5, wherein the component (B) is contained in an amount of 0.01 to 5 parts by mass based on 100 parts by mass of the component (A).

7. The composition for forming a film according to claim 5, further comprising (D) a photo-acid generator or (E) a thermal acid generator.

8. A method for forming an organic film to be used in a manufacturing process of a semiconductor device, the method comprising:
spin-coating a substrate to be processed with the composition for forming a film according to any one of claims 5 to 7; and
forming a cured film by heating the substrate coated with the composition for forming a film at a temperature of 100°C or higher and 600°C or lower for 10 to 600 seconds.

9. A patterning process comprising:
forming an organic film on a body to be processed by using the composition for forming a film according to claim 5;
forming a resist middle layer film on the organic film by using a silicon-containing resist middle layer film material;
forming a resist upper layer film on the resist middle layer film by using a resist upper layer film material comprising a photoresist composition;
forming a circuit pattern in the resist upper layer film;
transferring the pattern to the resist middle layer film by etching while using the resist upper layer film having the formed circuit pattern as a mask;
transferring the pattern to the organic film by etching while using the resist middle layer film having the transferred pattern as a mask; and
further transferring the pattern in the body to be processed by etching while using the organic film having the transferred pattern as a mask, or
a patterning process comprising:
forming an organic film on a body to be processed by using the composition for forming a film according to claim 5;
forming a resist middle layer film on the organic film by using a silicon-containing resist middle layer film material;
forming an organic antireflective film or an adhesive film on the resist middle layer film;
forming a resist upper layer film on the organic antireflective film or the adhesive film by using a resist upper layer film material comprising a photoresist composition;
forming a circuit pattern in the resist upper layer film;
transferring the pattern to the organic antireflective film or the adhesive film and to the resist middle layer film by etching while using the resist upper layer film having the formed circuit pattern as a mask;
transferring the pattern to the organic film by etching while using the resist middle layer film having the transferred pattern as a mask; and
further transferring the pattern to the body to be processed by etching while using the organic film having the transferred pattern as a mask, or
a patterning process comprising:
forming an organic film on a body to be processed by using the composition for forming a film according to claim 5;
forming an inorganic hard mask selected from the group consisting of a silicon oxide film, a silicon nitride film, and a silicon oxynitride film on the organic film;
forming a resist upper layer film on the inorganic hard mask by using a resist upper layer film material comprising a photoresist composition;
forming a circuit pattern in the resist upper layer film;
transferring the pattern to the inorganic hard mask by etching while using the resist upper layer film having the formed circuit pattern as a mask;
transferring the pattern to the organic film by etching while using the inorganic hard mask having the transferred pattern as a mask; and
further transferring the pattern to the body to be processed by etching while using the organic film having the transferred pattern as a mask, preferably, wherein the inorganic hard mask is formed by a CVD method or an ALD method, or
a patterning process comprising:
forming an organic film on a body to be processed by using the composition for forming a film according to claim 5;
forming an inorganic hard mask selected from the group consisting of a silicon oxide film, a silicon nitride film, and a silicon oxynitride film on the organic film;
forming an organic antireflective film or an adhesive film on the inorganic hard mask;
forming a resist upper layer film on the organic antireflective film or the adhesive film by using a resist upper layer film material comprising a photoresist composition;
forming a circuit pattern in the resist upper layer film;
transferring the pattern to the organic antireflective film or the adhesive film and to the inorganic hard mask by etching while using the resist upper layer film having the formed circuit pattern as a mask;
transferring the pattern to the organic film by etching while using the inorganic hard mask having the transferred pattern as a mask; and
further transferring the pattern to the body to be processed by etching while using the organic film having the transferred pattern as a mask, preferably, wherein the inorganic hard mask is formed by a CVD method or an ALD method.

10. The patterning process according to claim 9, wherein the circuit pattern is formed by a lithography using light having a wavelength of 10 nm or more and 300 nm or less, a direct writing with an electron beam, nanoimprinting, or a combination thereof.

11. The patterning process according to claim 9, wherein the circuit pattern is developed with an alkaline development or an organic solvent.

12. The patterning process according to claim 9, wherein the body to be processed is a semiconductor device substrate or the semiconductor device substrate coated with any of a metal film, a metal carbide film, a metal oxide film, a metal nitride film, a metal oxycarbide film, and a metal oxynitride film, preferably, wherein the metal constituting the body to be processed is silicon, titanium, tungsten, hafnium, zirconium, chromium, germanium, copper, silver, gold, aluminum, indium, gallium, arsenic, palladium, iron, tantalum, iridium, molybdenum, or an alloy thereof.

13. A patterning process comprising:
forming a resist film on a substrate by using the composition for forming a film according to any one of claims 5 to 7;
exposing the resist film to a high-energy beam; and
developing the exposed resist film by using a developer, preferably,
wherein the high-energy beam is a g-line, an i-line, a KrF excimer laser beam, an ArF excimer laser beam, an ultraviolet ray, an electron beam, or an extreme ultraviolet ray having a wavelength of 3 to 15 nm.

14. A monomer represented by the following general formula (a5), 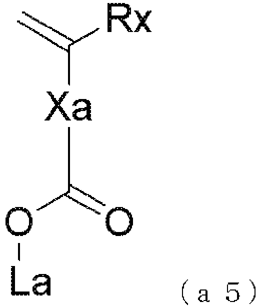
wherein Rₓ represents a hydrogen atom or a methyl group; Xₐ represents a single bond, a phenylene group, a naphthylene group, or a linking group having 1 to 12 carbon atoms and having an ester bond, an ether bond, or a lactone ring, and may have an oxygen atom, a nitrogen atom, a sulfur atom, and/or a halogen atom; and La represents an acid-labile group and has an aromatic group having at least one pentafluorosulfanyl group as a substituent, preferably, the monomer is represented by the following general formula (a6), 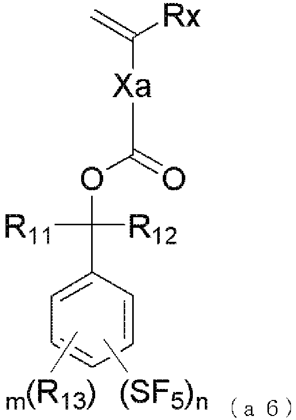
wherein Xₐ and Rₓ represent the same defined above; R₁₁ and R₁₂ each independently represent a linear, branched, or cyclic alkyl group having 1 to 12 carbon atoms, a linear, branched, or cyclic alkenyl group having 2 to 12 carbon atoms, a linear, branched, or cyclic alkynyl group having 2 to 12 carbon atoms, or an aryl group having 5 to 10 carbon atoms, each of which may have an oxygen atom and/or a sulfur atom, and R₁₁ and R₁₂ may bond to each other to form a ring; R₁₃ each independently represents a hydrogen atom, a halogen atom, an alkyl group having 1 to 4 carbon atoms, or an alkoxy group; "m" represents an integer of 0 to 4; and "n" represents an integer of 1 to 3.

15. A polymer having a repeating unit represented by the following general formula (a7) and having a weight-average molecular weight in a range of 100 to 500,000, 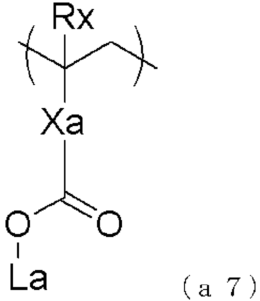
wherein, Rₓ represents a hydrogen atom or a methyl group; Xₐ represents a single bond, a phenylene group, a naphthylene group, or a linking group having 1 to 12 carbon atoms and having an ester bond, an ether bond, or a lactone ring, and may have an oxygen atom, a nitrogen atom, a sulfur atom, and/or a halogen atom; La represents an acid-labile group and has an aromatic group having at least one pentafluorosulfanyl group as a substituent, preferably, the polymer has a repeating unit represented by the following general formula (a8) and has a weight-average molecular weight in a range of 100 to 500,000, 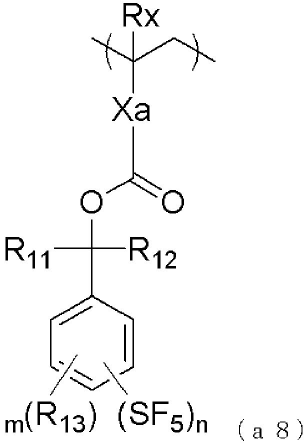
wherein Xₐ and Rₓ represent the same defined above; R₁₁ and R₁₂ each independently represent a linear, branched, or cyclic alkyl group having 1 to 12 carbon atoms, a linear, branched, or cyclic alkenyl group having 2 to 12 carbon atoms, a linear, branched, or cyclic alkynyl group having 2 to 12 carbon atoms, or an aryl group having 5 to 10 carbon atoms, each of which may have an oxygen atom and/or a sulfur atom, and R₁₁ and R₁₂ may bond to each other to form a ring; R₁₃ each independently represents a hydrogen atom, a halogen atom, an alkyl group having 1 to 4 carbon atoms, or an alkoxy group; "m" represents an integer of 0 to 4; and "n" represents an integer of 1 to 3.
